(19) Europäisches Patentamt
European Patent Office
Office européen des brevets

(11) **EP 2 363 399 A1**

(12) **EUROPEAN PATENT APPLICATION**

(43) Date of publication:
**07.09.2011 Bulletin 2011/36**

(21) Application number: **11167370.3**

(22) Date of filing: **27.07.2007**

(51) Int Cl.:
***C07D 471/04*** *(2006.01)* ***C07D 219/06*** *(2006.01)*
***A61K 31/165*** *(2006.01)* ***C07D 209/42*** *(2006.01)*
***C07D 215/54*** *(2006.01)* ***C07D 217/26*** *(2006.01)*
***C07D 235/24*** *(2006.01)* ***C07D 237/26*** *(2006.01)*
***C07D 333/68*** *(2006.01)* ***C07C 233/62*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **27.07.2006 FR 0653138**

(62) Document number(s) of the earlier application(s) in accordance with Art. 76 EPC:
**07805258.6 / 2 046 789**

(71) Applicants:
- **INSTITUT NATIONAL DE LA SANTE ET DE LA RECHERCHE MEDICALE (INSERM)
75013 Paris (FR)**
- **Université d'Auvergne
63001 Clermont-Ferrand (FR)**

(72) Inventors:
- **Madelmont, Jean-Claude
63540 ROMAGNAT (FR)**
- **Chezal, Jean-Michel
63400 CHAMALIERES (FR)**
- **Chavignon, Olivier
63730 LES MARTRES DE VEYRE (FR)**
- **Teulade, Jean-Claude
34430 SAINT JEAN DE VEDAS (FR)**
- **Moins, Nicole
63000 CLERMONT FERRAND (FR)**

(74) Representative: **Le Coupanec, Pascale A.M.P.
Nony & Associés
3, rue de Penthièvre
75008 Paris (FR)**

Remarks:
This application was filed on 24-05-2011 as a divisional application to the application mentioned under INID code 62.

(54) **Labelled analogues of halobenzamides as radiopharmaceuticals**

(57) The present invention relates to the use of a compound of formula (I):

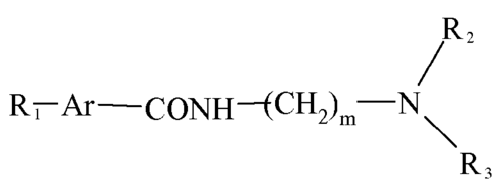

(I)

in which
$R_1$ represents a radionuclide,
Ar represents an aromatic nucleus,
m is an integer varying from 2 to 4,
$R_2$ and $R_3$ represent, independently of one another, a hydrogen atom, a ($C_1$-$C_6$)alkyl group, a ($C_1$-$C_6$)alkenyl group or an aryl group chosen from a phenyl, benzyl, imidazolyl, pyridyl, pyrimidinyl, pyrazinyl, indolyl, indazolyl, furyl and thienyl group, and their addition salts with pharmaceutically acceptable acids, in the preparation of a radiopharmaceutical composition intended for the diagnosis and/or treatment of melanoma.

EP 2 363 399 A1

## Description

The present invention relates to the use of aromatic and heteroaromatic analogues of halobenzamides labelled with a suitable radioactive isotope as radiopharmaceuticals for the diagnosis by gamma scintigraphic and positron imaging or for the internal radiotherapeutic treatment of melanoma, and also to some novel aromatic and heteroaromatic analogues of halobenzamides.

Melanoma is one of the most dangerous skin tumours with a steadily increasing incidence. Specifically, the current incidence of cutaneous melanoma is close to 10 000 new cases diagnosed per year in France. This is a highly invasive cancer, the development of which is rapidly fatal at the metastatic stage. 5-year survival does not exceed 14%, except in the case where the thickness of the tumour is less than 0.76 mm. In the case where the lesion exceeds this thickness, this tumour gives metastases in an unpredictable and silent fashion. This is why a search is currently underway for a method of investigation which makes possible the early evaluation both of the local extension and of the distant extension of the tumour.

During the last decade, a number of radiopharmaceutical products, selected for their potential melanin affinity, have been experimented with but few have had a satisfactory clinical development.

It should be explained that radiopharmaceutical products comprise two functional components, one being radioactive and the other not being radioactive. The radioactive component makes possible the detection of the product in the context of the diagnosis and it constitutes the active agent in the case of therapeutic use. It is composed of a radionuclide with appropriate physical properties. The nonradioactive component, for its part, is composed of a molecule or tracer, optionally biological, intended to accumulate in the target organ, in this case, in the context of the present invention, in the melanoma, and to ensure the absorption of radioactivity by the latter. This nonradioactive component is determining for the biological behaviour of the radiopharmaceutical product in the body, in particular regarding the specificity and the pharmacokinetic profile.

More particularly in imaging, monoclonal antibodies which specifically target melanoma have been developed. In addition, various molecules exhibiting an affinity for melanoma have been studied in man, after labelling with $^{123}I$ : methylene blue, N-[3-(4-morpholino)propyl]-*N*-methyl-2-hydroxy-5-iodo-3-methylbenzylamine (ERC9) and benzamide structures.

Furthermore, the document EP 458 886 describes compounds of use in the diagnosis and treatment of malignant melanoma. *N*-(2-Diethylaminoethyl)-4-iodobenzamide (BZA) forms in particular the subject of more detailed studies, and also *N*-(2-diethylaminoethyl)-2-iodobenzamide (BZA2), in the medical imaging application and more particularly for the scintigraphic detection of primary ocular melanoma and metastases of cutaneous and ocular melanomas.

The document US 5 911 970 for its part describes, inter alia, other benzamide- derived compounds exhibiting a high specificity and affinity with regard to the surface of cancer cells.

There thus exists, on the one hand, a need to have available a specific tracer which makes it possible, from the time of the diagnosis, to carry out an assessment of extension of the disease and then, subsequently, monitoring. Such a tracer should advantageously make possible the differential diagnosis of ocular melanoma, primary lesion, which is often difficult to identify.

On the other hand, treatments remain defeated with regard to disseminated melanoma and the development of novel specific therapeutic approaches for the treatment of melanoma is essential.

This is because, while the surgical treatment of melanoma remains the best weapon (operable primary cutaneous tumours and isolated secondary sites), the low effectiveness of the treatments provided to date with regard to the disseminated disease illustrates the need to develop a treatment of melanoma by vectorized internal radiotherapy. To date, the first experimental results, carried out with an $\alpha$-MSH analogue, are very preliminary and the ideal tracer remains to be determined.

Thus, the object of the present invention is the targeting of melanotic lesions by the development of molecules which, administered to the body, will make it possible to vectorize a radioisotope. This object results in two fields of applications, namely imaging and radiotherapy.

The compounds in accordance with the invention are thus of advantage both for their use in imaging, namely for the diagnosis of malignant melanoma, and in vectorized internal radiotherapy, targeting more particularly secondary lesions and primary ocular lesions. One of the major advantages of the compounds according to the present invention lies precisely in their mixed potentiality. In other words, according to the chemical variations under consideration, their respective behaviours in the body destine them more particularly for use in medical imaging, for use in radiotherapy or else, and this category of compounds may prove to be particularly attractive, for use both in medical imaging and in radiotherapy.

The examples reported below clearly illustrate, for a few compounds, these various behaviours in terms of specificity, of duration of radioactivity or of antitumour effectiveness.

Finally, some compounds corresponding to the general formula of the compounds in accordance with the invention, when they are unlabelled, may also exhibit an innate antitumour activity. Thus, in this specific case, this

amounts to saying that the nonradioactive component defined above can play, in addition to its role of targeting the melanoma and/or of promoting absorption of radioactivity, a role of cytotoxic agent per se.

Consequently, this particular category of compounds, the use of which is the subject-matter of the present invention, for some already known for their anticancer activity when they are devoid of radionuclides, in particular via the mode of action of intercalating agents, may exhibit the advantage of making possible a radiotherapeutic application which can be combined with an application in chemotherapy. The reference is then to compounds intended for an application in radiochemotherapy. However, it is clearly the activity via labelling by a radioactive isotope which is targeted in the present invention. In other words, the possible activity related to the mechanism of actions innate to the unlabelled compound is generally only to be regarded as an additional advantage.

Mention may in particular be made, among unlabelled compounds exhibiting an innate cytotoxic activity which can, after labelling, be used in the radiochemotherapy of melanoma, of certain compounds described in the documents WO 93/24096, WO 98/17649 or US 6 821 983 as analogues of acridinecarboxamide ("DACA"). The targeting of melanoma had not until now been demonstrated for these compounds. The two following publications :

- Osman, S., Rowlinson-Busza, G., Luthra, K. S., Aboagye, E. O., Brown, G. D., Brady, F., Myers, R., Gamage, S. A., Denny, W. A., Baguley, B. C., Price, P. M., Cancer Res., 2001, 61, 2935-2944, and
- Saleem, A., Harte, R. J., Matthews, J. C., Osman, S., Brady, F., Luthra, S. K., Brown, G. D., Bleehen, N., Connors, T., Jones, T., Price, P. M., and Aboagye, E. O., J. Clin. Oncology, 2001, 19, 1421-1429,

describe the biodistribution of DACA labelled with $^{11}C$. However, these two experiments relate to short times (< 1 h) and do not demonstrate a specific fixing of DACA for the melanotic tumours evaluated.

Derivatives of phenazine type are described in particuar in the document WO 2005/118580 and in the publication S. A. Gamage et al., Bioorganic & Medicinal Chemistry, 2006, 14, 1160-1168, for their innate anticancer activity. However, the use of such phenazine derivatives for the targeting of melanoma has never been demonstrated.

According to a first aspect, a subject-matter of the present invention is the use of a compound of formula (I):

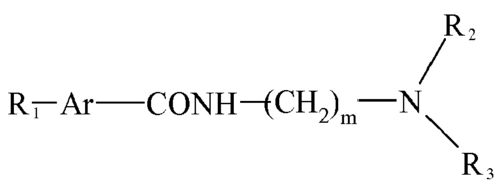

(I)

in which
$R_1$ represent a radionuclide,
Ar represents an aromatic nucleus,
m is an integer varying from 2 to 4,
$R_2$ and $R_3$ represent, independently of one another, a hydrogen atom, a $(C_1-C_6)$alkyl group, a $(C_1-C_6)$alkenyl group or an aryl group chosen from a phenyl, benzyl, imidazolyl, pyridyl, pyrimidinyl, pyrazinyl, indolyl, indazolyl, furyl and thienyl group, in which the aromatic nucleus denotes an aryl group chosen from the naphthyl, phenanthryl and anthryl group or a heteroaryl group, it being possible for the said heteroaryl group to be

- mono- or disubstituted by:
  - an optionally labelled halogen atom,
  - a hydroxyl group,
  - a $(C_1-C_4)$alkyl group
  - a $(C_1-C_4)$alkoxy group,
  - an $-NO_2$ group,
  - an $-NR_5R_6$ group, where $R_5$ and $R_6$ can independently represent a hydrogen atom or a $(C_1-C_4)$alkyl group,
  - an $-NHCONH_2$ group,
  - an -SH group,
  - an $-NHCOOR_7$, $-NHCONHR_7$ or $-SR_7$ group, where $R_7$ represents a $(C_1-C_4)$alkyl group,
  - an oxo group, or
- monosubstituted by an anilino group which can itself be substituted by 1 to 3 groups which can be chosen from a $(C_1-C_4)$alkyl or $(C_1-C_4)$alkoxy group, a hydroxyl group, a halogen atom or an $NHR^e$ group where $R^e$ represents a

hydrogen, or a $COR^a$ group, a $COOR^a$ group or an $SO_2R^a$ group, where $R^a$ represents an aryl group or a $(C_1-C_{10})$ alkyl group optionally substituted by an oxo group,

and in which $R_1$ is bonded to the aromatic nucleus as such or, when the substituent of the aromatic nucleus is an anilino group, $R_1$ can be bonded to the phenyl group of the anilino group,
and their addition salts with pharmaceutically acceptable acids, in the preparation of a radiopharmaceutical composition intended for the diagnosis and/or treatment of melanoma.

According to another aspect, a subject-matter of the invention is a product chosen from the compounds of formula (I) as defined above for the diagnosis and/or treatment of melanoma.

In the context of the present invention, the term "halogen" is understood to mean chlorine, fluorine, bromine, iodine or astatine.

It is specified that, in the context of the present invention, the term "aromatic nucleus" is distinct from a phenyl group.

The term "heteroaryl" denotes a 5- or 6-membered aromatic ring comprising 1 or 2 heteroatoms or a bi- or tricyclic aromatic nucleus comprising from 1 to 4 heteroatoms, and at least one of the rings of which has 6 ring members, the other fused ring or rings having 5 or 6 ring members.

The term "heteroatom" is understood to mean nitrogen, oxygen or sulphur.

Within the meaning of the present invention, the term "radionuclide" is understood to mean an isotope of natural or artificial origin which demonstrates radioactive properties. The radionuclide can be a radioisotope chosen from $^{123}I$, $^{124}I$, $^{125}I$, $^{131}I$ $^{75}Br$, $^{76}Br$, $^{77}Br$, $^{18}F$, $^{210}At$ or $^{211}At$.

Advantageously, $R_1$ is an iodine atom chosen from $^{123}I$, $^{124}I$, $^{125}I$ and $^{131}I$.

According to a specific embodiment, the heteroaryl comprises at least one nitrogen atom. Preferably, the heteroaryl does not comprise an oxygen atom. Finally, very particularly preferably, the heteroaryl comprises only nitrogen as heteroatom(s). Thus, advantageously, the heteroaryl comprises from 1 to 4 nitrogen atoms, in particular 2 or 3 nitrogen atoms.

Mention may be made, as example of 5- or 6-membered aromatic ring comprising 1 or 2 heteroatom(s), of pyrrole, imidazole, pyridine, pyrimidine, pyrazine, pyridazine and thiazole.

Mention may be made, as example of bi- or tricyclic aromatic nucleus in accordance with the invention, of the nuclei having benzene as one of the rings, including indole, isoindole, quinoline, isoquinoline, quinoxaline, benzimidazole, indazole, phthalazine, quinazoline, cinnoline or benzothiophene for the bicycles and carbazole, phenanthridine, acridine, phenothiazine, phenoxazine, phenazine, phenanthroline, carboline, perimidine and benzisoquinoline for the tricycles.

Mention may be made, as example of bi- or tricyclic aromatic nucleus in accordance with the invention, each of the rings of which, taken in isolation, is an aromatic nucleus comprising at least one heteroatom, of naphthyridine, quinolizine, purine, imidazopyridine, indolizine, pteridine, imidazotriazine or pyrazinopyridazine for the bicycles.

Within the meaning of the present invention, the heteroaryl can be partially hydrogenated. However, for the bi- or tricycles, each of the rings forming them, taken in isolation, comprises at least one double bond. Thus, for example, the compound of formula (I) for which Ar is a dihydrobenzofuran is excluded from the scope of the present invention.

In the context of the present invention, the terms "aromatic nucleus", "aryl", and "heteroaryl" include all the positional isomers.

According to another preferred embodiment, the compound of formula (I) exhibits a bi- or tricyclic aromatic nucleus as defined above and the $R_1$ group is bonded to one of the rings and the group

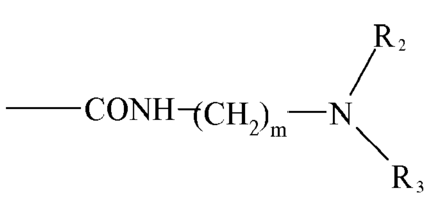

is bonded to the other ring or to one of the other rings.

According to a favoured embodiment, an additional subject-matter of the present invention is the use of a compound, referred to as preferred compound, of formula (I),
in which
$R_1$ is a radionuclide as defined above,
Ar is an aryl group or a heteroaryl group,
m is an integer varying from 2 to 4,
$R_2$ and $R_3$ represent, independently of one another, a hydrogen atom, a $(C_1-C_6)$alkyl group or a $(C_1-C_6)$alkenyl group,

the aryl group being chosen from the naphthyl, phenanthryl and anthryl group, and
the heteroaryl group being a 5- or 6-membered aromatic ring comprising 1 or 2 nitrogen atoms or a bi- or tricyclic aromatic nucleus comprising from 1 to 4 nitrogen atoms or comprising a sulphur atom, at least one of the rings of which has 6 ring members, the other fused ring or rings having 5 or 6 ring members, it being possible for the said heteroaryl group to be monosubstituted by:

- an optionally labelled halogen atom,
- a $(C_1-C_4)$alkoxy group,
- a $(C_1-C_4)$alkyl group,
- an oxo group or
- an anilino group which can itself be substituted by 1 to 3 groups which can be chosen from a $(C_1-C_4)$alkyl or $(C_1-C_4)$ alkoxy group, a halogen atom, a hydroxyl group or an $NHR^e$ group where $R^e$ represents a hydrogen, or a $COR^a$ group, a $COOR^a$ group or an $SO_2R^a$ group, where $R^a$ represents an aryl group or a $(C_1-C_{10})$alkyl group optionally substituted by an oxo group;

and in which $R_1$ is bonded to the aromatic nucleus as such or, when the substituent of the aromatic nucleus is an anilino group, $R_1$ can be bonded to the phenyl group of the anilino group,
and its addition salts with pharmaceutically acceptable acids, in the preparation of a radiopharmaceutical composition intended for the diagnosis and/or treatment of melanoma.

Preference is given, among these preferred compounds of formula (I), to those for which the heteroaryl group does not comprise an oxygen atom, indeed even to those for which the heteroaryl group comprises only nitrogen as heteroatom(s).

Thus, according to the preferred embodiment, a subject-matter of the present invention is the use of a preferred compound of formula (I), characterized in that the heteroaryl group is chosen from an indolyl, isoindolyl, quinolyl, isoquinolyl, quinoxalinyl, benzimidazolyl, indazolyl, phthalazinyl, quinazolinyl, cinnolinyl, carbazolyl, phenanthridinyl, acridinyl, phenazinyl, phenanthrolinyl, carbolinyl, perimidinyl, benzisoquinolinyl, naphthyridinyl, quinolizinyl, purinyl, imidazopyridyl, indolizinyl, pteridinyl, imidazotriazinyl and pyrazinopyridazinyl group, it being possible for the said heteroaryl group to be substituted as described above, in the preparation of a radiopharmaceutical composition intended for the diagnosis and/or treatment of melanoma.

Very particular preference is given to the use of the preferred compounds of formula (I) for which Ar is chosen from a naphthyl, pyridyl, phenazinyl, naphthyridinyl, indolyl, imidazopyridyl, benzimidazolyl, quinolyl, quinolonyl, isoquinolyl, quinoxalinyl, benzothienyl, acridinyl or acridonyl group, it being possible for the said group to be monosubstituted by a methyl group, a methoxy group or an optionally labelled halogen atom, and an acridinyl group substituted by an anilino group which can itself be substituted by 1 to 3 groups which can be chosen from a $(C_1-C_4)$alkyl or $(C_1-C_4)$alkoxy group, a hydroxyl group, a halogen atom or an $NHR^e$ group where $R^e$ represents a hydrogen, or a $COR^a$ group, a $COOR^a$ group or an $SO_2R^a$ group, where $R^a$ represents an aryl group or a $(C_1-C_{10})$alkyl group optionally substituted by an oxo group.

In the context of the present invention, quinolone is quinoline substituted by an oxo group in the 4-position and acridone is an acridine substituted by an oxo group in the 9-position.

According to a favoured embodiment of the present invention, when Ar comprises only one ring, the preferred compound of formula (I) exhibits the $R_1$ group in the para position with respect to the group

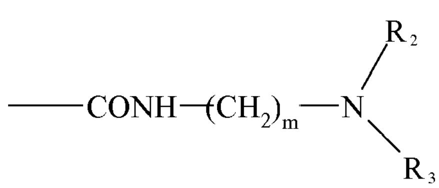

According to another favoured embodiment of the present invention, when Ar is a bi- or tricycle, the preferred compound of formula (I) exhibits the $R_1$ group bonded to one of the rings and the group

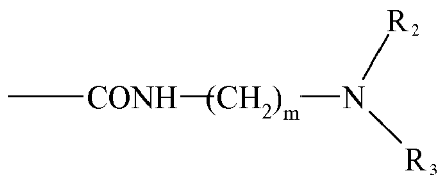

bonded to the other ring or to one of the other rings.

According to yet another favoured embodiment of the present invention, preference is given to the use of a compound of formula (I), characterized in that Ar is a bi-or tricyclic heteroaryl as defined above and in that $R_1$ is bonded to the ring, taken in isolation, not comprising a heteroatom or comprising the least thereof and the group

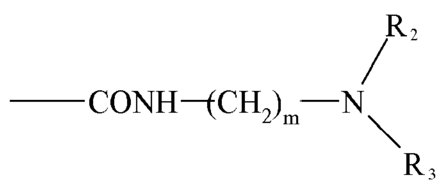

is bonded to another ring comprising the greater number of heteroatom(s).

An additional subject-matter of the present invention is the use of a compound of formula (I')

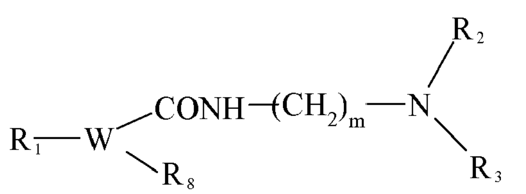

(I')

in which W is chosen from a phenazinyl, imidazopyridyl, benzimidazolyl, quinolyl, quinoxalinyl, naphthyridinyl, acridinyl and acridonyl group, it being possible for the said acridinyl group to be substituted by an anilino group itself substituted by three groups,

- at least one of the substituents representing the $(C_1\text{-}C_4)$alkoxy group,
- at least one of the substituents being chosen from an $NHR^e$ group where $R^e$ represents a hydrogen, or a $COR^a$ group, a $COOR^a$ group or an $SO_2R^a$ group, where $R^a$ represents an aryl group or a $(C_1\text{-}C_{10})$alkyl group optionally substituted by an oxo group, and
- the remaining substituent representing a hydrogen or halogen atom,

$R_1$, $R_2$, $R_3$ and m have the same meaning as above, and
$R_8$ represents a hydrogen atom, a $(C_1\text{-}C_4)$alkyl or $(C_1\text{-}C_4)$alkoxy group, an optionally labelled halogen atom, an -SH group, an -OH group or an $-NR_5R_6$ group where $R_5$ and $R_6$ can independently represent a hydrogen atom or a $(C_1\text{-}C_4)$ alkyl group,
in the preparation of a composition intended for the diagnosis and/or treatment of melanoma.

According to another aspect, a subject-matter of the invention is a product chosen from the compounds of formula (I') as defined above for the diagnosis and/or treatment of melanoma.

In the formulae which follow, the groups of variable definition, in particular the $R_1$ and $R_8$ radicals, can take any position on the heterocycle. According to a specific embodiment, the $R_1$ group is bonded to one of the rings and the group

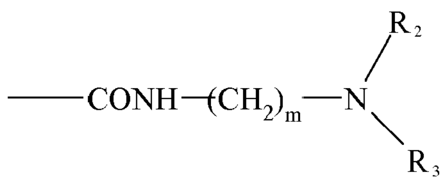

is bonded to the other ring or to one of the other rings.

According to yet another embodiment, the $R_8$ radical is situated, when this is possible, on a ring other than that carrying the $R_1$ group.

A subject-matter of the present invention is more particularly the use of the compound of formula (Ia)

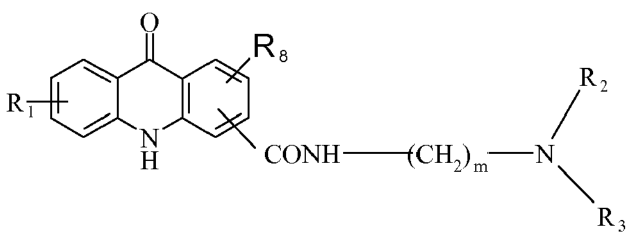

(Ia)

in which

$R_1$, $R_2$, $R_3$ and m have the same meaning as above, and

$R_8$ represents a hydrogen atom, a ($C_1$-$C_4$)alkyl group, a ($C_1$-$C_4$)alkoxy group, an optionally labelled halogen atom, an -SH group, an -OH group or an -$NR_5R_6$ group where $R_5$ and $R_6$ can independently represent a hydrogen atom or a ($C_1$-$C_4$)alkyl group,

in the preparation of a composition intended for the diagnosis and/or treatment of melanoma.

In addition, a subject-matter of the present invention is more particularly the use of a compound of formula (Ib)

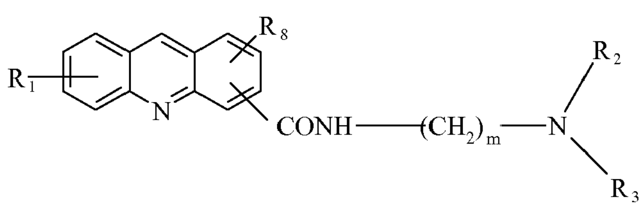

(Ib)

in which

$R_1$, $R_2$, $R_3$ and m have the same meaning as above, and

$R_8$ is as defined above,

in the preparation of a composition intended for the diagnosis and/or treatment of melanoma.

A subject-matter of the present invention is also more particularly the use of a compound of formula (Ic)

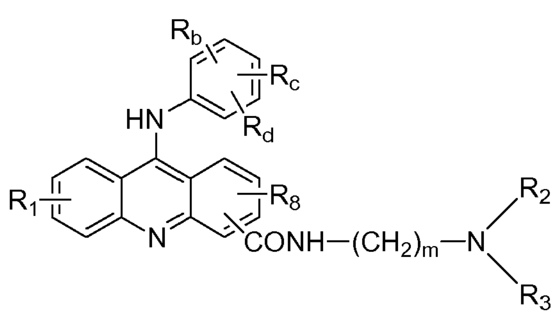

(Ic)

in which
$R_1$, $R_2$, $R_3$ and m have the same meaning as above, and
$R_8$ is as defined above,
at least one of the substituents chosen from $1^0$, $R^c$ and $R^d$ represents a $(C_1\text{-}C_4)$alkoxy group,
at least one of the substituents chosen from $R^b$, $R^c$ and $R^d$ is chosen from an $NHR^e$ group where $R^e$ represents a hydrogen, or a $COR^a$ group, a $COOR^a$ group or an $SO_2R^a$ group, where $R^a$ represents an aryl group or a $(C_1\text{-}C_{10})$alkyl group optionally substituted by an oxo group, and
the remaining substituent, chosen from $R^b$, $R^c$ and $R^d$, represents a hydrogen or halogen atom,
in the preparation of a composition intended for the diagnosis and/or treatment of melanoma.

Another subject-matter of the present invention is more particularly the use of a compound of formula (If)

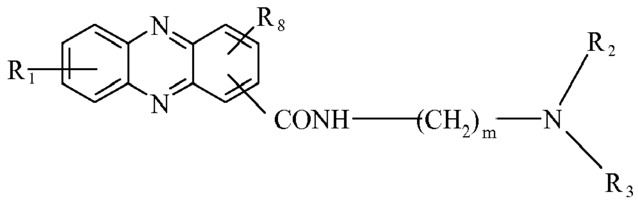

(If)

in which
$R_1$, $R_2$, $R_3$ and m have the same meaning as above, and
$R_8$ is as defined above,
in the preparation of a composition intended for the diagnosis and/or treatment of melanoma.

In addition, a subject-matter of the present invention is more particularly the use of a compound of formula (Ig)

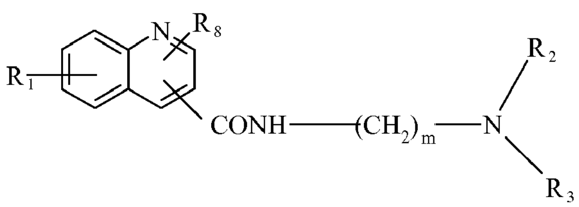

(Ig)

in which
$R_1$, $R_2$, $R_3$ and m have the same meaning as above, and
$R_8$ is as defined above,
in the preparation of a composition intended for the diagnosis and/or treatment of melanoma.

A subject-matter of the present invention is also more particularly the use of a compound of formula (Ih)

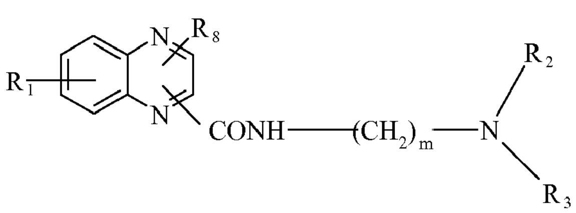

(Ih)

in which
$R_1$, $R_2$, $R_3$ and m have the same meaning as above, and
$R_8$ is as defined above,
in the preparation of a composition intended for the diagnosis and/or treatment of melanoma.

A subject-matter of the present invention is also more particularly the use of a compound of formula (Ii)

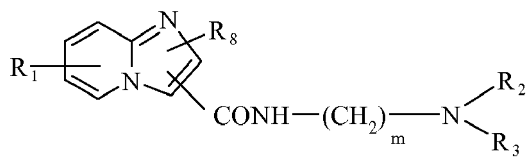

(Ii)

in which
$R_1$, $R_2$, $R_3$ and m have the same meaning as above, and
$R_8$ is as defined above,
in the preparation of a composition intended for the diagnosis and/or treatment of melanoma.

In addition, a subject-matter of the present invention is more particularly the use of a compound of formula (Io)

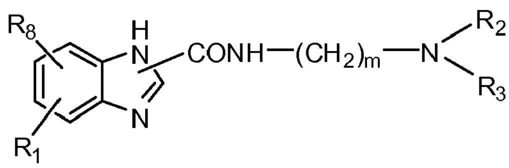

(Io)

in which
$R_1$, $R_2$, $R_3$ and m have the same meaning as above, and
$R_8$ is as defined above,
in the preparation of a composition intended for the diagnosis and/or treatment of melanoma.

Another subject-matter of the present invention is more particularly the use of a compound of formula (Ip)

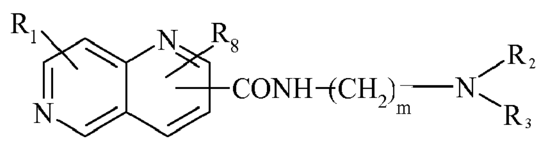

(Ip)

in which
$R_1$, $R_2$, $R_3$ and m have the same meaning as above, and
$R_8$ is as defined above,
in the preparation of a composition intended for the diagnosis and/or treatment of melanoma.

Another subject-matter of the present invention is a product chosen from the compounds of formulae (Ia), (Ib), (Ic), (If), (Ig), (Ih), (Ii), (Io) and (Ip) as defined above for the diagnosis and/or treatment of melanoma.

Finally, according to a second aspect, a subject-matter of the present invention is novel compounds of formula (II)

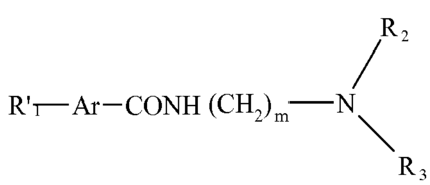

(II)

in which
$R'_1$ represents a hydrogen atom or an optionally labelled halogen atom,
$R_2$, $R_3$ and m have the same meaning as above, and
Ar is chosen from the naphthyl, pyridyl, benzothienyl, indolyl, isoindolyl, quinolyl, isoquinolyl, quinoxalinyl, benzimidazolyl, indazolyl, phthalazinyl, quinazolinyl, cinnolinyl, carbazolyl, phenanthrolinyl, carbolinyl, perimidinyl, benzisoquinolinyl, naphthyridinyl, quinolizinyl, purinyl, imidazopyridinyl, indolizinyl, pteridinyl, imidazotriazinyl and pyrazinopyridazinyl group, it being possible for the said group to be mono- or disubstituted by a $(C_1-C_4)$alkyl or $(C_1-C_4)$alkoxy group, an

optionally labelled halogen atom, a hydroxyl group or an $-NR_5R_6$ group where $R_5$ and $R_6$ can independently represent a hydrogen atom or a $(C_1-C_4)$alkyl group.

Preference is given, in the context of the present invention, to the compounds of formula (II) for which Ar is chosen from a naphthyl, pyridyl, indolyl, imidazopyridinyl, benzimidazolyl, quinolyl, quinolonyl, isoquinolyl, quinoxalinyl, naphthyridinyl and benzothienyl group, it being possible for the said group to be monosubstituted by a methyl group, a methoxy group, an optionally labelled halogen atom, an -OH group or an $-NR_5R_6$ group where $R_5$ and $R_6$ can independently represent a hydrogen atom or a $(C_1-C_4)$alkyl group.

$R'_1$ is preferably a radionuclide within the meaning of the invention.

The preferences relating to the respective positions of the $R_1$ substituent and of the group

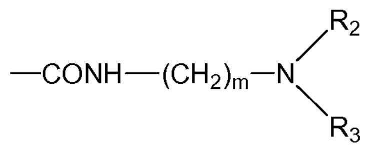

described above relating to the compounds of formula (I) also apply in defining the compounds of formula (II).

In addition, a subject-matter of the present invention is more particularly, according to a third aspect, novel compounds of formula (I")

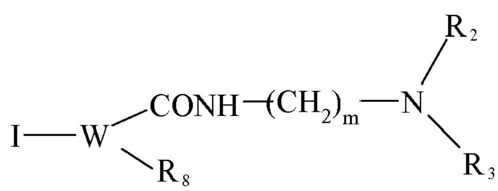

(I'')

in which
$R_2$, $R_3$ and m have the same meaning as above,
$R_8$ is as defined above and
W has the same meaning as above, it being possible for the iodine atom to be labelled.

A subject-matter of the present invention is more particularly novel compounds of formula (Id)

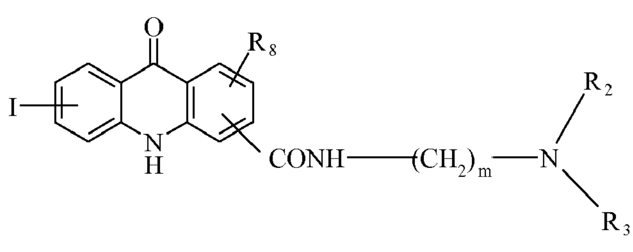

(Id)

in which
$R_2$, $R_3$ and m have the same meaning as above, and
$R_8$ is as defined above, it being possible for the iodine atom to be labelled.

In addition, a subject-matter of the present invention is more particularly novel compounds of formula (Ie)

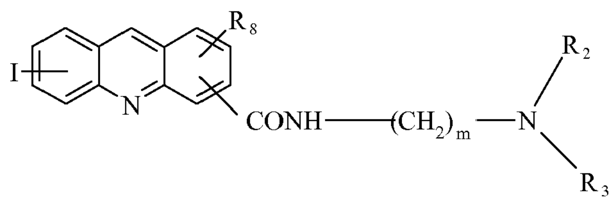

(Ie)

in which
$R_2$, $R_3$ and m have the same meaning as above, and
$R_8$ is as defined above.

Another subject-matter of the present invention is more particularly novel compounds of formula (Ij)

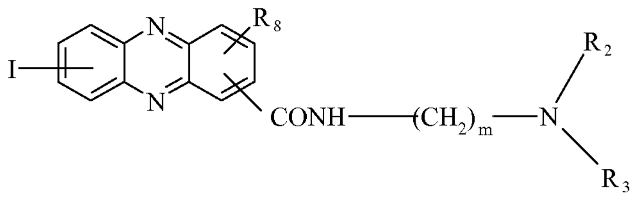

(Ij)

in which
$R_2$, $R_3$ and m have the same meaning as above, and
$R_8$ is as defined above, it being possible for the iodine atom to be labelled.

In addition, a subject-matter of the present invention is more particularly novel compounds of formula (Ik)

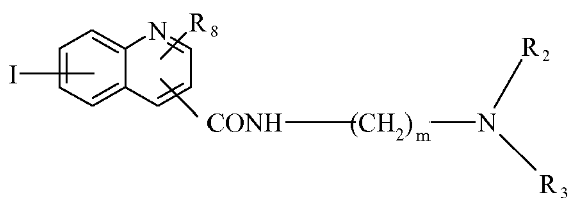

(Ik)

in which
$R_2$, $R_3$ and m have the same meaning as above, and
$R_8$ is as defined above, it being possible for the iodine atom to be labelled.

In addition, a subject-matter of the present invention is more particularly novel compounds of formula (Im)

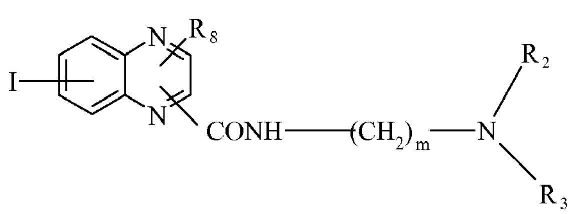

(Im)

in which
$R_2$, $R_3$ and m have the same meaning as above, and
$R_8$ is as defined above, it being possible for the iodine atom to be labelled.

In addition, a subject-matter of the present invention is more particularly novel compounds of formula (In)

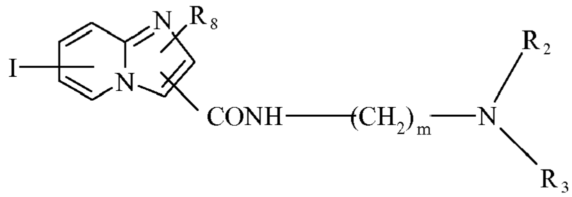

(In)

in which
$R_2$, $R_3$ and m have the same meaning as above, and
$R_8$ is as defined above, it being possible for the iodine atom to be labelled.

Another subject-matter of the present invention is more particularly novel compounds of formula (Iq)

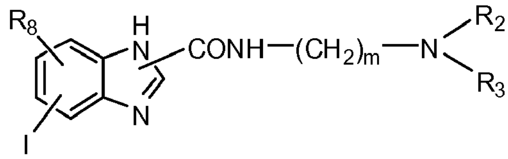

(Iq)

in which
$R_2$, $R_3$ and m have the same meaning as above, and
$R_8$ is as defined above, it being possible for the iodine atom to be labelled.

In addition, a subject-matter of the present invention is more particularly novel compounds of formula (Ir)

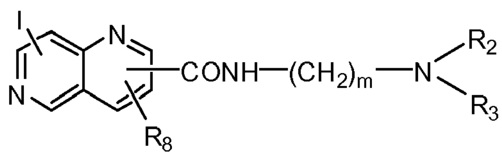

(Ir)

in which
$R_2$, $R_3$ and m have the same meaning as above, and
$R_8$ is as defined above, it being possible for the iodine atom to be labelled.

All the substitutions, including those represented for the compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq) and (Ir) by the $R_1$ and $R_8$ groups, the iodine atom or

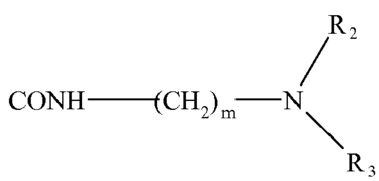

can take place in all the possible positions of the Ar group.

For the compounds of formulae (Ia), (Ib), (Ic), (Id) and (Ie), the substitution by the group

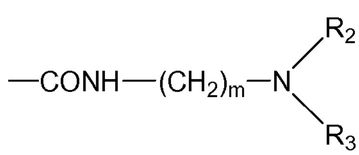

can take place in particular in the 4-position or in the 9-position of the acridine or in the 4-position of the acridone, which are the preferred positions.

For these same compounds, the $R_1$ group or the iodine atom is preferably in the 2-, 5- or 7-position of the acridine or of the acridone.

According to a preferred embodiment of the present invention, the compound is chosen from:

- *N*-(2-diethylaminoethyl)-6-iodoimidazo[1,2-*a*]pyridine-2-carboxamide (21);
- *N*-(2-diethylaminoethyl)-6-iodoquinoline-2-carboxamide (31);
- *N*-(4-dipropylaminobutyl)-6-iodoquinoline-2-carboxamide (49);
- *N*-(2-diethylaminoethyl)-6-iodoquinoxaline-2-carboxamide (46);
- *N*-(2-diethylaminoethyl)-5-iodobenzimidazole-2-carboxamide (26);
- *N*-(2-diethylaminoethyl)-9,10-dihydro-7-iodo-9-oxoacridine-4-carboxamide (84);
- *N*-(2-diethylaminoethyl)-5-iodoacridine-4-carboxamide (102);
- *N*-(2-diethylaminoethyl)-7-iodoacridine-4-carboxamide (111);
- *N*-(2-diethylaminoethyl)-8-iodonaphthyridine-2-carboxamide (142);
- *N*-(4-dipropylaminobutyl)-6-iodoquinoxaline-2-carboxamide (144);
- *N*-(2-diethylaminoethyl)-7-iodophenazine-1-carboxamide (151);
- and their pharmaceutically acceptable salts.

For the group of the compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II), m is preferably equal to 2 or 4.

The compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) can comprise one or more asymmetric carbon atoms. They can thus exist in the form of enantiomers or of diastereoisomers. These enantiomers, diastereoisomers and their mixtures, including the racemic mixtures, form part of the invention.

The pharmaceutically acceptable salts of the compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) include the addition salts with pharmaceutically acceptable acids, such as inorganic acids, for example hydrochloric, hydrobromic, phosphoric or sulphuric acid, and organic acids, such as acetic, trifluoroacetic, propionic, oxalic, succinic, fumaric, malic, tartaric, citric, ascorbic, maleic, glutamic, benzoic, toluenesulphonic, methanesulphonic, stearic and lactic acid.

The compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) or their salts can form solvates (namely hydrates); the invention includes such solvates.

In comparison with the compounds described in the document EP 458 886 and more particularly with *N*-(2-diethylaminoethyl)-4-iodobenzamide (BZA), improvements in terms of specificity and/or of affinity for the target organ, namely the melanoma, and/or in terms of pharmacokinetic profile and/or of antitumour effectiveness could be observed for some labelled compounds in accordance with the present invention.

When the compounds in accordance with the invention of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) are used for medical imaging purposes, $R_1$ is preferably a radionuclide possessing $\gamma$ or $\beta^+$ emission which can be detected according to conventional radioimaging techniques, for example scintigraphic imaging by single photon emission tomography (SPET) and by positron emission tomography (PET).

Such a radionuclide possessing $\gamma$ or $\beta^+$ emission advantageously exhibits an optimum energy for the measurement by means of a $\gamma$-camera or PET camera. Mention may in particular be made, as radionuclides acceptable for medical imaging, of $^{123}I$, $^{124}I$, $^{125}I$, $^{131}I$, $^{18}F$ , $^{75}Br$, $^{76}Br$ and $^{77}Br$.

$^{123}I$ is particularly suitable for SPET scintigraphic diagnosis and $^{124}I$ for a PET scintigraphic diagnosis.

When the compounds of the invention of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) are used for therapeutic purposes, $R_1$ is preferably a radionuclide possessing $\alpha$, $\beta^-$ or Auger electron emission. The radionuclides suitable in this context, capable of providing a cytotoxic effect, can be chosen from $^{131}I$, $^{125}I$, $^{211}At$ and $^{210}At$.

$^{131}I$ is particularly suitable for an application in the treatment of melanoma in internal radiotherapy. Furthermore, $^{125}I$, due to its Auger electron emission, can be used in internal radiotherapy provided that it is internalized in the cell.

The compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) can be prepared by condensation of an ester of formula (III)

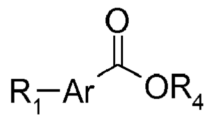

(III)

in which Ar has the same meaning as in the formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) and $R_4$ represents a ($C_1$-$C_6$)alkyl, aryl or heteroaryl group, for example chosen from benzene, pentafluorobenzene, *p*-nitrobenzene, triazole, benzotriazole, 7-azabenzotriazole and succinimide, with a diamine of formula (IV)

$H_2N$-$(CH_2)_m$-$NR_2R_3$ (IV)

in which m, $R_2$ and $R_3$ have the same meaning as in the formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II).

The condensation of the amine (IV) with the ester (III) can preferably be carried out in the presence or absence of trimethylaluminium by heating at reflux of the dichloromethane, of the toluene or of any other appropriate solvent.

This process of synthesis forms part of the invention as regards the compounds of formulae (I") and (II), according to a fourth aspect.

The compounds of formulae (I) and (II) in which $R_2$ or $R_3$ is a hydrogen can preferably be prepared by condensation of the amine (IV) with the ester (III) in which $R_4$ is a *p*-nitrophenyl. This reaction can preferably be carried out in tetrahydrofuran at ambient temperature.

The compounds of formula (III) can be synthesized from the compounds (V) according to various methodologies:

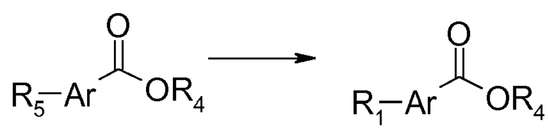

(V) (III)

When $R_5$ is a hydrogen atom: by direct iodination of the aromatic or heteroaromatic part preferably using *N*-iodosuccinimide at reflux of the acetonitrile or of any other appropriate solvent. This iodination can also be carried out in the presence of diiodine, sodium periodate and sulphuric acid. The iodination can also be obtained after formation of an organolithium compound at low temperature, followed by treatment with diiodine.

When $R_5$ is a halogen atom: by direct exchange in the presence of alkali metal iodide, in an acidic medium, and in the presence or absence of a catalyst, such as copper sulphate. The halogen/iodine exchange can also be obtained after passing through an organometallic compound (organolithium compound, organomagnesium compound, and the like) at low temperature, followed by treating the latter with diiodine.

When $R_5$ is an $NH_2$ group: by a diazotization reaction, the amine being treated at 0°C with sodium nitrite in an acidic medium, and then, after formation of the diazonium salt, by addition of alkali metal iodide and heating. This diazotization can also be carried out in an organic medium, the amino derivative being treated with tert-butyl nitrite in the presence of diiodomethane or of diiodine.

When $R_5$ is an $NO_2$ or NO group: by reduction, either by catalytic hydrogenation or by using a metal (Fe, Sn, and the like) in the presence of an acid (HCl, HBr, acetic acid, and the like). The amine thus obtained is treated according to the protocol described above.

In the case where Ar is an acridinyl group, the compounds (IIIb) are preferably obtained by reduction of the acridone (IIIa) to give acridane (VI) in the presence of a borane/tetrahydrofuran complex or of any other complexing agent, such as tert-butylamine, diethylamine, dimethylamine, morpholine, pyridine, trimethylamine, triethylamine, triphenylphosphine, dimethyl sulphoxide or dimethyl sulphide and at reflux of the tetrahydrofuran or of any other appropriate solvent. This methodology exhibits the advantage of reducing acridone to acridane without affecting the iodine or the ester functional group, in contrast to the conventional methods using, for example, as reducing agent, lithium aluminium hydride ($LiAlH_4$), which reduces the ester to alcohol, or the Al/Hg (or Na/Hg or Na/ROH) amalgam, which results in the substitution of the iodine by a hydrogen.

The acridanes (VI) are subsequently oxidized to acridines (IIIb) by ferric chloride hexahydrate at 50°C or by any other appropriate oxidizing agent, according to the scheme which follows:

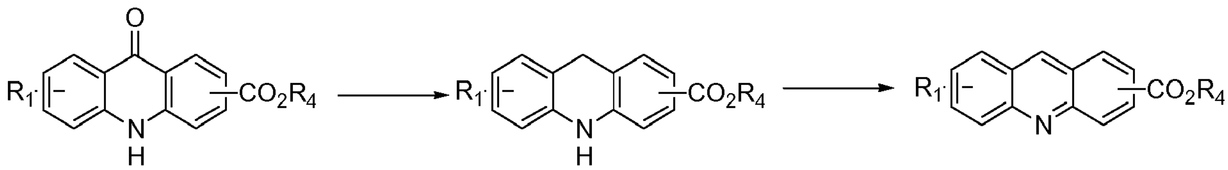

(IIIa) (VI) (IIIb)

This process is novel and also forms part of the invention.

Thus, the present invention relates, according to a fifth aspect, to the process for the preparation of a compound of formula (VI)

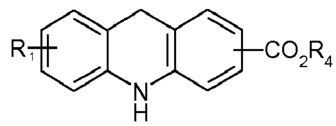

in which $R_1$ and $R_4$ are as defined above, of use as synthetic intermediate in the preparation of the compounds of formula (Ia),
characterized in that it comprises a stage of reduction of the acridone of formula (IIIa)

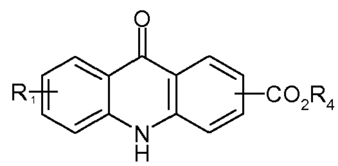

in which $R_1$ and $R_4$ are as defined above, in the presence of a complexing agent.

The process can be followed by a stage of oxidation of the acridanes of formula (VI) thus obtained in order to obtain an acridine of formula (IIIb).

The labelling of the compound of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) can be carried out by several techniques. For example, it can be carried out by exchange in an acidic medium between the nonradioactive iodinated molecule and a radioactive alkali metal halide. The exchange can be carried out by heating, at reflux, an aqueous solution of the compound of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) in a buffered medium or of the compound of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) in acetic acid and of the radioactive halide, in the presence or absence of copper sulphate. The labelling can also take place between a trialkylstannane precursor of the compound of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) and an alkali metal halide, such as $Na^{125}I$ or $Na^{131}I$, in the presence of an oxidizing agent, such as chloramine-T, peracetic acid or aqueous hydrogen peroxide solution, and an acid, such as hydrochloric acid, acetic acid or an acidic buffer, preferably at ambient temperature and in an appropriate solvent.

The trialkylstannane precursor compounds of the compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) also form part of the present invention. They are defined in the same way as the compounds of the abovementioned formulae, except for the $R_1$ group, which is an $-Sn[(C_1-C_6)alkyl]_3$ group and which can in particular be an $-SnBu_3$ group. In the continuation of the description, they are referred to as compounds of formula (VII)

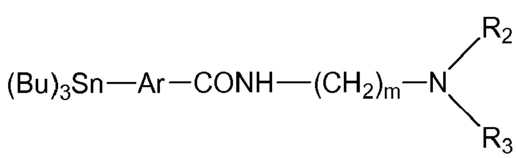

(VII)

in which $R_2$, $R_3$ and m have the same meaning as above.

The following examples illustrate the invention.

The melting points, uncorrected, were determined on an Electrothermal model IA 9300 capillary apparatus. The NMR spectra were recorded on an Avance DRX-200 (200 MHz for $^1H$ and 50 MHz for $^{13}C$) and a Bruker AC 400 (400 MHz for $^1H$ and 100 MHz for $^{13}C$). The chemical shifts $\delta$ are expressed in ppm (parts per million) and the coupling constants in Hz. The following abbreviations are used: s (singlet), bs (broad singlet), d (doublet), dd (split doublet), t (triplet), q (quartet), st (sextet), td (split triplet), m (multiplet). The infrared spectra were recorded on a Nicolet Impact 410 FTIR and Vector 22 FT spectrophotometer. The direct introduction mass spectra were recorded on a Hewlett Packard 5989A device coupled with a 5890 series 2 GC. The electrospray ionization mass spectra (ESI-MS) were obtained on an Esquire-LC, Brucker. In the case of the organotin compounds, the tin fragments are given for the 120 isotope of tin (isotopic abundance = 33%). The elemental analyses for C, H and N were carried out at the Service Central d'Analyse [Central Analytical Service] (CNRS, Vernaison, France). The thin layer chromatograms (TLC) were run on alumina plates (60 F254, type E, Merck) or on silica plates (Kieselgel 60 F254, Merck) and visualized under a UV lamp or with sublimed iodine. The column purification of the compounds was carried out using alumina (Merck aluminium oxide 90, standardized (activity stage II-III)) or silica (SDS, 60Å C.C, 35-70 $\mu$m, Chromagel) as support.

## EXAMPLE 1

Synthesis of N-(2-diethylaminoethyl)-6-iodo-2-naphthamide hydrochloride **(3)**

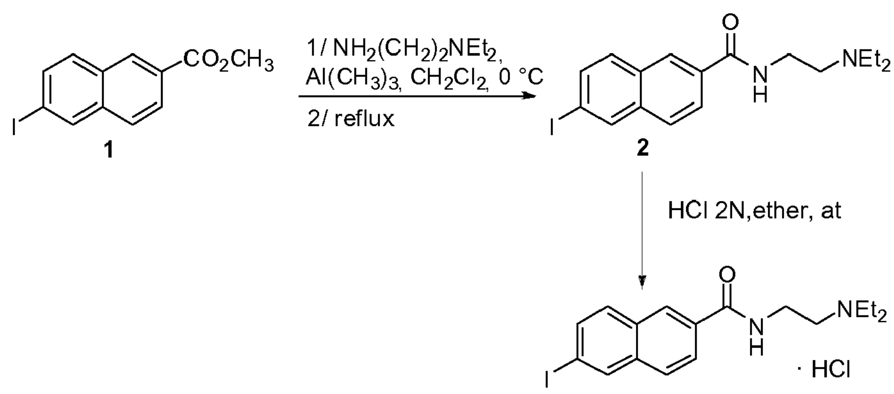


1

2

3

### Stage A: *N*-(2-diethylaminoethyl)-6-iodo-2-naphthamide (2)

A 2M solution of trimethylaluminium in toluene (7.2 ml, 14.4 mmol) is added to a solution of *N*,*N*-diethylethylenediamine (2 ml, 14.1 mmol) in anhydrous dichloromethane (110 ml) under argon at 0˚C. Methyl 6-iodo-2-naphthoate (**1**) (Adcock, W. and Wells, P. R., Aust. J. Chem., 1965, 18, 1351-1364) (3.12 g, 10 mmol), in solution in anhydrous dichloromethane (88 ml), is rapidly added. The medium is heated at reflux for 16 hours and then the reaction is halted with water (130 ml). The reaction mixture is extracted with dichloromethane (3 $\times$ 100 ml). The organic phase is dried over magnesium sulphate and evaporated under vacuum. The crude reaction product is purified by chromatography on alumina eluted with a dichloromethane/ethanol (97/3, v/v) mixture, to result in the amide **2** (1.58 g, 3.99 mmol). Yield: 40%; Rf: 0.58 ($Al_2O_3$, dichloromethane/ethanol (97/3, v/v)); melting point: 105-107˚C; $^1H$ NMR (400 MHz, $CDCl_3$) $\delta$ 1.05 (t, 6H, *J*= 7 Hz), 2.58 (q, 4H, *J*= 7 Hz), 2.68 (t, 2H, *J*= 6 Hz), 3.53 (q, 2H, *J*= 6 Hz), 7.40 (m, 1H), 7.54 (d, 1H, *J*= 8.5 Hz), 7.68 (d, 2H, 8,5 Hz), 7.83 (d, 1H, *J*= 8.5 Hz), 8.18 (s, 1H), 8.23 (s, 1H); $^{13}C$ NMR (100 MHz, $CDCl_3$) $\delta$ 11.8 (2C), 37.3, 46.6 (2C), 51.1, 93.6, 124.4, 127.0, 127.3, 130.2, 131.1, 132.3, 135.0, 135.7, 136.2, 166.9; IR (KBr) $\nu$ $cm^{-1}$: 1537, 1614, 1630, 3303; MS (m/z, %) 281 ($M^+$-115.6), 126 (9), 86 (100), 58 (14).

### Stage B: *N*-(2-diethylaminoethyl)-6-iodo-2-naphthamide hydrochloride (3)

*N*-(2-diethylaminoethyl)-6-iodo-2-naphthamide (**2**) (1.00 g, 2.52 mmol) is dissolved in anhydrous dichloromethane (22 ml) under argon at ambient temperature. A 2N solution of hydrochloric acid in ether (22 ml) is added to the reaction medium with stirring. The latter is stirred for 10 minutes and then the solvent is evaporated. The solid obtained is taken up in anhydrous ether (50 ml). The solution is stirred under argon and at ambient temperature for 24 hours. The precipitate obtained is filtered off, to result in the hydrochloride **3** (1.03 g, 2.38 mmol). Yield : 94% ; melting point:

151-152°C; ¹H NMR (200 MHz, $d_6$-DMSO) δ 1.22 (t, 6H, J= 7 Hz), 3.19 (m, 6H), 3.68 (m, 2H), 7.83 (m, 1H), 7.97 (m, 2H), 8.46 (s, 1H), 8.53 (s, 1H), 9.13 (m, 1H), 10.33 (m, 1H); IR (KBr) ν $cm^{-1}$: 1297, 1533, 1655, 3252. Anal. Calculated for $C_{17}H_{21}IN_2O \cdot HCl \cdot H_2O$: C, 45.30; H, 5.37; N, 6.22. Found: C, 45.26; H, 5.16; N, 6.40.

## EXAMPLE 2

Synthesis of *N*-(2-diethylaminoethyl)-6-iodonicotinamide dihydrochloride (7)

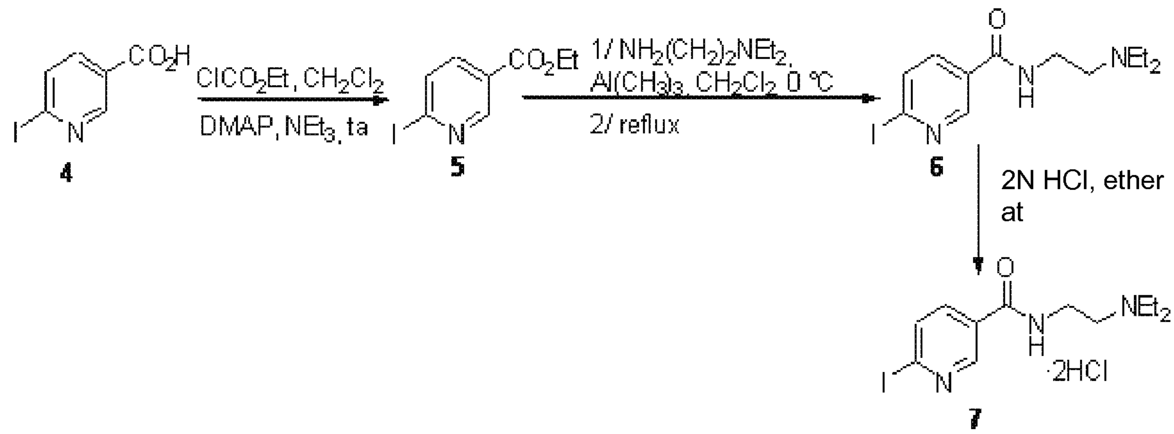


**Stage A: Ethyl 6-iodonicotinate (5)**

Triethylamine (1.67 µl, 1.19 mmol), ethyl chloroformate (0.70 ml, 7.32 mmol) and 4-dimethylaminopyridine (0.45 g, 3.68 mmol) are added, at 0°C, to a solution of 6-iodonicotinic acid (**4**) (Newkome, G. R., Moorfield, C. N. and Sabbaghian, B., J. Org. Chem., 1986, 51, 953-954) (250 mg, 1.00 mmol) in anhydrous dichloromethane (25 ml). After returning to ambient temperature, the reaction mixture is brought to reflux for 2 hours. The solution is evaporated to dryness and then the residue is purified on a column of alumina eluted with dichloromethane, to result in the ester **5** (211 mg, 0.76 mmol) ; yield: 76%; Rf: 0.92 ($Al_2O_3$, dichloromethane); melting point: 46-48°C; ¹H NMR (200 MHz, $CDCl_3$) δ 1.32 (t, 3H, *J* = 3 Hz), 4.32 (q, 2H, *J*= 7 Hz), 7.78 (m, 2H), 8.82 (d, 1H, *J*= 2 Hz); ¹³C NMR (50 MHz, $CDCl_3$) δ 14.2, 61.6, 123.3, 125.7, 134.7, 138.0, 151.4, 164.7; IR (KBr) ν $cm^{-1}$: 1268, 1292, 1577, 1711, 3082; MS (m/z, %) 277 ($M^+$, 61), 232 (21), 204 (20), 150 (100), 127 (16), 77 (43), 51 (22).

**Stage B: *N*-(2-diethylaminoethyl)-6-iodonicotinamide (6)**

The compound **6** was prepared according to the procedure described for the preparation of the compound **2**, using ethyl 6-iodonicotinate (**5**) as starting material and heating the reaction medium at reflux for 20 hours. Yield: 98%; viscous liquid: Rf: 0.60 ($Al_2O_3$, dichloromethane/ethanol (97/3, v/v)); ¹H NMR (200 MHz, $CDCl_3$) 8 0.83 (t, 6H, *J* = 7 Hz), 2.37 (q, 4H, *J* = 7 Hz), 2.47 (t, 2H, *J* = 6 Hz), 3.28 (q, 2H, *J* = 6 Hz), 7.38 (m, 1H), 7.78 (m, 2H), 8.73 (m, 1H); ¹³C NMR (50 MHz, $CDCl_3$) δ 11.7 (2C), 37.2, 46.4 (2C), 50.8, 120.9, 129.5, 134.5, 136.1, 148.6, 164.4; IR ($CCl_4$) ν $cm^{-1}$: 1538, 1630, 2927, 2965, 3301; MS (m/z, %) 348 ($M^+$ +1. 1), 86 (100), 77 (8), 58 (16).

**Stage C: *N*-(2-diethylaminoethyl)-6-iodonicotinamide dihydrochloride (7)**

The compound **7** was prepared according to the procedure described for the preparation of the compound **3,** using N-(2-diethylaminoethyl)-6-iodonicotinamide (**6**) as starting material. Yield: 70%; melting point: 127-129°C; ¹H NMR (200 MHz, *$d_6$*-DMSO) δ 1.26 (t, 6H, *J* = 7 Hz), 3.25 (m, 6H), 3.68 (m, 4H), 8.00 (m, 2H), 8.87 (m, 1H), 9.25 (m, 1H), 10.24 (m, 1H); IR (KBr) ν $cm^{-1}$: 1278, 1529, 1588, 1661, 2661, 3232. Anal. Calculated for $C_{12}H_{18}IN_3O \cdot 2HCl$: C, 34.31; H, 4.80; N, 10.00. Found: C, 34.43; H, 4.76; N, 10.08.

## EXAMPLE 3

Synthesis of *N*-(2-diethylaminoethyl)-5-iodoindole-2-carboxamide hydrochloride **(10)**

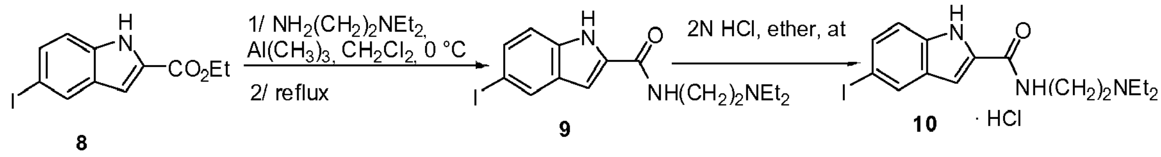


8 9 10

**Stage A: *N*-(2-diethylaminoethyl)-5-iodoindole-2-carboxamide (9)**

The compound **9** was prepared according to the procedure described for the preparation of the compound **2,** using ethyl 5-iodoindole-2-carboxylate (**8**) (Beshore, D. C. and Dinsmore, C. J., Synth. Commun., 2003, 33, 2423-2427) as starting material and heating the reaction medium at reflux for 3 hours. Yield: 50%; melting point: 208-210˚C; Rf 0.53 ($Al_2O_3$, dichloromethane/ethanol (97/3, v/v)); $^1$H NMR (200 MHz, $d_6$-DMSO) δ 1.03 (t, 6H, *J* = 7 Hz), 2.57 (m, 6H), 3.37 (m, 2H), 7.11 (s, 1H), 7.42 (AB spectrum, 2H, *J* = 8.5 Hz), 8.09 (s, 1H), 8.52 (t, 1H, *J* = 6 Hz), 11.82 (s, 1H); $^{13}$C NMR (50 MHz, $d_6$-DMSO) δ 11.9 (2C), 37.3, 46.8 (2C), 51.5, 83.3, 101.2, 114.7, 129.8, 129.9, 131.0, 132.8, 135.3, 160.6; IR (KBr) ν $cm^{-1}$: 1411, 1547, 1635, 2801, 2965, 3250, 3418; MS (m/z, %) 385 ($M^+$, 2), 86 (100), 58 (2).

**Stage B: *N*-(2-diethylaminoethyl)-5-iodoindole-2-carboxamide**

**hydrochloride (10)**

The compound **10** was prepared according to the procedure described for the preparation of the compound **3,** using *N*-(2-diethylaminoethyl)-5-iodoindole-2-carboxamide **(9)** as starting material. Yield: 81%; melting point: 217-219˚C; $^1$H NMR (400 MHz, $CDCl_3$) δ 1.29 (m, 6H), 3.28 (m, 6H), 3.72 (m, 2H), 7.22 (s, 1H), 7.31 (d, 1H, *J*= 8.5 Hz), 7.48 (d, 1H, *J*= 8.5 Hz), 8.06 (s, 1H), 9.16 (bs, 1H), 10.59 (bs, 1H), 11.94 (s, 1H); IR (KBr) ν $cm^{-1}$: 1544, 1646, 2468, 2569, 3228. Anal. Calculated for $C_{15}H_{20}IN_3O{\cdot}HCl$; C, 42.72; H, 5.02; N, 9.96. Found: C, 43.47; H, 5.20; N, 10.12.

## EXAMPLE 4

Synthesis of *N*-(2-diethylaminoethyl)-4-iodobenzo[*b*]thiophene-2-carboxamide hydrochloride (**13**)

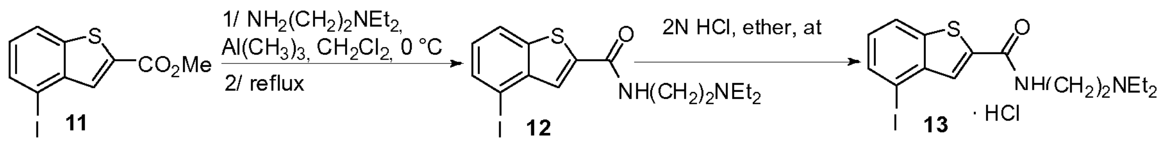


11 12 13

**Stage A: *N*-(2-diethylaminoethyl)-4-iodobenzo[*b*]thiophene-2-carboxamide** (12)

The compound **12** was prepared according to the procedure described for the preparation of the compound **2**, using methyl 4-iodobenzo[*b*]thiophene-2-carboxylate **(11)** (Bridges, A. J., Lee, A., Maduakor, E. C. and Schwartz, C. E., Tetrahedron Lett., 1992, 33, 7499-7502) as starting material and heating the reaction medium at reflux for 24 hours. Yield: 72%; Rf: 0.58 ($Al_2O_3$, dichloromethane/ethanol (97/3, v/v)); melting point: 76-78˚C; $^1$H NMR (400 MHz, $CDCl_3$) δ 1.09 (t, 6H, *J*= 7 Hz), 2.60 (q, 4H, *J*= 7 Hz), 2.68 (t, 2H, *J*= 7 Hz), 3.50 (q, 2H, *J*= 7 Hz), 7.08 (bs, 1H), 7.11 (t, 1H, *J*= 8 Hz), 7.80 (m, 3H); $^{13}$C NMR (100 MHz, $CDCl_3$) δ 12.2 (2C), 37.5, 46.9 (2C), 51.1, 91.4, 122.7, 127.2, 128.4, 134.9, 139.5, 140.0, 142.2, 161.7; IR (KBr) ν $cm^{-1}$: 1560, 1625, 2963, 3287; MS (m/z, %) 402 ($M^+$,1), 86 (100).

Stage B: ***N*-(2-diethylaminoethyl)-4-iodobenzo[b]thiophene-2-carboxamide**

**hydrochloride (13)**

The compound **13** was prepared according to the procedure described for the preparation of the compound **3**, using *N*-(2-diethylaminoethyl)-4-iodobenzo[b]thiophene-2-carboxamide (**12**) as starting material. Yield: 72%; melting point: 163-165°C; $^1$H NMR (200 MHz, $d_6$-DMSO) δ 1.28 (t, 6H, *J*= 7 Hz), 3.25 (m, 6H), 3.70 (m, 2H), 7.26 (t, 1H, *J*= 8 Hz), 7.92 (d, 1H, *J* = 8 Hz), 8.00 (d, 1H, *J* = 8 Hz), 8.22 (s, 1H), 9.42 (m, 1H), 10.29 (m, 1H); IR (KBr) ν cm$^{-1}$: 1535, 1648, 3249. Anal. Calculated for $C_{15}H_{19}IN_2OS \cdot HCl$: C, 41.06; H, 4.59; N, 6.39. Found: C, 41.14; H, 4.79; N, 6.21.

**EXAMPLE 5**

Syntheses of *N*-(2-diethylaminoethyl)-3-iodoimidazo[1,2-a]pyridine-2-carboxamide dihydrochloride (**16**) and of *N*-(2-diethylaminoethyl)-3-(tributylstannyl)imidazo[1,2-*a*]pyridine-2-carboxamide (**17**)

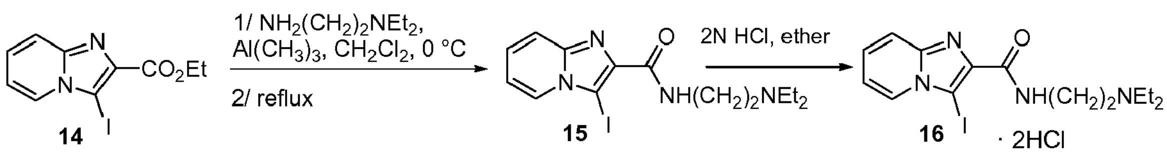

**14** **15** **16** · 2HCl

**Stage A: *N*-(2-diethylaminoethyl)-3-iodoimidazo[1,2-*a*]pyridine-2-carboxamide (15)**

The compound **15** was prepared according to the procedure described for the preparation of the compound **2,** using ethyl 3-iodoimidazo[1,2-*a*]pyridine-2-carboxylate **(14)** (Enguehard, C., Renou, J. L., Collot, V., Hervet, M., Rault, S. and Gueiffier, A., J. Org. Chem., 2000, 65, 6572-6575) as starting material and heating the reaction medium at reflux for 5 hours. Yield: 70%; viscous liquid; Rf: 0.41 ($Al_2O_3$, dichloromethane/ethanol (98/2, v/v)); $^1$H NMR (200 MHz, $CDCl_3$) δ 0.99 (t, 6H, *J* = 7 Hz), 2.54 (q, 4H, *J* = 7 Hz), 2.63 (t, 2H, *J* = 6.5 Hz), 3.48 (q, 2H, *J* = 6.5 Hz), 6.89 (t, 1H, *J* = 7 Hz), 7.24 (m, 1H), 7.48 (d, 1H, *J*= 9 Hz), 7.77 (m, 1H), 8.19 (d, 1H, *J*= 7 Hz); $^{13}$C NMR (50 MHz, $CDCl_3$) δ 12.2 (2C), 37.2, 47.2 (2C), 51.9, 64.1, 113.7, 117.9, 126.4, 126.6, 139.1, 146.7, 161.5; IR ($CCl_4$) ν cm$^{-1}$: 1217, 1250, 1546, 1662, 2924, 3450; MS (m/z, %): 387 ($M^+$ + 1, 1), 271 (10), 259 (12), 243 (5), 116 (6), 99 (6), 86 (100), 58 (13).

**Stage B: *N*-(2-diethylaminoethyl)-3-iodoimidazo[1,2-a]pyridine-2-carboxamide dihydrochloride (16)**

The compound **16** was prepared according to the procedure described for the preparation of the compound **3**, using *N*-(2-diethylaminoethyl)-3-iodoimidazo[1,2-a]pyridine-2-carboxamide (**15**) as starting material. Yield: 60%; melting point: 128-130°C; $^1$H NMR (200 MHz, $d_6$-DMSO) δ 1.31 (t, 6H, *J*= 7 Hz), 3.25 (m, 6H), 3.72 (m, 2H), 7.25 (t, 1 H, *J* = 7 Hz), 7.59 (m, 1H), 7.72 (d, 1H, *J* = 9 Hz), 8.56 (d, 1H, *J* = 7 Hz), 8.92 (bs, 1H), 10.49 (bs, 1H); IR (KBr) ν cm$^{-1}$: 1535, 1669, 2660, 2974, 3265, 3422. Anal. Calculated for $C_{14}H_{19}IN_4O$-2HCl: C, 36.62; H, 4.61; N, 12.20. Found: C, 36.40; H, 4.92; N, 11.97.

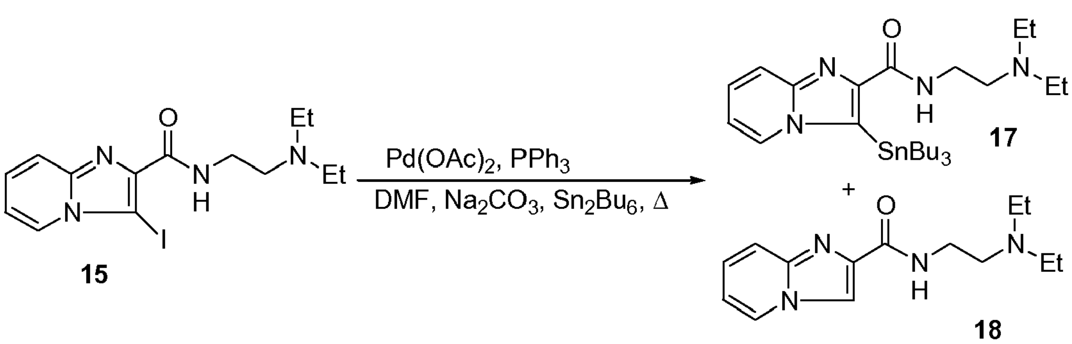

**15** **17** + **18**

**Stage C: *N*-(2-diethylaminoethyl)-3-(tributylstannyl)imidazo[1,2-*a*]pyridine-2-carboxamide (17)**

Bis(tributyltin) (1.30 ml, 2.57 mmol), triphenylphosphine (36 mg, 0.14 mmol), sodium carbonate (149 mg, 1.41 mmol) and palladium diacetate (20 mg, 0.09 mmol) are added, under argon, to a solution of iodinated compound **15** (546 mg, 1.41 mmol) in anhydrous dimethylformamide (8 ml). The solution is heated at 100˚C for 4 hours and then at 135˚C for 24 hours (the solution becomes black). After returning to ambient temperature, the solution is filtered through Celite® 521 and the filter residue is washed with dimethylformamide (8 ml). The filtrate is evaporated under vacuum. The residue obtained is chromatographed on a column of alumina eluted with a dichloromethane/ethanol (99/l, v/v) mixture, to result, in order to elution, in:

***N*-(2-diethylaminoethyl)-3-(tributylstannyl)imidazo[1,2-a]pyridine-2-carboxamide (17)**

(45 mg, 0.08 mmol); yield: 6%; viscous liquid; Rf: 0.26 ($Al_2O_3$, dichloromethane/ethanol: 99/1 (v/v)); $^1H$ NMR (400 MHz, $CDCl_3$) δ 0.86 (t, 9H, *J*= 7 Hz), 1.04 (t, 6H, *J* = 7 Hz), 1.37-1.17 (m, 12H), 1.53 (m, 6H), 2.62 (q, 4H, *J* = 7 Hz), 2.69 (t, 2H, *J* = 6 Hz), 3.51 (q, 2H, *J* = 6 Hz), 6.74 (t, 1H, *J* = 7 Hz), 7.17 (m, 1H), 7.56 (d, 1H, *J* = 9 Hz), 7.63 (bs, 1H), 8.20 (d, 1H, *J* = 7 Hz); $^{13}C$ NMR (100 MHz, $CDCl_3$) δ 11.8 (2C), 11.9 (3C, $^1J_{Sn-C}$= 365 Hz), 13.7 (3C), 27.3 (3C, $^3J_{Sn-C}$= 65 Hz), 29.1 (3C, $^2J_{S-C}$= 21 Hz), 37.0, 46.9 (2C), 51.9, 112.4, 118.1, 125.2, 127.7, 128.3, 148.1, 148.3, 164.1; MS (m/z, %): 493 ($M^+$-57.9), 100 (41), 86 (100), 58 (20).

**N-(2-diethylaminoethyl)imidazo[1,2-a]pyridine-2-carboxamide (18)**

(193 mg, 0.74 mmol); yield: 52%; viscous liquid; Rf 0.16 ($Al_2O_3$, dichloromethane/ethanol (99/1, (v/v)); $^1H$ NMR (400 MHz, $CDCl_3$) δ 1.09 (t, 6H, *J* = 7 Hz), 2.69 (m, 6H), 3.58 (q, 4H, *J* = 6 Hz),), 6.87 (t, 1H, *J* = 7 Hz), 7.28 (m, 1H), 7.60 (d, 1H, *J*= 9 Hz), 7.80 (bs, 1H), 8.19 (m, 2H); $^{13}C$ NMR (100 MHz, $CDCl_3$) δ 10.6 (2C), 35.9, 45.9 (2C), 50.8, 111.8, 113.2, 117.1, 124.9, 125.5, 139.1, 143.5, 161.8; IR ($CCl_4$) ν $cm^{-1}$: 1489, 1565, 1669, 2971; MS (m/z, %): 260 ($M^+$, 2), 145 (9), 99 (21), 86 (100), 58 (18).

**<u>EXAMPLE 6</u>**

Synthesis of *N*-(2-diethylaminoethyl)-6-iodoimidazo[1,2-*a*]pyridine-2-carboxamide dihydrochloride (**21**)

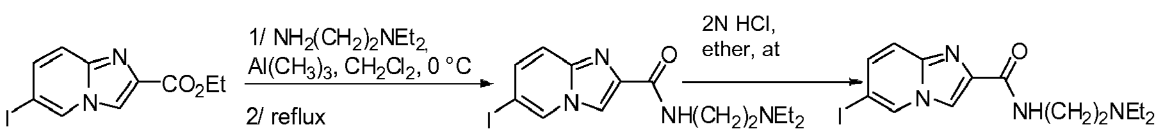


19 20 21 · 2HCl

**Stage A: N-(2-diethylaminoethyl)-6-iodoimidazo[1,2-*a*]pyridine-2-carboxamide (20)**

The compound **20** was prepared according to the procedure described for the preparation of the compound **2,** using ethyl 6-iodoimidazo[1,2-*a*]pyridine-2-carboxylate (**19**) (Sunberg, R. J., Biswas, S., Murthi, K. K., Rowe, D., McDall, J. W. and Dzimianski, M. T., J. Med. Chem., 1998, 41, 4317-4328) as starting material and heating the reaction medium at reflux for 72 hours. Yield: 83%; viscous liquid; Rf: 0.55 ($Al_2O_3$, dichloromethane/ethanol (97/3, v/v)); $^1H$ NMR (200 MHz, $CDCl_3$) δ 1.03 (t, 6H, *J*= 7 Hz), 2.57 (q, 4H, *J* = 7 Hz), 2.66 (t, 2H, *J* = 6.5 Hz), 3.51 (q, 2H, *J* = 6.5 Hz), 7.34 (m, 2H), 7.68 (m, 1H), 8.06 (s, 1H), 8.39 (t, 1H, J = 1.5 Hz); $^{13}C$ NMR (50 MHz, $CDCl_3$) δ 12.2 (2C), 37.3, 47.2 (2C), 51.9, 76.5, 113.4, 118.9, 130.9, 133.5, 140.2, 142.6, 161.8; IR ($CCl_4$) ν $cm^{-1}$: 1218, 1251, 1562, 1670, 2971, 3430; MS (m/z, %): 99 ($M^+$ - 287, 11), 86 (100), 58 (11).

**Stage B: *N*-(2-diethylaminoethyl)-6-iodoimidazo[1,2-*a*]pyridine-2-carboxamide dihydrochloride (21)**

The compound **21** was prepared according to the procedure described for the preparation of the compound **3**, using *N*-(2-diethylaminoethyl)-6-iodoimidazo[1,2-a]pyridine-2-carboxamide (**20**) as starting material. Yield: 80%; melting point: 208-210˚C; $^1H$ NMR (200 MHz, $d_6$-DMSO) δ 1.21 (t, 6H, *J*= 7 Hz), 3.17 (m, 6H), 3.58 (m, 2H), 7.57 (m, 2H), 8.41 (s, 1H), 9.00 (t, 1H, *J*= 5 Hz), 9.09 (s, 1H), 9.95 (m, 1H); IR (KBr) ν $cm^{-1}$: 1289, 1600, 1659, 2650, 3100-2900,

3426. Anal. Calculated for $C_{14}H_{19}IN_{40}O \cdot 2HCl \cdot 2H_2O$: C, 33.96; H, 5.09; N, 11.31. Found: C, 34.33; H, 5.20; N, 11.06.

**EXAMPLE 7**

Synthesis of *N*-(2-diethylaminoethyl)-5-iodobenzimidazole-2-carboxamide dihydrochloride **(26)**

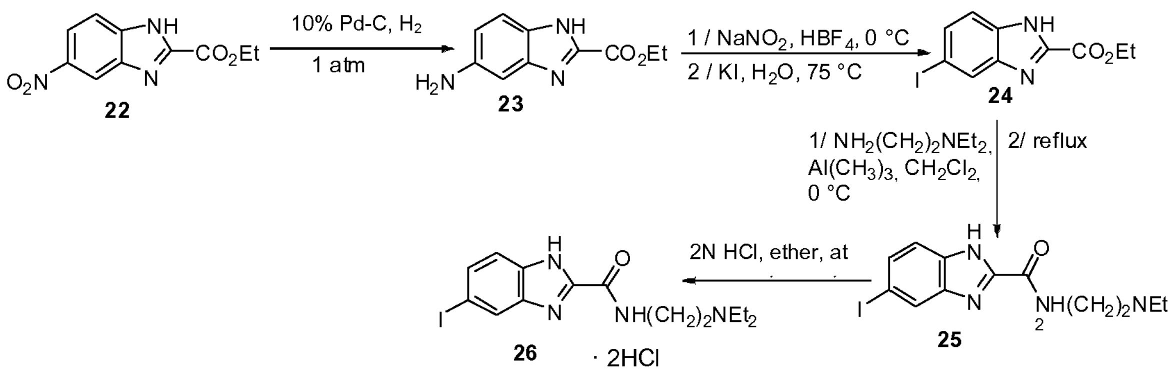


22 23 24 25 26 · 2HCl

**Stage A: Ethyl 5-aminobenzimidazole-2-carboxylate (23)**

10% palladium-on-charcoal (760 mg) is added to a solution of ethyl 5-nitrobenzimidazole-2-carboxylate (**22**) (Buchel, K. H. Z., Naturforsch., B. 1970, 25, 945-953) (7.00 g, 29.7 mmol) in ethyl acetate (213 ml). The solution is hydrogenated at atmospheric pressure for 18 hours. The reaction medium is filtered through Celite® 521, the filter residue is washed with ethanol and the filtrates are then evaporated to dryness. The orange solid obtained is purified by chromatography on alumina eluted with a dichloromethane/ethanol (97/3, v/v) mixture, to result in the pale orange solid **23** (2.22 g, 10.8 mmol). Yield: 36%; Rf: 0.25 ($Al_2O_3$, dichloromethane/ethanol (97/3, v/v)); melting point: 147-149°C; $^1H$ NMR (200 MHz, $CDCl_3$) δ 1.41 (t, 3H, *J*= 7 Hz), 4.46 (q, 2H, *J*= 7 Hz), 6.76 (m, 2H), 7.58 (d, 1H, *J* = 9 Hz); IR (KBr) ν $cm^{-1}$: 1269, 1701, 3370; MS (m/z, %) 205 ($M^+$, 53), 159 (100), 133 (52), 131 (44), 105 (35), 83 (22), 78 (20), 52 (21).

**Stage B: Ethyl 5-iodobenzimidazole-2-carboxylate (24)**

A solution of sodium nitrite (750 mg, 11.0 mmol) in water (3 ml) is added dropwise to a solution of the amine **23** (2.20 g, 10.7 mmol) in 50% tetrafluoroboric acid (40 ml, 32.1 mmol) at 0°C. The solution is stirred at 0°C for 1 hour and then filtered, to result in a white precipitate corresponding to the diazonium salt (3.26 g, 10.7 mmol). The latter is dissolved in water (67 ml) and then a solution of potassium iodide (2.79 g, 16.8 mmol) in water (14 ml) is added. The mixture is heated to 70°C. When evolution of gas has ceased (2 hours) and after returning to ambient temperature, the medium is basified using a saturated aqueous sodium carbonate solution (pH = 8-9). The solution is extracted with dichloromethane (2 × 80 ml) and the combined organic extracts are washed with a 5% aqueous sodium hydrogensulphite solution (2 × 40 ml), dried over magnesium sulphate, filtered and evaporated to give a brown powder. The product is purified by chromatography on alumina eluted with a dichloromethane/ethanol (99/1, v/v) mixture, to result in the yellow precipitate **24** (407 mg, 1.29 mmol). Yield: 12%; Rf 0.57 ($Al_2O_3$, dichloromethane/ethanol (99/1 v/v)); melting point: 166-168°C; $^1H$ NMR (200 MHz, $d_6$-DMSO) δ 1.40 (t, 3H, *J*= 7 Hz), 4.50 (q, 2H, *J*= 7 Hz), 7.51 (d, 1H, *J*= 9 Hz), 7.66 (dd, 1H, *J* = 9, 2 Hz), 8.09 (d, 1H, *J* = 2 Hz); IR (KBr) ν $cm^{-1}$: 1246, 1310, 1515, 1697, 3287; MS (m/z, %) 316 ($M^+$, 56), 270 (30), 244 (100), 117 (38), 90 (25), 63 (19).

**Stage C: *N*-(2-diethylaminoethyl)-5-iodobenzimidazole-2-carboxamide** (25)

The compound **25** was prepared according to the procedure described for the preparation of the compound **2**, using ethyl 5-iodobenzimidazole-2-carboxylate (**24**) as starting material and heating the reaction medium at reflux for 6 hours. Yield 82%; Rf: 0.38 ($Al_2O_3$, dichloromethane/ethanol (97/3, v/v)); melting point: 136-138°C; $^1H$ NMR (200 MHz, $CDCl_3$) δ 1.04 (t, 6H, *J*= 7 Hz), 2.64 (q, 4H, *J*= 7 Hz), 2.76 (m, 2H), 3.62 (m, 2H), 7.36 (d, 1H, *J* = 8.5 Hz), 7.52 (d, 1H, *J* = 8.5), 7.96 (bs, 1H), 8.28 (m, 1H); IR ν $cm^{-1}$: 1420, 1551, 1664, 2966, 3175; MS (m/z, %) 386 ($M^+$, 1), 86 (100), 58 (11).

**Stage D: *N*-(2-diethylaminoethyl)-5-iodobenzimidazole-2-carboxamide dihydrochloride (26)**

The compound **26** was prepared according to the procedure described for the preparation of the compound **3**, using *N*-(2-diethylaminoethyl)-5-iodobenzimidazole-2-carboxamide (**25**) as starting material. Yield: 82%; melting point: 217-219˚C; $^1$H NMR (200 MHz, $d_6$-DMSO) δ 1.27 (t, 6H, *J*= 7 Hz), 3.22 (m, 6H), 3.74 (m, 2H), 7.52 (d, 1H, *J* = 8.5 Hz), 7.64 (dd, 1H, *J* = 8.5, 1 Hz), 8.03 (d, 1H, *J* = 1 Hz), 9.36 (m, 1H), 10.45 (m, 1H); IR (KBr) ν cm$^{-1}$: 1593, 1683, 2979. Anal. Calculated for $C_{14}H_{19}IN_4O·2HCl·H_2O$: C, 35.24; H, 4.86; N, 11.74. Found: C, 35.38; H, 4.77; N, 11.79.

**EXAMPLE 8**

Syntheses of *N*-(2-diethylaminoethyl)-6-iodoquinoline-2-carboxamide dihydrochloride (**31**) and of *N*-(2-diethylaminoethyl)-6-(tributylstannyl)quinoline-2-carboxamide (**32**)

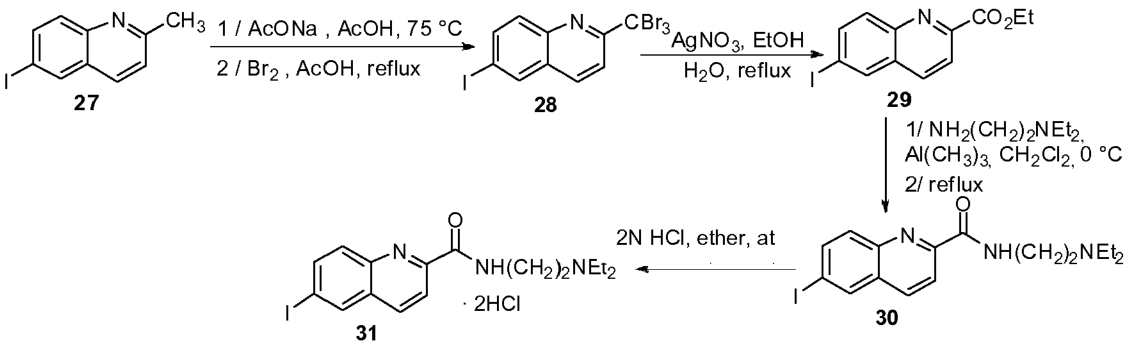


**Stage A: 2-tribromomethyl-6-iodoquinoline (28)**

6-Iodo-2-methylquinoline (**27**) (Petrow, V. and Sturgeon, B., J. Chem. Soc., 1954, 570-574) (7.12 g, 26.5 mmol) and sodium acetate (12.1 g) are dissolved in acetic acid (15 ml). The mixture is heated under argon at 75˚C for 10 minutes. A solution of dibromine (4.45 ml, 86.6 mmol) in acetic acid (13 ml) is added dropwise. On completion of the addition, the reaction medium is heated at reflux for 2 hours and then cooled to ambient temperature. The contents of the round-bottomed flask are poured onto crushed ice (200 g) and stirred at ambient temperature for 18 hours. The precipitate formed is filtered off, washed with water (60 ml) and placed in a dessicator for 4 hours, to result in the orange solid **28** (9.10 g, 18.0 mmol). Yield: 68%; Rf 0.86 ($Al_2O_3$ cyclohexane/ethyl acetate (8/2, v/v)); melting point: 124-126˚C; $^1$H NMR (400 MHz, $CDCl_3$) δ 7.92 (d, 1H, *J* = 8.5 Hz), 8.03 (d, 1H, *J* = 8.5 Hz), 8.14 (d, 1H, *J* = 9 Hz), 8.26 (m, 2H); $^{13}$C NMR (100 MHz, $CDCl_3$) δ 40.8, 94.5, 118.6, 129.1, 131.7, 135.9, 136.4, 139.3, 144.0, 159.0; IR (KBr) ν cm$^{-1}$: 1293, 1480, 1584; MS (m/z, %) 509 ($M^+$+6, 1), 507 ($M^+$+4, 2), 505 ($M^+$+2, 3), 503 ($M^+$, 1), 428 (40), 426 (77), 424 (45), 348 (81), 346 (81), 255 (100), 140 (97), 127 (90), 82 (90), 57 (89).

**Stage B: Ethyl 6-iodoquinoline-2-carboxylate (29)**

A solution of siliver nitrate (9.05 g, 53.8 mmol) in water (83 ml) is added dropwise to a solution of the iodinated compound **28** (9.10 g, 18.0 mmol) in ethanol (132 ml). The mixture is heated at reflux for 30 minutes. The solution is cooled to ambient temperature, filtered and acidified with an aqueous hydrochloric acid solution (1.5M, 5 ml). The filtrate is evaporated by half, basified with a saturated aqueous sodium carbonate solution (100 ml) and extracted with ether, and the ether extract is dried over magnesium sulphate and evaporated. The product is purified by chromatography on alumina eluted with an ethyl acetate/cyclohexane (8/2, v/v) mixture, to result in a dark yellow solid **29** (3.96 g, 12.1 mmol). Yield: 67%; Rf: 0.97 ($Al_2O_3$, ethyl acetate/cyclohexane (8/2, v/v)); melting point: 119-121˚C; $^1$H NMR (200 MHz, $d_6$-DMSO) δ 1.42 (t, 3H, *J* = 7 Hz), 4.46 (q, 2H, *J* = 7 Hz), 7.97 (d, 1H, *J* = 9 Hz), 8.16 (m, 2H), 8.55 (d, 1H, *J* = 9 Hz), 8.64 (s, 1H); $^{13}$C NMR (100 MHz, $CDCl_3$) δ 14.4, 62.4, 95.0, 121.8, 130.7, 132.2, 135.6, 136.4, 139.1, 146.4, 148.7, 165.0; IR (KBr) ν cm$^{-1}$: 1307, 1714; MS (m/z, %) 327 ($M^+$, 17), 283 (19), 255 (100), 127 (28), 100 (11).

**Stage C: *N*-(2-diethylaminoethyl)-6-iodoquinoline-2-carboxamide (30)**

The compound **30** was prepared according to the procedure described for the preparation of the compound

**2**, using ethyl 6-iodoquinoline-2-carboxylate (**29**) as starting material and heating the reaction medium at reflux for 6 hours. Yield: 69%; Rf: 0.56 ($Al_2O_3$, dichloromethane/ethanol (97/3, v/v)); melting point: 75-77°C; $^1H$ NMR (400 MHz, $CDCl_3$) δ 1.09 (t, 6H, *J*= 7 Hz), 2.63 (q, 4H, *J*= 7 Hz), 2.73 (t, 2H, *J*= 7 Hz), 3.58 (q, 2H, *J* = 7 Hz), 7.81 (d, 1H, *J* = 9 Hz), 7.98 (dd, 1H, *J* = 9 and 2 Hz), 8.16 (d, 1H, *J* = 8.5 Hz), 8.26 (d, 1H, *J* = 2 Hz), 8.30 (d, 1H, *J* = 8.5 Hz), 8.57 (bs, 1H); $^{13}C$ NMR (100 MHz, $CDCl_3$) δ 12.1 (2C), 37.5, 47.2 (2C), 51.7, 93.7, 119.6, 130.7, 131.8, 136.1, 136.5, 139.4, 145.4, 150.5, 164.1; IR (KBr) ν $cm^{-1}$: 1526, 1663, 2963, 3400; MS (m/z, %) 386 ($M^+$, 1), 86 (100), 58 (11).

**Stage D: *N*-(2-diethylaminoethyl)-6-iodoquinoline-2-carboxamide dihydrochloride (31)**

The compound **31** was prepared according to the procedure described for the preparation of the compound **3,** using *N*-(2-diethylaminoethyl)-6-iodoquinoline-2-carboxamide (**30**) as starting material. Yield: 69%; melting point: 104-106°C; $^1H$ NMR (200 MHz, $d_6$-DMSO) δ 1.28 (t, 6H, *J*= 7 Hz), 3.24 (m, 6H), 3.77 (q, 2H, *J*= 7 Hz), 7.92 (d, 1H, *J*= 9 Hz), 8.21 (m, 2H), 8.57 (d, 1H, *J*= 9 Hz), 8.65 (s, 1H), 9.34 (m, 1H), 10.08 (m, 1H); IR ν $cm^{-1}$: 1384, 1523, 1684, 3415. Anal. Calculated for $C_{16}H_{20}IN_3O{\cdot}2HCl{\cdot}2.5H_2O$: C, 37.30; H, 5.28; N, 8.16. Found: C, 37.35; H, 5.15; N, 8.23.

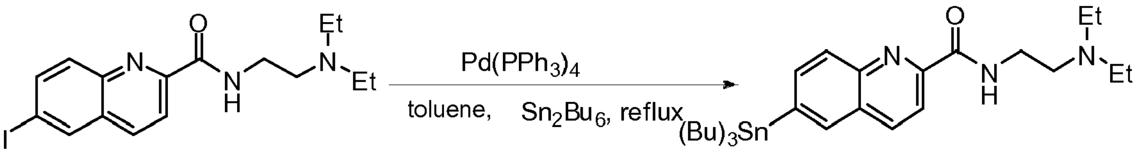


30 32

**Stage E: *N*-(2-diethylaminoethyl)-6-(tributylstannyl)quinoline-2-carboxamide (32)**

Bis(tributyltin) (443 μl, 1.18 mmol) and a spatula tip of tetrakis(triphenylphosphine)palladium are added, under argon, to a solution of the iodinated compound **30** (332 mg, 0.87 mmol) in anhydrous toluene (15 ml) degassed beforehand under argon. The solution is heated at reflux for 8 hours. After returning to ambient temperature, the solution is filtered through Celite® 521 and the filtrate is evaporated under vacuum. The residue obtained is chromatographed on a column of alumina eluted with an ethyl acetate/cyclohexane (6/4, v/v) mixture, to result in the tributylstannane **32** (282 mg, 0.50 mmol); yield: 58%; viscous liquid; Rf: 0.86 (ethyl acetate/cyclohexane (6/4, v/v)); $^1H$ NMR (400 MHz, $CDCl_3$) δ 0.90 (t, 9H, *J* = 7 Hz), 1.14 (m, 12H), 1.36 (m, 6H), 1.57 (m, 6H), 2.69 (q, 4H, *J*= 7 Hz), 2.80 (t, 2H, *J*= 6 Hz), 3.63 (q, 2H, *J* = 6 Hz), 7.84 (d, 1H, *J* = 8.5 Hz), 7.96 (s, 1H), 8.04 (d, 1H, *J* = 8.5 Hz), 8.29 (AB spectrum, 2H, *J*= 8.5 Hz), 8.67 (m, 1H); $^{13}C$ NMR (100 MHz, $CDCl_3$) δ 10.0 (3C, $^1J_{Sn\text{-}C}$= 333 Hz), 11.8 (2C), 13.7 (3C), 27.4 (3C, $^3J_{Sn\text{-}C}$= 56 Hz), 29.2 (3C, $^2J_{Sn\text{-}C}$= 20 Hz), 37.4, 47.4 (2C), 51.8, 118.7, 128.2, 128.9, 136.1, 136.9, 137.3, 143.4, 146.5, 149.6, 164.8; IR ($CCl_4$) ν $cm^{-1}$: 1519, 1680, 2926, 2960; ESI-MS m/z 562.2 $[M+H]^+$.

**EXAMPLE 9**

Synthesis of *N*-(2-diethylaminoethyl)-1,4-dihydro-6-iodo-4-oxoquinoline-3-carboxamide hydrochloride (**35**)

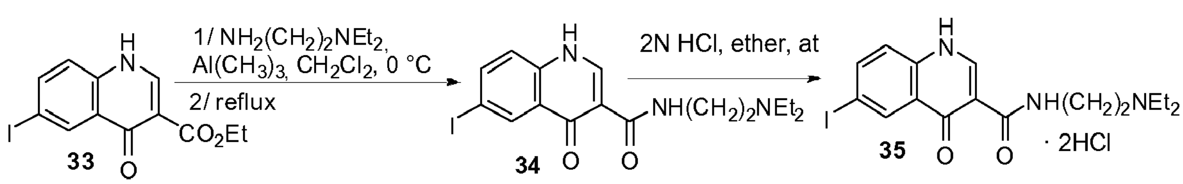


33 34 35

**Stage A: *N*-(2-diethylaminoethyl)-1,4-dihydro-6-iodo-4-oxoquinoline-3-carboxamide (34)**

The compound **34** was prepared according to the procedure described for the prpearation of the compound **2**, using ethyl 1,4-dihydro-6-iodo-4-oxoquinoline-3-carboxylate (**33**) (Lin, A. J. and Loo, T. L., J. Med. Chem., 1978, 21, 268-272) as starting material and heating the reaction medium at reflux for 5 hours. Yield: 81%; Rf 0.04 ($Al_2O_3$, dichloromethane/ethanol (97/3, v/v)); melting point: 234-236°C; $^1H$ NMR (400 MHz, $d_6$-DMSO) δ 1.13 (t, 6H, *J* = 7 Hz), 2.85

(m, 6H), 3.69 (q, 2H, *J* = 6 Hz), 7.33 (d, 1H, *J* = 8.5 Hz), 7.87 (d, 1H, *J* = 8.5 Hz), 8.49 (s, 1H), 8.67 (s, 1H), 10.32 (bs, 1H); $^{13}C$ NMR (100 MHz, $d_6$-DMSO) δ 11.4 (2C), 36.4, 46.6 (2C), 51.4, 89.6, 111.3, 121.5, 127.9, 133.8, 138.7, 140.5, 144.0, 164.2, 174.4; IR (KBr) ν $cm^{-1}$: 1507, 1551, 1633, 2965, 3061; MS (m/z, %) 413 ($M^+$, 1), 86 (100), 58 (10).

**Stage B: *N*-(2-diethylaminoethyl)-1,4-dihydro-6-iodo-4-oxoquinoline-3-carboxamide dihydrochloride (35)**

The compound **35** was prepared according to the procedure described for the preparation of the compound **3**, using *N*-(2-diethylaminoethyl)-1,4-dihydro-6-iodo-4-oxoquinoline-3-carboxamide (**34**) as starting material. Yield: 92%; melting point: 164-166°C; $^1H$ NMR (200 MHz, $d_6$-DMSO) δ 1.27 (t, 6H, *J* = 7 Hz), 3.24 (m, 6H), 3.73 (m, 2H), 7.62 (d, 1H, *J* = 9 Hz), 8.11 (dd, 1H, *J* = 9 and 1.5 Hz), 8.5 (d, 1H, *J* = 1.5 Hz), 8.80 (d, 1H, *J*= 6.5 Hz), 10.07 (m, 2H); IR (KBr) ν $cm^{-1}$: 1521, 1618, 1654, 3028, 3514. Anal. Calculated for $C_{16}H_{20}IN_3O_2 \cdot 2HCl \cdot 1.5H_2O$: C, 37.45; H, 4.91; N, 8.19. Found: C, 37.42; H, 5.08; N, 8.12.

**EXAMPLE 10**

Synthesis of *N*-(2-diethylaminoethyl)-5-iodoisoquinoline-3-carboxamide dihydrochloride (**39**)

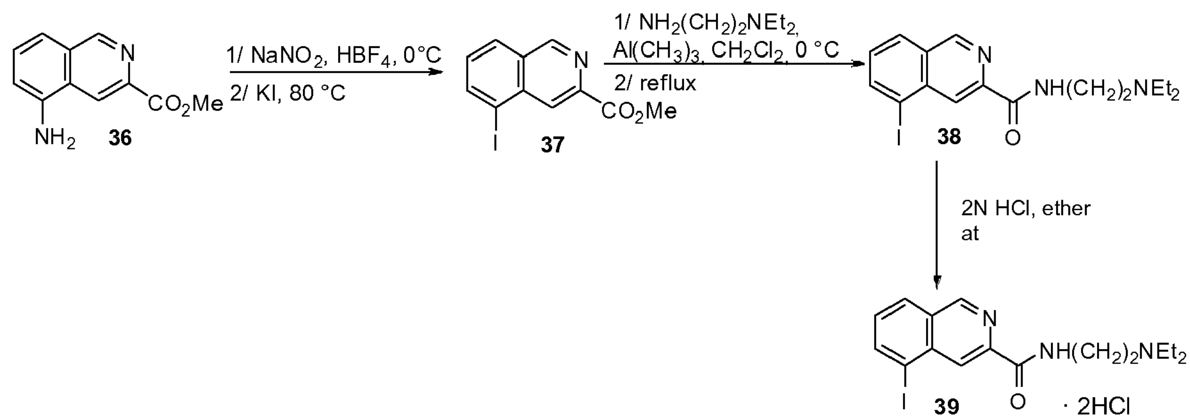

36
37
38
39

**Stage A: Methyl 5-iodoisoquinoline-3-carboxylate (37)**

Concentrated hydrochloric acid (576 μl) is added to a suspension of methyl 5-aminoisoquinoline-3-carboxylate (**36**) (Lee, C. H., Bayburt, E. K., DiDomenico, S., Drizin, I., Gomtsyan, A. R., Koenig, J. R., Perner, R. J., Schmidt, R. G., Turner, S. C., White, T. K. and Zheng, G. Z., US Patent 656417, 2003) (500 mg, 2.47 mmol) in water (4 ml). The solution is cooled to 0°C and then a solution of sodium nitrite (166 mg, 2.47 mmol) in water (3 ml) is rapidly added. After stirring at 0°C for 30 minutes, 50% tetrafluoroboric acid (311 μl, 2.47 mmol) is added. Stirring is continued for 30 minutes. The precipitate formed is filtered off. The diazonium salt obtained is dissolved in water (12 ml) and then a solution of potassium iodide (453 mg, 3.10 mmol) in water (5 ml) is rapidly added. The reaction medium is heated to 80°C. When the evolution of gas has ceased (2 hours) and after returning to ambient temperature, the reaction mixture is extracted with dichloromethane (3 × 30 ml) and the organic extract is washed with a 5% aqueous sodium hydrogensulphite solution (40 ml), dried over magnesium sulphate, filtered and evaporated. The product is purified by chromatography on alumina eluted with a dichloromethanol/ethanol (99/1, v/v) mixture, to result in an orange precipitate **37** (190 mg, 0.61 mmol). Yield: 25%; Rf 0.64 ($Al_2O_3$, dichloromethane/ethanol (99/1, v/v)); melting point: 165-167°C; $^1H$ NMR (400 MHz, $d_6$-DMSO) δ 4.00 (s, 3H), 7.65 (t, 1H, *J*= 8 Hz), 8.32 (d, 1H, *J*= 8 Hz), 8.50 (d, 1H, *J*= 8 Hz), 8.55 (s, 1H), 9.37 (s, 1H); $^{13}C$ NMR (100 MHz, $d_6$-DMSO) δ 52.6, 99.6, 126.1, 128.5, 130.5, 131.3, 136.4, 142.2, 142.8, 153.8, 165.2; IR (KBr) ν $cm^{-1}$: 1249, 1304, 1710; MS (m/z, %) 313 ($M^+$, 313), 283 (20), 255 (100), 127 (41), 100 (18), 74 (11).

**Stage B: N-(2-diethylaminoethyl)-5-iodoisoquinoline-3-carboxamide (38)**

The compound **38** was prepared according to the procedure described for the preparation of the compound **2**, using methyl 5-iodoisoquinoline-3-carboxylate (**37**) as starting material and heating the reaction medium at reflux for 4 hours. Yield: 72%; Rf: 0.47 ($Al_2O_3$, dichloromethane/ethanol (97/3, v/v)); viscous liquid; $^1H$ NMR (400 MHz, $CDCl_3$) δ

1.08 (t, 6H, *J*= 7 Hz), 2.64 (q, 4H, *J*= 7 Hz), 2.74 (t, 2H, *J*= 7 Hz), 3.62 (q, 2H, *J* = 7 Hz), 7.40 (t, 1H, *J* = 8 Hz), 8.00 (d, 1H, *J* = 8 Hz), 8.31 (d, 1H, *J* = 8 Hz), 8.57 (m, 1H), 8.76 (s, 1H), 9.05 (s, 1H); $^{13}$C NMR (100 MHz, $CDCl_3$) δ 11.9 (2C), 37.5, 47.3 (2C), 51.8, 99.4, 123.8, 128.3, 129.7, 130.6, 138.1, 142.0, 145.8, 152.0, 164.5; IR (KBr) ν cm$^{-1}$: 1516, 1677, 2971; MS (m/z, %) 397 ($M^+$, 1), 86 (100), 58 (7).

**Stage C: *N*-(2-diethylaminoethyl)-5-iodoisoquinoline-3-carboxamide dihydrochloride (39)**

The compound **39** was prepared according to the procedure described for the preparation of the compound **3**, using *N*-(2-diethylaminoethyl)-5-iodoisoquinoline-2-carboxamide (**38**) as starting material. Yield: 72%; melting point: 152-154˚C; $^1$H NMR (200 MHz, $d_6$-DMSO) δ 1.22 (t, 6H, *J*= 7 Hz), 3.25 (m, 6H), 3.74 (m, 2H), 7.63 (t, 1H, *J*= 8 Hz), 8.33 (d, 1H, *J* = 8 Hz), 8.49 (d, 1H, *J* = 8 Hz), 8.56 (s, 1H), 9.38 (m, 2H), 9.76 (m, 1H); IR (KBr) ν cm$^{-1}$: 1526, 1684, 3447, 3927. Anal. Calculated for $C_{16}H_{20}IN_3O{\cdot}2HCl$: C, 40.87; H, 4.72; N, 8.94. Found: C, 40.91; H, 4.75; N, 8.84.

**EXAMPLE 11**

Synthesis of *N*-(2-diethylaminoethyl)-6-iodoquinoxaline-2-carboxamide dihydrochloride (**46**) and of *N*-(2-diethylaminoethyl)-6-(tributylstannyl)quinoxaline-2-carboxamide (**47**)

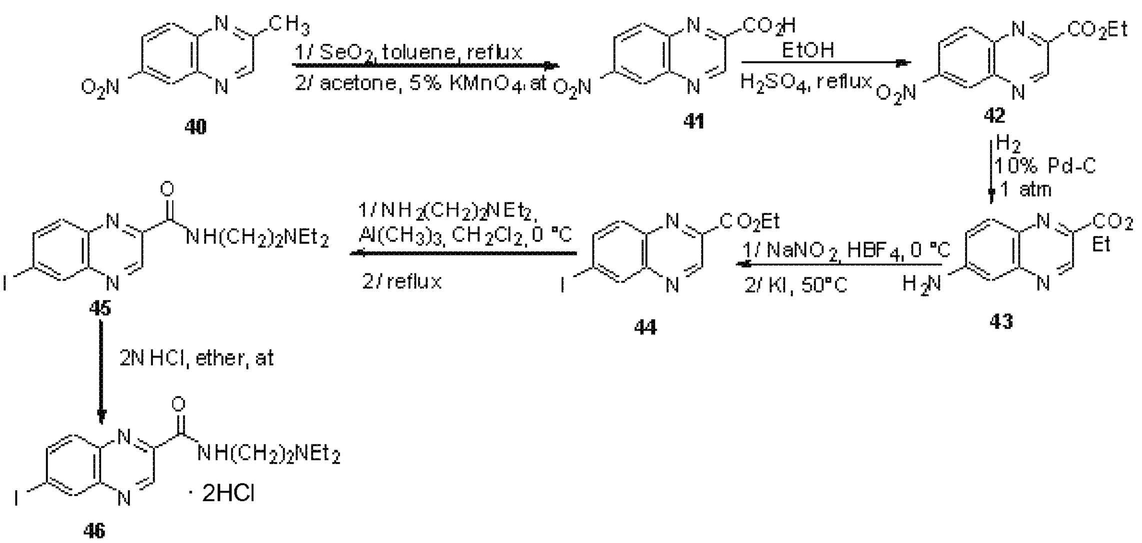


**Stage A: 6-nitroquinoxaline-2-carboxylic acid (41)**

Selenium dioxide (16.0 g, 0.14 mol) is added to a solution of 2-methyl-6-nitroquinoxaline (**40**) (Higashida, S., Sakurai, M., Yabe, Y., Nishihgaki, T., Komai, T. and Handa, H. ; Patent EP 0 587 311, 1994) (15.14 g, 80.1 mmol) in toluene (240 ml). The solution is heated at reflux for 2 hours. After returning to ambient temperature, the precipitate obtained is filtered off and washed with toluene (40 ml) and dichloromethane (50 ml). The precipitate (aldehyde) obtained is dissolved in acetone (400 ml) and a 5% aqueous sodium permanganate solution (540 ml) is added fractionwise. The solution is stirred at ambient temperature for 3 hours and then filtered. The filtrate is reduced to half under vacuum and the solution is extracted with ether. The aqueous phase is acidified (pH = 1) with concentrated hydrochloric acid. The precipitate thus obtained is filtered off and placed in a dessicator for 4 days, to result in the acid **41** (5.26 g, 24.0 mmol). Yield: 30%; melting point: 212-214˚C; $^1$H NMR (200 MHz, $d_6$-DMSO) δ 8.51 (d, 1H, *J*= 9 Hz), 8.67 (dd, 1H, *J* = 2.5, 9 Hz), 9.01 (d, 1H, *J* = 2.5 Hz), 9.62 (s, 1H); $^{13}$C NMR (100 MHz, $d_6$-DMSO) δ 124.3, 124.9, 132.1, 141.6, 143.2, 146.3, 147.3, 148.8, 164.6; IR (KBr) ν cm$^{-1}$: 1147, 1347, 1526, 1706, 3200-3500; MS (m/z, %) 219 ($M^+$, 37), 175 (100), 129 (37), 102 (39), 75 (37).

**Stage B: Ethyl 6-nitroquinoxaline-2-carboxylate (42)**

Concentrated sulphuric acid (750 μl) is added to a solution of the acid **41** (5.00 g, 22.8 mmol) in anhydrous

ethanol (50 ml) under argon. The solution is heated at reflux for 7 hours. After returning to ambient temperature, the solution is evaporated under vacuum, a saturated aqueous sodium bicarbonate solution (50 ml) is added and then the mixture is extracted with dichloromethane. The organic phase is dried over magnesium sulphate and evaporated under vacuum, to produce the ester **42** (3.79 g, 15.3 mmol). Yield: 67%; melting point: 221-223°C; $^1$H NMR (200 MHz, $CDCl_3$) δ 1.58 (t, 3H, *J* = 7 Hz), 4.68 (q, 2H, *J* = 7 Hz), 8.53 (d, 1H, *J* = 9 Hz), 8.68 (dd, 1H, *J* = 2.5, 9 Hz), 9.14 (d, 1H, *J* = 2.5 Hz), 9.72 (s, 1H); IR (KBr) ν $cm^{-1}$: 1282, 1347, 1531, 1741; MS (m/z, %) 248 ($M^+$+1, 4), 203 (36), 175 (100), 128 (23), 101 (32), 75 (24).

**Stage C: Ethyl 6-aminoquinoxaline-2-carboxylate (43)**

10% palladium-on-charcoal (300 mg) is added to a solution of the nitrated ester **42** (2.93 g, 11.9 mmol) in ethanol (500 ml). The solution is hydrogenated at atmospheric pressure for 3 hours 45. The reaction medium is filtered through Celite® 545, the filter residue is washed with ethanol and then the filtrates are evaporated to dryness. The orange solid obtained is purified by chromatography on alumina eluted with a dichloromethane/ethanol (99/1, v/v) mixture, to result in the amine **43** (1.69 g, 7.79 mmol). Yield: 66%; Rf: 0.55 ($Al_2O_3$, dichloromethane/ethanol (99/1, v/v)); melting point: 181-183°C; $^1$H NMR (200 MHz, $CDCl_3$) δ 1.49 (t, 3H, *J*= 7 Hz), 4.38 (m, 2H), 4.56 (q, 2H, *J*= 7 Hz), 7.16 (d, 1H, *J* = 2.5 Hz), 7.26 (dd, 1H, *J* = 2.5, 9 Hz), 8.05 (d, 1H, *J* = 9 Hz), 9.35 (s, 1H); $^{13}$C NMR (100 MHz, $CDCl_3$) δ 14.4, 62.1, 106.9, 123.1, 131.9, 136.6, 138.4, 145.6, 145.9, 150.4, 164.7; IR (KBr) ν $cm^{-1}$: 1299, 1487, 1613, 1701, 3202, 3431; MS (m/z, %) 217 ($M^+$, 23), 145 (100), 117 (19), 90 (16), 63 (14).

**Stage D: Ethyl 6-iodoquinoxaline-2-carboxylate (44)**

A solution of sodium nitrite (480 mg, 6.96 mmol) in water (3 ml) is added dropwise at 0°C to a solution of the amine **43** (1.37 g, 6.31 mmol) in 50% tetrafluoroboric acid (10 ml). The mixture is stirred at 0°C for one hour and then a solution of potassium iodide (1.57 g, 9.46 mmol) in water (5 ml) is added. The solution is stirred at 0°C for one hour and then at 50°C for one hour. After returning to ambient temperature, the reaction mixture is basified with a saturated aqueous sodium bicarbonate solution (60 ml). The solution is extracted with dichloromethane. The organic phase is washed with a 5% aqueous sodium hydrogensulphite solution (2 x 30 ml). The organic phase is dried over magnesium sulphate and evaporated under vacuum. The residue is deposited on a column of alumina eluted with dichloromethane, to result in the iodinated ester **44** (0.68 g, 2.07 mmol). Yield: 33%; Rf: 0.80 ($Al_2O_3$, dichloromethane); melting point: 160-162°C; $^1$H NMR (400 MHz, $CDCl_3$) δ 1.51 (t, 3H, *J*= 7 Hz), 4.59 (q, 2H, *J*= 7 Hz), 7.99 (d, 1H, *J* = 9 Hz), 8.10 (dd, 1H, *J* = 2 and 9 Hz), 8.62 (d, 1H, *J* = 2 Hz), 9.51 (s, 1H); $^{13}$C NMR (100 MHz, $CDCl_3$) δ 14.3, 62.7, 99.2, 131.6, 138.5, 140.0, 140.7, 143.0, 144.1, 145.7, 163.9; IR (KBr) ν $cm^{-1}$: 1103, 1151, 1230, 1237, 1717; MS (m/z, %) 328 ($M^+$, 19), 284 (43), 256 (100), 128 (29), 101 (50), 75 (30).

**Stage E: *N*-(2-diethylaminoethyl)-6-iodoquinoxaline-2-carboxamide (45)**

The compound **45** was prepared according to the procedure described in the preparation of the compound **2**, using ethyl 6-iodoquinoxaline-2-carboxylate (**44**) as starting material and heating the reaction medium at reflux for 7 hours. Yield: 77%; melting point: 60-62°C; Rf 0.64 ($Al_2O_3$, dichloromethane/ethanol (97/3, v/v)); $^1$H NMR (400 MHz, $CDCl_3$) δ 1.00 (t, 6H, *J*= 7 Hz), 2.54 (q, 4H, *J*= 7 Hz), 2.64 (t, 2H, *J*= 6 Hz), 3.49 (q, 2H, *J* = 6 Hz), 7.71 (d, 1H, *J* = 9 Hz), 7.96 (dd, 1H, *J* = 2 and 9 Hz), 8.31 (m, 1H), 8.49 (d, 1H, *J*= 2 Hz), 9.55 (s, 1H); $^{13}$C NMR (100 MHz, $CDCl_3$) δ 12.1 (2C), 37.4, 47.1 (2C), 51.5, 97.9, 130.8, 138.5, 139.5, 139.6, 144.1, 144.3, 144.6, 162.9; IR (KBr) ν $cm^{-1}$: 1517, 1661, 3397; MS (m/z, %) 398 ($M^+$, 1), 86 (100), 58 (15).

**Stage F: *N*-(2-diethylaminoethyl)-6-iodoquinoxaline-2-carboxamide dihydrochloride (46)**

The compound **46** was prepared according to the procedure described for the preparation of the compound **3**, using *N*-(2-diethylaminoethyl)-6-iodoquinoxaline-2-carboxamide (**45**) as starting material. Yield: 76%; melting point: 201-203°C; $^1$H NMR (200 MHz, $d_6$-DMSO) δ 1.28 (t, 6H, *J*= 7 Hz), 3.28 (m, 6H), 3.78 (m, 2H), 7.97 (d, 1H, *J* = 9 Hz), 8.30 (d, 1H, *J* = 9 Hz), 8.68 (s, 1H), 9.47 (m, 2H), 10.43 (bs, 1H); IR (KBr) ν $cm^{-1}$: 1516, 1674, 2459, 3326. Anal. Calculated for $C_{15}H_{19}IN_4O·2HCl$: C, 38.24; H, 4.49; N, 11.89. Found: C, 38.25; H, 4.45; N, 11.81.

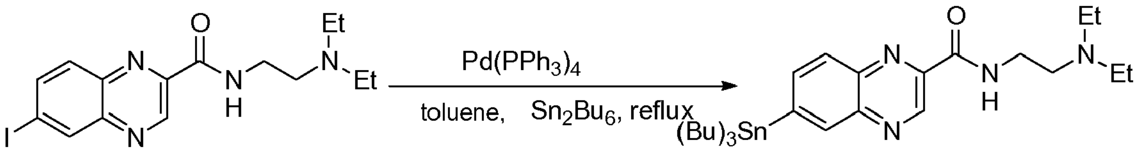


45 47

**Stage G: *N*-(2-diethylaminoethyl)-6-(tributylstannyl)quinoxaline-2-carboxamide (47)**

The compound **47** was prepared according to the procedure described for the preparation of the compound **32**, using *N*-(2-diethylaminoethyl)-6-iodoisoquinoxaline-2-carboxamide (**45**) as starting material. The residue obtained is chromatographed on a column of alumina eluted with an ethyl acetate/cyclohexane (7/3, v/v) mixture; yield: 47%; viscous liquid; Rf: 0.84 ($Al_2O_3$, ethyl acetate/cyclohexane (7/3, v/v)); $^1$H NMR (200 MHz, $CDCl_3$) δ 0.82 (t, 9H, *J*= 7 Hz), 1.07 (t, 6H, *J*= 7 Hz), 1.19 (m, 6H), 1.30 (m, 6H), 1.51 (m, 6H), 2.58 (q, 4H, *J* = 7 Hz), 2.68 (t, 2H, *J* = 6 Hz), 3.54 (q, 2H, *J* = 6 Hz) 7.85 (d, 1H, *J* = 8 Hz), 7.98 (d, 1H, *J* = 8 Hz), 8.24 (s, 1H), 8.41 (t, 1H, *J* = 6 Hz), 9.59 (s, 1H); $^{13}$C NMR (100 MHz, $CDCl_3$) δ 9.9 (3C, $^1J_{Sn\text{-}C}$= 348 Hz), 11.8 (2C), 13.6 (3C), 27.3 (3C, $^3J_{Sn\text{-}C}$= 56 Hz), 29.0 (3C, $^2J_{Sn\text{-}C}$= 21 Hz), 37.2, 47.3 (2C), 51.6, 128.2, 137.7, 137.9, 140.3, 142.9, 143.4, 143.6, 149.0, 163.6; IR ($CCl_4$) ν $cm^{-1}$: 1521, 1684, 2929, 2961; ESI-MS m/z 563.3 $[M+H]^+$.

<u>**EXAMPLE 12**</u>

Synthesis of *N*-(4-dipropylaminobutyl)-6-iodoquinoline-2-carboxamide dihydrochloride (**49**)

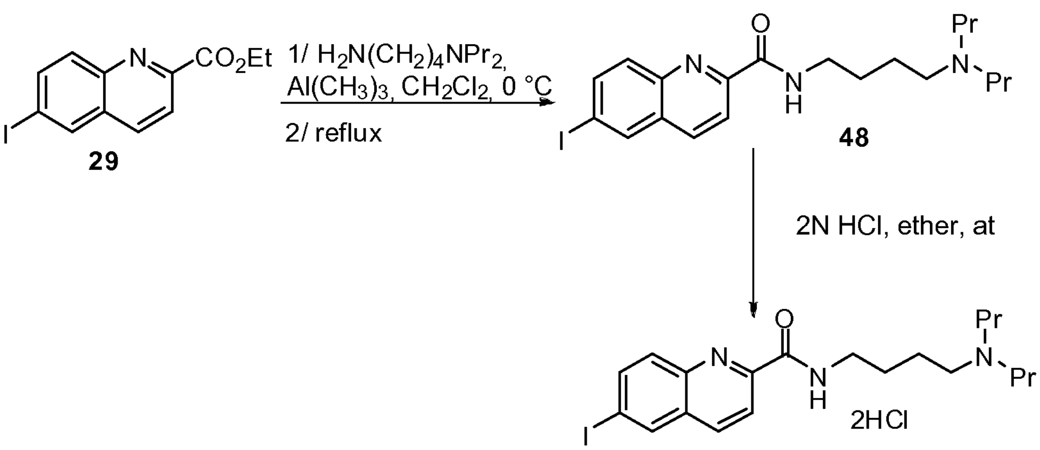


29 48 49

**Stage A : *N*-(4-dipropylaminobutyl)-6-iodoquinoline-2-carboxamide (48)**

The compound **48** was prepared according to the procedure described for the preparation of the compound **2**, using ethyl 6-iodoquinoline-2-carboxylate (**29**) and 4-amino-*N,N*-dipropylbutylamine (Seguin, H., Gardette, D., Moreau, M. F., Madelmont, J. C. and Gramain, J. C., Synth. Commun., 1998, 28, 4257-4272) as starting materials. The reaction medium is heated at reflux for 8 hours (360 mg, 0.82 mmol). Yield: 39%; viscous liquid; Rf: 0.68 ($Al_2O_3$, dichloromethane / ethanol (97/3, v/v)); $^1$H NMR ($CDCl_3$, 400 MHz) δ 0.80 (t, 6H, *J* = 7 Hz), 1.44 (st, 4H, *J* = 7 Hz), 1.60 (m, 4H), 2.39 (t, 4H, *J*= 7 Hz), 2.50 (t, 2H, *J*= 7 Hz), 3.47 (q, 2H, *J*= 6.5 Hz), 7.74 (d, 1H, *J*= 9 Hz), 7.90 (dd, 1H, *J* = 9.2 Hz), 8.10 (d, 1H, *J* = 8 Hz), 8.19 (d, 1H, *J* = 2 Hz), 8.22 (d, 1H, *J* = 8 Hz); $^{13}$C NMR ($CDCl_3$, 100 MHz) δ 11.9 (2C), 19.5 (2C), 24.1, 27.6, 39.4, 53.5, 55.8 (2C), 93.9, 119.6, 130.7, 131.2, 136.2, 136.5, 138.8, 145.4, 150.3, 164.1; IR (KBr) ν $cm^{-1}$: 1532, 1668, 2956; MS (m/z, %) 453 ($M^+$, 5), 424 (33), 353 (20), 254 (34), 127 (23), 114 (100).

**Stage B : N-(4-dipropylaminobutyl)-6-iodoquinoline-2-carboxamide dihydrochloride (49)**

The compound **49** was prepared according to the procedure described for the preparation of the compound **3**, using *N*-(4-dipropylaminobutyl)-6-iodoquinoline-2-carboxamide (**48**) as starting material. Yield: 97%; melting point: 133-135 ˚C; $^1$H NMR (400 MHz, $d_6$-DMSO) δ 0.87 (t, 6H, *J*= 7 Hz), 1.66 (m, 8H), 2.94 (m, 4H), 3.05 (m, 2H), 3.38 (m, 2H), 7.89 (d, 1H, *J* = 9 Hz), 8.12 (d, 1H, *J* = 9 Hz), 8.16 (d, 1H, *J* = 8.5 Hz), 8.51 (d, 1H, *J* = 8.5 Hz), 8.58 (s, 1H), 9.05 (m, 1H), 10.55 (bs, 1H); IR (KBr) ν $cm^{-1}$: 1534, 1663, 2962, 3250-3600. Anal. Calculated for $C_{20}H_{28}IN_3O{\cdot}2HCl$: C, 45.64; H, 5.75; N, 7.98. Found: C, 46.51; H, 5.99; N, 8.16.

## EXAMPLE 13

Synthesis of *N*-(2-diethylaminoethyl)-9,10-dihydro-1-iodo-9-oxoacridine-4-carboxamide hydrochloride (**55**)

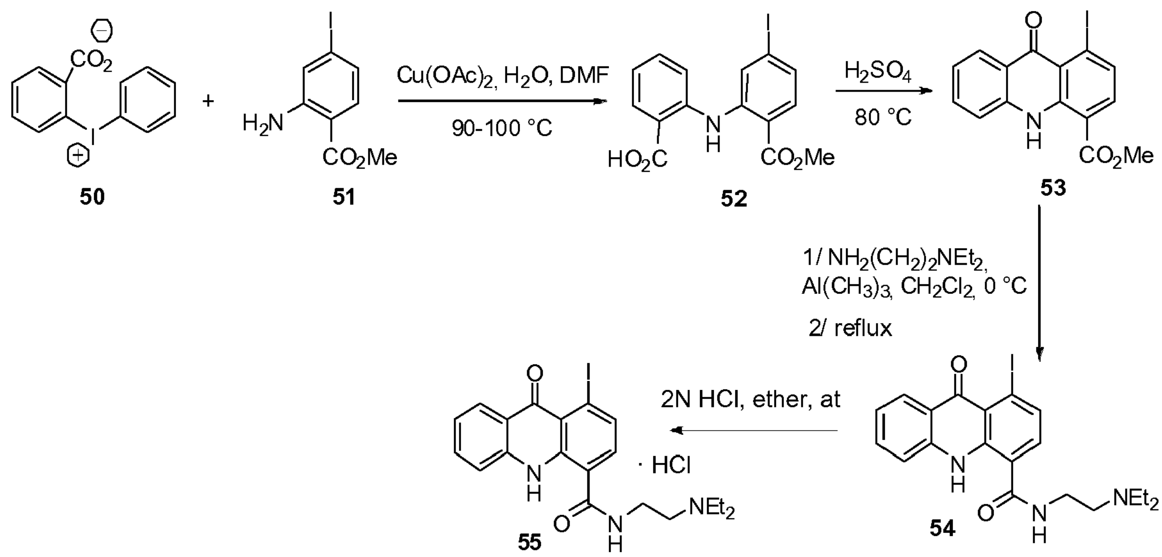


**Stage A: 2-(5-iodo-2-(methoxycarbonyl)phenylamino)benzoic acid (52)**

Diphenyliodonium-2-carboxylate (**50**) (Fieser, L. F.; Haddadin, M. J. Org. Synth. 1966, 46, 107-112) (0.88 g, 2.72 mmol) and then copper acetate monohydrate (12 mg) are added, at ambient temperature and with moderate stirring, to a solution of methyl 4-iodoanthranilate (**51**) (Allison, B. D., Hack, M. D., Phuong, V. K., Rabinowitz, M. H. and Rosen, M. D., U.S. Patent 2005/0038032, 2005) (0.50 g, 1.80 mmol) in dimethylformamide (30 ml). The green mixture obtained is heated at 90-100˚C, with stirring, for 12 hours. The solvent is subsequently evaporated under vacuum. Ethyl acetate (18 ml) and then a 0.1N hydrochloric acid solution (18 ml) are successively added to the crude product. The organic phase is then separated by settling and then extracted with a 0.1N aqueous ammonia solution (2 x 9 ml). The aqueous phase recovered is acidified to pH = 6 with a 0.1N hydrochloric acid solution. The mixture is then cooled in an ice bath and then filtered under vacuum. The precipitate obtained is washed with hot water (5 ml). Yield: 80%; melting point: 206-208˚C; $^1$H NMR (400 MHz, $d_6$-DMSO) δ 3.83 (s, 3H), 7.04 (t, 1H, *J* = 7.5 Hz), 7.30 (d, 1H, *J* = 8.5 Hz), 7.47 (m, 2H), 7.62 (d, 1H, *J* = 8.5 Hz), 7.74 (s, 1H), 7.92 (d, 1H, *J* = 7.5 Hz), 10.70 (s, 1H), 13.20 (m, 1H); $^{13}$C NMR (100 MHz, $d_6$-DMSO) δ 52.1, 101.9, 115.6, 118.2 (2C), 121.1, 125.3, 128.5, 131.8, 132.8, 133.5, 142.4, 144.4, 166.5, 168.3; IR (KBr) ν $cm^{-1}$: 1220, 1254, 1575, 1685, 3000-3600; MS (m/z, %) 397 ($M^+$, 100), 321 (88), 194 (59), 166 (95), 139 (62), 63 (77), 55 (61).

**Stage B: Methyl 9,10-dihydro-1-iodo-9-oxoacridine-4-carboxylate (53)**

A solution of 2-(5-iodo-2-(methoxycarbonyl)phenylamino)benzoic acid (**52**) (1.00 g, 2.52 mmol) in concentrated sulphuric acid (2 ml) is heated, under argon, in an oil bath at 80˚C for 30 minutes. After returing to ambient temperature, the green solution is cooled with an ice bath and diluted with water (15 ml). The green precipitate obtained is then filtered

off under vacuum, washed with water (4 ml), taken up in anhydrous ethanol and then dried by evaporation under vacuum. The product is purified by chromatography on silica eluted with a dichloromethane / ethanol (99.5/0.5, v/v) mixture, to result in the yellow precipitate **53** (92 mg, 0.24 mmol). Yield: 85%; Rf: 0.65 ($SiO_2$, dichloromethane/ethanol (99.5/0.5, v/v)); melting point: 189-191°C; $^1$H NMR (200 MHz, $CDCl_3$) δ 4.01 (s, 3H), 7.29 (m, 2H), 7.67 (t, 1H, *J* = 8.5 Hz), 7.91 (m, 2H), 8.42 (d, 1H, *J*= 8.5 Hz), 12.07 (bs, 1H); $^{13}$C NMR (100 MHz, $CDCl_3$) δ 52.8, 102.4, 113.4, 117.3, 119.8, 121.4, 122.9, 127.5, 134.1, 135.1, 135.3, 138.7, 142.4, 168.6, 176.2; IR (KBr) ν $cm^{-1}$: 1273, 1508, 1613, 1640, 1685, 3217; MS (m/z, %) 379 ($M^+$, 71), 347 (81), 192 (24), 164 (100), 127 (24), 88 (24), 76 (24), 63 (24).

**Stage C: *N*-(2-diethylaminoethyl)-9,10-dihydro-1-iodo-9-oxoacridine-4-carboxamide (54)**

The compound **54** was prepared according to the procedure described for the preparation of the compound **2**, using methyl 9,10-dihydro-1-iodo-9-oxoacridine-4-carboxylate (**53**) as starting material and heating the reaction medium at reflux for 5 hours. Yield: 64%; Rf: 0.40 ($Al_2O_3$, dichloromethane / ethanol (99/1, v/v)); melting point: 248-250°C; $^1$H NMR (400 MHz, $CDCl_3$) δ 1.04 (t, 6H, *J* = 6.5 Hz), 2.60 (q, 4H, *J* = 6.5 Hz), 2.70 (m, 2H), 3.48 (m, 2H), 7.19 (t, 1H, *J* = 7 Hz), 7.25 (d, 1H, *J* = 8.5 Hz), 7.36 (d, 1H, *J* = 8 Hz), 7.57 (m, 2H), 7.81 (d, 1H, *J* = 8 Hz), 8.34 (d, 1H, *J* = 8 Hz), 12.75 (s, 1H); $^{13}$C NMR (100 MHz, $CDCl_3$) δ 11.7 (2C), 37.1, 46.9 (2C), 51.2, 99.0, 117.5 (2C), 119.9, 121.3, 122.5, 127.4, 130.9, 134.0, 134.8, 139.1, 142.1, 168.4, 176.5; IR (KBr) ν $cm^{-1}$: 1513, 1615, 3281; MS (m/z, %) 463 ($M^+$, 1), 86 (100), 58 (16).

**Stage D: *N*-(2-diethylaminoethyl)-9,10-dihydro-1-iodo-9-oxoacridine-4-carboxamide hydrochloride (55)**

The compound **55** was prepared according to the procedure described for the preparation of the compound **3**, using *N*-(2-diethylaminoethyl)-9,10-dihydro-1-iodo-9-oxoacridine-4-carboxamide (**54**) as starting material. Yield: 81%; melting point: 269-271°C; $^1$H NMR (400 MHz, $d_6$-DMSO) δ 1.26 (t, 6H, *J*= 7 Hz), 3.22 (m, 4H), 3.33 (m, 2H, *J* = 7 Hz), 3.75 (m, 2H), 7.33 (t, 1H, *J* = 7 Hz), 7.62 (d, 1H, *J* = 8 Hz), 7.76 (d, 1H, *J* = 7 Hz), 7.96 (m, 2H), 8.20 (d, 1H, *J*= 8 Hz), 9.49 (bs, 1H), 10.39 (bs, 1H), 12.70 (bs, 1H); $^{13}$C NMR (100 MHz, $d_6$-DMSO-) δ 8.3 (2C), 34.3, 46.5 (2C), 49.6, 99.0, 117.7, 118.0, 118.8, 120.4, 122.3, 126.3, 133.0, 134.1, 134.3, 138.6, 141.3, 168.2, 175.2; IR (KBr) ν $cm^{-1}$: 1513, 1577, 1613, 3057, 3100-3500. Anal. Calculated for $C_{20}H_{22}IN_3O_2\cdot HCl\cdot 3H_2O$ : C, 43.38; H, 5.28; N, 7.59. Found : C, 43.74; H, 4.99; N, 7.68.

<u>**EXAMPLE 14**</u>

Synthesis of *N*-(2-diethylaminoethyl)-9,10-dihydro-2-iodo-9-oxoacridine-4-carboxamide hydrochloride (**60**)

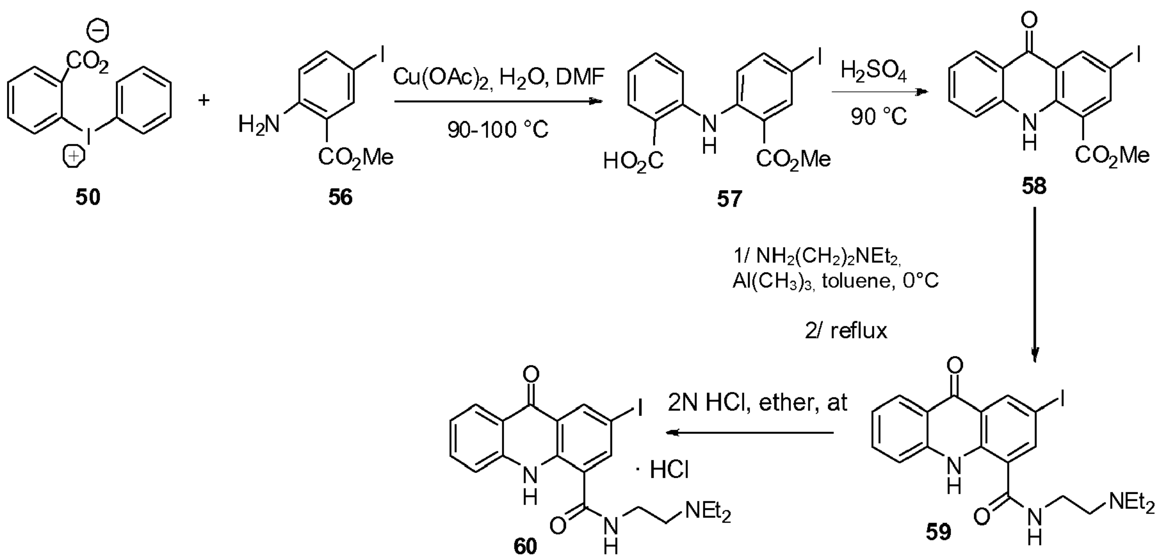


50 56 57 58 59 60

**Stage A: 2-(4-iodo-2-(methoxycarbonyl)phenylamino)benzoic acid (57)**

The compound **57** was prepared according to the procedure described for the preparation of the compound **52**, using methyl 5-iodoanthranilate (**56**) (Sy, W. W., Synth. Commun., 1992, 22, 3215-3219) as starting material. Yield: 59%; Rf: 0.86 ($Al_2O_3$, dichloromethane / ethanol (9/1, v/v)); melting point: 230-232°C; $^1$H NMR (400 MHz, *d$_6$*-DMSO) δ 3.86 (s, 3H), 7.00 (m, 1H), 7.33 (d, 1H, *J*= 9 Hz), 7.46 (m, 2H), 7.74 (d, 1H, *J*= 9 Hz), 7.93 (d, 1H, *J* = 8 Hz), 8.14 (s, 1H), 10.78 (s, 1H), 13.21 (m, 1H); $^{13}$C NMR (100 MHz, *d$_6$*-DMSO) δ 52.2, 81.5, 117.8, 118.0, 118.7, 119.9, 120.7, 131.7, 133.4, 139.2, 141.8, 142.7, 143.1, 165.5, 168.4; IR (KBr) ν cm$^{-1}$: 1209, 1269, 1508, 1577, 1683, 1719, 2800-3200; MS (m/z, %) 397 ($M^+$, 100), 320 (67), 194 (54), 166 (77), 139 (59), 63 (72).

**Stage B: methyl 9,10-dihydro-2-iodo-9-oxoacridine-4-carboxylate (58)**

The compound **58** was prepared according to the procedure described for the preparation of the compound **53**, using 2-(4-iodo-2-(methoxycarbonyl)phenylamino)-benzoic acid (**52**) as starting material. The residue obtained is chromatographed on a column of silica eluted with a dichloromethane/ethanol (99.5/0.5, v/v) mixture, to result, in order of elution, in:

**Methyl 9,10-dihydro-2-iodo-9-oxoacridine-4-carboxylate (58)**

Yield: 85%; Rf 0.41 ($SiO_2$, dichloromethane / ethanol (99.5/0.5, v/v)); melting point: 275-277°C; $^1$H NMR (200 MHz, $CDCl_3$) δ 4.03 (s, 3H), 7.32 (t, 1H, *J* = 8.5 Hz), 7.38 (d, 1H, *J*= 8.5 Hz), 7.70 (t, 1H, *J*= 8.5 Hz), 8.42 (d, 1H, *J*= 8.5 Hz), 8.65 (d, 1H, *J*= 2 Hz), 8.99 (d, 1H, *J* = 2 Hz), 11.65 (bs, 1H); $^{13}$C NMR (100 MHz, $CDCl_3$) δ 52.9, 81.9, 115.6, 117.8, 121.7, 122.8, 124.2, 127.3, 134.4, 139.9, 140.9, 142.4, 144.5, 167.3, 176.5; IR (KBr) ν cm$^{-1}$: 1282, 1430, 1508, 1694, 3262; MS (m/z, %) 379 ($M^+$, 100), 347 (52), 164 (30).

**Methyl 9,10-dihydro-9-oxoacridine-4-carboxylate** (Rewcasttle, G. W. and Denny, W. A., Synthesis, 1985, 220-222).

Yield: 8%; Rf: 0.21 ($SiO_2$, dichloromethane / ethanol (99.5/0.5, v/v)); melting point: 170-172°C (lit.: 172°C).

**Stage C: *N*-(2-diethylaminoethyl)-9,10-dihydro-2-iodo-9-oxoacridine-4-carboxamide (59)**

The compound **59** was prepared according to the procedure described for the preparation of the compound **2**, using methyl 9,10-dihydro-2-iodo-9-oxoacridine-4-carboxylate (**58**) as starting material and heating the reaction medium at reflux for 1.5 hours in anhydrous toluene. Yield: 88%; Rf: 0.43 ($Al_2O_3$, dichloromethane / ethanol (99/1, v/v)); melting point: 250-252°C; $^1$H NMR (200 MHz, $CDCl_3$) δ 1.08 (t, 6H, *J* = 7 Hz), 2.67 (q, 4H, *J*= 7 Hz), 2.75 (t, 2H, *J*= 6 Hz), 3.56 (m, 2H), 7.21 (t, 1H, *J*= 8 Hz), 7.30 (d, 1H, *J* = 8 Hz), 7.61 (t, 1H, *J* = 8 Hz), 7.65 (bs, 1H), 8.08 (d, 1H, *J* = 2 Hz), 8.32 (d, 1H, *J*= 8 Hz), 8.75 (d, 1H, *J*= 2 Hz), 12.15 (bs, 1H); $^{13}$C NMR (100 MHz, $CDCl_3$) δ 11.8 (2C), 37.4, 46.9 (2C), 51.2, 82.0, 117.9, 120.0, 121.2, 122.3, 124.0, 126.8, 134.0, 139.7, 139.8, 140.0 (2C), 166.9, 176.5; IR (KBr) ν cm$^{-1}$: 1512, 1617, 3422; MS (m/z, %) 463 ($M^+$, 6), 348 (18), 320 (14), 193 (11), 164 (11), 86 (100), 58 (23).

**Stage D: *N*-(2-diethylaminoethyl)-9,10-dihydro-2-iodo-9-oxoacridine-4-carboxamide hydrochloride (60)**

The compound *60* was prepared according to the procedure described for the preparation of the compound *3* using *N*-(2-diethylaminoethyl)-9,10-dihydro-2-iodo-9-oxoacridine-4-carboxamide (**59**) as starting material. Yield: 71%; melting point: 298-300°C; $^1$H NMR (400 MHz, *d$_6$*-DMSO) δ 1.27 (m, 6H), 3.23 (m, 4H), 3.33 (m, 2H), 3.75 (m, 2H), 7.33 (t, 1H, *J* = 7 Hz), 7.75 (m, 2H), 8.23 (d, 1H, *J* = 8 Hz), 8.60 (s, 1H), 8.68 (s, 1H), 9.42 (bs, 1H), 10.27 (bs, 1H), 12.19 (bs, 1H); $^{13}$C NMR (100 MHz, *d$_6$*-DMSO) δ 8.3 (2C), 34.2, 46.4 (2C), 49.5, 83.2, 118.6, 120.5 (2C), 122.3, 123.3, 125.9, 134.3, 138.2, 139.3, 139.8, 140.7, 166.8, 175.3; IR (KBr) ν cm$^{-1}$: 1300, 1517, 1560, 1617, 3200-3400. Anal. Calculated for $C_{20}H_{22}IN_3O_2 \cdot HCl \cdot 1.75H_2O$ : C, 45.21; H, 5.03; N, 7.91. Found : C, 44.91; H, 4.66; N, 8.07.

**EXAMPLE 15**

Synthesis of *N*-(2-diethylaminoethyl)-9,10-dihydro-3-iodo-9-oxoacridine-4-carboxamide hydrochloride (**65**)

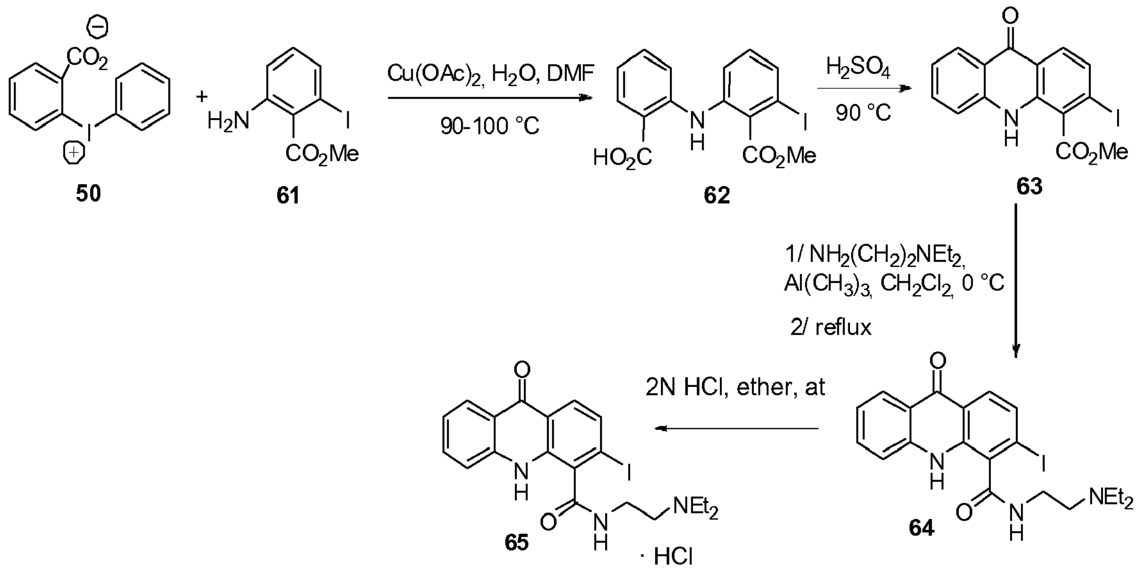

50 61 62 63 64 65

**Stage A: 2-(3-iodo-2-methoxycarbonylphenylamino)benzoic acid (62)**

The compound **62** was prepared according to the procedure described for the preparation of the compound **52,** using methyl 6-iodoanthranilate (**61**) (Seltzman, H. H. and Berrang, B. D., Tetrahedron Lett., 1993, 34, 3083-3086) as starting material. Yield: 67%; melting point: 191-193˚C; $^1$H NMR (200 MHz, $d_6$-DMSO) δ 3.85 (s, 3H), 6.85 (t, 1H, *J*= 8 Hz), 7.18 (m, 2H), 7.40 (t, 1H, *J*= 8 Hz), 7.50 (d, 1H, *J*= 8 Hz), 7.59 (d, 1H, *J*= 8 Hz), 7.90 (d, 1H, *J*= 8 Hz), 9.93 (bs, 1H); $^{13}$C NMR (100 MHz, $d_6$-DMSO) δ 52.6, 93.6, 113.6, 114.4, 118.7, 121.1, 131.8, 131.9, 133.0, 133.4, 134.2, 139.0, 145.8, 167.6, 169.9; IR (KBr) ν cm$^{-1}$: 1247, 1269, 1444, 1575, 1657, 1740, 2700-3200, 3318; MS (m/z, %) 397 ($M^+$, 73), 321 (100), 194 (36), 166 (41), 139 (18).

**Stage B: methyl 9,10-dihydro-3-iodo-9-oxoacridine-4-carboxylate (63)**

The compound **63** was prepared according to the procedure described for the preparation of the compound **53,** using 2-(3-iodo-2-(methoxycarbonyl)phenylamino)benzoic acid (**62**) as starting material. Yield: 65%; Rf: 0.41 ($SiO_2$, dichloromethane / ethanol (99.5/0.5, v/v)); melting point: 202-204˚C; $^1$H NMR (200 MHz, $CDCl_3$) δ 4.07 (s, 3H), 7.31 (m, 2H), 7.70 (t, 1H, *J* = 7 Hz), 7.88 (d, 1H, *J* = 8.5 Hz), 8.23 (d, 1H, *J* = 8.5 Hz), 8.41 (d, 1H, *J* = 8 Hz), 10.56 (bs, 1H); $^{13}$C NMR (100 MHz, $d_6$-DMSO) δ 53.3, 102.0, 118.1, 120.4, 120.5, 122.2, 125.8, 127.8, 128.9, 131.1, 134.1, 137.4, 140.7, 167.0, 176.1; IR (KBr) ν cm$^{-1}$: 1281, 1431, 1583, 1617, 3000-3600; MS (m/z, %) 379 ($M^+$, 60), 347 (100), 164 (54), 139 (25), 63 (29).

**Stage C: *N*-(2-diethylaminoethyl)-9,10-dihydro-3-iodo-9-oxoacridine-4-carboxamide (64)**

The compound **64** was prepared according to the procedure described for the preparation of the compound **2**, using methyl 9,10-dihydro-3-iodo-9-oxoacridine-4-carboxylate (**63**) as starting material and heating the reaction medium at reflux for 6 hours. Yield: 38%; Rf: 0.40 ($Al_2O_3$, dichloromethane / ethanol (99/1, v/v)); melting point: 215-217˚C; $^1$H NMR (200 MHz, $CDCl_3$) δ 1.49 (t, 6H, *J*= 7 Hz), 3.37 (m, 4H), 3.52 (m, 2H), 4.08 (m, 2H), 7.00 (d, 1H, *J* = 8 Hz), 7.19 (t, 1H, *J* = 8 Hz), 7.59 (m, 3H), 8.21 (d, 1H, *J* = 8 Hz), 8.77 (bs, 1H), 10.09 (s, 1H); $^{13}$C NMR (100 MHz, $d_6$-DMSO) δ 10.8 (2C), 39.5, 46,8 (2C), 50.3, 102.3, 118.2, 120.3, 120.4, 121.9, 125.7, 127.6, 130.9, 132.0, 133.9, 137.2, 140.9, 166.5, 176.3; IR (KBr) ν cm$^{-1}$: 1556, 1614, 2960-3230; MS (m/z, %) 463 ($M^+$, 1), 86 (100), 58 (12).

**Stage D: *N*-(2-diethylaminoethyl)-9,10-dihydro-3-iodo-9-oxoacridine-4-carboxamide hydrochloride (65)**

The compound **65** was prepared according to the procedure described for the preparation of the compound **3,** using *N*-(2-diethylaminoethyl)-9,10-dihydro-3-iodo-9-oxoacridine-4-carboxamide (**64**) as starting material. Yield: 87%; melting point: 262-264˚C; $^1$H NMR (400 MHz, $d_6$-DMSO) δ 1.27 (t, 6H, *J* = 7 Hz), 3.23 (m, 4H), 3.32 (m, 2H), 3.76 (m, 2H), 7.32 (t, 1H, *J* = 8 Hz), 7.75 (m, 2H), 7.95 (d, 1H, *J* = 8.5 Hz), 7.99 (d, 1H, *J* = 8.5 Hz), 8.21 (d, 1H, *J* = 8 Hz), 9.05 (m, 1H), 10.17 (bs, 1H), 10.79 (bs, 1H); $^{13}$C NMR (100 MHz, $d_6$-DMSO) δ 8.4 (2C), 34.5, 46.6 (2C), 49.3, 102.6, 118.3, 120.4 (2C), 121.9, 125.7, 127.8, 130.9, 131.6, 133.8, 137.2, 141.0, 166.9, 176.3; IR (KBr) ν cm$^{-1}$: 1427, 1590, 1618,

3200-3400. Anal. Calculated for $C_{20}H_{22}IN_3O_2 \cdot HCl \cdot H_2O$ : C, 46.39; H, 4.87; N, 8.12. Found : C, 46.59; H, 4.89; N, 8.06.

**EXAMPLE 16**

Synthesis of *N*-(2-diethylaminoethyl)-9,10-dihydro-5-iodo-9-oxoacridine-4-carboxamide hydrochloride (**69**)

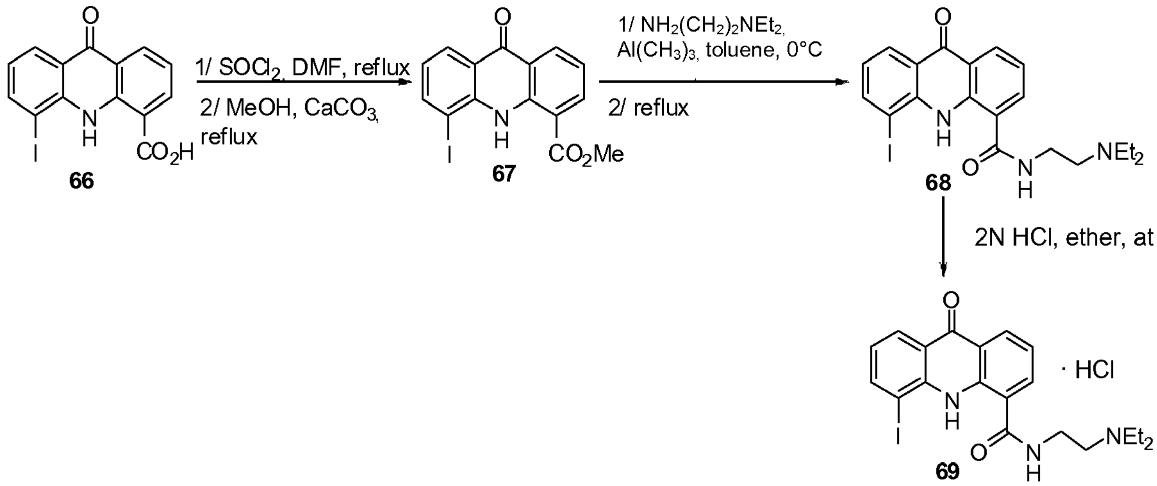


66 67 68 69

**Stage A: methyl 9,10-dihydro-5-iodo-9-oxoacridine-4-carboxylate (67)**

Dimethylformamide (3 drops) is added to a solution of 9,10-dihydro-5-iodo-9-oxoacridine-4-carboxylic acid (**66**) (Rewcastle, G. W. and Denny, W. A., Synthesis, 1985, 2, 217-220) (1.00 g, 2.74 mmol) in thionyl chloride (30 ml). The reaction medium is heated at reflux for 15 minutes and then the thionyl chloride is driven off by evaporation under vacuum. The acid chloride is taken up in anhydrous toluene (20 ml) in order to be once again evaporated to dryness. The precipitate is dissolved in anhydrous methanol (30 ml) and then calcium carbonate is added (1.20 g). The mixture is subsequently brought to reflux for 3 hours. After returning to ambient temperature, the calcium carbonate is filtered off. The precipitate is taken up in a 1N hydrochloric acid solution (25 ml) and filtered. The aqueous phase is extracted with dichloromethane (3 x 50 ml) and then all the organic phases are combined, dried over magnesium sulphate, filtered and evaporated to dryness. The residue obtained is chromatographed on a column of alumina eluted with dichloromethane, to result in the ester **67** (0.59 g, 1.56 mmol). Yield: 57%; Rf: 0.78 ($Al_2O_3$, dichloromethane); melting point: 174-176°C; $^1H$ NMR (400 MHz, $CDCl_3$) δ 4.07 (s, 3H), 7.06 (t, 1H, *J* = 7.5 Hz), 7.30 (t, 1H, *J* = 8 Hz), 8.17 (d, 1H, *J* = 7.5 Hz), 8.46 (m, 2H), 8.69 (d, 1H, *J* = 8 Hz), 12.11 (s, 1H); $^{13}C$ NMR (100 MHz, $CDCl_3$) δ 52.9, 86.2, 114.3, 120.8, 121.9, 122.5, 123.6, 127.7, 133.8, 137.0, 140.7, 141.5, 144.0, 168.0, 177.6; IR (KBr) ν $cm^{-1}$: 1141, 1279, 1430, 1518, 1608, 3197; MS (m/z, %) 379 ($M^+$, 80), 347 (100), 164 (99), 75 (71), 63 (40).

**Stage B: *N*-(2-diethylaminoethyl)-9,10-dihydro-5-iodo-9-oxoacridine-4-carboxamide (68)**

The compound **68** was prepared according to the procedure described for the preparation of the compound **2**, using methyl 9,10-dihydro-5-iodo-9-oxoacridine-4-carboxylate (**67**) as starting material and heating the reaction medium at reflux for 2 hours in anhydrous toluene. Yield: 73%; Rf: 0.23 ($Al_2O_3$, dichloromethane / ethanol (99/1, v/v)); melting point: 148-150°C; $^1H$ NMR (400 MHz, $CDCl_3$) δ 1.05 (t, 6H, *J*= 7 Hz), 2.61 (q, 4H, *J* = 7 Hz), 2.72 (m, 2H), 3.56 (m, 2H), 6.95 (t, 1H, *J* = 8 Hz), 7.21 (t, 1H, *J* = 8 Hz), 7.58 (bs, 1H), 7.92 (d, 1H, *J* = 8 Hz), 8.08 (d, 1H, *J* = 8 Hz), 8.36 (d, 1H, *J* = 8 Hz), 8.53 (d, 1H, *J* = 8 Hz), 12.80 (s, 1H); $^{13}C$ NMR (100 MHz, $CDCl_3$) δ 11.8 (2C), 37.3, 46.9 (2C), 51.2, 86.3, 118.1, 120.6, 122.2, 122.3, 123.2, 127.6, 131.8, 132.0, 141.1, 141.2, 143.9, 167.9, 177.8; IR (KBr) ν $cm^{-1}$: 1521, 1610, 3200-3500; MS (m/z, %) 463 ($M^+$, 1), 86 (100), 58 (7).

**Stage C: *N*-(2-diethylaminoethyl)-9,10-dihydro-5-iodo-9-oxoacridine-4-carboxamide hydrochloride (69)**

The compound **69** was prepared according to the procedure described for the preparation of the compound

**3**, using *N*-(2-diethylaminoethyl)-9,10-dihydro-5-iodo-9-oxoacridine-4-carboxamide (**68**) as starting material. Yield: 82%; melting point: 251-253°C; $^1$H NMR (400 MHz, $d_6$-DMSO) δ 1.27 (t, 6H, *J* = 7 Hz), 3.24 (m, 4H), 3.36 (m, 2H), 3.79 (m, 2H), 7.12 (t, 1H, *J* = 8 Hz), 7.43 (t, 1H, *J* = 7.5 Hz), 8.26 (d, 1H, *J* = 8 Hz), 8.32 (d, 1H, *J*= 7 Hz), 8.46 (d, 1H, *J*= 8 Hz), 8.55 (d, 1H, *J*= 7 Hz), 9.55 (bs, 1H), 10.34 (bs, 1H), 12.97 (bs, 1H); $^{13}$C NMR (100 MHz, $d_6$-DMSO) δ 8.3 (2C), 34.2, 46.5 (2C), 49.8, 87.5, 117.4, 120.7, 121.1, 121.3, 123.5, 126.5, 130.8, 133.8, 140.5 (2C), 144.0, 168.1, 176.3; IR (KBr) ν cm$^{-1}$: 1301, 1428, 1522, 1611, 2650, 2946, 3200-3400. Anal. Calculated for $C_{20}H_{22}IN_3O_2 \cdot HCl \cdot H_2O$ : C, 46.39; H, 4.87; N, 8.12. Found : C, 46.03; H, 4.80; N, 8.06.

**EXAMPLE 17**

Synthesis of *N*-(2-diethylaminoethyl)-9,10-dihydro-6-iodo-9-oxoacridine-4-carboxamide hydrochloride (**78**)

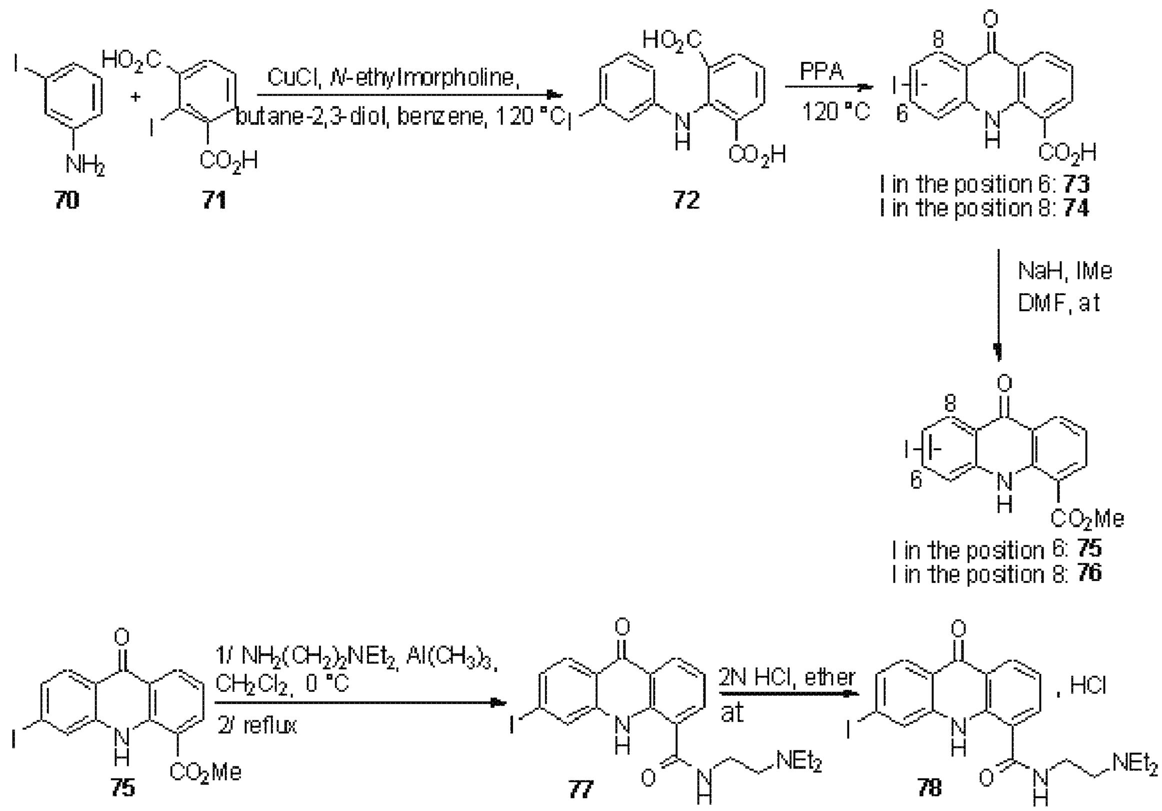


**70** **71** **72** **75** **77** **78**

**Stage A: 2-(3-iodophenylamino)isophthalic acid (72)**

3-iodoaniline (**70**) (6.74 g, 30.8 mmol) and copper chloride (2.03 g, 20.5 mmol) are added, under argon, to a solution of 2-iodoisophthalic acid (**71**) (Rewcastle G. W. and Denny, W. A., Synthesis, 1985, 217-220) (6.00 g, 20.6 mmol) in a mixture of anhydrous butane-2,3-diol (25 ml) and anhydrous benzene (45 ml). The reaction mixture is immersed in an oil bath heated beforehand to 120°C. When the internal temperature reaches approximately 100°C, the anhydrous N-ethylmorpholine (5.88 ml, 45.9 mmol) is added and then the reaction mixture is stirred at 120°C (external) for 4 hours. After returning to ambient temperature, the solution is treated with a 0.5N aqueous ammonia solution (100 ml) and then with active charcoal (6 g). The mixture is subsequently filtered through Celite® 521 and the filtered residue is washed several times with water (2 x 50 ml). The filtrates are acidified with 2N hydrochloric acid (80 ml) and then the aqueous phase is extracted with ethyl acetate (3 x 100 ml). The organic phase is filtered through Celite® 521 in order to remove an inorganic precipitate and is then extracted with a 0.5N aqueous ammonia solution (2 x 100 ml). The aqueous phase is acidified with concentrated hydrochloric acid and then concentrated to 2/3 under vacuum. The precipitate formed is filtered off and then washed with hot water. The precipitate is subsequently taken up in anhydrous

ethanol (50 ml) to be dried under vacuum, to result in the acid **72** (2.12 g, 5.34 mmol). Yield: 26%; melting point: 214-216˚C; $^1$H NMR (400 MHz, $d_6$-DMSO) δ 6.89 (d, 1H, *J* = 8 Hz), 6.98 (t, 1H, *J* = 8 Hz), 7.08 (t, 1H, *J* = 8 Hz), 7.21 (d, 1H, *J* = 8 Hz), 7.25 (s, 1H), 7.95 (d, 2H, *J* = 8 Hz), 9.54 (bs, 1H), 13.10 (m, 2H); $^{13}$C NMR (100 MHz, $d_6$-DMSO) δ 95.1, 116.4, 120.2, 122.0 (2C), 125.2, 129.6, 130.9, 135.0 (2C), 142.8, 145.5, 168.4 (2C); IR (KBr) ν $cm^{-1}$: 1249, 1432, 1584, 1665, 1693, 2800-3200; MS (m/z, %) 383 ($M^+$, 100), 321 (38), 194 (48), 166 (53), 139 (21).

**Stage B: 9,10-dihydro-6-iodo-9-oxoacridine-4-carboxylic acid (73) and 9,10-dihydro-8-iodo-9-oxoacridine-4-carboxylic acid (74)**

2-(3-iodophenylamino)isophthalic acid (**72**) (0.89 g, 23.2 mmol) is dissolved in polyphosphoric acid (PPA) (26 g) at 120˚C (internal). The mixture is stirred at 120˚C (internal) for 2 hours and then poured into boiling water (86 ml). The precipitate formed is filtered off and then dissolved in a 1/1 (v/v) mixture of methanol and a 1N aqueous sodium hydroxide solution (140 ml). The solution is heated to 60˚C (internal) and then filtered while hot. The filtrate is acidified (pH = 5) with glacial acetic acid (10 ml), concentrated to 2/3 under vacuum and cooled on an ice bath until precipitation occurs. The yellow precipitate formed is filtered off and then taken up in ethanol (20 ml) to be dried under vacuum, to result in a mixture (1/1, dislayed by $^1$H NMR) of the acids **73** and **74** (0.71 g, 1.95 mmol).

**Stage C: methyl 9,10-dihydro-6-iodo-9-oxoacridine-4-carboxylate (75) and methyl 9,10-dihydro-8-iodo-9-oxoacridine-4-carboxylate (76)**

60% sodium hydride in mineral oil (10.4 mg, 0.26 mmol) is added to a mixture of 9,10-dihydro-6-iodo-9-oxoacridine-4-carboxylic acid (**73**) and 9,10-dihydro-8-iodo-9-oxoacridine-4-carboxylic acid (**74**) (95.0 mg, 0.26 mmol) in anhydrous dimethylformamide (5 ml), stirred beforehand for 10 minutes, at ambient temperature under argon. The mixture is then stirred at ambient temperature for 30 minutes. Methyl iodide (16.2 μl, 0.26 mmol) is subsequently added to the solution, which is stirred at ambient temperature for 18 hours. The red solution obtained is then evaporated under vacuum. The mixture is subsequently diluted with water (10 ml), basified with a saturated aqueous sodium carbonate solution (10 ml) and extracted with ethyl acetate (3 x 20 ml). The organic phases obtained are combined, dried over magnesium sulphate, filtered and evaporated under vacuum. The residue is subsequently separated by chromatography on a column of silica eluted with dichloromethane, to result, in order of elution, in the following products:

**methyl 9,10-dihydro-8-iodo-9-oxoacridine-4-carboxylate (76)**

(50 mg, 0.13 mmol); yield: 51%; Rf 0.58 ($SiO_2$, dichloromethane); melting point: 239-241˚C; $^1$H NMR (400 MHz, $CDCl_3$) δ 4.01 (s, 3H), 7.23 (m, 2H), 7.34 (d, 1H, *J*= 8 Hz), 7.92 (d, 1H, *J* = 7.5 Hz), 8.37 (d, 1H, *J* = 7 Hz), 8.67 (d, 1H, *J* = 7.5 Hz), 11.64 (bs, 1H); $^{13}$C NMR (100 MHz, $CDCl_3$) δ 52.6, 92.5, 113.3, 118.6, 119.0, 120.5, 122.3, 133.8, 134.4, 136.6, 137.2, 140.7, 141.3, 168.4, 176.1; IR (KBr) ν $cm^{-1}$: 1261, 1592, 1611, 1692, 2926, 2961; MS (m/z, %) 379 ($M^+$, 79), 347 (82), 220 (28), 192 (34), 164 (100), 75 (43), 63 (33).

**Methyl 9,10-dihydro-6-iodo-9-oxoacridine-4-carboxylate (75)**

(32 mg, 0.08 mmol); yield: 32%; Rf 0.38 ($SiO_2$, dichloromethane); melting point: 258-260˚C; $^1$H NMR (200 MHz, $CDCl_3$) δ 4.03 (s, 3H), 7.30 (t, 1H, *J* = 8 Hz), 7.61 (d, 1H, *J* = 8.5 Hz), 7.85 (s, 1H), 8.13 (d, 1H, *J* = 8.5 Hz), 8.46 (d, 1H, *J* = 7.5 Hz), 8.71 (d, 1H, *J* = 7.5 Hz), 11.77 (bs, 1H); $^{13}$C NMR (50 MHz, $CDCl_3$) δ 52.7, 101.5, 120.5, 120.8, 121.3, 122.7, 126.4, 128.5, 131.6, 134.0, 136.9, 140.7, 141.7, 168.5, 174.6; IR (KBr) ν $cm^{-1}$: 1280, 1589, 1609, 1640, 1685, 3271; MS (m/z, %) 379 ($M^+$, 95), 347 (100), 220 (32), 192 (29), 164 (99), 137 (26), 75 (58), 63 (42).

**Stage D: *N*-(2-diethylaminoethyl)-9,10-dihydro-6-iodo-9-oxoacridine-4-carboxamide (77)**

The compound **77** was prepared according to the procedure described for the preparation of the compound **2**, using methyl 9,10-dihydro-6-iodo-9-oxoacridine-4-carboxylate (**75**) as starting material and heating the reaction medium at reflux for 2 hours. Yield: 65%; Rf: 0.25 ($Al_2O_3$, dichloromethane / ethanol (99/1, v/v)); melting point: 208-210˚C; $^1$H NMR (400 MHz, $CDCl_3$) δ 1.25 (t, 6H, *J*= 7 Hz), 2.95 (m, 4H), 3.04 (m, 2H), 3.72 (m, 2H), 7.22 (t, 1H, *J* = 7.5 Hz), 7.47 (d, 1H, *J* = 8.5 Hz), 7.70 (s, 1H), 8.00 (d, 1H, *J* = 8.5 Hz), 8.25 (d, 1H, *J* = 7 Hz), 8.51 (d, 1H, *J* = 8 Hz), 8.67 (bs, 1H), 12.40 (s, 1H); $^{13}$C NMR (100 MHz, $CDCl_3$) δ 9.9 (2C), 35.9, 47.9 (2C), 52.5, 101.3, 116.7, 120.5, 120.6, 122.7, 126.5, 128.3, 131.0, 132.0, 133.0, 140.7, 141.2, 168.7, 177.7; IR (KBr) ν $cm^{-1}$: 1301, 1449, 1522, 1560, 1580, 1610, 2966, 3343; MS (m/z, %) 463 ($M^+$, 1), 86 (100), 58 (12).

**Stage E: *N*-(2-diethylaminoethyl)-9,10-dihydro-6-iodo-9-oxoacridine-4-carboxamide hydrochloride (78).**

The compound **78** was prepared according to the procedure described for the preparation of the compound **3**, using *N*-(2-diethylaminoethyl)-9,10-dihydro-6-iodo-9-oxoacridine-4-carboxamide (**77**) as starting material. Yield: 46%; melting point: 266-268°C; $^1$H NMR (400 MHz, $d_6$-DMSO) δ 1.27 (t, 6H, *J* = 7 Hz), 3.23 (m, 4H), 3.34 (m, 2H), 3.76 (m, 2H), 7.37 (t, 1H, *J* = 7.5 Hz), 7.61 (t, 1H, *J* = 8.5 Hz), 7.93 (d, 1H, *J* = 8.5 Hz), 8.26 (s, 1H), 8.38 (d, 1H, *J* = 7.5 Hz), 8.42 (d, 1H, *J* = 8 Hz), 9.43 (m, 1H), 10.41 (bs, 1H), 12.21 (bs, 1H); $^{13}$C NMR (100 MHz, *$d_6$*-DMSO) δ 8.3 (2C), 34.2, 46.5 (2C), 49.7, 102.2, 118.6, 119.5, 120.4, 121.7, 126.8, 127.4, 130.2, 130.6, 133.6, 139.9, 140.6, 167.9, 176.2; IR (KBr) ν cm$^{-1}$: 1309, 1450, 1523, 1577, 1611, 3066, 3250-3450. Anal. calculated for $C_{20}H_{22}IN_3O_2 \cdot HCl \cdot 1.5H_2O$ : C, 45.60; H, 4.97; N, 7.98. Found: C, 45.80; H, 4.71; N, 7.92.

**EXAMPLE 18**

Syntheses of *N*-(2-diethylaminoethyl)-9,10-dihydro-7-iodo-9-oxoacridine-4-carboxamide hydrochloride (**84**) and of *N*-(2-diethylaminoethyl)-9,10-dihydro-9-oxo-7-(tributylstannyl)acridine-4-carboxamide (**85**).

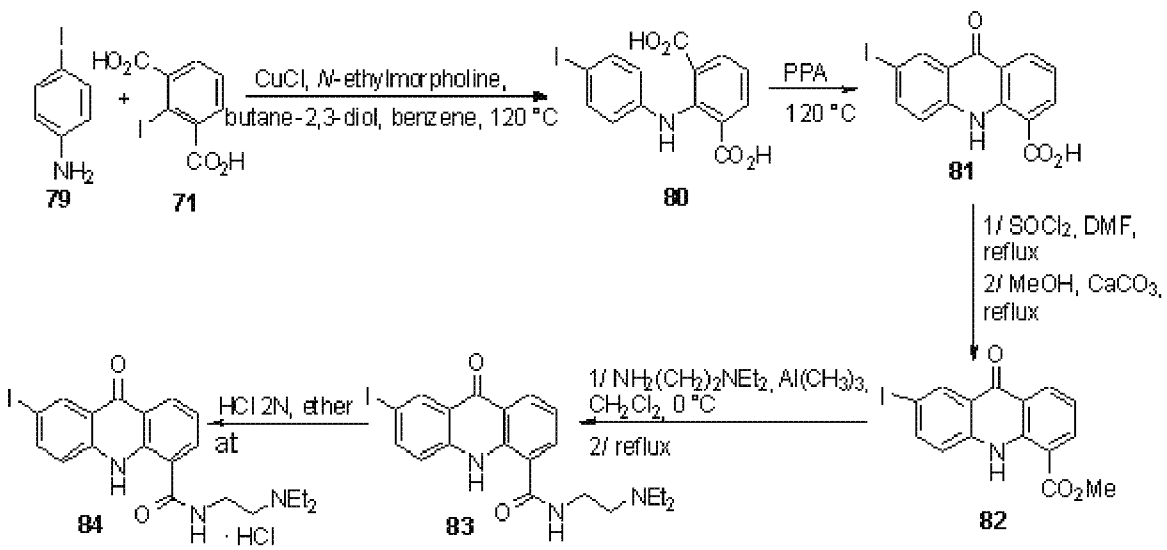


**Stage A: 2-(4-iodophenylamino)isophtalic acid (80)**

The compound **80** was prepared according to the procedure described for the preparation of the compound **72**, using 4-iodoaniline (**79**) as starting material. Yield: 62%; melting point: 241-243°C; $^1$H NMR (200 MHz, *$d_6$*-DMSO) δ 6.75 (d, 2H, *J*= 9 Hz), 7.04 (t, 1H, *J* = 8 Hz), 7.49 (d, 2H, *J* = 9 Hz), 7.94 (d, 2H, *J* = 8 Hz), 9.60 (s, 1H); $^{13}$C NMR (100 MHz, *$d_6$*-DMSO) δ 83.8, 119.7 (2C), 119.8, 121.4 (2C), 135.1 (2C), 137.4 (2C), 143.3, 143.9, 168.5 (2C); IR (KBr) ν cm$^{-1}$: 1243, 1408, 1505, 1592, 1689, 2800-3200; MS (m/z, %) 383 ($M^+$, 100), 194 (86), 166 (35), 139 (22).

**Stage B: 9,10-dihydro-7-iodo-9-oxoacridine-4-carboxylic acid (81)**

The compound **81** was prepared according to the procedure described for the preparation of the compound **73**, using 2-(4-iodophenylamino)isophtalic acid (**80**) as starting material. Yield: 63%; melting point: 355-357°C; $^1$H NMR (200 MHz, *$d_6$*-DMSO) δ 7.25 (t, 1H, *J* = 8 Hz), 7.46 (d, 1H, *J* = 9 Hz), 7.86 (d, 1H, *J* = 9 Hz), 8.34 (m, 3H), 11.93 (bs, 1H); $^{13}$C NMR (100 MHz, *$d_6$*-DMSO) δ 85.8, 115.9, 120.4, 121.0, 121.4, 122.2, 131.9, 134.0, 136.9, 139.0, 140.9, 141.6, 169.0, 175.1; IR (KBr) ν cm$^{-1}$: 1148, 1446, 1518, 1609, 1693, 2800-3200; MS (m/z, %) 365 ($M^+$, 95), 347 (100), 319 (23), 164 (33).

**Stage C: methyl 9,10-dihydro-7-iodo-9-oxoacridine-4-carboxylate (82)**

The compound **82** was prepared according to the procedure described for the preparation of the compound **67**, using 9,10-dihydro-7-iodo-9-oxoacridine-4-carboxylic acid (**81**) as starting material. Yield: 60%; Rf: 0.85 ($Al_2O_3$, dichloromethane); melting point: 224-226°C; NMR $^1H$ (200 MHz, $CDCl_3$) δ 4.01 (s, 3H), 7.14 (d, 1H, *J* = 8.5 Hz), 7.25 (t, 1H, *J* = 8 Hz), 7.89 (dd, 1H, *J* = 8.5, 2 Hz), 8.41 (d, 1H, *J* = 8 Hz), 8.67 (d, 1H, *J* = 8 Hz), 8.72 (d, 1H, *J* = 2 Hz), 11.76 (s, 1H); $^{13}C$ NMR (100 MHz, $CDCl_3$) δ 52.7, 85.4, 113.9, 119.6, 120.4, 122.5, 123.2, 134.0, 136.0, 136.9, 139.3, 141.6, 142.3, 168.4, 176.5; IR (KBr) ν $cm^{-1}$: 1137, 1440, 1518, 1589, 1614, 1692, 3247; MS (m/z, %) 379 ($M^+$, 91), 347 (100), 220 (29), 164 (94), 75 (44), 63 (34).

**Stage D: *N*-(2-diethylaminoethyl)-9,10-dihydro-7-iodo-9-oxoacridine-4-carboxamide (83)**

The compound **83** was prepared according to the procedure described for the preparation of the compound **2**, using methyl 9,10-dihydro-7-iodo-9-oxoacridine-4-carboxylate (**82**) as starting material and heating the reaction medium at reflux for 6 hours. Yield: 69%; Rf: 0.27 ($Al_2O_3$, dichloromethane / ethanol (99/1, v/v)); melting point: 140-142°C; $^1H$ NMR (400 MHz, $CDCl_3$) δ 1.30 (t, 6H, *J*= 7 Hz), 3.01 (m, 4H), 3.10 (m, 2H), 3.76 (m, 2H), 7.07 (d, 1H, *J*= 9 Hz), 7.24 (t, 1H, *J*= 8 Hz), 7.81 (d, 1H, *J*= 9 Hz), 8.25 (d, 1H, *J* = 8 Hz), 8.53 (d, 1H, *J* = 8 Hz), 8.65 (m, 2H), 12.48 (s, 1H); $^{13}C$ NMR (100 MHz, $CDCl_3$) δ 10.0 (2C), 35.9, 47.6 (2C), 52.2, 84.9, 116.8, 119.7, 120.4, 122.4, 122.8, 131.9, 132.9, 135.6, 139.2, 141.0, 141.9, 168.6, 176.5; IR (KBr) ν $cm^{-1}$: 1300, 1516, 1613, 1647, 2800-3500; MS (m/z, %) 463 ($M^+$, 4), 86 (100), 58 (7).

**Stage E: N-(2-diethylaminoethyl)-9,10-dihydro-7-iodo-9-oxoacridine-4-carboxamide hydrochloride (84)**

The compound **84** was prepared according to the procedure described for the preparation of the compound **3,** using N-(2-diethylaminoethyl)-9,10-dihydro-7-iodo-9-oxoacridine-4-carboxamide (**83**) as starting material. Yield: 99%; melting point: 220-222°C; $^1H$ NMR (400 MHz, $d_6$-DMSO) δ 1.27 (t, 6H, *J* = 7 Hz), 3.23 (m, 4H), 3.34 (m, 2H), 3.76 (m, 2H), 7.38 (t, 1H, *J* = 8 Hz), 7.63 (d, 1H, *J* = 9 Hz), 8.00 (d, 1H, *J* = 9 Hz), 8.44 (m, 2H), 8.49 (s, 1H), 9.46 (bs, 1H), 10.40 (bs, 1H), 12.38 (bs, 1H); $^{13}C$ NMR (100 MHz, $d_6$-DMSO) δ 8.3 (2C), 34.2, 46.5 (2C), 49.7, 85.7, 118.5, 120.4, 121.0, 121.6, 122.2, 130.4, 133.7, 134.1, 139.2, 140.1, 141.7, 168.0, 175.2; IR (KBr) ν $cm^{-1}$: 1303, 1517, 1617, 3200-3400. Anal. Calculated for $C_{20}H_{22}IN_3O_2 \cdot HCl \cdot 2H_2O$ : C, 44.83; H, 5.08; N, 7.84. Found: C, 44.67; H, 4.43; N, 7.76.

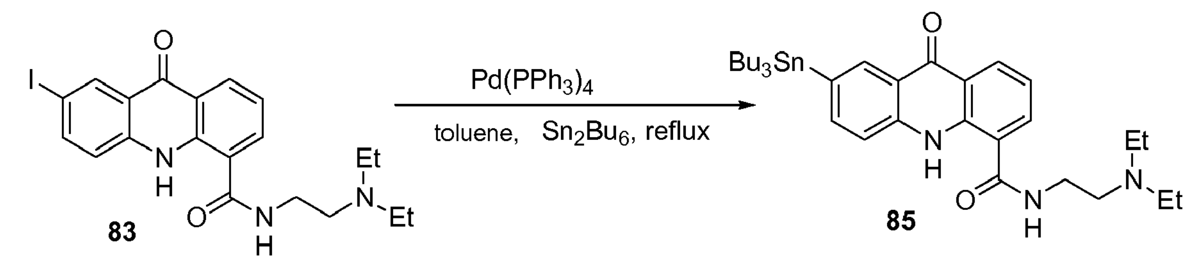


83

85

**Stage F : *N*-(2-diethylaminoethyl)-9,10-dihydro-9-oxo-7-(tributylstannyl)-acridine-4-carboxamide (85)**

The compound **85** was prepared according to the procedure described for the preparation of the compound **32**, using *N*-(2-diethylaminoethyl)-9,10-dihydro-7-iodo-9-oxoacridine-4-carboxamide (**83**) as starting material and heating the reaction medium at reflux for 4 hours. The residue obtained is chromatographed on a column of alumina eluted with ethyl acetate. Yield: 69%; Rf 0.20 ($Al_2O_3$, ethyl acetate); viscous liquid; $^1H$ NMR (200 MHz, $CDCl_3$) δ 0.88 (t, 9H, *J* = 7 Hz), 1.05 (m, 12H), 1.24 (m, 6H), 1.55 (m, 6H), 2.57 (q, 4H, *J*= 7 Hz), 2.70 (t, 2H, *J*= 7 Hz), 3.52 (m, 2H), 7.19 (t, 1H, *J*= 8 Hz), 7.37 (d, 1H, *J* = 8 Hz), 7.52 (bs, 1H), 7.75 (d, 1H, *J* = 8 Hz), 7.90 (d, 1H, *J* = 8 Hz), 8.54 (s, 1H), 8.65 (d, 1H, *J*= 8 Hz), 12.40 (bs, 1H); $^{13}C$ NMR (50 MHz, $CDCl_3$) δ 9.9 (3C, $^1J_{Sn-c}$ = 324 Hz), 12.1 (2C), 13.7 (3C), 27.4 (3C, $^3J_{Sn-C}$ = 55 Hz), 29.2 (3C, $^2J_{Sn-C}$ = 20 Hz), 37,3, 46.9 (2C), 51.1, 117.2, 117.8, 119.5, 121.1, 123.3, 131.3, 131.8, 134.8, 135.1, 140.4, 141.3, 141.4, 168.4, 178.2; IR ($CCl_4$) ν $cm^{-1}$: 1150, 1508, 1608, 1652, 2928, 2960; ESI-MS m/z 628.2 $[M+H]^+$.

**EXAMPLE 19**

Synthesis of *N*-(2-diethylaminoethyl)-9,10-dihydro-8-iodo-9-oxoacridine-4-carboxamide hydrochloride (**87**)

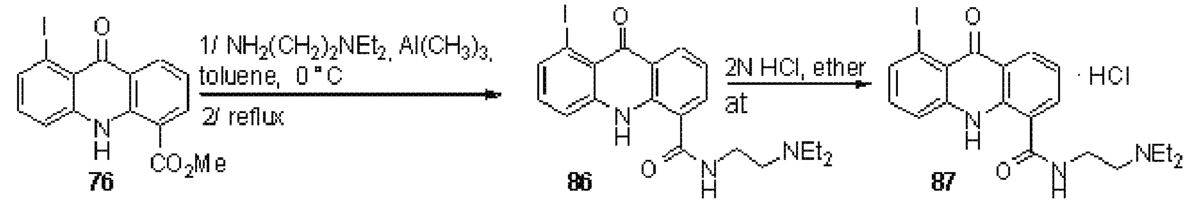


**Stage A: *N*-(2-diethylaminoethyl)-9,10-dihydro-8-iodo-9-oxoacridine-4-carboxamide (86)**

The compound **86** was prepared according to the procedure described for the preparation of the compound **2**, using methyl 9,10-dihydro-8-iodo-9-oxoacridine-4-carboxylate (**76**) as starting material and heating the reaction medium at reflux for 4 hours in anhydrous toluene. Yield: 63%; unstable product; Rf 0.30 ($Al_2O_3$, dichloromethane / ethanol (99/1, v/v)); melting point: 144-146°C; $^1H$ NMR (200 MHz, $CDCl_3$) δ 1.15 (t, 6H, *J*= 7 Hz), 2.76 (q, 4H, *J*= 7 Hz), 2.86 (t, 2H, *J*= 6 Hz), 3.59 (m, 2H), 7.17 (m, 2H), 7.31 (d, 1H, *J*= 8 Hz), 7.80 (bs, 1H), 7.85 (d, 1H, *J*= 7.5 Hz), 7.95 (d, 1H, *J*= 7.5 Hz), 8.54 (d, 1H, *J* = 7 Hz), 11.30 (bs, 1H); $^{13}C$ NMR (100 MHz, $CDCl_3$) δ 11.3 (2C), 36.6, 47.0 (2C), 51.3, 92.2, 116.8, 118.5, 118.7, 120.3, 122.4, 131.8, 132.1, 133.6, 136.6, 140.1, 141.3, 168.1, 176.1; IR (KBr) ν $cm^{-1}$: 1298, 1514, 1606, 1645, 2800-3500; MS (m/z, %) 463 ($M^+$, 1), 86 (100), 58 (12).

**Stage B: *N*-(2-diethylaminoethyl)-9,10-dihydro-8-iodo-9-oxoacridine-4-carboxamide hydrochloride (87)**

The compound **87** was prepared according to the procedure described for the preparation of the compound **3**, using *N*-(2-diethylaminoethyl)-9,10-dihydro-8-iodo-9-oxoacridine-4-carboxamide (**86**) as starting material. Yield: 91%; melting point: 228-230°C; $^1H$ NMR (400 MHz, $d_6$-DMSO) δ 1.25 (t, 6H, *J* = 7 Hz), 3.30 (m, 6H), 3.71 (m, 2H), 7.37 (m, 2H), 7.71 (d, 1H, *J* = 8 Hz), 7.90 (d, 1H, *J* = 7.5 Hz), 8.28 (d, 1H, *J* = 7.5 Hz), 8.43 (d, 1H, *J* = 7 Hz), 9.23 (bs, 1H), 9.44 (m, 1H), 12.26 (bs, 1H); $^{13}C$ NMR (100 MHz, $d_6$-DMSO) δ 8.3 (2C), 34.2, 46.5 (2C), 49.7, 92.4, 117.5, 117.8, 119.3, 120.4, 121.6, 130.9, 133.4, 134.2, 136.3, 139.3, 141.2, 168.0, 175.0; IR (KBr) ν $cm^{-1}$: 1295, 1458, 1517, 1560, 1607, 2925, 3200-3400. Anal. Calculated for $C_{20}H_{22}IN_3O_2 \cdot HCl \cdot 0.5H_2O$ : C, 47.21; H, 4.74; N, 8.26. Found : C, 47.70; H, 5.12; N, 8.29.

## EXAMPLE 20

Synthesis of *N*-(2-diethylaminoethyl)-1-iodoacridine-4-carboxamide dihydrochloride (**91**)

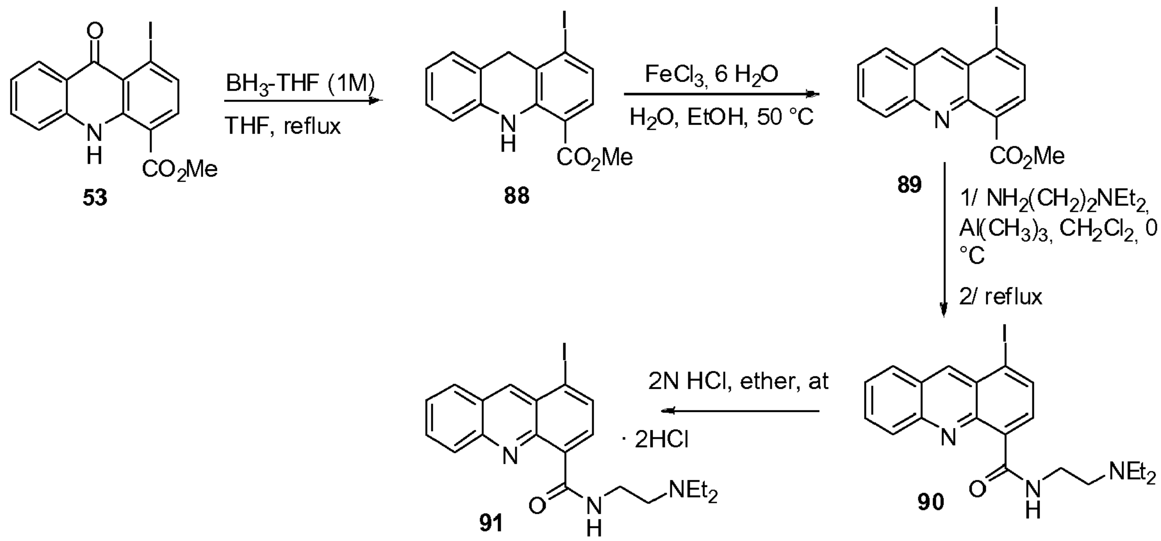

**Stage A: methyl 1-iodoacridane-4-carboxylate (88)**

A 1M solution of $BH_3$-THF (0.82 ml, 0.82 mmol) is added dropwise at reflux, under argon, to a solution of methyl 9,10-dihydro-1-iodo-9-oxoacridine-4-carboxylate (53) (0.26 g, 0.69 mmol) in anhydrous THF (10 ml). The medium is heated at reflux for 45 minutes. After returning to ambient temperature, the reaction is halted with a 3N hydrochloric acid solution (5 ml) and then the medium is basified using a saturated aqueous sodium carbonate solution (pH = 8-9). The reaction mixture is extracted with dichloromethane (3 x 100 ml). The organic phase is dried over magnesium sulphate and evaporated under vacuum. The crude reaction product is purified by chromatography on silica eluted with dichloromethane, to result in the acridane **88** (188 mg, 0.52 mmol); yield: 75%; Rf 0.91 ($SiO_2$, dichloromethane); melting point: 119-121˚C; $^1H$ NMR (400 MHz, $CDCl_3$) δ 3.91 (s, 3H), 4.13 (s, 2H), 6.74 (d, 1H, *J* = 7.5 Hz), 6.88 (t, 1H, *J*= 7.5 Hz), 7.10 (m, 2H), 7.26 (d, 1H, *J* = 8.5 Hz), 7.41 (d, 1H, *J* = 8.5 Hz), 9.93 (bs, 1H); $^{13}C$ NMR (100 MHz, $CDCl_3$) δ 38.1, 52.0, 109.0, 109.8, 114.5, 119.5, 121.8, 124.3, 127.6, 128.6, 128.8, 130.1, 137.9, 143.6, 168.8; IR (KBr) ν $cm^{-1}$: 1252, 1269, 1431, 1498, 1585, 1690, 2948, 3304; MS (m/z, %) 365 ($M^+$, 100), 332 (78), 206 (52), 178 (53), 151 (23).

**Stage B: methyl 1-iodoacridine-4-carboxylate (89)**

Methyl 1-iodoacridane-4-carboxylate (**88**) (140 mg, 0.38 mmol) is dissolved in a mixture of water (2 ml) and ethanol (10 ml) and then $FeCl_3 \cdot 6H_2O$ (0.30 g) is added. The solution is stirred at 50˚C for 30 minutes and then a saturated aqueous sodium carbonate solution (20 ml) is added. The aqueous phase is subsequently extracted with dichloromethane (3 x 30 ml). The organic phase is dried over magnesium sulphate, filtered and evaporated to dryness. The product obtained **89** is very unstable and is used without purification (121 mg, 0.33 mmol); yield: 87%; $^1H$ NMR (400 MHz, $CDCl_3$) δ 4.00 (s, 3H), 7.53 (t, 1H, *J* = 7 Hz), 7.66 (d, 1H, *J* = 7 Hz), 7.76 (t, 1H, *J* = 7 Hz), 7.99 (d, 1H, *J* = 8 Hz), 8.07 (d, 1H, *J* = 7 Hz), 8.21 (d, 1H, *J* = 8 Hz), 8.91 (s, 1H).

**Stage C : *N*-(2-diethylaminoethyl)-1-iodoacridine-4-carboxamide (90)**

The compound **90** was prepared according to the procedure described for the preparation of the compound **2**, using methyl 1-iodoacridine-4-carboxylate (**89**) as starting material and heating the reaction medium at reflux for 4 hours. Yield: 80%; Rf 0.40 ($Al_2O_3$, dichloromethane / ethanol (99/1, v/v)); melting point: 103-105 ˚C; $^1H$ NMR (400 MHz, $CDCl_3$) δ 1.15 (t, 6H, *J*= 7 Hz), 2.78 (q, 4H, *J*= 7 Hz), 2.88 (t, 2H, *J*= 7 Hz), 3.83 (m, 2H), 7.63 (t, 1H, *J* = 7 Hz), 7.85 (t, 1H, *J* = 8 Hz), 8.07 (d, 1H, *J* = 8.5 Hz), 8.24 (d, 1H, *J* = 8 Hz), 8.29 (d, 1H, *J* = 8.5 Hz), 8.60 (d, 1H, *J* = 7.5 Hz), 9.04 (s, 1H), 11. 82 (bs, 1H); $^{13}C$ NMR (100 MHz, $CDCl_3$) δ 11.6 (2C), 38.0, 47.1 (2C), 51.9, 103.8, 126.9, 127.0, 128.0, 128.3, 128.7, 129.4, 131.9, 135.4, 137.1, 142.6, 145.8, 148.1, 165.4; IR (KBr) ν $cm^{-1}$: 1517, 1649, 1654, 2967, 3186, 3300-3500; MS (m/z, %) 447 ($M^+$, 8), 332 (18), 177 (17), 86 (100), 58 (15).

**Stage D: *N*-(2-diethylaminoethyl)-1-iodoacridine-4-carboxamide dihydrochloride (91)**

The compound **91** was prepared according to the procedure described for the preparation of the compound **3**, using *N*-(2-diethylaminoethyl)-1-iodoacridine-4-carboxamide (**90**) as starting material. Yield: 71%; melting point: 219-221˚C; $^1H$ NMR (400 MHz, $d_6$-DMSO) δ 1.27 (t, 6H, *J*= 7 Hz), 3.27 (m, 4H), 3.43 (m, 2H), 4.00 (m, 2H), 7.80 (t, 1H, *J* = 7.5 Hz), 8.06 (d, 1H, *J* = 7.5 Hz), 8.40 (d, 1H, *J* = 7.5 Hz), 8.46 (m, 2H), 8.58 (d, 1H, *J* = 9 Hz), 9.34 (s, 1H), 10.25 (s, 1H), 11.30 (s, 1H); $^{13}C$ NMR (100 MHz, $d_6$-DMSO) δ 8.5 (2C), 34.5, 46.9 (2C), 54.9, 105.4, 126.6, 127.4, 127.5, 128.3, 128.8, 132.7, 134.8, 136.8, 142.9, 144.7, 147.4, 165,4, one carbon not observed; IR (KBr) ν $cm^{-1}$: 1394, 1549, 1577, 1624, 2472, 2638, 2975, 3200-3500. Anal. Calculated for $C_{20}H_{22}IN_3O \cdot 2HCl$: C, 46.18; H, 4.65; N, 8.08. Found : C, 46.38; H, 4.79; N, 7.99.

**<u>EXAMPLE 21</u>**

Synthesis of *N*-(2-diethylaminoethyl)-2-iodoacridine-4-carboxamide dihydrochloride (**95**)

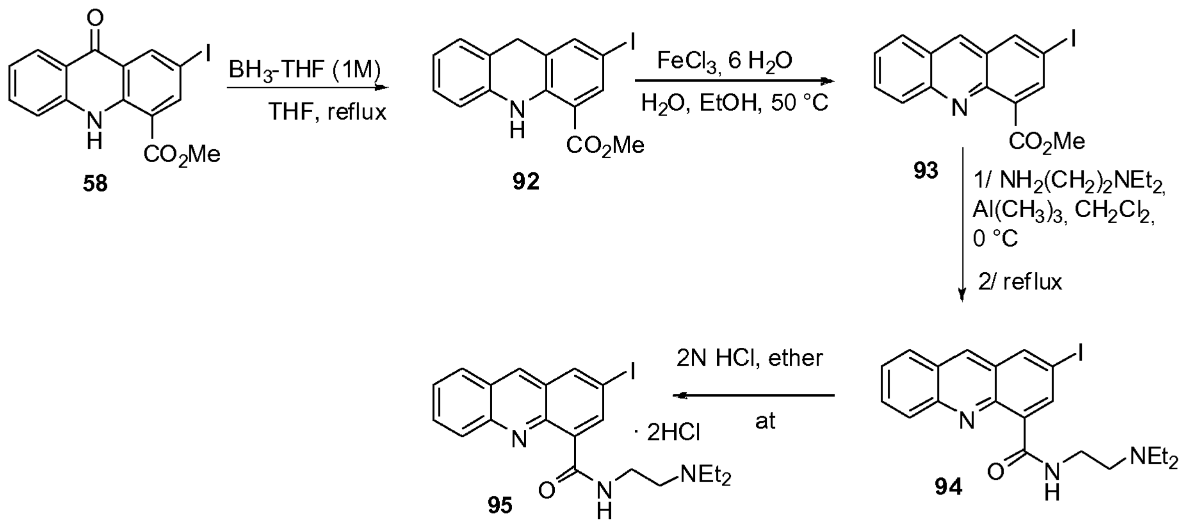


**Stage A: methyl 2-iodoacridane-4-carboxylate (92)**

The compound **92** was prepared according to the procedure described for the preparation of the compound **88**, using methyl 9,10-dihydro-2-iodo-9-oxoacridine-4-carboxylate (**58**) as starting material. Yield: 58%; Rf: 0.93 ($SiO_2$, dichloromethane); melting point: 148-150°C; $^1H$ NMR (400 MHz, $CDCl_3$) δ 3.92 (s, 3H), 4.06 (s, 2H), 6.77 (d, 1H, *J* = 7.5 Hz), 6.90 (t, 1H, *J* = 7.5 Hz), 7.06 (d, 1H, *J* = 7.5 Hz), 7.12 (t, 1H, *J* = 7.5 Hz), 7.48 (s, 1H), 8.08 (s, 1H), 9.77 (bs, 1H); $^{13}C$ NMR (100 MHz, $CDCl_3$) δ 31.0, 52.2, 79.2, 112.3, 114.9, 119.4, 121.9, 124.4, 127.5, 128.4, 137.5, 138.3, 141.3, 143.3, 167.7; IR (KBr) ν $cm^{-1}$: 1253, 1263, 1501, 1676, 2941, 3344; MS (m/z, %) 365 ($M^+$, 100), 332 (23), 178 (58), 151 (20), 127(22).

**Stage B: methyl 2-iodoacridine-4-carboxylate (93)**

The compound **93** was prepared according to the procedure described for the preparation of the compound **89**, using methyl 2-iodoacridane-4-carboxylate (**92**) as starting material. Yield: 93%; very unstable product; $^1H$ NMR (200 MHz, $CDCl_3$) δ 4.14 (s, 3H), 7.62 (t, 1H, *J* = 7.5 Hz), 7.88 (t, 1H, *J* = 7.5 Hz), 8.05 (d, 1H, *J* = 8 Hz), 8.36 (m, 2H), 8.59 (s, 1H), 8.86 (s, 1H).

**Stage C : *N*-(2-diethylaminoethyl)-2-iodoacridine-4-carboxamide (94)**

The compound **94** was prepared according to the procedure described for the preparation of the compound **2**, using methyl 2-iodoacridine-4-carboxylate (**93**) as starting material and heating the reaction medium at reflux for 4 hours. Yield: 80%; Rf 0.41 ($Al_2O_3$, dichloromethane / ethanol (99/1, v/v)); melting point: 108-110°C; $^1H$ NMR (400 MHz, $CDCl_3$) δ 1.19 (t, 6H, *J*= 7 Hz), 2.85 (m, 4H), 2.96 (m, 2H), 3.88 (m, 2H), 7.61 (t, 1H, *J* = 7 Hz), 7.87 (t, 1H, *J* = 8 Hz), 8.01 (d, 1H, *J* = 8.5 Hz), 8.29 (d, 1H, *J* = 8.5 Hz), 8.51 (s, 1H), 8.71 (s, 1H), 9.10 (s, 1H), 11.88 (bs, 1H); $^{13}C$ NMR (100 MHz, $CDCl_3$) δ 11.2 (2C), 37.7, 47.2 (2C), 51.7, 91.0, 126.2, 127.1, 128.2, 128.4, 129.4, 129.6, 131.8, 136.3, 140.7, 143.4, 145.0, 147.9, 164.8; IR (KBr) ν $cm^{-1}$: 1516, 1637, 2965, 3182; MS (m/z, %) 447 ($M^+$, 1), 177 (8), 86 (100), 58 (13).

**Stage D: *N*-(2-diethylaminoethyl)-2-iodoacridine-4-carboxamide**

**dihydrochloride (95)**

The compound **95** was prepared according to the procedure described for the preparation of the compound **3**, using *N*-(2-diethylaminoethyl)-2-iodoacridine-4-carboxamide (**94**) as starting material. Yield: 76%; melting point: 215-217°C; $^1H$ NMR (200 MHz, $d_6$-DMSO) δ 1.26 (t, 6H, *J*= 7 Hz), 3.24 (m, 4H), 3.41 (m, 2H), 3.83 (m, 2H), 7.73 (m, 1H), 8.01 (t, 1H, *J* = 8 Hz), 8.23 (d, 1H, *J* = 8.5 Hz), 8.52 (d, 1H, *J* = 8.5 Hz), 8.79 (s, 1H), 8.90 (s, 1H), 9.27 (s, 1H), 10.49 (m, 1H), 11.33 (bs, 1H); $^{13}C$ NMR (100 MHz, $d_6$-DMSO) δ 8.5 (2C), 34.5, 46.9 (2C), 49.6, 91.2, 125.8, 127.4, 128.0, 128.6, 128.7, 129.1, 132.4, 138.0, 141.0, 142.0, 143.5, 146.9, 164.5; IR (KBr) ν $cm^{-1}$: 1572, 1625, 1649, 2651, 2975, 3200-3500. Anal. Calculated for $C_{20}H_{22}IN_3O·2HCl·1.5H_2O$ : C, 43.90; H, 4. 97; N,7.68. Found : C, 43.77; H, 4.77; N, 7.54.

**EXAMPLE 22**

Synthesis of *N*-(2-diethylaminoethyl)-3-iodoacridine-4-carboxamide dihydrochloride (**98**)

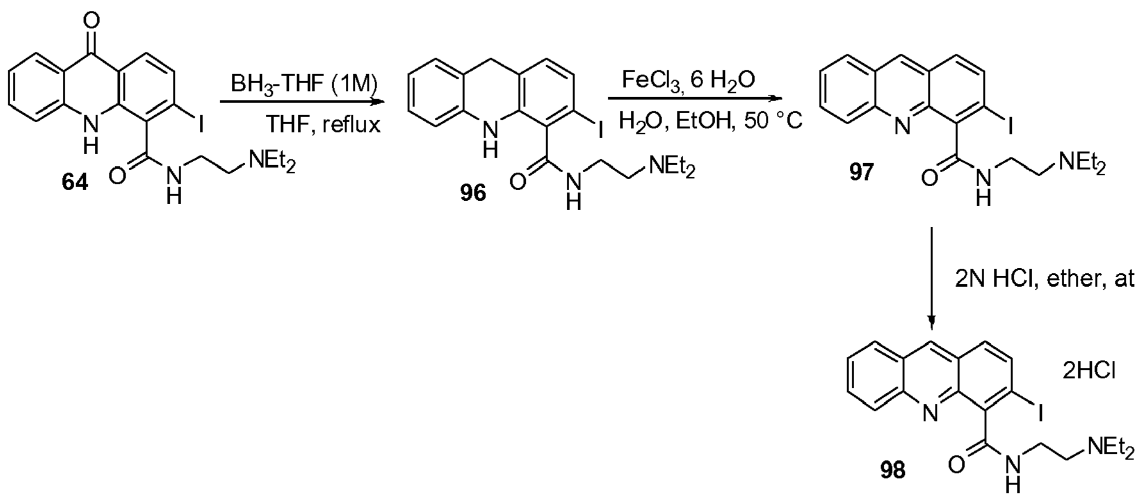


**Stage A : *N*-(2-diethylaminoethyl)-3-iodoacridane-4-carboxamide (96)**

The compound 96 was prepared according to the procedure described for the preparation of the compound **88**, using methyl *N*-(2-diethylaminoethyl)-9,10-dihydro-3-iodo-9-oxoacridine-4-carboxylate (**64**) as starting material. Yield: 60%; unstable product; melting point: 106-108˚C; $^1$H NMR (200 MHz, $CDCl_3$) δ 1.26 (t, 6H, *J*= 7 Hz), 2.99 (m, 6H), 3.91 (q, 2H, *J* = 6.5 Hz), 3.98 (s, 2H), 6.48 (m, 1H), 6.71 (d, 1H, *J* = 8 Hz), 6.79 (t, 1H, *J*= 8 Hz), 6.89 (m, 1H), 7.09 (m, 2H), 7.26 (m, 2H); $^{13}$C NMR (50 MHz, $CDCl_3$) δ 8.5 (2C), 31.1, 35.1, 53.4 (2C), 57.0, 90.1, 114.6, 119.2, 121.3, 121.5, 124.5, 127.3, 128.3, 130.9, 131.3, 138.8, 139.6, 168.8; IR (KBr) ν $cm^{-1}$: 1164, 1449, 1636, 2346, 3268; MS (m/z, %) 449 ($M^+$, 1), 86 (100), 58 (10).

**Stage B: *N*-(2-diethylaminoethyl)-3-iodoacridine-4-carboxamide (97)**

The compound **97** was prepared according to the procedure described for the preparation of the compound **89**, using *N*-(2-diethylaminoethyl)-3-iodoacridane-4-carboxamide (**96**) as starting material. Yield: 73%; Rf: 0.13 ($Al_2O_3$, ethyl acetate / pentane (1/1, v/v)); melting point: 81-83˚C; $^1$H NMR (400 MHz, $CDCl_3$) δ 1.04 (t, 6H, *J*= 7 Hz), 2.69 (m, 4H), 2.91 (m, 2H), 3.81 (m, 2H), 6.95 (bs, 1H), 7.54 (d, 1H, *J* = 8 Hz), 7.65 (d, 1H, *J* = 9 Hz), 7.76 (t, 1H, *J* = 8 Hz), 7.80 (d, 1H, *J* = 9 Hz), 7.94 (d, 1H, *J* = 8.5 Hz), 8.17 (d, 1H, *J* = 9 Hz), 8.68 (s, 1H); $^{13}$C NMR (100 MHz, $CDCl_3$) δ 11.3 (2C), 37.2, 46.8 (2C), 51.6, 97.1, 125.3, 126.5, 126.6, 128.0, 129.5, 130.1, 130.7, 135.1, 136.0, 142.5, 146.2, 149.2, 169.2; IR (KBr) ν $cm^{-1}$: 1458, 1654, 2925, 3200-3500; MS (m/z, %) 447 ($M^+$, 1), 177 (11), 86 (100), 58 (12).

**Stage C: *N*-(2-diethylaminoethyl)-3-iodoacridine-4-carboxamide**

**dihydrochloride (98)**

The compound **98** was prepared according to the procedure described for the preparation of the compound **3**, using *N*-(2-diethylaminoethyl)-3-iodoacridine-4-carboxamide (**97**) as starting material. Yield: 83%; melting point: 134-136˚C; $^1$H NMR (200 MHz, $CD_3OD$) δ 1.47 (t, 6H, *J*= 7 Hz), 3.49 (q, 4H, *J*= 7 Hz), 3.62 (t, 2H, *J*= 7 Hz), 3.97 (t, 2H, *J* = 7Hz), 7.79 (m, 1H), 8.06 (m, 3H), 8.35 (m, 2H), 9.40 (s, 1H); $^{13}$C NMR (100 MHz, *$d_6$-DMSO*) δ 8.8 (2C), 34.1, 46.9 (2C), 49.4, 97.9, 124.9, 126.2, 126.6, 128.7, 128.9, 130.0, 131.6, 135.0, 137.0, 142.3, 145.6, 148.3 169.0; IR (KBr) ν $cm^{-1}$: 1458, 1560, 1636, 1654, 2924, 3200-3500. Anal. Calculated for $C_{20}H_{22}IN_3O$, 2HCl, $H_2O$ : C, 44.63; H, 4.87; N, 7.81. Found : C, 44.58; H, 4.92; N, 7.42.

**EXAMPLE 23**

Syntheses of *N*-(2-diethylaminoethyl)-5-iodoacridine-4-carboxamide dihydrochloride (**102**) and of *N*-(2-diethylaminoethyl)-5-(tributylstannyl)acridine-4-carboxamide (**103**)

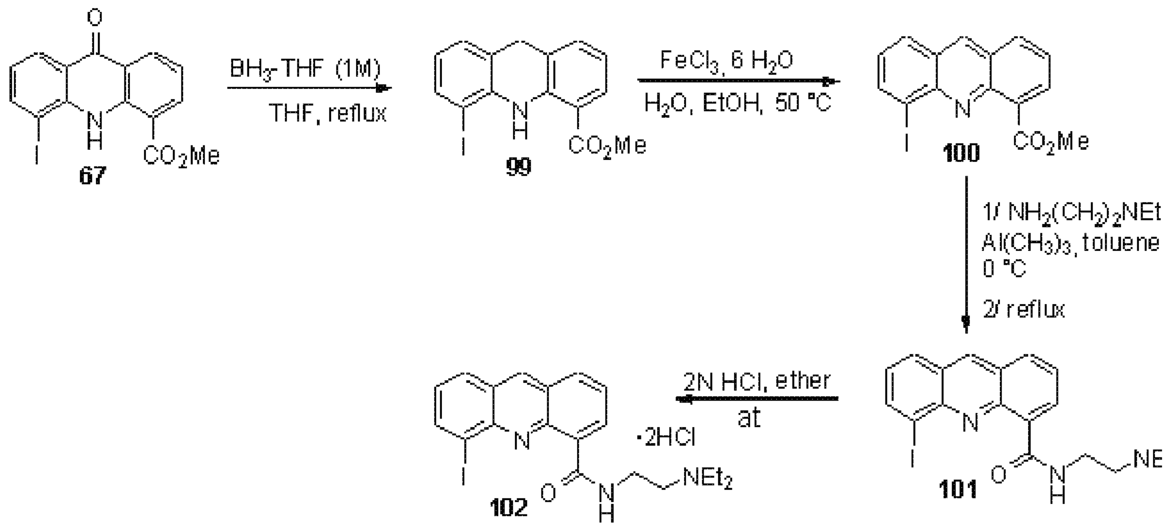


**Stage A: methyl 5-iodoacridane-4-carboxylate (99)**

The compound **99** was prepared according to the procedure described for the preparation of the compound **88**, using methyl 9,10-dihydro-5-iodo-9-oxoacridine-4-carboxylate (**67**) as starting material. Yield: 98%; Rf: 0.94 ($SiO_2$, dichloromethane); melting point: 105-107˚C; $^1H$ NMR (400 MHz, $CDCl_3$) δ 3.97 (s, 3H), 4.08 (s, 2H), 6.61 (t, 1H, *J* = 7.5 Hz), 6.83 (t, 1H, *J* = 7.5 Hz), 7.03 (d, 1H, *J* = 7 Hz), 7.24 (d, 1H, *J* = 7 Hz), 7.58 (d, 1H, *J* = 8 Hz), 7.84 (d, 1H, *J* = 8 Hz), 10.16 (bs, 1H); $^{13}C$ NMR (100 MHz, $CDCl_3$) δ 32.2, 52.2, 84.3, 111.3, 119.6, 121.1, 121.7, 122.7, 128.3, 129.5, 133.3, 137.3, 140.1, 143.0, 168.5; IR (KBr) ν $cm^{-1}$: 1259, 1443, 1491, 1689, 2924, 3260; MS (m/z, %) 365 ($M^+$, 100), 332 (56), 206 (53), 177 (81), 151 (63), 103 (36), 89 (37), 75 (46).

**Stage B: methyl 5-iodoacridine-4-carboxylate (100)**

The compound **100** was prepared according to the procedure described for the preparation of the compound **89**, using methyl 5-iodoacridane-4-carboxylate (**99**) as starting material. Yield: 97%; very unstable product; $^1H$ NMR (200 MHz, $CDCl_3$) δ 4.20 (s, 3H), 7.17 (dd, 1H, *J*= 8.5, 7 Hz), 7.51 (dd, 1H, *J*= 8.5, 7 Hz), 7.87 (d, 1H, *J*= 8.5 Hz), 8.03 (d, 1H, *J* = 8.5 Hz), 8.11 (d, 1H, *J* = 7 Hz), 8.39 (d, 1H, *J* = 7 Hz), 8.60 (s, 1H).

**Stage C : *N*-(2-diethylaminoethyl)-5-iodoacridine-4-carboxamide (101)**

The compound **101** was prepared according to the procedure described for the preparation of the compound **2**, using methyl 5-iodoacridine-4-carboxylate (**100**) as starting material and heating the reaction medium at reflux for 3 hours in anhydrous toluene. Yield: 73%; Rf 0.17 ($Al_2O_3$, dichloromethane / ethanol (99/1, v/v)); melting point: 64-66˚C; $^1H$ NMR (200 MHz, $CDCl_3$) δ 1.11 (t, 6H, *J* = 7 Hz), 2.69 (q, 4H, *J* = 7 Hz), 2.92 (m, 2H), 3.82 (m, 2H), 7.19 (dd, 1H, *J* = 8.5, 7 Hz), 7.56 (dd, 1H, *J* = 8.5, 7 Hz), 7.86 (d, 1H, *J* = 8.5 Hz), 7.98 (d, 1H, *J* = 8.5 Hz), 8.32 (d, 1H, *J* = 7 Hz), 8.60 (s, 1H), 8.88 (d, 1H, *J*= 7 Hz), 11.76 (s, 1H); $^{13}C$ NMR (100 MHz, $CDCl_3$) δ 11.8 (2C), 38.4, 47.5 (2C), 52.4, 102.6, 125.8, 126.1, 127.0, 127.1, 128.2, 129.5, 132.9, 136.0, 138.6, 141.6, 145.8, 146.3, 165.4; IR (KBr) ν $cm^{-1}$: 1648, 1654, 2925, 3200, 3300-3500; MS (m/z, %) 447 ($M^+$, 1), 177 (9), 86 (100), 58 (12).

**Stage D: *N*-(2-diethylaminoethyl)-5-iodoacridine-4-carboxamide**

**dihydrochloride (102)**

The compound **102** was prepared according to the procedure described for the preparation of the compound **3**, using *N*-(2-diethylaminoethyl)-5-iodoacridine-4-carboxamide (**101**) as starting material. Yield: 69%; melting point: 144-146°C; $^1$H NMR (400 MHz, $d_6$-DMSO) δ 1.27 (t, 6H, *J*= 7 Hz), 3.26 (m, 4H), 3.43 (m, 2H), 4.10 (m, 2H), 7.48 (t, 1H, *J* = 7.5 Hz), 7.83 (t, 1H, *J* = 7.5 Hz), 8.27 (d, 1H, *J* = 8.5 Hz), 8.46 (d, 1H, *J* = 8 Hz), 8.60 (d, 1H, *J* = 7 Hz), 8.84 (d, 1H, *J* = 7 Hz), 9.36 (s, 1H), 10.72 (bs, 1H), 12.27 (bs, 1H); $^{13}$C NMR (100 MHz, $d_6$-DMSO) δ 8.4 (2C), 34.8, 46.4 (2C), 49.9, 104.0, 125.9, 126.0, 126.9, 127.0, 127.8, 129.3, 133.2, 135.7, 140.1, 141.8, 145.6, 145.8, 165.2; IR (KBr) ν cm$^{-1}$: 1546, 1578, 1654, 2660, 2928, 3300-3500. Anal. Calculated for $C_{20}H_{22}IN_3O{\cdot}2HCl{\cdot}H_2O$: C, 44.63; H, 4.87; N, 7.81. Found: C, 44.80; H, 4.73; N, 7.63.

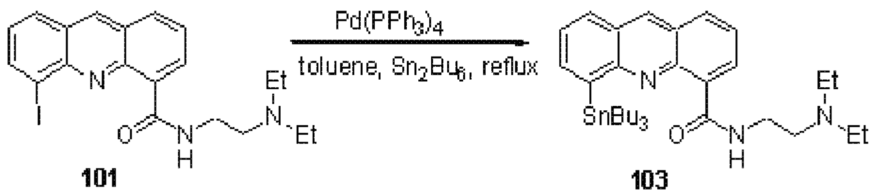


**Stage E : *N*-(2-diethylaminoethyl)-5-(tributylstannyl)acridine-4-carboxamide (103)**

The compound **103** was prepared according to the procedure described for the preparation of the compound **32**, using *N*-(2-diethylaminoethyl)-5-iodoacridine-4-carboxamide (**101**) as starting material and heating at reflux for 32 hours. The residue obtained is chromatographed on a column of alumina eluted with a mixture of ethyl acetate and pentane (1/1, v/v), resulting, in order of elution, in:

***N*-(2-diethylaminoethyl)-5-(tributylstannyl)acridine-4-carboxamide (103)**

Yield: 25%; Rf 0.79 ($Al_2O_3$, ethyl acetate / pentane (1/1, v/v)); viscous liquid; $^1$H NMR (200 MHz, $CDCl_3$) δ 0.79 (t, 9H, *J* = 7 Hz), 1.3 (m, 18H), 1.45 (m, 6H), 2.76 (m, 4H), 2.88 (m, 2H), 3.73 (m, 2H), 7.54 (t, 1H, *J* = 7.5 Hz), 7.64 (dd, 1H, *J* = 7.5, 8.5 Hz), 8.00 (m, 2H), 8.15 (d, 1H, *J* = 8.5 Hz), 8.88 (s, 1H), 8.93 (d, 1H, *J* = 7.5 Hz), 10.59 (bs, 1H); $^{13}$C NMR (50 MHz, $CDCl_3$) δ 10.1 (3C, $^1J_{Sn\text{-}C}$ = 343 Hz), 12.2 (2C), 13.7 (3C), 27.4 (3C, $^3J_{Sn\text{-}C}$ = 60 Hz), 29.3 (3C, $^2J_{Sn\text{-}C}$ = 20 Hz), 39.1, 47.8 (2C), 52.1, 125.1, 126.0, 126.5, 127.0, 128.8, 129.3, 132.7, 135.3, 139.3, 141.0, 145.0, 146.4, 153.0, 166.7; IR ($CCl_4$) ν cm$^{-1}$: 1286, 1662, 2927, 2959; ESI-MS m/z 612.3 [M+H]$^+$.

***N*-(2-diethylaminoethyl)acridine-4-carboxamide** (Atwell, G. J.; Rewcastle, G. W., Baguley, B. C. and Denny, W. A., J. Med. Chem., 1987, 30, 664-669). Yield 29%; Rf 0.33 ($Al_2O_3$, ethyl acetate / pentane (1/1, v/v)); melting point 148-150°C.

**EXAMPLE 24**

Synthesis of *N*-(2-diethylaminoethyl)-6-iodoacridine-4-carboxamide dihydrochloride (**107**)

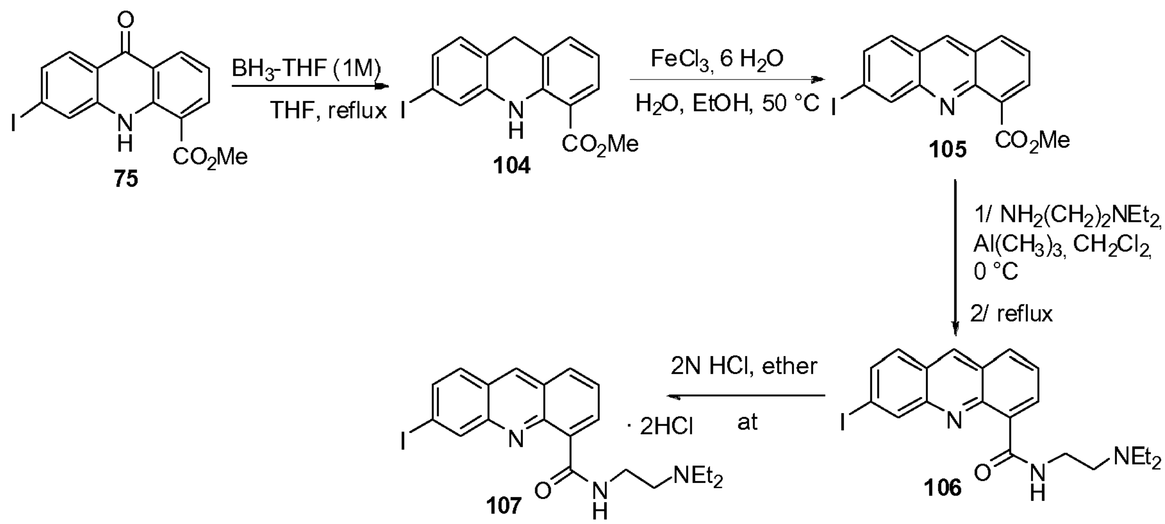


75 104 105 106 107

**Stage A: methyl 6-iodoacridane-4-carboxylate (104)**

The compound **104** was prepared according to the procedure described for the preparation of the compound **88**, using methyl 9,10-dihydro-6-iodo-9-oxoacridine-4-carboxylate (**75**) as starting material. Yield: 76%; unstable product; melting point: 108-110˚C; $^1$H NMR (400 MHz, $CDCl_3$) δ 3.94 (s, 3H), 4.01 (s, 2H), 6.80 (m, 2H), 7.14 (s, 1H), 7.18 (d, 1H, *J* = 8 Hz), 7.22 (d, 1H, *J* = 7.5 Hz), 7.79 (d, 1H, *J* = 8 Hz), 9.82 (bs, 1H); $^{13}$C NMR (100 MHz, $CDCl_3$) δ 31.2, 52.0, 91.6, 110.8, 119.2, 119.5, 121.3, 123.3, 129.4, 129.9, 130.2, 133.6, 140.5, 143.0, 168.8; IR (KBr) ν $cm^{-1}$: 1266, 1429, 1464, 1507, 1594, 1684, 2942, 3342.

**Stage B: methyl 6-iodoacridine-4-carboxylate (105)**

The compound **105** was prepared according to the procedure described for the preparation of the compound **89**, using methyl 6-iodoacridane-4-carboxylate (**104**) as starting material. Yield: 88%; very unstable product; $^1$H NMR (200 MHz, $CDCl_3$) δ 4.12 (s, 3H), 7.54 (dd, 1H, *J*= 8.5, 7 Hz), 7.67 (m, 2H), 8.06 (d, 1H, *J*= 8.5 Hz), 8.12 (d, 1H, *J*= 7 Hz), 8.70 (s, 1H), 8.77 (s, 1H).

**Stage C : *N*-(2-diethylaminoethyl)-6-iodoacridine-4-carboxamide (106)**

The compound **106** was prepared according to the procedure described for the preparation of the compound **2**, using methyl 6-iodoacridine-4-carboxylate (**105**) as starting material and heating the reaction medium at reflux for 4 hours. Yield: 52%; Rf: 0.37 ($Al_2O_3$, dichloromethane / ethanol (99/1, v/v)); melting point: 80-82 ˚C; $^1$H NMR (200 MHz, $CDCl_3$) δ 1.16 (t, 6H, *J*= 7 Hz), 2.79 (m, 6H), 3.77 (q, 2H, *J*= 6 Hz), 7.65 (dd, 1H, *J* = 8.5, 7 Hz), 7.70 (d, 1H, *J* = 9 Hz), 7.78 (d, 1H, *J* = 9 Hz), 8.06 (d, 1H, *J* = 8.5 Hz), 8.78 (s, 1H), 8.81 (s, 1H), 8.97 (d, 1H, *J* = 7 Hz), 11.90 (bs, 1H); $^{13}$C NMR (50 MHz, $CDCl_3$) δ 12.0 (2C), 38.0, 47.0 (2C), 51.9, 98.1, 124.6, 126.0, 127.0, 128.9, 129.0, 132.1, 134.8, 135.8, 137.6, 138.4, 146.6, 147.7, 165.5; IR (KBr) ν $cm^{-1}$: 1458, 1559, 1654, 2924, 3300-3500; MS (m/z, %) 447 ($M^+$, 3), 177 (9), 86 (100), 58 (15).

**Stage D: *N*-(2-diethylaminoethyl)-6-iodoacridine-4-carboxamide dihydrochloride(107)**

The compound **107** was prepared according to the procedure described for the preparation of the compound **3**, using *N*-(2-diethylaminoethyl)-6-iodoacridine-4-carboxamide (**106**) as starting material. Yield: 72%; melting point: 201-203˚C; $^1$H NMR (400 MHz, $d_6$-DMSO) δ 1.27 (t, 6H, *J* = 7 Hz), 3.24 (m, 4H), 3.38 (m, 2H), 3.97 (q, 2H, *J*= 6 Hz), 7.79 (dd, 1H, *J* = 8.5 and 7 Hz), 7.95 (d, 1H, *J* = 9 Hz), 8.02 (d, 1H, *J* = 9 Hz), 8.40 (d, 1H, *J* = 8.5 Hz), 8.77 (d, 1H, *J* = 7 Hz), 9.11 (s, 1H), 9.37 (s, 1H), 10.47 (bs, 1H), 11.33 (t, 1H, *J*= 6 Hz); $^{13}$C NMR (100 MHz, $d_6$-DMSO) δ 8.5 (2C), 34.4, 46.9 (2C), 49.8, 100.5, 124.5, 125.6, 126.5, 127.7, 129.8, 133.4, 134.9, 135.5, 137.2, 139.5, 145.3, 147.2, 165.6; IR (KBr) ν $cm^{-1}$: 1609, 1637, 2970, 3300-3500. Anal. Calculated for $C_{20}H_{22}IN_3O{\cdot}2HCl{\cdot}0.5\ H_2O$ : C, 45.39; H, 4.76; N, 7.94. Found : C, 45.23; H, 4.70; N, 7.72.

**EXAMPLE 25**

Syntheses of *N*-(2-diethylaminoethyl)-7-iodoacridine-4-carboxamide dihydrochloride (**111**) and of *N*-(2-diethylaminoethyl)-7-(tributylstannyl)acridine-4-carboxamide (**152**).

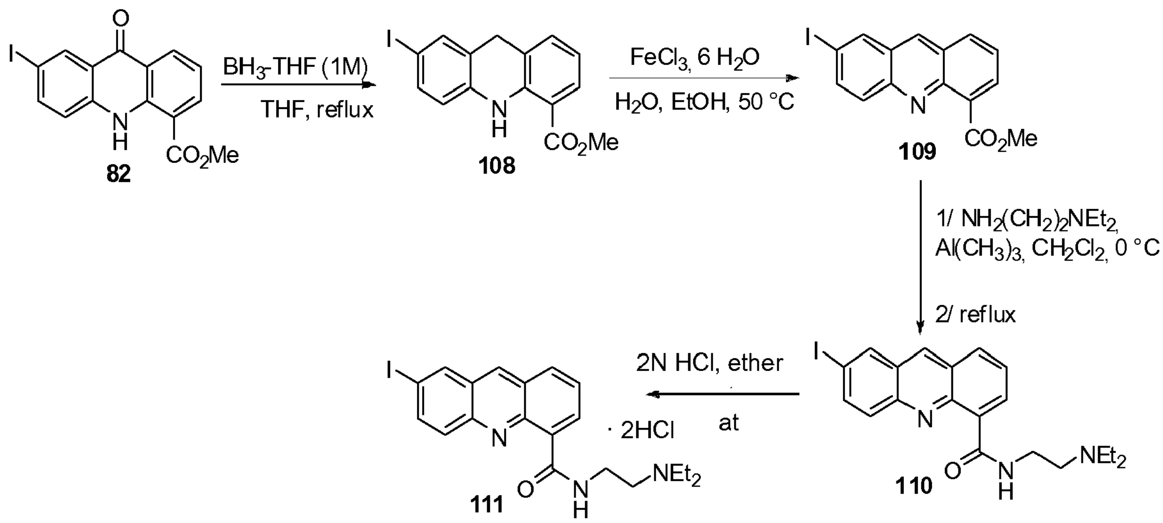


**Stage A: methyl 7-iodoacridane-4-carboxylate (108)**

The compound **108** was prepared according to the procedure described for the preparation of the compound **88,** using methyl 9,10-dihydro-7-iodo-9-oxoacridine-4-carboxylate (**82**) as starting material. Yield: 95%; Rf: 0.90 ($SiO_2$, dichloromethane); melting point: 122-124°C; $^1$H NMR (400 MHz, $CDCl_3$) δ 3.91 (s, 3H), 4.04 (s, 2H), 6.54 (d, 1H, *J* = 8.5 Hz), 6.79 (t, 1H, *J* = 7.5 Hz), 7.22 (d, 1H, *J* = 7 Hz), 7.37 (m, 2H), 7.77 (d, 1H, *J* = 7.5 Hz), 9.83 (bs, 1H); $^{13}$C NMR (100 MHz, $CDCl_3$) δ 30.4, 52.0, 82.9, 110.7, 116.7, 119.1, 121.1, 122.4, 129.4, 133.7, 136.0, 136.8, 138.8, 143.1, 168.9; IR (KBr) ν $cm^{-1}$: 1194, 1269, 1430, 1466, 1502, 1594, 1682, 2948, 3320; MS (m/z, %) 365 ($M^+$, 100), 333 (50), 206 (40), 177 (65), 151 (44), 127 (30), 103 (29), 89 (33), 75 (42).

**Stage B: methyl 7-iodoacridine-4-carboxylate (109)**

The compound **109** was prepared according to the procedure described for the preparation of the compound **89**, using methyl 7-iodoacridane-4-carboxylate (**108**) as starting material. Yield: 95%; very unstable product; $^1$H NMR (200 MHz, $CDCl_3$) δ 4.13 (s, 3H), 7.48 (dd, 1H, *J* = 8.5 and 7 Hz), 7.98 (m, 3H), 8.09 (d, 1H, *J* = 7 Hz), 8.29 (s, 1H), 8.53 (s, 1H).

**Stage C: *N*-(2-diethylaminoethyl)-7-iodoacridine-4-carboxamide (110)**

The compound **110** was prepared according to the procedure described for the preparation of the compound **2**, using methyl 7-iodoacridine-4-carboxylate (**109**) as starting material and heating the reaction medium at reflux for 6 hours. Yield: 53%; Rf: 0.33 ($Al_2O_3$, dichloromethane / ethanol (99/1, v/v)); melting point: 176-178°C; $^1$H NMR (200 MHz, $CDCl_3$) δ 1.26 (t, 6H, *J*= 7 Hz), 2.98 (m, 6H), 3.98 (m, 2H), 7.70 (dd, 1H, *J*= 8.5 and 7 Hz), 8.06 (d, 1H, *J*= 9 Hz), 8.16 (m, 2H), 8.47 (s, 1H), 8.78 (s, 1H), 8.98 (d, 1H, *J*= 7 Hz), 12.01 (bs, 1H); $^{13}$C NMR (100 MHz, $CDCl_3$) δ 10.8 (2C), 37.2, 47.2 (2C), 51.6, 92.2, 126.1, 127.0, 127.5, 128.3, 130.9, 132.6, 135.8, 136.4, 136.8, 139.8, 146.3, 146.5, 166.1; IR (KBr) ν $cm^{-1}$: 1457, 1515, 1562, 2965, 1665, 3300-3500; MS (m/z, %) 447 ($M^+$, 2), 332 (10), 305 (12), 177 (8), 86 (100), 58 (8).

**Stage D: *N*-(2-diethylaminoethyl)-7-iodoacridine-4-carboxamide dihydrochloride (111)**

The compound **111** was prepared according to the procedure described for the preparation of the compound **3**, using *N*-(2-diethylaminoethyl)-7-iodoacridine-4-carboxamide (**110**) as starting material. Yield: 67%; melting point: 213-215°C; $^1$H NMR (400 MHz, *$d_6$-DMSO*) δ 1.27 (t, 6H, *J* = 7 Hz), 3.25 (m, 4H), 3.41 (m, 2H), 3.92 (m, 2H), 7.79 (t,

1H, $J$ = 7.5 Hz), 8.19 (d, 1H, $J$ = 9 Hz), 8.36 (d, 1H, $J$ = 9 Hz), 8.41 (d, 1H, $J$ = 8.5 Hz), 8.74 (m, 2H), 9.28 (s, 1H), 10.57 (bs, 1H), 11.32 (bs, 1H); $^{13}$C NMR (100 MHz, $d_6$-*DMSO*) δ 8.5 (2C), 34.4, 46.9 (2C), 49.7, 93.3, 125.8, 126.5, 127.2, 128.0, 130.6, 133.3, 135.1, 136.7, 137.8, 139.8, 145.3, 145.7; IR (KBr) ν $cm^{-1}$: 1403, 1585, 1628, 2665, 2953, 3222, 3200-3500. Anal. Calculated for $C_{20}H_{22}IN_3O \cdot 2HCl \cdot 2.5H_2O$ : C, 42.50; H, 5.17; N, 7.43. Found : C, 42.78; H, 4.96; N, 7.35.

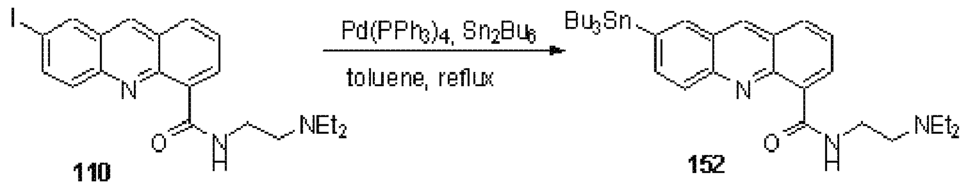


110 152

**Stage E : *N*-(2-diethylaminoethyl)-7-(tributylstannyl)acridine-4-carboxamide (152)**

The compound **152** was prepared according to the procedure described for the preparation of the compound **32**, using *N*-(2-diethylaminoethyl)-7-iodoacridine-4-carboxamide (**110**) as starting material and heating the reaction medium at reflux for 5 hours. The residue obtained is chromatographed on a column of alumina eluted with a mixture of ethyl acetate and pentane (7/3, v/v). Yield: 40%; Rf: 0.29 ($Al_2O_3$, ethyl acetate / pentane (7/3, v/v)); viscous liquid; $^1$H NMR (400 MHz, $CDCl_3$) δ 0.90 (t, 9H, $J$ = 7 Hz), 1.13 (t, 6H, $J$= 7 Hz), 1.21 (m, 6H), 1.37 (st, 6H, $J$= 7 Hz), 1.60 (m, 6H), 2.74 (q, 4H, $J$= 7 Hz), 2.85 (t, 2H, $J$ = 6.5 Hz), 3.80 (q, 2H, $J$ = 6.5 Hz), 7.62 (m, 1H), 7.90 (d, 1H, $J$ = 8.5 Hz), 8.12 (m, 2H), 8.21 (d, 1H, $J$ = 8.5 Hz), 8.82 (s, 1H), 8.96 (d, 1H, $J$ = 7 Hz), 12.04 (bs, 1H); $^{13}$C NMR (100 MHz, $CDCl_3$) δ 9.8 (3C, $^1J_{Sn-C}$ = 333 Hz), 11.9 (2C), 13.7 (3C), 27.4 (3C, $^3J_{Sn-C}$ = 55 Hz), 29.1 (3C, $^2J_{Sn-C}$ = 20 Hz), 38.1, 47.1 (2C), 52.0, 125.2, 126.0, 126.9, 127.7, 128.6, 132.4, 135.0, 136.7, 136.9, 138.0, 141.4, 146.3, 147.7, 166.0; IR ($CCl_4$) ν $cm^{-1}$: 1464, 1514, 1559, 1657, 2854, 2873, 2928, 2961, 3150-3250; ESI-MS m/z 612.2 $[M+H]^+$.

**EXAMPLE 26**

Synthesis of *N*-(2-diethylaminoethyl)-8-iodoacridine-4-carboxamide dihydrochloride (**115**)

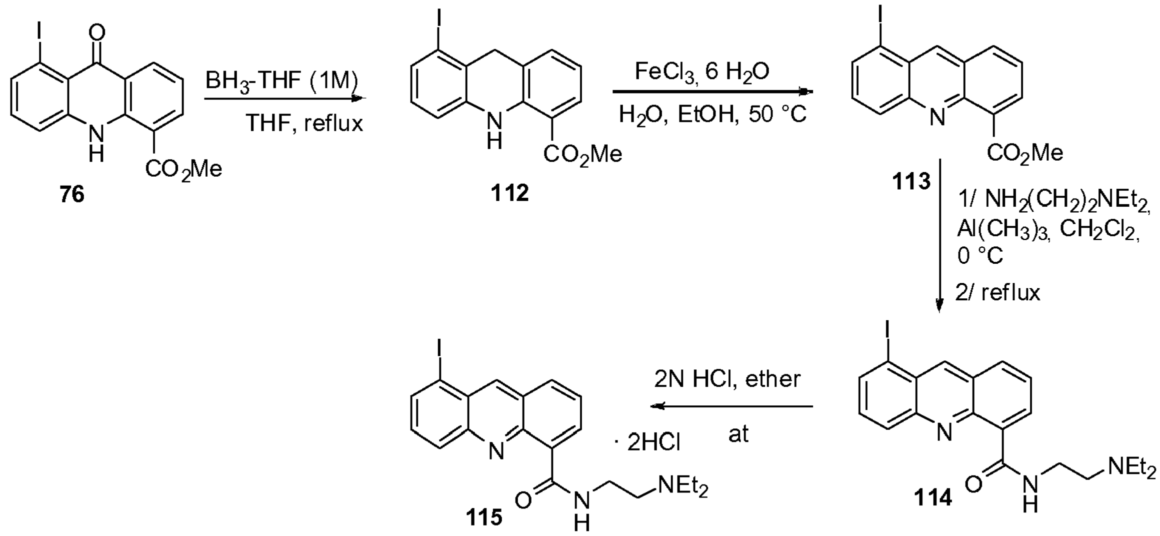


76 112 113 114 115

**Stage A: methyl 8-iodoacridane-4-carboxylate (112)**

The compound **112** was prepared according to the procedure described for the preparation of the compound **88**, using methyl 9,10-dihydro-8-iodo-9-oxoacridine-4-carboxylate (**76**) as starting material. Yield: 92%; Rf: 0.97 ($SiO_2$, dichloromethane); melting point: 108-110°C; $^1$H NMR (400 MHz, $CDCl_3$) δ 3.90 (s, 3H), 4.09 (s, 2H), 6.69 (d, 1H, $J$ = 8

Hz), 6.77 (m, 2H), 7.24 (d, 1H, *J* = 7.5 Hz), 7.36 (d, 1H, *J* = 8 Hz), 7.77 (d, 1H, *J* = 8 Hz), 9.77 (bs, 1H); $^{13}$C NMR (100 MHz, $CDCl_3$) δ 37.5, 51.9, 101.2, 110.3, 114.8, 115.7, 119.0, 122.5, 128.9, 129.4, 131.4, 133.9, 139.5, 142.8, 168.9; IR (KBr) ν $cm^{-1}$: 1262, 1442, 1501, 1603, 1685, 2960, 3319; MS (m/z, %) 365 ($M^+$, 100), 333 (53), 332 (55), 206 (81), 178 (90), 177 (95), 151 (71), 127 (42), 103 (42), 89 (32), 75 (66), 63 (42).

**Stage B: methyl 8-iodoacridine-4-carboxylate (113)**

The compound **113** was prepared according to the procedure described for the preparation of the compound **89**, using methyl 8-iodoacridane-4-carboxylate (**112**) as starting material. Yield: 90%; very unstable product; $^1$H NMR (200 MHz, $CDCl_3$) δ 4.11 (s, 3H), 7.51 (dd, 1H, *J* = 8.5 and 7 Hz), 7.62 (dd, 1H, *J* = 8.5 and 7 Hz), 8.22 (m, 3H), 8.32 (d, 1H, *J*= 8.5 Hz), 9.02 (s, 1H).

**Stage C: N-(2-diethylaminoethyl)-8-iodoacridine-4-carboxamide (114)**

The compound **114** was prepared according to the procedure described for the preparation of the compound **2**, using methyl 8-iodoacridine-4-carboxylate (**113**) as starting material and heating the reaction medium at reflux for 16 hours in anhydrous dichloromethane. Yield: 55%; Rf 0.35 ($Al_2O_3$, dichloromethane / ethanol (99/1, v/v)); melting point: 78-80˚C; $^1$H NMR (400 MHz, $CDCl_3$) δ 1.16 (t, 6H, *J*= 7 Hz), 2.80 (q, 4H, *J*= 7 Hz), 2.91 (m, 2H), 3.85 (m, 2H), 7.50 (t, 1H, *J*= 8 Hz), 7.69 (t, 1H, *J*= 7.5 Hz), 8.16 (m, 2H), 8.27 (d, 1H, *J* = 8.5 Hz), 8.96 (s, 1H), 8.98 (d, 1H, *J* = 7 Hz), 11.76 (bs, 1H); $^{13}$C NMR (100 MHz, $CDCl_3$) δ 11.4 (2C), 37.7, 47.1 (2C), 51.8, 98.4, 126.1, 127.5, 127.7, 128.2, 130.2, 131.6, 132.4, 135.9, 137.4, 142.4, 146.7, 147.4, 165.6; IR (KBr) ν $cm^{-1}$: 1508, 1546, 1654, 2925, 3200; MS (m/z, %) 447 ($M^+$, 1), 177 (11), 86 (100), 58 (11).

**Stage D: *N*-(2-diethylaminoethyl)-8-iodoacridine-4-carboxamide dihydrochloride (115)**

The compound **115** was prepared according to the procedure described for the preparation of the compound **3**, using *N*-(2-diethylaminoethyl)-8-iodoacridine-4-carboxamide (**114**) as starting material. Yield: 72%; melting point: 128-130˚C; $^1$H NMR (400 MHz, *$d_6$-DMSO*) δ 1.28 (t, 6H, *J* = 7 Hz), 3.25 (m, 4H), 3.41 (m, 2H), 3.99 (m, 2H), 7.71 (t, 1H, *J* = 7.5 Hz), 7.82 (t, 1H, *J* = 7 Hz), 8.33 (d, 1H, *J* = 7 Hz), 8.60 (m, 2H), 8.76 (d, 1H, *J* = 7 Hz), 9.25 (s, 1H), 10.73 (m, 1H), 11.26 (bs, 1H); $^{13}$C NMR (100 MHz, *$d_6$-DMSO*) δ 8.5 (2C), 34.4, 46.9 (2C), 49.7, 99.2, 125.9, 127.1, 127.3, 127.6, 130.0, 132.6, 133.4, 135.3, 137.8, 142.7, 145.6, 147.1, 165.5; IR (KBr) ν $cm^{-1}$: 1507, 1559, 1653, 3300-3500. Anal. Calculated for $C_{20}H_{22}IN_3O{\cdot}2HCl{\cdot}1.5H_2O$ : C, 43.90; H, 4.97; N, 7.68. Found : C, 43.87; H, 4.77; N, 7.38.

**<u>EXAMPLE 27</u>**

Synthesis of *N*-(2-diethylaminoethyl)-2-iodoacridine-9-carboxamide dihydrochloride (**120**)

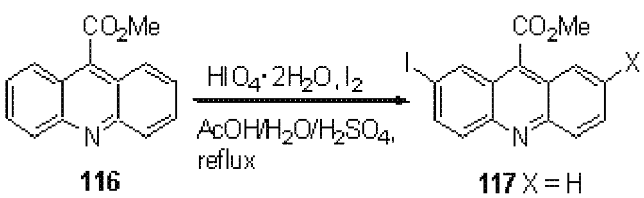


**116** **117** X = H **118** X = I

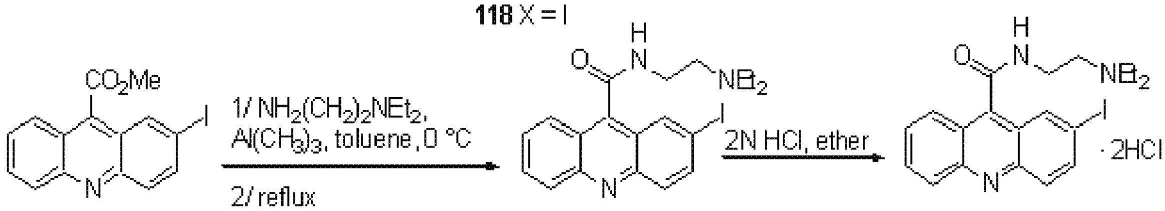


**117** **119** **120**

**Stage A : methyl 2-iodoacridine-9-carboxylate (117) and methyl 2,7-diiodoacridine-9-carboxylate (118)**

Periodic acid dihydrate (76 mg, 0.33 mmol) and diiodine (216 mg, 0.85 mmol) are added to a solution of methyl acridine-9-carboxylate (**116**) (Renotte, R., Sarlet, G., Thunus, L. and Lejeune, R., Luminescence, 2000, 15, 311-320) (0.40 g, 1.69 mmol) in a mixture of concentrated sulphuric acid (80 μl), water (320 μl) and acetic acid (1 ml). The solution is stirred at reflux for 8 hours. After returning to ambient temperature, water (2 ml) and then a saturated aqueous sodium carbonate solution (10 ml) are added. The medium is extracted with dichloromethane (3 x 20 ml). The organic phase is dried with magnesium sulphate, filtered and then evaporated to dryness. The residue obtained is purified by chromatography on a column of silica eluted with dichloromethane, to result, in order of elution, in the following products:

**Methyl 2,7-diiodoacridine-9-carboxylate (118)**

(50 mg, 0.10 mmol); yield: 6%; Rf: 0.50 ($SiO_2$, dichloromethane); melting point: 201-203°C; $^1H$ NMR (400 MHz, $CDCl_3$) δ 4.24 (s, 3H), 8.04 (m, 4H), 8.42 (d, 2H, *J*= 2 Hz); $^{13}C$ NMR (100 MHz, $CDCl_3$) δ 53.5, 94.7 (2C), 123.9 (2C), 130.8 (2C), 134.1 (2C), 139.8, 146.8 (2C), 166.7; IR (KBr) ν $cm^{-1}$: 1220, 1720; MS (m/z, %) 489 ($M^+$, 100), 458 (29), 430 (18), 347 (10), 303 (17), 164 (14).

**Methyl 2-iodoacridine-9-carboxylate (117)**

(98 mg, 0.27 mmol); yield: 16%; Rf 0.30 ($SiO_2$, dichloromethane); melting point: 106-108°C; $^1H$ NMR (400 MHz, $CDCl_3$) δ 4.20 (s, 3H), 7.58 (m, 1H), 7.79 (m, 1H), 7.95 (m, 2H), 7.98 (d, 1H, *J* = 8 Hz), 8.20 (d, 1H, *J* = 8.5 Hz), 8.39 (s, 1H); $^{13}C$ NMR (100 MHz, $CDCl_3$) δ 53.2, 93.6, 122.5, 123.7, 125.3, 127.8, 130.0, 130.7, 131.3, 134.0, 135.4, 138.9, 147.1, 148.8, 167.4; IR (KBr) ν $cm^{-1}$: 1218, 1728; MS (m/z, %) 363 ($M^+$, 100), 332 (33), 304 (28), 177 (33).

**Stage B : *N*-(2-diethylaminoethyl)-2-iodoacridine-9-carboxamide (119)**

The compound **119** was prepared according to the procedure described for the preparation of the compound **2**, using methyl 2-iodoacridine-9-carboxylate (**117**) as starting material and heating the reaction medium at reflux for 24 hours in anhydrous toluene. Yield: 58%; Rf: 0.61 ($Al_2O_3$, dichloromethane / ethanol (99/1, v/v)); melting point: 83-85°C; $^1H$ NMR (400 MHz, $CDCl_3$) δ 1.00 (t, 6H, *J* = 7 Hz), 2.58 (q, 4H, *J* = 7 Hz), 2.78 (t, 2H, *J*= 6 Hz), 3.79 (q, 2H, *J*= 6 Hz), 6.85 (m, 1H), 7.55 (m, 1H), 7.80 (m, 1H), 7.91 (d, 1H, *J* = 9 Hz), 7.96 (d, 1H, *J* = 9 Hz), 8.08 (d, 1H, *J* = 9 Hz), 8.17 (d, 1H, *J* = 8.5 Hz), 8.48 (s, 1H); $^{13}C$ NMR (50 MHz, $CDCl_3$) δ 11.6 (2C), 37.6, 46.8 (2C), 51.8, 93.1, 122.3, 123.6, 125.6, 127.3, 129.7, 130.7, 131.1, 134.2, 138.9, 140.1, 147.2, 148.7, 166.5; IR (KBr) ν $cm^{-1}$: 1636, 2956, 3200-3400; MS (m/z, %) 447 ($M^+$, 1), 177 (3), 86 (100), 58 (9).

**Stage C: *N*-(2-diethylaminoethyl)-2-iodoacridine-9-carboxamide dihydrochloride (120)**

The compound **120** was prepared according to the procedure described for the preparation of the compound **3**, using N-(2-diethylaminoethyl)-2-iodoacridine-9-carboxamide (**119**) as starting material. Yield: 78%; melting point: 121-123°C; $^1H$ NMR (400 MHz, *$d_6$-DMSO*) δ 1.29 (t, 6H, *J* = 7 Hz), 3.22 (m, 4H), 3.41 (m, 2H), 3.96 (m, 2H), 7.79 (m, 1H), 8.06 (t, 1H, *J* = 7.5 Hz), 8.13 (m, 2H), 8.24 (d, 1H, *J* = 9 Hz), 8.33 (d, 1H, *J*= 9 Hz), 8.45 (s, 1H), 9.55 (t, 1H, *J* = 6 Hz), 10.79 (m, 1H); $^{13}C$ NMR (50 MHz, $d_6$-DMSO) δ 8.5 (2C), 34.3, 46.6 (2C), 49.3, 94.6, 122.0, 123.2, 126.2, 126.7, 128.8, 128.5, 133.0, 133.9, 140.6, 142.9, 144.4, 146.0, 165.3; IR (KBr) ν $cm^{-1}$: 1420, 1464, 1654, 2400-2600, 2976, 3200-3500. Anal. Calculated for $C_{20}H_{22}IN_3O \cdot 2HCl \cdot 1.5H_2O$ : C, 43.90; H, 4.97; N, 7.68. Found : C, 43.73; H, 4.72; N, 7.58.

<u>**EXAMPLE 28**</u>

Synthesis of *N*-(2-diethylaminoethyl)-2,7-diiodoacridine-9-carboxamide dihydrochloride (**122**)

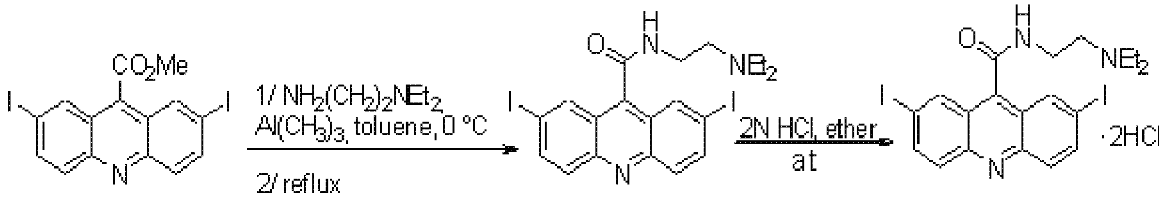


118 121 122

**Stage A: *N*-(2-diethylaminoethyl)-2,7-diiodoacridine-9-carboxamide (121)**

The compound **121** was prepared according to the procedure described for the preparation of the compound **2**, using methyl 2,7-diiodoacridine-9-carboxylate (**118**) as starting compound and heating the reaction medium at reflux for 20 hours in anhydrous toluene. Yield: 57%; Rf 0.72 ($Al_2O_3$, dichloromethane / ethanol (99/1, v/v)); melting point: 209-211°C; $^1H$ NMR (400 MHz, $CDCl_3$) δ 1.03 (t, 6H, *J*= 7 Hz), 2.63 (q, 4H, *J*= 7 Hz), 2.80 (t, 2H, *J*= 6 Hz), 3.81 (q, 2H, *J*= 6 Hz), 6.90 (m, 1H), 7.88 (d, 2H, *J*= 9 Hz), 7.98 (d, 2H, *J* = 9 Hz), 8.46 (s, 2H); $^{13}C$ NMR (100 MHz, $CDCl_3$) δ 11.7 (2C), 37.8, 46.7 (2C), 51.7, 94.1 (2C), 123.6 (2C), 130.9 (2C), 134.4 (2C), 138.9, 139.3 (2C), 147.1 (2C), 165.9; IR (KBr) ν $cm^{-1}$: 1637, 2924, 3300-3500; MS (m/z, %) 573 ($M^+$, 1), 458 (2), 430 (3), 303 (7), 86 (100), 58 (12).

**Stage B: *N*-(2-diethylaminoethyl)-2,7-diiodoacridine-9-carboxamide dihydrochloride (122)**

The compound **122** was prepared according to the procedure described for the preparation of the compound **3**, using *N*-(2-diethylaminoethyl)-2,7-diiodoacridine-9-carboxamide (**121**) as starting material. Yield: 92%; melting point: 224-226°C; $^1H$ NMR (200 MHz, $d_6$-DMSO) δ 1.31 (t, 6H, *J*= 7 Hz), 3.24 (m, 4H), 3.42 (m, 2H), 3.96 (m, 2H), 8.03 (d, 2H, *J* = 9 Hz), 8.21 (d, 2H, *J* = 9 Hz), 8.42 (s, 2H), 9.56 (m, 1H), 10.74 (bs, 1H); $^{13}C$ NMR (50 MHz, $d_6$-DMSO) δ 8.6 (2C), 34.4, 46.6 (2C), 49.3, 95.3 (2C), 123.4 (2C), 129.6 (2C), 133.9 (3C), 140.3 (2C), 145.5 (2C), 165.0; IR (KBr) ν $cm^{-1}$: 1420, 1663, 2575, 3200-3500. Anal. Calculated for $C_{20}H_{22}IN_3O \cdot 2HCl$ : C, 37.18; H, 3.59; N, 6.50. Found : C, 37.57; H, 3.76; N, 6.18.

**<u>EXAMPLE 29</u>**

Synthesis of *N*-(2-diethylaminoethyl)-2-iodo-9-(4-methanesulphonamido-2-methoxyanilino)acridine-4-carboxamide dihydrochloride (**125**)

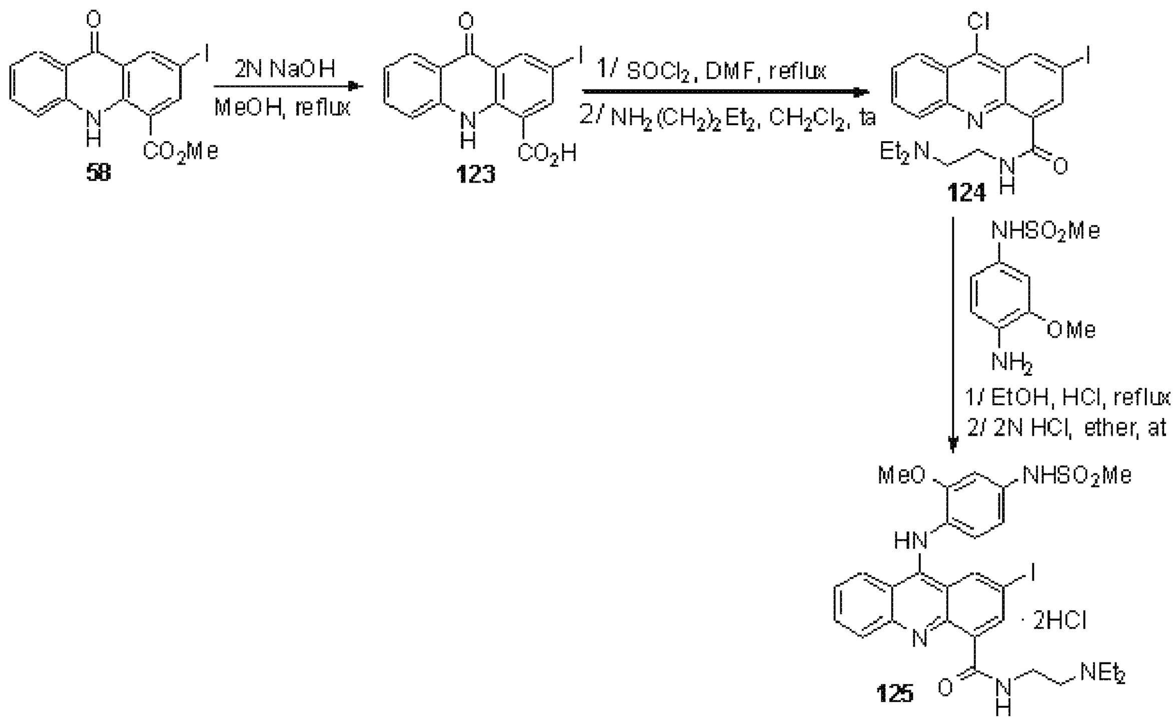


58 123 124 125

### Stage A : 9,10-dihydro-2-iodo-9-oxoacridine-4-carboxylic acid (123)

A 2N aqueous sodium hydroxide solution (30 ml) is added to a solution of ester **58** (0.30 g, 0.79 mmol) in ethanol (30 ml). The reaction mixture is brought to reflux for 10 minutes and then filtered while hot. After returning to ambient temperature, the filtrate is acidified with glacial acetic acid (pH = 5), concentrated to 2/3 and cooled in an ice bath until precipitation has occurred. The precipitate formed is filtered off and washed with water, then taken up in anhydrous ethanol (30 ml) and, finally, dried under vacuum, to result in the acid **123** (0.26 g, 0.71 mmol); yield: 90%; melting point: > 400˚C; $^{1}$H NMR (400 MHz, $d_6$-DMSO) δ 7.31 (t, 1H, *J* = 7.5 Hz), 7.64 (d, 1H, *J* = 7.5 Hz), 7.77 (t, 1H, *J* = 7.5 Hz), 8.25 (d, 1H, *J* = 7.5 Hz), 8.59 (m, 2H), 14.45 (bs, 1H); $^{13}$C NMR (100 MHz, $d_6$-DMSO) δ 83.3, 118.4, 120.4, 121.5, 123.1, 124.8, 126.0, 133.9, 136.5, 139.9, 140.7, 143.2, 167.8, 175.5; IR (KBr) ν $cm^{-1}$: 1165, 1514, 1615, 3000-3400; MS (m/z, %) 365 ($M^+$, 5), 164 (10), 84 (27), 69 (64), 55 (100).

### Stage B: 9-chloro-N-(2-diethylaminoethyl)-2-iodoacridine-4-carboxamide (124)

A suspension comprising 9,10-dihydro-2-iodo-9-oxoacridine-4-carboxylic acid (**123**) (0.88 g, 2.41 mmol), thionyl chloride (5 ml) and dimethylformamide (1 drop) is brought to reflux for 45 minutes. The thionyl chloride is driven off by evaporating under vacuum (temperature of the bath of the evaporator < 40˚C). The acid chloride thus formed is taken up in anhydrous toluene (10 ml) in order to be once again evaporated to dryness. It is subsequently cooled to -5˚C and then a solution of *N,N*-diethylethylenediamine (1.32 ml, 9.24 mmol) in anhydrous dichloromethane (10 ml) is added, under argon, at -5˚C. The reaction medium is stirred at ambient temperature for 2 hours. The organic phase is washed with a 10% aqueous sodium carbonate solution (2 x 20 ml) and a saturated aqueous sodium chloride solution (20 ml). The organic phase is dried over magnesium sulphate, filtered and evaporated to dryness. The chlorinated derivative **124** obtained is very unstable and is used without further purification (0.93 g, 1.93 mmol); yield: 80%; $^{1}$H NMR (200 MHz, $CDCl_3$) δ 1.23 (t, 6H, *J* = 7 Hz), 2.95 (m, 6H), 3.93 (m, 2H), 7.70 (t, 1H, *J* = 7.5 Hz), 7.91 (t, 1H, *J*= 7.5 Hz), 8.38 (m, 2H), 8.98 (d, 1H, *J*= 2 Hz), 9.12 (d, 1H, *J*= 2 Hz), 11.77 (bs, 1H).

### Stage C: *N*-(2-diethylaminoethyl)-2-iodo-9-(4-methanesulphonamido-2-methoxyanilino)acridine-4-carboxamide dihydrochloride (125)

4-Methanesulphonamido-2-methoxyaniline (Ferlin, M. G., Marzano, C., Chiarelotto, G., Baccichetti, F. and Bordin, F., Eur. J. Med. Chem., 2000, 35, 827-837) (97 mg, 0.45 mmol) and concentrated hydrochloric acid (40 μl) are added to the compound 9-chloro-*N*-(2-diethylaminoethyl)-2-iodoacridine-4-carboxamide (**124**) (0.22 g, 0.46 mmol) in solution in anhydrous ethanol (10 ml). The reaction medium is stirred at reflux for 17 hours. After returning to ambient

temperature, a saturated aqueous sodium carbonate sodium (20 ml) is added. The aqueous phase is subsequently extracted with dichloromethane (3 x 50 ml). The organic phases are combined, dried over magnesium sulphate, filtered and then evaporated to dryness. The precipitate obtained is purified on a column of alumina eluted with a dichloromethane / ethanol, 99/1 (v/v), mixture. The red solid obtained is taken up in dichloromethane (5 ml) and then a solution of hydrochloric acid (2N) in anhydrous ether (5 ml). The mixture is stirred at ambient temperature for 5 minutes and then the solvents are driven off by evaporation under vacuum. The residue is taken up in anhydrous ether and stirred at ambient temperature under argon overnight. The hydrochloride obtained is filtered off and then washed with anhydrous ether, to result in the compound **125** (96 mg, 0.13 mmol). Yield: 28%; melting point: 235-237°C; $^1$H NMR (400 MHz, $d_6$-DMSO) δ 1.29 (t, 6H, *J* = 7 Hz), 3.14 (s, 3H), 3.24 (m, 4H), 3.37 (m, 2H), 3.48 (s, 3H), 3.80 (m, 2H), 7.04 (d, 1H, *J* = 8.5 Hz), 7.09 (s, 1H), 7.46 (t, 1H, *J* = 7.5 Hz), 7.52 (d, 1H, *J* = 8 Hz), 8.00 (t, 1H, *J* = 7.5 Hz), 8.16 (d, 1H, *J* = 8.5 Hz), 8.20 (bs, 1H), 8.85 (s, 1H), 8.89 (bs, 1H), 9.80 (bs, 1H), 10.20 (s, 1H), 10.75 (bs, 1H), 11.75 (m, 1H), 13.83 (bs, 1H); $^{13}$C NMR (50 MHz, $d_6$-DMSO) δ 8.5 (2C), 34.7, 39.5, 46.6 (2C), 49.8, 56.0, 87.0, 104.1, 112.0, 113.7, 116.0, 120.4, 121.8, 123.8, 124.8, 125.2, 127.7, 136.2, 137.3, 137.6, 139.1, 140.4, 142.7, 153.6, 155.3, 166.3; IR (KBr) ν cm$^{-1}$: 1150, 1323, 1510, 1618, 2933, 3200-3500; MS (of the basic form) (m/z, %) 661 (M$^+$, 1), 582 (11), 509 (11), 86 (100), 58 (21). Anal. Calculated for $C_{28}H_{32}IN_5O_4S{\cdot}2HCl{\cdot}2.5H_2O$ : C, 43.14; H, 5.04; N, 8.98. Found : C, 43.06; H, 4.74; N, 8.90.

## EXAMPLE 30

Synthesis of *N*-(2-diethylaminoethyl)-5-iodo-9-(4-methanesulphonamido-2-methoxyanilino)acridine-4-carboxamide dihydrochloride (**127**)

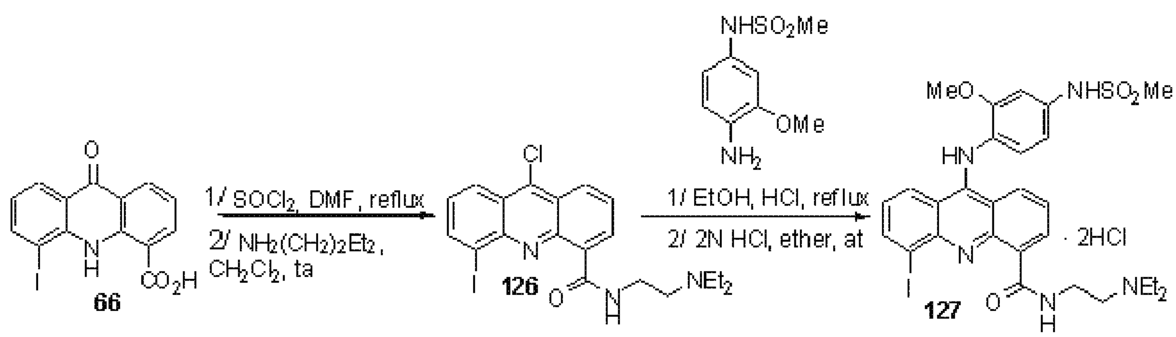

66 126 127

**Stage A: 9-chloro-*N*-(2-diethylaminoethyl)-5-iodoacridine-4-carboxamide (126)**

The compound **126** was prepared according to the procedure described for the preparation of the compound **124**, using 9,10-dihydro-5-iodo-9-oxoacridine-4-carboxylic acid (**66**) as starting material. Yield: 93%; very unstable compound; $^1$H NMR (200 MHz, $CDCl_3$) δ 1.38 (t, 6H, *J*= 7 Hz), 3.17 (q, 4H, *J*= 7 Hz), 3.37 (m, 2H), 4.22 (m, 2H), 7.41 (dd, 1H, *J* = 9 and 7 Hz), 7.78 (dd, 1H, *J* = 8.5 and 7 Hz), 8.44 (d, 1H, *J* = 8.5 Hz), 8.51 (d, 1H, *J* = 7 Hz), 8.60 (d, 1H, *J*= 8.5 Hz), 8.99 (d, 1H, *J* = 7 Hz), 12.40 (m, 1H).

**Stage B: *N*-(2-diethylaminoethyl)-5-iodo-9-(4-methanesulphonamido-2-methoxyanilino)acridine-4-carboxamide dihydrochloride (127)**

The compound **127** was prepared according to the procedure described for the preparation of the compound **125**, using 9-chloro-*N*-(2-diethylaminoethyl)-5-iodoacridine-4-carboxamide (**126**) as starting material. Yield: 38%; melting point: 212-214°C; $^1$H NMR (400 MHz, $d_6$-DMSO) δ 1.28 (t, 6H, *J*= 7 Hz), 3.13 (s, 3H), 3.25 (q, 4H, *J*= 7 Hz), 3.40 (m, 2H), 3.48 (s, 3H), 3.85 (m, 2H), 7.02 (m, 2H), 7.21 (t, 1H, *J* = 8 Hz), 7.55 (m, 2H), 8.39 (m, 1H), 8.54 (m, 2H), 8.89 (d, 1H, *J*= 7 Hz), 10.06 (s, 1H), 10.25 (bs, 1H), 10.81 (m, 1H), 12.00 (bs, 1H), 14.70 (bs, 1H); $^{13}$C NMR (100 MHz, $d_6$-DMSO) δ 8.3 (2C), 34.4, 39.5, 46.4 (2C), 49.5, 55.7, 89.5, 103.7, 111.6, 113.8, 114.2, 117.8, 122.7, 123.5, 125.2, 125.4, 127.5, 129.6, 135.9, 138.9, 139.3, 140.0, 146.0, 153.2, 156.9, 167.6; IR (KBr) ν cm$^{-1}$: 1151, 1325, 1513, 1585, 1613, 2900-3100, 3200-3500; MS (of the basic form) (m/z, %) 661 (M$^+$, 1), 582 (9), 509 (15), 446 (13), 383 (12), 86 (100). Anal. Calculated for $C_{28}H_{32}IN_5O_4S{\cdot}2HCl{\cdot}3H_2O$ : C, 42.65; H, 5.11; N, 8.88. Found : C, 42.72; H, 4.81; N, 8.68.

## EXAMPLE 31

Syntheses of *N*-(2-diethylaminoethyl)-7-iodo-9-(4-methanesulphonamido-2-methoxyanilino)acridine-4-carboxamide dihydrochloride (**129**) and of *N*-(2-diethylaminoethyl)-7-(tributylstannyl)-9-(4-methanesulphonamido-2-methoxyanilino) acridine-4-carboxamide (**131**)

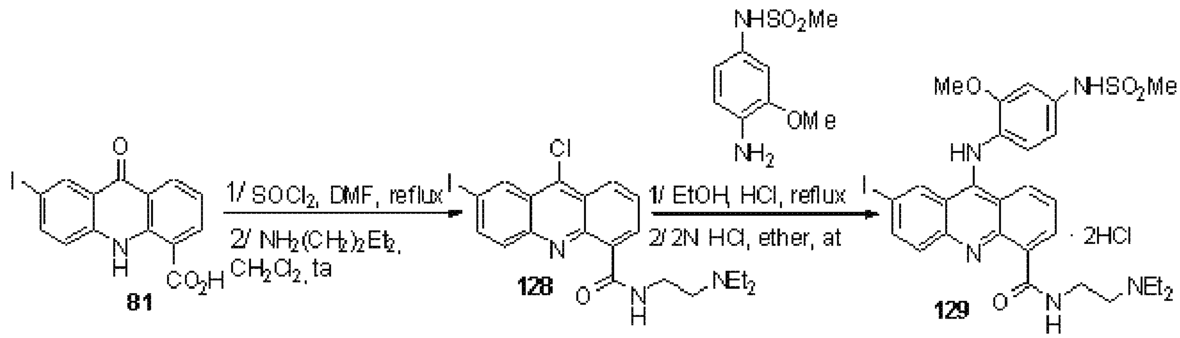


**81** **128** **129**

### Stage A: 9-chloro-*N*-(2-diethylaminoethyl)-7-iodoacridine-4-carboxamide (128)

The compound **128** was prepared according to the procedure described for the preparation of the compound **124**, using 9,10-dihydro-7-iodo-9-oxoacridine-4-carboxylic acid (**81**) as starting material. Yield: 96%; very unstable compound; $^1$H NMR (200 MHz, $CDCl_3$) δ 1.12 (t, 6H, *J*= 7 Hz), 2.75 (m, 6H), 3.77 (q, 2H, *J*= 6 Hz), 7.72 (dd, 1H, *J*= 8.5 and 7 Hz), 8.00 (m, 2H), 8.49 (d, 1H, *J* = 8.5 Hz), 8.74 (s, 1H), 8.98 (d, 1H, *J* = 7 Hz), 11.60 (m, 1H).

### Stage B: *N*-(2-diethylaminoethyl)-7-iodo-9-(4-methanesulphonamido-2-methoxyanilino)acridine-4-carboxamide dihydrochloride (129)

The compound **129** was prepared according to the procedure described for the preparation of the compound **125**, using 9-chloro-*N*-(2-diethylaminoethyl)-7-iodoacridine-4-carboxamide (**128**) as starting material. Yield: 33%; melting point: 208-210˚C; $^1$H NMR (400 MHz, $d_6$-DMSO) δ 1.28 (t, 6H, *J* = 7 Hz), 3.13 (s, 3H), 3.24 (m, 4H), 3.37 (m, 2H), 3.49 (s, 3H), 3.80 (m, 2H), 7.06 (d, 1H, *J*= 8.5 Hz), 7.09 (s, 1H), 7.54 (m, 2H), 7.97 (d, 1H, *J* = 9 Hz), 8.19 (d, 1H, *J* = 9 Hz), 8.58 (m, 2H), 8.70 (d, 1H, *J* = 7.5 Hz), 9.95 (t, 1H, *J*= 6 Hz), 10.26 (s, 1H), 10.85 (m, 1H), 11.80 (m, 1H), 14.04 (bs, 1H); $^{13}$C NMR (100 MHz, $d_6$-DMSO) δ 8.3 (2C), 34.3, 39.5, 46.4 (2C), 49.5, 55.7, 89.2, 103.7, 111.7, 114.2, 114.8, 119.9, 121.9, 122.7, 123.4, 127.5, 129.1, 133.3, 135.7, 137.9, 138.2, 140.2, 143.2, 153.3, 155.0, 167.2; IR (KBr) ν $cm^{-1}$: 1150, 1324, 1511, 1586, 1618, 2926, 3300-3500; MS (of the basic form) (m/z, %) 661 ($M^+$, 7), 582 (47), 509 (62), 466 (29), 439 (21), 383 (17), 86 (100), 58 (13). Anal. Calculated for $C_{29}H_{32}IN_5O_4S{\cdot}2HCl{\cdot}2H_2O$: C, 43.65; H, 4.97; N, 9.09. Found: C, 43.37; H, 4.64; N, 8.93.

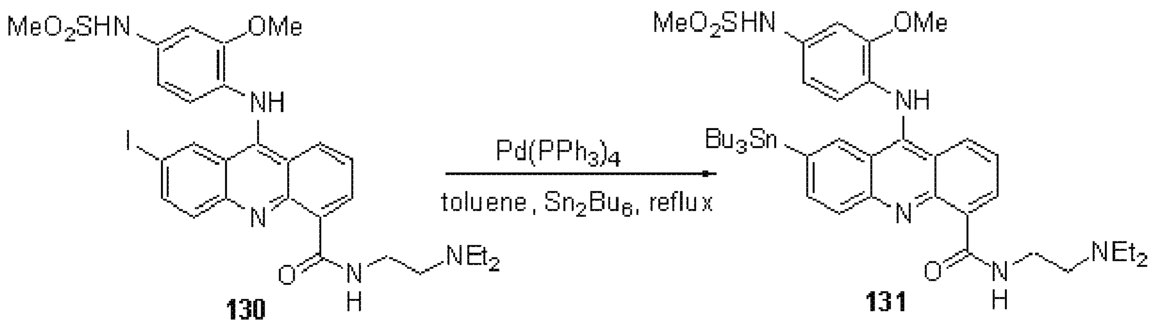


**130** **131**

**Stage C: *N*-(2-diethylaminoethyl)-7-(tributylstannyl)-9-(4-methanesulphon-amido-2-methoxyanilino)acridine-4-carboxamide (131)**

The compound **131** was prepared according to the procedure described for the preparation of the compound **32**, using *N*-(2-diethylaminoethyl)-7-iodo-9-(4-methanesulphonamido-2-methoxyanilino)acridine-4-carboxamide (**130**) (freshly prepared from the dihydrochloride **129** treated with a saturated aqueous sodium carbonate solution and then extracted with dichloromethane) as starting material and heating at reflux for 4.5 hours. The residue obtained is chromatographed on a column of alumina eluted with a mixture of ethyl acetate and methanol (96/4, v/v). Yield: 34%; Rf: 0.48 ($Al_2O_3$, ethyl acetate / ethanol (96/4, v/v)); melting point: 139-141˚C; IR (KBr) ν $cm^{-1}$: 1152, 1457, 1507, 1592, 2924; ESI-MS m/z 826.5 $[M+H]^+$.

**EXAMPLE 32**

Synthesis of *N*-(2-diethylaminoethyl)-9-(5-iodo-4-methanesulphonamido-2-methoxyanilino)acridine-4-carboxamide dihydrochloride (**137**)

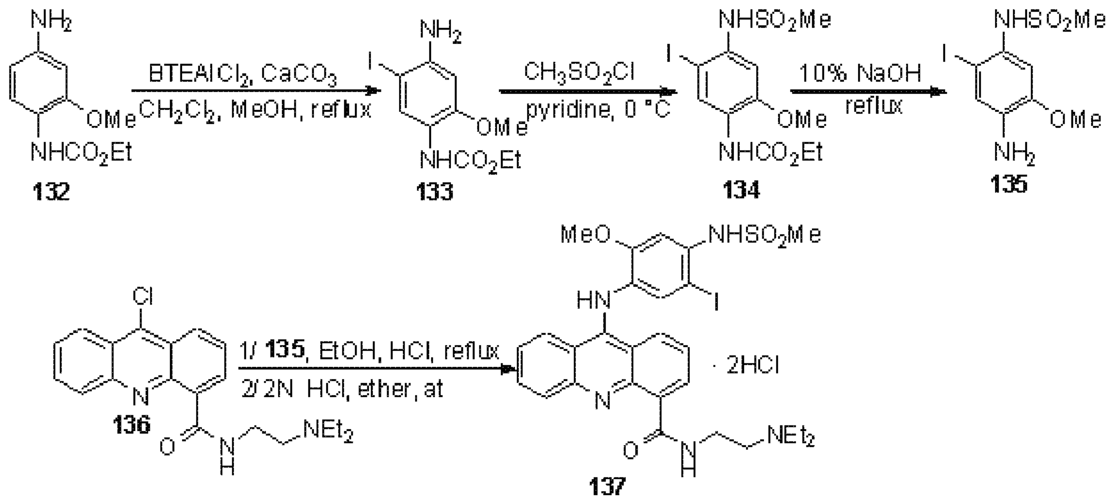


132 133 134 135

136 137

**Stage A: ethyl N-(4-amino-5-iodo-2-methoxyphenyl)carbamate (133)**

$BTEAICl_2$ (Kajigaeshi, S., Kakinami, T., Yamasaki, H., Fujisaki, S. and Okamoto, T., Bull. Chem. Soc. Jpn., 1988, 61, 600-602) (0.81 g, 2.09 mmol) and calcium carbonate (0.32 mg, 3.08 mmol) are added to the aniline derivative **132** (Ferlin, M. G., Marzano, C., Chiarelotto, G., Baccichetti, F. and Bordin, F., Eur. J. Med. Chem., 2000, 35, 827-837) (0.40 g, 1.90 mmol) in solution in a mixture of methanol (15 ml) and dichloromethane (40 ml). The reaction medium is stirred at reflux for 45 minutes. After returning to ambient temperature, the mixture is filtered through Celite® 521. The filtrate is subsequently concentrated to 1/3 under vacuum and then the organic phase is washed with a 5% aqueous sodium bisulphite solution (10 ml), a saturated aqueous sodium bicarbonate solution (10 ml), water (10 ml) and finally a saturated aqueous NaCl solution (10 ml). The organic phase is dried over magnesium sulphate, filtered and then evaporated to dryness. The residue obtained is chromatographed on a column of alumina eluted with an acetate / pentane (7/3, v/v) mixture, to result in the iodinated compound **133** (102 mg, 0.30 mmol). Yield: 16%; viscous liquid; Rf: 0.79 ($Al_2O_3$, acetate / pentane (7/3, v/v)); $^1H$ NMR (200 MHz, $CDCl_3$) δ 1.29 (t, 3H, *J*= 7 Hz), 3.78 (s, 3H), 4.01 (m, 2H), 4.19 (q, 2H, *J*= 7 Hz), 6.31 (s, 1H), 6.84 (bs, 1H), 8.25 (bs, 1H); $^{13}C$ NMR (100 MHz, $CDCl_3$) δ 14.7, 55.8, 61.2, 72.8, 97.8, 120.5, 128.4, 142.8, 149.8, 153.7; IR ($CCl_4$) ν $cm^{-1}$: 1221, 1529, 3200-3500; MS (m/z, %) 336 ($M^+$, 100), 290 (16), 264 (18), 249 (18), 108 (32), 94 (20), 52 (20).

**Stage B: ethyl *N*-(5-iodo-4-methanesulphonamido-2-methoxyphenyl)-carbamate (134)**

To the aminated derivative **133** (0.64 g, 1.90 mmol) in solution in anhydrous pyridine (5 ml) is added dropwise, at -15˚C, under argon, methanesulphonyl chloride (0.16 ml, 2.09 mmol) (internal temperature < -5˚C). The reaction

medium is stored overnight in a refrigerator and then concentrated to 2/3 under vacuum. The mixture is subsequently taken up with water and then the remaining pyridine is neutralized by addition of concentrated hydrochloric acid. The aqueous phase is extracted with dichloromethane (3 x 30 ml). The organic phase is dried over sodium sulphate, filtered and then evaporated to dryness. The residue obtained is chromatographed on a column of alumina eluted with dichloromethane, to result in the compound **134** (0.43 g, 1.05 mmol). Yield: 55%; melting point: 153-155°C; Rf: 0.08 ($Al_2O_3$, dichloromethane); $^1H$ NMR (400 MHz, $CDCl_3$) δ 1.31 (t, 3H, J = 7 Hz), 2.94 (s, 3H), 3.87 (s, 3H), 4.21 (q, 2H, J = 7 Hz), 6.45 (s, 1H), 7.14 (s, 1H), 7.19 (s, 1H), 8.53 (bs, 1H); $^{13}C$ NMR (100 MHz, $CDCl_3$) δ 14.5, 39.8, 56.1, 61.6, 82.4, 106.8, 126.8, 127.4, 132.1, 148.6, 153.2; IR (KBr) ν $cm^{-1}$: 1150, 1337, 1526, 1708, 3195, 3353; MS (m/z, %) 414 ($M^+$, 40), 335 (100), 291 (13), 263 (40), 136 (12).

**Stage C: 5-iodo-4-methanesulphonamido-2-methoxyaniline (135)**

The ethyl carbamate **134** (0.60 g, 1.45 mmol) is brought to reflux for 1.5 hours in the presence of a 10% aqueous sodium hydroxide solution (10 ml). After returning to ambient temperature, the reaction medium is adjusted to pH = 8 by addition of concentrated hydrochloric acid. The aqueous phases are extracted with dichloromethane (4 × 50 ml). The organic phases are combined, dried, filtered and then evaporated to dryness, to result in the amine **135** (0.44 g, 1.29 mmol). Yield: 89%; melting point: 110-112°C; Rf: 0.08 ($Al_2O_3$, dichloromethane); $^1H$ NMR (400 MHz, $d_6$-DMSO) δ 2.96 (s, 3H), 3.75 (s, 3H), 5.07 (s, 2H), 6.76 (s, 1H), 7.08 (s, 1H), 8.94 (s, 1H); $^{13}C$ NMR (100 MHz, $d_6$-DMSO) δ 40.9, 55.5, 89.7, 111.7, 121.7, 126.0, 138.8, 146.3; IR (KBr) ν $cm^{-1}$: 1221, 1529, 1723, 3356, 3430; MS (m/z, %) 342 ($M^+$, 19), 263 (100), 136 (18), 121 (12).

**Stage D: *N*-(2-diethylaminoethyl)-9-(5-iodo-4-methanesulphonamido-2-methoxyanilino)acridine-4-carboxamide dihydrochloride (137)**

The compound **137** was prepared according to the procedure described for the preparation of the compound **125,** using 9-chloro-*N*-(2-diethylaminoethyl)acridine-4-carboxamide **(136)** (Atwell, G. J., Cain, B. F., Baguley, B. C., Finlay, G. J. and Denny, W. A., J. Med. Chem., 1984, 27, 1481-1485) and 5-iodo-4-methanesulphonamido-2-methoxyaniline **(135)** as starting materials. Yield: 33%; melting point: 213-215°C; $^1H$ NMR (400 MHz, $d_6$-DMSO) δ 1.28 (t, 6H, *J*= 7 Hz), 3.16 (s, 3H), 3.24 (q, 4H, *J*= 7 Hz), 3.38 (m, 2H), 3.47 (s, 3H), 3.82 (m, 2H), 7.15 (s, 1H), 7.48 (m, 1H), 7.57 (m, 1H), 8.02 (m, 1H), 8.11 (s, 1H), 8.19 (d, 1H, *J* = 8 Hz), 8.28 (d, 1H, *J* = 8 Hz), 8.61 (d, 1H, *J* = 8Hz), 8.74 (d, 1H, *J* = 7Hz), 9.50 (bs, 1H), 9.95 (bs, 1H), 10.85 (bs, 1H), 11.80 (bs, 1H), 14.20 (bs, 1H); $^{13}C$ NMR (50 MHz, $d_6$-DMSO) δ 8.3 (2C), 34.3, 41.4 (2C), 46.4, 49.5, 56.0, 86.4, 111.1, 113.6, 114.5, 119.7, 120.2, 122.6, 124.5, 124.9, 128.0, 129.1, 133.6, 135.9 (2C), 138.3, 138.8, 139.2, 153.0, 156.0, 167.3; IR (KBr) ν $cm^{-1}$: 1151, 1320, 1522, 1572, 1623, 2927, 3300-3500; MS (of the basic form) (m/z, %) 661 ($M^+$, 2), 582 (24), 509 (25), 456 (74), 383 (73), 340 (40), 128 (27), 86 (100), 58 (19). Anal. Calculated for $C_{28}H_{32}IN_5O_4S{\cdot}2HCl{\cdot}2H_2O$ : C, 43.65; H, 4.97; N, 9.09. Found : C, 43.51; H, 4.76; N, 9.03.

**EXAMPLE 33**

Synthesis of *N*-(2-diethylaminoethyl)-8-iodo-1,6-naphthyridine-2-carboxamide dihydrochloride **(142)**

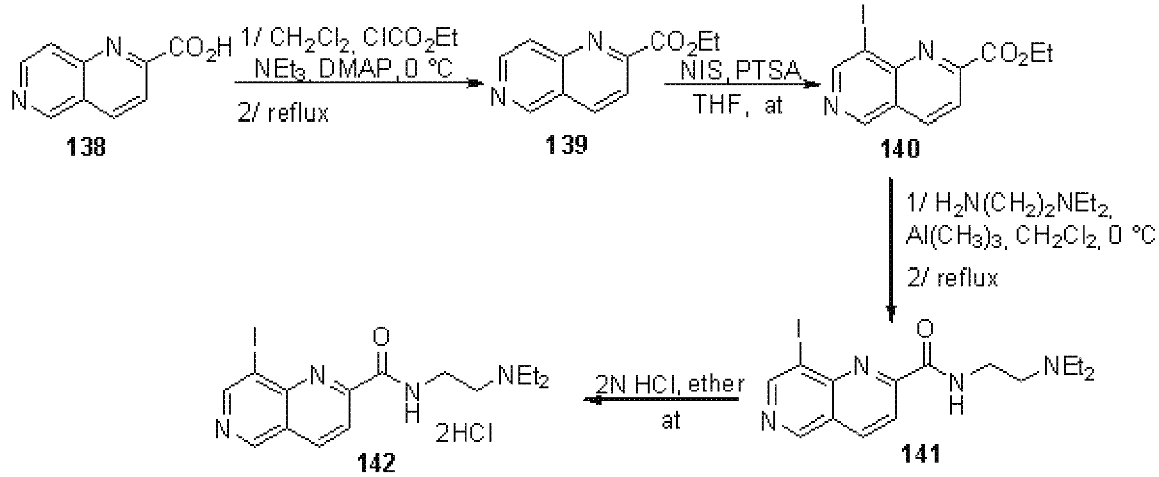


138 139 140 141 142

**Stage A: ethyl 1,6-naphthyridine-2-carboxylate (139)**

Triethylamine (3.03 ml, 21.8 mmol), ethyl chloroformate (3.73 ml, 39.0 mmol) and 4-dimethylaminopyridine (DMAP) (2.40 g, 19.6 mmol) are added, at 0˚C, to a solution of 1,6-naphthyridine-2-carboxylic acid (**138**) (Chan, L., Jin, H., Stefanac, T., Lavallée, J.F., Falardeau, G., Wang, W., Bédard, J., May, S. and Yuen, L., J. Med. Chem., 1999, 42, 3023-3025) (1.00 g, 5.75 mmol) in anhydrous dichloromethane (75 ml). After returning to ambient temperature, the reaction mixture is brought to reflux for 5 hours, the solution is evaporated to dryness and then the residue is purified on a column of alumina eluted with ethyl acetate to result in the ester **139** (517 mg, 2.56 mmol). Yield: 45%; Rf: 0.86 ($Al_2O_3$, ethyl acetate); melting point: 106-108˚C; $^1H$ NMR (400 MHz, $CDCl_3$) δ 1.41 (t, 3H, *J* = 7 Hz), 4.49 (q, 2H, *J* = 7 Hz), 8.02 (d, 1H, *J* = 6 Hz), 8.21 (d, 1H, *J* = 8.5 Hz), 8.39 (d, 1H, *J* = 8.5 Hz), 8.75 (d, 1H, *J* = 6 Hz), 9.29 (s, 1H); $^{13}C$ NMR (100 MHz, $CDCl_3$) δ 14.3, 62.0, 122.4, 122.7, 124.0, 137.2, 147.5, 149.7, 152.3, 152.9, 164.6; IR (KBr) *v* $cm^{-1}$: 1256, 1734; MS (m/z, %) 202 ($M^+$, 3), 158 (18), 130 (100), 102 (15), 75 (22), 51 (16).

Stage B: ethyl 8-iodo-1,6-naphthyridine-2-carboxylate (140)

*N*-iodosuccinimide (NIS) (691 mg, 3.07 mmol) and para-toluenesulphonic acid (PTSA) (176 mg, 1.02 mmol) are added, at -10˚C, to a solution of the ester **139** (517 mg, 2.56 mmol) in tetrahydrofuran (50 ml). The reaction mixture is stirred at -10˚C for 10 minutes and then continued, after returning to ambient temperature, for 24 hours. *N*-iodosuccinimide (576 mg, 2.56 mmol) is then added to the solution. After stirring at ambient temperature for 21 hours, the solution is evaporated under vacuum and then the residue obtained is taken up in a saturated aqueous sodium carbonate solution (50 ml). The aqueous phase is extracted with ethyl acetate (3 × 20 ml). The organic phases are combined, dried over magnesium sulphate, filtered and then evaporated under vacuum. The residue is purified on a column of alumina eluted with an ethyl acetate/cyclohexane (7/3, v/v) mixture, to result in the iodinated ester **140** (527 mg, 1.61 mmol). Yield: 63%; Rf: 0.83 ($Al_2O_3$, ethyl acetate / cyclohexane (7/3, v/v)); melting point: 135-137˚C; $^1H$ NMR (200 MHz, $CDCl_3$) δ 1.51 (t, 3H, *J*= 7 Hz), 4.55 (q, 2H, *J* = 7 Hz), 8.32 (d, 1H, *J* = 8 Hz), 8.39 (d, 1H, *J* = 8 Hz), 9.23 (s, 1H), 9.28 (s, 1H); $^{13}C$ NMR (100 MHz, $CDCl_3$) δ 14.2, 62.7, 100.9, 123.4, 125.2, 137.9, 149.3, 152.8, 153.2, 155.2, 164.3; IR ($CCl_4$) v $cm^{-1}$: 1126, 1274, 1742; MS (m/z, %) 328 ($M^+$, 18), 284 (18), 256 (100), 228 (8), 129 (29), 101 (52), 75 (25).

**Stage C: N-(2-diethylaminoethyl)-8-iodo-1,6-naphthyridine-2-carboxamide (141)**

The compound **141** was prepared according to the procedure described for the preparation of the compound **2**, using ethyl 8-iodo-1,6-naphthyridine-2-carboxylate (**140**) as starting material and heating the reaction medium at reflux for 3 hours. The residue is chromatographed on a column of alumina eluted with ethyl acetate. Yield: 73%; melting point: 44-46 ˚C; Rf: 0.67 ($Al_2O_3$, AcOEt); $^1H$ NMR (200 MHz, $CDCl_3$) δ 1.07 (t, 6H, *J* = 7 Hz), 2.60 (q, 4H, *J* = 7 Hz), 2.70 (t, 2H, *J* = 6 Hz), 3.57 (q, 2H, *J* = 6 Hz), 8.35 (d, 1H, *J*= 8.5 Hz), 8.44 (d, 1H, *J* = 8.5 Hz), 8.83 (m, 1H), 9.17 (s, 1H), 9.19 (s, 1H); $^{13}C$ NMR (50 MHz, $CDCl_3$) δ 12.3 (2C), 37.4, 46.9 (2C), 51.4, 100.2, 121.5, 125.3, 138.1, 148.1, 152.8, 154.7, 154.8, 162.8; IR (KBr) v $cm^{-1}$: 1460, 1516, 1689, 2801, 2926, 2965, 3300-3400; MS (m/z, %) 398 ($M^+$, 1), 86 (100), 58 (15).

**Stage D: *N*-(2-diethylaminoethyl)-8-iodo-1,6-naphthyridine-2-carboxamide dihydrochloride (142)**

The compound **142** was prepared according to the procedure described for the preparation of the compound **3**, using *N*-(2-diethylaminoethyl)-8-iodo-1,6-naphthyridine-2-carboxamide (**141**) as starting material. Yield: 72%; melting point: 215-217˚C; $^1H$ NMR (200 MHz, $d_6$-DMSO) δ 1.27 (t, 6H, *J* = 7 Hz), 3.25 (m, 6H), 3.82 (m, 2H), 8.34 (d, 1H, *J* = 8.5 Hz), 8.81 (d, 1H, *J* = 8.5 Hz), 8.95 (m, 1H), 9.29 (s, 1H), 9.29 (s, 1H), 10.75 (bs, 1H); IR (KBr) ν $cm^{-1}$: 1450, 1528, 1624, 1681, 2043, 2240-2750, 2939, 3045, 3250-3550. Anal. Calculated for $C_{15}H_{19}IN_4O \cdot 2HCl \cdot 15H_2O$: C, 36.16; H, 4.86; N, 11.25. Found: C, 36.50; H, 4.56; N, 11.03.

## EXAMPLE 34

Synthesis of *N*-(4-dipropylaminobutyl)-6-iodoquinoxaline-2-carboxamide dihydrochloride (**144**)

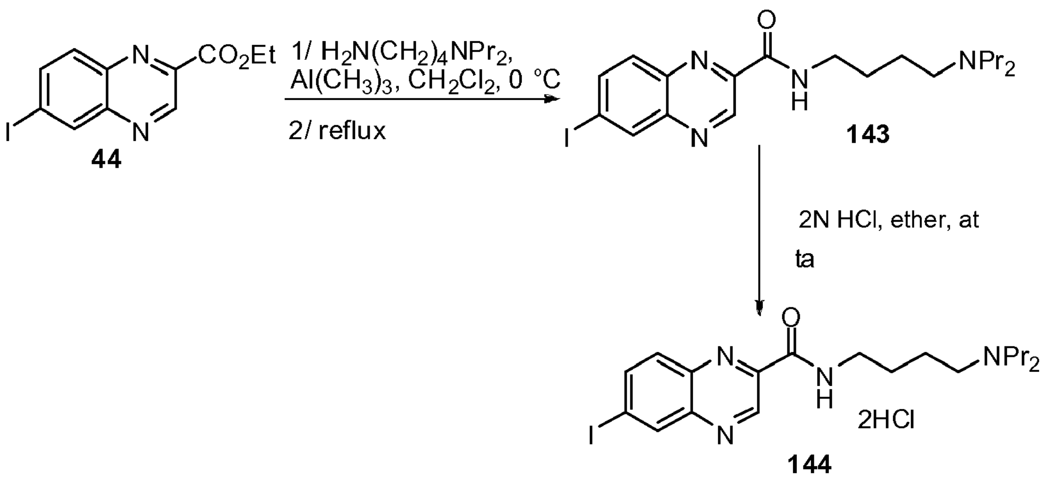


44 143 144

**Stage A: *N*-(4-dipropylaminobutyl)-6-iodoquinoxaline-2-carboxamide (143)**

The compound **143** was prepared according to the procedure described for the preparation of the compound **2**, using ethyl 6-iodoquinoxaline-2-carboxylate (**44**) and 4-amino-*N,N*-dipropylbutylamine (Seguin, H., Gardette, D., Moreau, M. F., Madelmont, J. C. and Gramain, J. C., Synth. Commun., 1998, 28, 4257-4272) as starting materials. The reaction medium is heated at reflux for 20 hours. Chromatography results, by order of elution, in the starting ethyl 6-iodoquinoxaline-2-carboxylate (**44**). Yield: 30%; and then in the carboxamide derivative **143**. Yield: 60%; melting point: 74-76˚C; Rf: 0.83 ($Al_2O_3$, dichloromethane / ethanol (97/3, v/v)); $^1H$ NMR ($CDCl_3$, 200 MHz) δ 0.80 (t, 6H, *J* = 7 Hz), 1.48 (m, 8H), 2.38 (m, 6H), 3.50 (q, 2H, *J* = 6.5 Hz), 7.72 (d, 1H, *J* = 9 Hz), 7.99 (dd, 1H, *J* = 9 and 2 Hz), 8.07 (m, 1H), 8.51 (d, 1H, *J* = 2 Hz), 9.57 (s, 1H); $^{13}C$ NMR ($CDCl_3$, 50 MHz) δ 11.9 (2C), 19.8 (2C), 24.6, 27.5, 39.5, 53.6, 56.0 (2C), 98.0, 130.5, 138.5, 139.3, 139.6, 143.9, 144.2, 144.6, 162.9; IR (KBr) *ν* $cm^{-1}$: 1539, 1685, 2802, 2869, 2955, 3240-3350; MS (m/z, %) 454 ($M^+$, 4), 425 (23), 354 (7), 255 (13), 128 (23), 114 (100), 72 (21), 70 (22).

**Stage B: *N*-(4-dipropylaminobutyl)-6-iodoquinoxaline-2-carboxamide dihydrochloride (144)**

The compound **144** was prepared according to the procedure described for the preparation of the compound **3**, using *N*-(4-dipropylaminobutyl)-6-iodoquinoxaline-2-carboxamide (**143**) as starting material. Yield: 81%; melting point: 175-177˚C; $^1H$ NMR (200 MHz, $d_6$-DMSO) δ 0.88 (t, 6H, *J* = 7 Hz), 1.67 (m, 8H), 2.95 (m, 6H), 3.39 (m, 2H), 7.93 (d, 1H, *J* = 9 Hz), 8.24 (dd, 1H, *J* = 9 and 2 Hz), 8.63 (d, 1H, *J* = 2 Hz), 9.19 (t, 1H, *J* = 5.5 Hz), 9.45 (s, 1H), 10.13 (bs, 1H); IR (KBr) ν $cm^{-1}$: 1475, 1541, 1683, 2600-2750, 2800-3000, 3273. Anal. Calculated for $C_{19}H_{27}IN_4O \cdot 2HCl$: C, 43.28; H, 5.54; N, 10.63. Found: C, 44.33; H, 5.59; N, 10.75.

**EXAMPLE 35**

Synthesis of *N*-(2-diethylaminoethyl)-7-iodophenazine-1-carboxamide dihydrochloride (**151**)

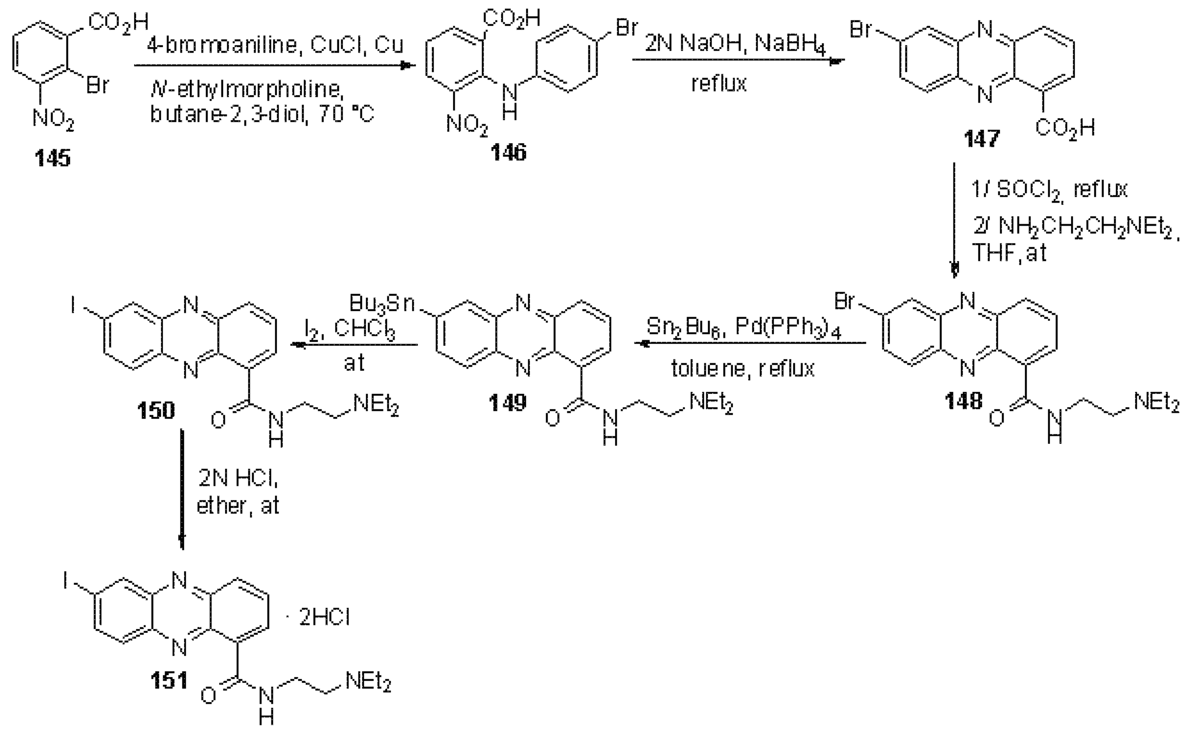


145 146 147 148 149 150 151

**Stage A: *N*-(4-bromophenyl)-3-nitroanthranilic acid (146)**

Butane-2,3-diol (10 ml), 2-bromo-3-nitrobenzoic acid (**145**) (Culhane, P.J., Organic Syntheses, Wiley, New York, 1944, Collect. Vol I, 125) (4.10 g, 16.7 mmol), cuprous chloride (206 mg) and copper powder (410 mg) are successively added to a solution of 4-bromoaniline (4.30 g, 24.5 mmol) in *N*-ethylmorpholine (6 ml, 46.9 mmol). The solution is heated at 70˚C for 7.5 hours. Heating is halted and a 0.5M aqueous ammonia solution (140 ml) is added with stirring. The medium is then rapidly filtered through Celite® 545. The Celite is washed with 0.5M aqueous ammonia solution (20 ml). The filtrate is poured, with vigorous stirring, onto a 2N aqueous hydrochloric acid solution (140 ml). After stirring for 10 minutes, the solution loses its colour and a pale yellow precipitate is formed. The solution is placed in a refrigerator overnight and the precipitate formed is filtered off and then dried in a desiccator, to result in the diphenylamine **146** (1.76 g, 5.22 mmol) Yield: 31%; melting point: 194-196˚C; $^1H$ NMR (400 MHz, $d_6$-DMSO) δ 6.86 (d, 2H, *J* = 9 Hz), 7.16 (t, 1H, *J*= 8 Hz), 7.38 (d, 2H, *J* = 9 Hz), 8.09 (d, 1H, *J* = 8 Hz), 8.18 (d, 1H, *J* = 8 Hz), 9.69 (bs, 1H); $^{13}C$ NMR (100 MHz, $CDCl_3$) δ 114.1, 119.8 (2C), 120.0, 122.0, 130.4, 131.8 (2C), 136.5, 137.8, 140.5, 141.4, 168.1; IR (KBr) *v* $cm^{-1}$: 1259, 1437, 1526, 1668, 3328; ESI-MS m/z 334.7 $[M-H]^+$.

**Stage B:7-bromophenazine-1-carboxylic acid (147)**

Sodium borohydride (505 mg, 13.3 mmol) is added to a solution of *N*-(4-bromophenyl)-3-nitroanthranilic acid (**146**) (1.50 g, 4.45 mmol) in 2N sodium hydroxide solution (75 ml). The reaction medium is heated at reflux for 2 hours. After returning to ambient temperature, the solution is cooled by means of an ice bath and then filtered. The precipitate is washed with precooled 2N sodium hydroxide solution (10 ml). The precipitate is taken up in water (100 ml) and the solution is acidified with stirring by addition of glacial acetic acid until precipitation has occurred (pH = 5). The precipitate obtained is filtered off and then dried in a desiccator, to result in the acid **147** (807 mg, 2.66 mmol). Yield: 60%; melting point: 268-269˚C; $^1H$ NMR (200 MHz, $d_6$-DMSO) δ; 8.12 (m, 2H), 8.34 (d, 1H, *J* = 9 Hz), 8.45 (m, 2H), 8.60 (d, 1H, *J* = 2H); IR (KBr) *v* $cm^{-1}$: 1396, 1739, 2600-2800; ESI-MS m/z 300.8 $[M+H]^+$.

**Stage C: *N*-(2-diethylaminoethyl)-7-bromophenazine-1-carboxamide (148)**

A solution of the acid **147** (885 mg, 2.92 mmol) in thionyl chloride (20 ml) under argon is heated at reflux for 30 minutes. The solution is evaporated under vacuum, then the residue is taken up in anhydrous toluene (5 ml) and again evaporated under vacuum. The acid chloride thus obtained is dissolved in anhydrous dichloromethane (30 ml). A solution of *N,N*-diethylethylenediamine (2.05 ml, 14.6 mmol) in anhydrous dichloromethane (15 ml) is added, under argon and at 0°C, to the solution. After returning to ambient temperature, the reaction medium is stirred for 22 hours. The solution is evaporated under vacuum and then the residue is chromatographed on a column of alumina eluted with ethyl acetate, to result in the compound **148** (1.13 g, 2.82 mmol). Yield: 96%; melting point: 107-109°C; Rf: 0.57 ($Al_2O_3$, ethyl acetate); $^1H$ NMR (200 MHz, $CDCl_3$) δ 1.27 (t, 6H, *J* = 7 Hz), 2.96 (q, 4H, *J* = 7 Hz), 3.06 (m, 2H), 4.00 (m, 2H), 7.97 (m, 2H), 8.43 (m, 3H), 8.98 (d, 1H, *J* = 7 Hz), 11.18 (bs, 1H); $^{13}C$ NMR (50 MHz, $CDCl_3$) δ 11.3 (2C), 37.7, 47.1 (2C), 51.7, 125.6, 129.4, 130.5, 130.7, 131.5, 133.6, 135.2, 135.6, 140.1, 140.8, 143.1, 143.6, 164.7; IR (KBr) ν $cm^{-1}$: 1515, 1653, 2874, 2936, 2966, 3260; ESI-MS m/z 400.7 $[M+H]^+$.

**Stage D: *N*-(2-diethylaminoethyl)-7-(tributylstannyl)phenazine-1-carboxamide (149)**

The compound **149** was prepared according to the procedure described for the preparation of the compound **32**, using *N*-(2-diethylaminoethyl)-7-bromophenazine-1-carboxamide (**148**) as starting material. The residue obtained is chromatographed on a column of alumina eluted with an ethyl acetate / cyclohexane (7/3, v/v) mixture; yield: 61%; viscous liquid; Rf: 0.84 ($Al_2O_3$, ethyl acetate / cyclohexane (7/3, v/v)); $^1H$ NMR (400 MHz, $CDCl_3$) δ 0.89 (t, 9H, *J* = 7.5 Hz), 1.17 (t, 6H, *J* = 7 Hz), 1.21 (m, 6H), 1.35 (st, 6H, *J* = 7.5 Hz), 1.59 (m, 6H), 2.81 (q, 4H, *J* = 7 Hz), 2.91 (t, 2H, *J* = 6 Hz), 3.85 (q, 2H, *J* = 6 Hz), 7.91 (dd, 1H, *J* = 7 and 8.5 Hz), 7.98 (d, 1H, *J* = 8.5 Hz), 8.25 (d, 1H, *J* = 8.5 Hz), 8.36 (d, 1H, *J* = 8.5 Hz), 8.39 (s, 1H), 8.95 (d, 1H, *J* =7Hz), 11.29 (bs, 1H); $^{13}C$ NMR (100 MHz, $CDCl_3$) δ 10.0 (3C, $^1J_{Sn-C}$ = 335 Hz), 11.4 (2C), 13.7 (3C), 27.4 (3C, $^3J_{Sn-C}$ = 56 Hz), 29.1 (3C, $^2J_{Sn-C}$ = 20 Hz), 37.7, 47.1 (2C), 51.7, 127.5, 129.2, 129.8, 133.7, 135.0, 138.2, 138.4, 140.9, 141.7, 142.2, 143.3, 148.7, 165.1; IR ($CCl_4$) *ν* $cm^{-1}$: 1465, 1508, 1660, 2854, 2873 2928, 2960; ESI-MS m/z 613.1 $[M+H]^+$.

**Stage E: *N*-(2-diethylaminoethyl)-7-iodophenazine-1-carboxamide (150)**

A solution of diiodine in chloroform (0.95 g, 3.74 mmol, 70 ml) is added dropwise over a period of 5 hours to a solution of the compound **149** (1.14 g, 1.87 mmol) in chloroform (30 ml). The reaction medium is stirred at ambient temperature for 18 hours. Diiodine (0.27 g, 1.06 mmol) is added and stirring is continued for 7 hours. A 5% aqueous sodium carbonate solution (100 ml) is added to the reaction medium. After separating by settling, the organic phase is washed with a 5% aqueous sodium bisulphite solution (2 × 40 ml), dried over magnesium sulphate, filtered and evaporated under vacuum. The residue obtained is chromatographed on a column of alumina eluted with an ethyl acetate / cyclohexane (7/3, v/v) mixture, to result in the iodonated derivative **150** (397 mg, 0.89 mmol); yield: 47%; melting point: 140-142°C; Rf: 0.56 ($Al_2O_3$, ethyl acetate / cyclohexane (7/3, v/v)); $^1H$ NMR (200 MHz, $CDCl_3$) δ 1.12 (t, 6H, *J* = 7 Hz), 2.76 (q, 4H, *J* = 7 Hz), 2.85 (m, 2H), 3.79 (q, 2H, *J* = 6 Hz), 7.93 (dd, 1H, *J* = 7 and 8.5 Hz), 8.07 (m, 2H), 8.30 (d, 1H, *J* = 8.5 Hz), 8.68 (s, 1H), 8.97 (d, 1H, *J* = 7 Hz), 11.0 (m, 1H); $^{13}C$ NMR (50 MHz, $CDCl_3$) δ 11.6 (2C), 37.8, 47.1 (2C), 51.7, 97.8, 129.5, 130.2, 130.7, 133.6, 135.7, 138.7, 140.1, 140.4, 141.0, 143.2, 143.5, 164.6; IR (KBr) *ν* $cm^{-1}$: 1510, 1646, 2872, 2936, 2963, 3243; ESI-MS m/z 448.9 $[M+H]^+$.

**Stage F: *N*-(2-diethylaminoethyl)-7-iodophenazine-1-carboxamide dihydrochloride (151)**

The compound **151** was prepared according to the procedure described for the preparation of the compound **3**, using *N*-(2-diethylaminoethyl)-7-iodophenazine-1-carboxamide (**150**) as starting material. Yield: 84%; melting point: 212-214°C; $^1H$ NMR (200 MHz, $d_6$-DMSO) δ 1.26 (t, 6H, *J* = 7 Hz), 3.24 (m, 4H), 3.39 (m, 2H), 3.91 (m, 2H), 8.10 (dd, 1H, *J* = 7 and 8.5 Hz), 8.37 (m, 3H), 8.69 (d, 1H, *J* = 7 Hz), 8.78 (s, 1H), 10.26 (bs, 1H), 10.46 (m, 1H); IR (KBr) *ν* $cm^{-1}$: 1508, 1654, 2361, 2588, 2948, 3255. Anal. Calculated for $C_{19}H_{21}IN_4O \cdot 2HCl$: C, 43.78; H, 4.45; N, 10.75. Found: C, 44.85; H, 4.42; N, 10.76.

The chemical structures and physical data of some precursor (unlabelled) iodinated compounds of formulae (I), (I'), (I''), (Ia), (Ib), (Ic), (Id), (Ie), (If), (Ig), (Ih), (Ii), (Ij), (Ik), (Im), (In), (Io), (Ip), (Iq) and (Ir) of the invention before their labelling described below and of some compounds of formula (II) according to the invention are illustrated in the following Table I. **Table I**

| 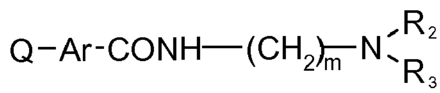 | | | | | | |
|---|---|---|---|---|---|---|
| **No.** | **Q-Ar-** | **m** | **$R_2$** | **$R_3$** | **Salt** | **M.p. (˚C)** |
| **2** | 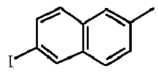 | | | | - | 105-107 |
| **3** | | 2 | Et | Et | HCl | 151-152 |
| **6** | 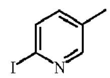 | | | | - | Liq |
| **7** | | 2 | Et | Et | 2HCl | 127-129 |
| **9** | 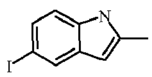 | | | | - | 208-210 |
| **10** | | 2 | Et | Et | HCl | 217-219 |
| **12** | 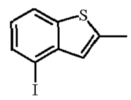 | | | | - | 76-78 |
| **13** | | 2 | Et | Et | HCl | 163-165 |
| **15** | 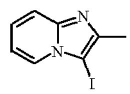 | | | | - | Liq |
| **16** | | 2 | Et | Et | 2HCl | 128-130 |
| **20** | 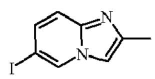 | | | | - | Liq |
| **21** | | 2 | Et | Et | 2HCl | 208-210 |
| **25** | 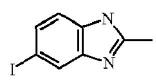 | | | | - | 136-138 |
| **26** | | 2 | Et | Et | 2HCl | 217-219 |
| **30** | 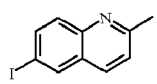 | | | | - | 75-77 |
| **31** | | 2 | Et | Et | 2HCl | 104-106 |
| **34** | 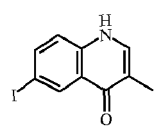 | | | | - | 234-236 |
| **35** | | 2 | Et | Et | 2HCl | 164-166 |
| **38** | 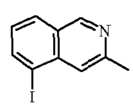 | | | | - | Liq |
| **39** | | 2 | Et | Et | 2HCl | 152-154 |
| **45** | 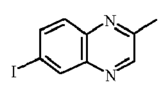 | | | | - | 60-62 |
| **46** | | 2 | Et | Et | 2HCl | 201-203 |
| **48** | 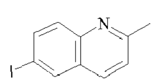 | | | | - | Liq |
| **49** | | 4 | Pr | Pr | 2HCl | 133-135 |

(continued)

| No. | Q-Ar- | m | $R_2$ | $R_3$ | Salt | M.p. (˚C) |
|---|---|---|---|---|---|---|
| **54** | 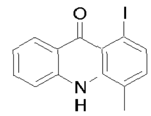 | | | | - | 248-250 |
| **55** | | 2 | Et | Et | 2HCl | 269-271 |
| **59** | 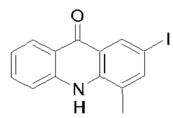 | | | | - | 250-252 |
| **60** | | 2 | Et | Et | 2HCl | 298-300 |
| **64** | 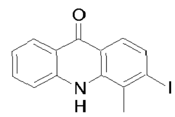 | | | | - | 215-217 |
| **65** | | 2 | Et | Et | 2HCl | 262-264 |
| **68** | 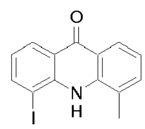 | | | | - | 148-150 |
| **69** | | 2 | Et | Et | 2HCl | 251-253 |
| **77** | 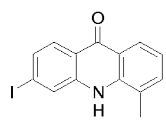 | | | | - | 208-210 |
| **78** | | 2 | Et | Et | 2HCl | 266-268 |
| **83** | 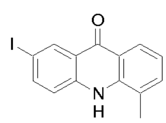 | | | | - | 140-142 |
| **84** | | 2 | Et | Et | 2HCl | 220-222 |
| **86** | 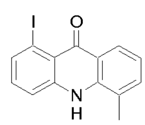 | | | | - | 144-146 |
| **87** | | 2 | Et | Et | 2HCl | 228-230 |
| **90** | 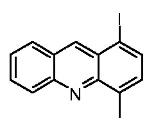 | | | | - | 103-105 |
| **91** | | 2 | Et | Et | 2HCl | 219-221 |
| **94** | 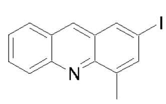 | | | | - | 108-110 |
| **95** | | 2 | Et | Et | 2HCl | 215-217 |
| **97** | 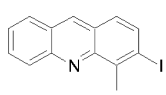 | | | | - | 81-83 |
| **98** | | 2 | Et | Et | 2HCl | 134-136 |
| **101** | 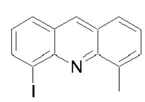 | | | | - | 64-66 |
| **102** | | 2 | Et | Et | 2HCl | 144-146 |

(continued)

| No. | Q-Ar- | m | $R_2$ | $R_3$ | Salt | M.p. (˚C) |
|---|---|---|---|---|---|---|
| **106** | 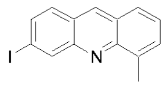 | 2 | Et | Et | - | 80-82 |
| **107** | | | | | 2HCl | 201-203 |
| **110** | 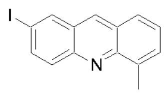 | 2 | Et | Et | - | 176-178 |
| **111** | | | | | 2HCl | 213-215 |
| **114** | 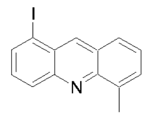 | 2 | Et | Et | - | 78-80 |
| **115** | | | | | 2HCl | 128-130 |
| **119** | 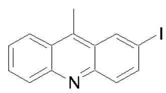 | 2 | Et | Et | - | 83-85 |
| **120** | | | | | 2HCl | 121-123 |
| **121** | 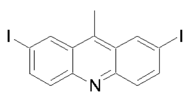 | 2 | Et | Et | - | 209-211 |
| **122** | | | | | 2HCl | 224-226 |
| **125** | 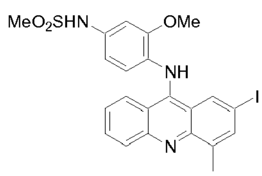 | 2 | Et | Et | -<br>2HCl | 235-237 |
| **128** | 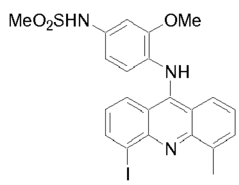 | 2 | Et | Et | -<br>2HCl | 212-214 |
| **129** | 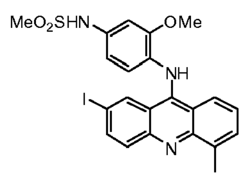 | 2 | Et | Et | -<br>2HCl | 208-210 |
| **137** | 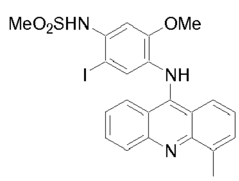 | 2 | Et | Et | -<br>2HCl | 213-215 |
| **141** | 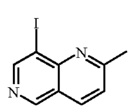 | 2 | Et | Et | - | Liq |
| **142** | | | | | 2HCl | 215-217 |

(continued)

| No. | Q-Ar- | m | $R_2$ | $R_3$ | Salt | M.p. (°C) |
|---|---|---|---|---|---|---|
| 143 | 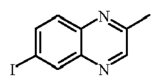 | 4 | Pr | Pr | - | 74-76 |
| 144 | | | | | 2HCl | 175-177 |
| 150 | 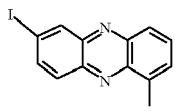 | 2 | Et | Et | - | 140-142 |
| 151 | | | | | 2HCl | 212-214 |

- Et denotes an ethyl radical,
- Pr denotes a propyl radical,
- "Liq" means that the product exists in the form of a liquid,
- M.p. denotes the melting point.

The structures of compounds of formula (VII) which are precursors of the compounds of formulae (I), (I'), (I"), (Ia), (Ib), (Ic), (Id), (Ie), (Ig), (Ih), (Ii), (Ik), (Im), (In) and (Il) are illustrated in the following Table II. **Table II**

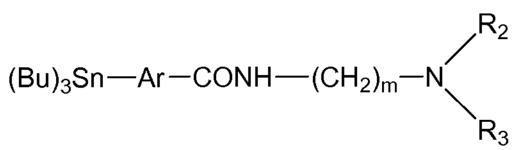 (VII)

| No. | $(Bu)_3$ Sn-Ar- | m | $R_2$ | $R_3$ | M.p. (°C) |
|---|---|---|---|---|---|
| 17 | 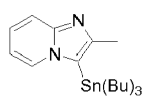 | 2 | Et | Et | Liq |
| 32 | 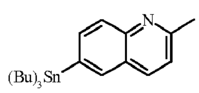 | 2 | Et | Et | Liq |
| 47 | 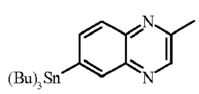 | 2 | Et | Et | Liq |
| 85 | 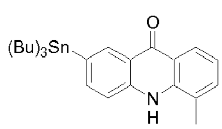 | 2 | Et | Et | Liq |
| 103 | 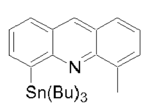 | 2 | Et | Et | Liq |

(continued)

| No. | $(Bu)_3$ Sn-Ar- | m | $R_2$ | $R_3$ | M.p. (°C) |
|---|---|---|---|---|---|
| **131** | 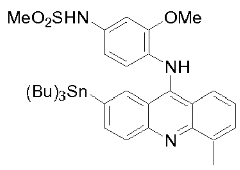 | 2 | Et | Et | Liq |
| **152** | 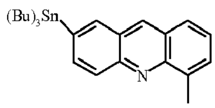 | 2 | Et | Et | Liq |
| - Bu denotes a butyl radica | | | | | |

**<u>EXAMPLE 36 :</u> Labelling**

<u>33.1. First alternative for the preparation of the labelled compounds of formula (II)</u>

<u>MATERIALS, EQUIPMENT AND METHODS</u>

The $Na^{125}I$ and the $Na^{131}I$ are supplied by Amersham. The Extrelut® and the sodium citrate/hydrochloric acid pH = 4 buffer originate from Merck. The radioactivity measurements were carried out, in the case of direct exchange, on an AMBIS 400 (Scanalytics, CSPI, San Diego, CA, USA). In the case of labellings of high specific activity, HPLC purifications were carried out on a Shimadzu device (LC 6A pump, SCL 6B controller, $CR_5A$ integrator) equipped with a Zorbax 80Å extend $C_{18}$ column (4.6 x 150 mm) connected in series with a UV spectrophotometer of Shimadzu SPD 6AV type and a Raytest Steffi gamma detector. The column is eluted with a water/methanol/0.2% aqueous ammonia mixture with a flow rate of 1 ml / minute and a linear elution gradient for methanol from 70 to 100% over a period of 10 minutes. The radiochemical purities were determined after HPLC analysis (HP1100, Hewlett Packard, Les Ulis, France). The Purospher $RP_{18}$ e column (5 μm) is coupled to a diode array detector and a Flow one $A_{500}$ Radiomatic radioactivity detector (Packard, Canberra, Australia). Chromatography is carried out using a water/methanol/0.2% aqueous ammonia mixture with a flow rate of 0.5 ml/minute and a linear elution gradient for methanol from 70 to 100% over a period of 10 minutes. The labelled products were identified by comparing their TLC (Rf) or HPLC (Rt) values with those obtained for the corresponding unlabelled compounds.

**General procedure for the preparation of the products labelled with $^{125}I$ iodine by direct exchange**

The hydrochloride (2-3 mg) is dissolved in 500 μl of a citrate/hydrochloric acid (pH = 4) buffer solution comprising 100 μl of an aqueous copper sulphate solution (5 mg/ml). An aqueous solution of sodium iodide $Na^{125}I$ exhibiting an activity of between 111 and 148 MBq is subsequently added to this mixture. The reaction mixture is heated at 120-150°C for one hour. The crude reaction product is taken up in 500 μl of deionized water and the reaction is monitored by thin layer chromatography (alumina, dichloromethane/ethanol (97/3, v/v)). A plate analyzer then makes it possible to count the radioactivity and to determine the percentage incorporation of $^{125}I$ in the molecule. After changing to basic pH (>10) using a 1N sodium hydroxide solution (100 μl), the mixture is purified on an Extrelut® cartridge (elution with 5 times 3 ml of dichloromethane). After evaporation, the residue is taken up in anhydrous dichloromethane (2 ml) and then in a 2N solution of hydrochloric acid in ether (5 ml), and then evaporated. The hydrochloride obtained is taken up in anhydrous ether (5 ml) and evaporated under vacuum. The radiolabelled compound is thus obtained with a yield and a radiochemical purity given in the following Table III:

Table III

| Starting hydrochloride | Heating temperature (Time) | Compound of formula (II) labelled with iodine-125 | Yield (%) | Purity[a] (%) |
|---|---|---|---|---|
| 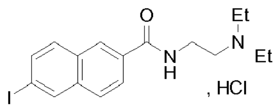 compound **3** | 150 °C (1 hour) | 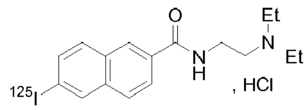 | 77 | 97 |
| 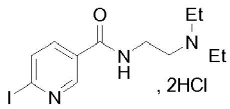 compound **7** | 120 °C (1 hour) | 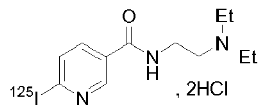 | 39 | 98 |
| 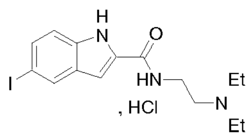 compound **10** | 150 °C (1 hour) | 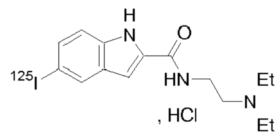 | 75 | 98 |
| 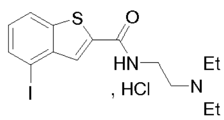 compound **13** | 150 °C (1 hour) | 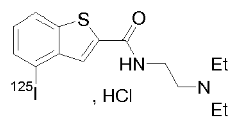 | 73 | 99 |
| 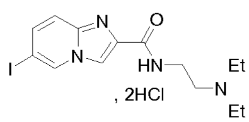 compound **21** | 150 °C (1 hour) | 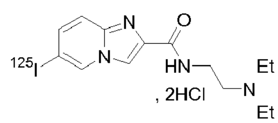 | 68 | 98 |
| 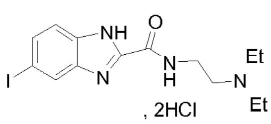 compound **26** | 150 °C (1 hour) | 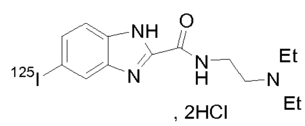 | 22 | 95 |
| 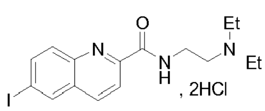 compound **31** | 150 °C (1 hour) | 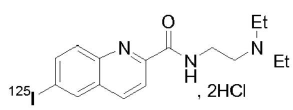 | 92 | 99 |
| 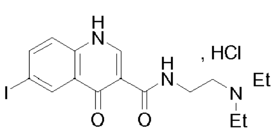 compound **35** | 150 °C (1 hour) | 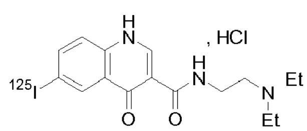 | 54 | 99[b] |
| 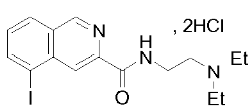 compound **39** | 150 °C (1 hour) | 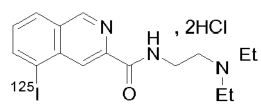 | 43 | 96 |

(continued)

| Starting hydrochloride | Heating temperature (Time) | Compound of formula (II) labelled with iodine-125 | Yield (%) | Purity[a] (%) |
|---|---|---|---|---|
| 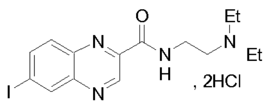 compound **46** | 150 °C (1 hour) | 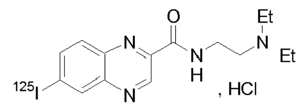 | 68 | 99 |
| 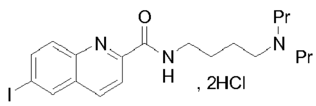 compound **49** | 150 °C (1 hour) | 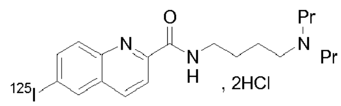 | 69 | 99 |
| 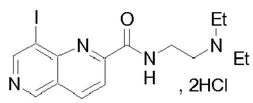 compound **142** | 130 °C (30 minutes) | 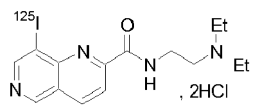 | 69 | 98[c] |
| 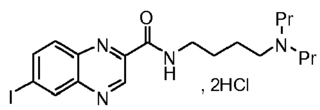 compound **144** | 140 °C (18 minutes) | 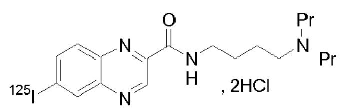 | 30[d] | 99 |
| 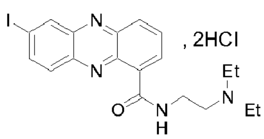 compound **151** | 130 °C (30 minutes) | 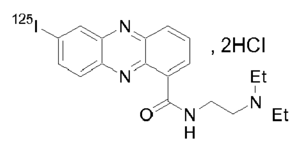 | 58 | 99[c] |

[a]Radiochemical purity after Extrelut®
[b]TLC, $Al_2O_3$, dichloromethane / ethanol (9/1, v/v)
[c]TLC, $Al_2O_3$, ethyl acetate
[d]purification by Extrelut® column and then by HPLC : retention time : 10.6 minutes (compound **144**)

<u>33.2. Second alternative for the preparation of the labelled compounds of formula (II)</u>

According to the second alternative, the compounds of formula (VII) are used as intermediates.

**Preparation of [$^{125}$I]-*N*-(2-diethylaminoethyl)-3-iodoimidazo[1,2-α]pyridine-2-carboxamide dihydrochloride [$^{125}$I]-(16) (labelling of high specific activity)**

An acetic acid/ethanol (1/1, v/v, 20 µl) mixture, Na$^{125}$I (5 µl, 18.9 MBq) and peracetic acid (20 µl) are added to a solution of the trialkylstannane 17 (0.43 mg) in ethanol (100 µl) in a vial. The vial is sealed and stirred, at ambient temperature, with a vortex for 30 minutes. A solution of sodium metabisulphite (20 mg) in water (100 µl) and then a 5N sodium hydroxide solution (100 µl) are added and the mixture is stirred with a vortex for a few minutes. The solution is deposited on an Extrelut® column and the vial is rinsed with a water/ethanol (1/1, v/v, 2 × 100 µl) mixture. After ten minutes of contact, the column is eluted with dichloromethane (5 × 2 ml). The organic phase is evaporated under vacuum and the residue is separated on an HPLC column (elution rate: 1 ml /minute). The retention times obtained are : 7.22 minutes (compound **15** labelled with $^{125}$I) and 16.7 minutes (compound **17**). The various fractions collected are evaporated under vacuum. The residue is taken up in anhydrous dichloromethane (1 ml) and then in a 2N solution of hydrochloric acid in ether (2 ml). The solution is evaporated under vacuum. The residue is taken up in anhydrous ether (2 ml) and evaporated under vacuum, to result in [$^{125}$I]-*N*-(2-diethylaminoethyl)-3-iodoimidazo[1,2-α]pyridine-2-carboxamide dihydrochloride ([$^{125}$I]-16) (7.96 MBq). Radiochemical yield: 57%; radiochemical purity: 99%.

**Preparation of [$^{131}$I]-*N*-(2-diethylaminoethyl)-6-iodoquinoline-2-carboxamide dihydrochloride [$^{131}$I]-(31) (labelling of high specific activity)**

A 0.5N hydrochloric acid solution (100 μl), Na$^{131}$I (100 μl, 1.30 GBq) and an aqueous solution of chloramine-T monohydrate (1 mg/ml, 100 μl) are successively added to a solution of the trialkylstannane **32** (0.45 mg) in ethanol (100 μl) in a vial. The vial is sealed and stirred, at ambient temperature, with a vortex for 30 minutes. A solution of sodium metabisulphite (20 mg) in water (100 μl) and then a 3N sodium hydroxide solution (150 μl) are added and the mixture is stirred with a vortex for a few minutes. The solution is deposited on an Extrelut® column and the vial is rinsed with a water/ethanol (1/1, v/v, 2 ×100 μl) mixture. After ten minutes, the column is eluted with dichloromethane (5 × 2 ml). The organic phase is evaporated under vacuum and the residue is chromatographed on an HPLC column (elution rate : 1 ml /minute). The retention times obtained are : 9.75 minutes (compound **30** labelled with $^{131}$**I**) and 19.9 minutes (compound **32**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{131}$I]-*N*-(2-diethylaminoethyl)-6-iodoquinoline-2-carboxamide dihydrochloride [$^{131}$I]-(**31**) (677 MBq). Radiochemical yield: 52%; radiochemical purity: 99%.

**Preparation of [$^{131}$I]-*N*-(2-diethylaminoethyl)-6-iodoquinoxaline-2-carboxamide hydrochloride [$^{131}$I]-(46) (labelling of high specific activity)**

A 0.5N hydrochloric acid solution (100 μl), Na$^{131}$I (120 μl, 1.49 GBq) and an aqueous solution of chloramine-T monohydrate (1 mg/ml, 100 μl) are successively added to a solution of the trialkylstannane **47** (0.45 mg) in ethanol (100 μl) in a vial. The vial is sealed and stirred, at ambient temperature, with a vortex for 20 minutes. A solution of sodium metabisulphite (20 mg) in water (100 μl) and then a 3N sodium hydroxide solution (150 μl) are added and the mixture is stirred with a vortex for a few minutes. The solution is deposited on an Extrelut® column and the vial is rinsed with a water/ethanol (1/1, v/v, 2 × 100 μl) mixture. After ten minutes, the column is eluted with dichloromethane (5 × 2 ml). The organic phase is evaporated under vacuum and the residue is chromatographed on an HPLC column (elution rate: 1 ml /minute). The retention times obtained are: 7.8 minutes (compound **45** labelled with $^{131}$**I**) and 17.2 minutes (compound **47**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{131}$I]-*N*-(2-diethylaminoethyl)-6-iodoquinoxaline-2-carboxamide hydrochloride [$^{131}$I]-(**46**) (1.02 GBq). Radiochemical yield: 68%; radiochemical purity: 98%.

**Preparation of [$^{125}$I]-*N*-(2-diethylaminoethyl)-9,10-dihydro-7-iodo-9-oxoacridine-4-carboxamide dihydrochloride [$^{125}$I]-(84) (labelling of high specific activity)**

A 1% ethanolic acetic acid solution (30 μl), Na$^{125}$**I** in sodium hydroxide solution (5 μl, 11.1 MBq) and an aqueous solution of chloramine-T monohydrate (0.4 mg/ml, 15 μl) are successively added to a solution of the trialkylstannane **85** (0.12 mg) in ethanol (30 μl) in a vial. The vial is sealed and stirred, at ambient temperature, with a vortex for 10 minutes. A 0.1N sodium hydroxide solution (20 μl) is added and the mixture is stirred with a vortex for a few minutes. The solution is deposited on an Extrelut® column and the vial is rinsed with a water/ethanol (1/1, v/v, 2 × 100 μl) mixture. After ten minutes, the column is eluted with dichloromethane (5 × 2 ml). The organic phase is evaporated under vacuum and the residue is chromatographed on an HPLC column (elution rate: 1 ml /minute). The retention times obtained are: 11.5 minutes (compound **83** labelled with $^{125}$I) and 19.8 minutes (compound **85**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{125}$I]-*N*-(2-diethylaminoethyl)-9,10-dihydro-7-iodo-9-oxoacridine-4-carboxamide dihydrochloride [$^{121}$I]-(**84**) (6.33 MBq). Radiochemical yield: 57%; radiochemical purity: 99.9%.

**Preparation of [$^{125}$I]-*N*-(2-diethylaminoethyl)-5-iodoacridine-4-carboxamide dihydrochloride [$^{125}$I]-(102) (labelling of high specific activity)**

A citrate/hydrochloric acid (pH = 4) buffer solution (20 μl), Na$^{125}$I in sodium hydroxide solution (8 μL, 29.6 MBq) and an aqueous solution of chloramine-T monohydrate (0.5 mg/ml, 15 μl) are successively added to a solution of the trialkylstannane **103** (0.12 mg) in ethanol (30 μl) in a vial. The vial is sealed and stirred, at ambient temperature, with a vortex for 10 minutes. A 0.1N sodium hydroxide solution (20 μl) is added and the mixture is stirred with a vortex for a few minutes. The solution is deposited on an Extrelut® column and the vial is rinsed with a water/ethanol (1/1, v/v, 2 × 100 μl) mixture. After ten minutes, the column is eluted with dichloromethane (5 × 2 ml). The organic phase is evaporated under vacuum and the residue is chromatographed on an HPLC column (elution rate: 1 ml /minute). The retention times obtained are: 11.0 minutes (compound **101** labelled with $^{125}$**I**) and 17.7 minutes (compound **103**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{125}$I]-*N*-(2-diethylaminoethyl)-5-iodoacridine-4-carboxamide dihydrochloride [$^{125}$**I**]-(**102**) (19.6 MBq). Radiochemical yield: 66%; radiochemical purity: 99%.

**Preparation of [$^{125}$I]-*N*-(2-diethylaminoethyl)-7-iodoacridine-4-carboxamide dihydrochloride [$^{125}$I]-(111) (labelling of high specific activity)**

A citrate/hydrochloric acid (pH = 4) buffer solution (20 μl), Na$^{125}$I in sodium hydroxide solution (70 μl, 221.3 MBq) and an aqueous solution of chloramine-T monohydrate (0.5 mg/ml, 10 μl) are successively added to a solution of the trialkylstannane **152** (0.12 mg) in ethanol (30 μl) in a vial. The vial is sealed and stirred, at ambient temperature, with a vortex for 10 minutes. A 0.1N sodium hydroxide solution (20μ l) is added and the mixture is stirred with a vortex for a few minutes. The solution is deposited on an Extrelut® column and the vial is rinsed with a water/ethanol (1/1, v/v, 2 × 100 μl) mixture. After ten minutes, the column is eluted with dichloromethane (5 × 2 ml). The organic phase is evaporated under vacuum and the residue is chromatographed on an HPLC column eluted with a water/methanol/0.2% aqueous ammonia mixture with a flow rate of 1 ml/minute and a linear elution gradient for methanol from 70 to 100% over a period of 20 minutes. The retention times obtained are: 15.1 minutes (compound **110** labelled with $^{125}$I) and 23.3 minutes (compound **152**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{125}$I]-*N*-(2-diethylaminoethyl)-7-iodoacridine-4-carboxamide dihydrochloride [$^{125}$I]-(**111**) (66.6MBq). Radiochemical yield: 31%; radiochemical purity: 99%.

**Preparation [$^{125}$I]-*N*-(2-diethylaminoethyl)-7-iodo-9-(4-methanesulphon-amido-2-methoxyanilino)acridine-4-carboxamide dihydrochloride [$^{125}$I]-(129) (labelling of high specific activity)**

A 1% ethanolic acetic acid solution (30 μl), Na$^{125}$I in sodium hydroxide solution (50 μl, 125.4 MBq) and an aqueous solution of chloramine-T monohydrate (0.25 mg/ml, 15 μl) are successively added to a solution of the trialkylstannane **131** (0.12 mg) in ethanol (30 μl) in a vial. The vial is sealed and stirred, at ambient temperature, with a vortex for 10 minutes. A 0.1N sodium hydroxide solution (20 μl) is added and the mixture is stirred with a vortex for a few minutes. The solution is deposited on an Extrelut® column and the vial is rinsed with a water/ethanol (1/1, v/v, 2 × 100 μl) mixture. After ten minutes, the column is eluted with dichloromethane (5 × 2 ml). The organic phase is evaporated under vacuum and the residue is chromatographed on an HPLC column (elution rate: 1 ml /minute). The retention times obtained are: 12.4 minutes (compound **130** labelled with $^{125}$I) and 17.8 minutes (compound **131**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{125}$I]-*N*-(2-diethylaminoethyl)-7-iodo-9-(4-methanesulphonamido-2-methoxyanilino)acridine-4-carboxamide dihydrochloride [$^{125}$I]-(129) (67.0 MBq). Radiochemical yield: 53%; radiochemical purity: 97%.

The compounds in accordance with the invention were subjected to pharmacological tests which demonstrated their usefulness in the treatment and/or diagnosis of malignant melanoma.

**EXAMPLE 37: Biodistribution of the molecules in the melanoma-bearing mouse**

*Experimental protocol:* The selection of the molecules with the pharmacokinetic profile best suited to an application in diagnosis or in therapy is based on the study of their biodistribution after labelling with $^{125}$I in the male C57BL6

mouse bearing grafted B16 F0 murine melanoma tumour. The distribution of the radioactivity of the various compounds in the body (tumour and other organs) is quantified using an Ambis 4000 image analyzer on sections of whole animals. While using a smaller number of animals than the conventional counting method, this technique appears to be more predictive of the scintigraphic imaging. All the *in vivo* experimentation is carried out within the context defined by French legislation relating to animal experimentation.

The B16 F0 melanoma cells are provided by the ATCC and cultured in monolayers in culture medium (MEM, Invitrogen) supplemented with 10% foetal calf serum and antibiotics. The cultures are maintained by subculturing after trypsinization. The cells of the first passages are frozen and stored in liquid nitrogen. For the transplanting, the cells in culture at confluence are detached by trypsinization and washed with phosphate buffer (PBS). They are resuspended in PBS and injected subcutaneously into the mice ($3 \times 10^5$ cells; 0.1 ml) in the left flank. After ten days, the tumours are palpables with a percentage of uptake of 98-100%.

The molecule labelled with $^{125}$I is injected via the caudal vein (0.1 μmol, 0.5-3.6 MBq/animal) at ten animals per product. At various times after administration (1, 3, 6, 24 or 72 hours), two mice are sacrificed by inhalation of $CO_2$ and rapidly frozen in liquid nitrogen. The animal is then cryosectioned at -22°C using a Reichert-Jung cryomicrotome (Leica Instruments, Rueil Malmaison, France) into sections with a thickness of 40 μm which are left to dehydrate under cold conditions for 48 hours.

The distribution of the radioactivity present in the sections is analyzed using an AMBIS 4000 analyzer (Scanalytics, CSPI, San Diego, CA), which is a proportional counter with a multiwire chamber validated and calibrated beforehand for the monitoring of iodine in the mouse. The measurements require acquisition times of 1000 minutes. The quantification of the radioactivity in the various organs is carried out from the two-dimensional image of the mouse cross section displayed on the screen, in the defined and delimited regions of interest. The value of radioactivity per unit of surface area (cpm/mm$^2$) is converted to concentration (kBq/g) and expressed as percentage of the injected dose/g of tissue (%ID/g).

In parallel with the autoradiographic study, the kinetics of the products are monitored by scintigraphic imaging of the mice, after injection of the labelled compound (3.7 MBq), using a dedicated gamma camera (Biospace gamma imager) which makes possible *in vivo* imaging and its repetition in the same animal.

*Results:*

The biodistribution of the compounds labelled with $^{125}$I, measured by the %ID/g, in the various organs of the melanoma-bearing mouse is represented in the following **Tables 1-19** (only the organs where the presence of radioactivity was displayed and quantified are given).

The radioactivity concentrations measured from 1 to 72 hours in the various organs after the *i.v.* administration of a compound labelled with $^{125}$I in the C57BL6 mouse bearing a subcutaneously grafted B 16 melanoma are summarized in **Tables 1 to 19.**

<u>Concentration of radioactivity in the tissues:</u> tumour (B 16), intestinal contents (Ic), stomach contents (Sc), skeletal muscle (muscle), blood (measured in the heart cavity), urine (bladder), pigmented tissues of the eye (uvea), gall bladder (GB), seminal vesicles (SV) or bone marrow (BM).

Table 1 : Biodistribution compound **3** (dose=1.50 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **12.99** | 2.72 | **11.38** | 2.20 | **8.95** | 0.72 | **6.97** | 3.87 | **1.52** | 0,46 |
| **brain** | 4.37 | 0.92 | 0.78 | 0.24 | 0.25 | 0.05 | - | - | - | - |
| **lc** | 7.82 | | 25.85 | 16.22 | 18.72 | 7.67 | 3.03 | 1.75 | - | - |
| **Sc** | 27.82 | 8.46 | 24.69 | 8.37 | 10.79 | 3.52 | 3.10 | 1.97 | - | - |
| **liver** | 9.46 | 1.18 | 4.75 | 0.60 | 2.73 | 0.36 | - | - | - | - |
| **muscle** | 1.92 | 0.55 | 0.83 | 0.17 | 0.38 | 0.05 | - | - | - | - |
| **pancreas** | 10.18 | 1.66 | 8.19 | - | - | - | - | - | - | - |
| **lungs** | 11.82 | 2.16 | 4.43 | 0.88 | 2.12 | 0.29 | - | - | - | - |
| **spleen** | 9.70 | 3.28 | 2.59 | 0.13 | 8.05 | 0.20 | - | - | - | - |
| **kidney** | 13.30 | 2.45 | 5.65 | 0.77 | 3.07 | 0.29 | - | - | - | - |
| **blood** | 2.97 | 0.55 | 1.77 | 0.09 | 1.74 | - | - | - | - | - |
| **uvea** | 13.21 | 1.68 | 13.90 | 1.79 | 12.76 | 2.26 | 12.28 | 6.22 | 7.84 | 9.38 |

Table 2 : Biodistribution compound 7 (dose=0.94 MBq)

| | **1H** | | **3H** | | **6H** | | **24 H** | | **72 H** | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **15.49** | 1.70 | **9.27** | 1.50 | **6.97** | 0.96 | **2.69** | 0.74 | **0.87** | 0.38 |
| **brain** | 0.64 | 0.20 | 0.22 | 0.14 | 0.12 | 0.05 | - | - | - | - |
| **lc** | 13.44 | 9.46 | 4.94 | 1.84 | 2.98 | 1.04 | - | - | - | - |
| **SV** | 6.79 | 0.95 | 3.78 | 0.38 | 3.55 | 0.55 | - | - | - | - |
| **Sc** | 34.80 | 8.68 | 42.71 | 10.70 | 50.21 | 5.45 | 1.46 | 0.51 | - | - |
| **liver** | 5.14 | 0.92 | 3.01 | 0.30 | 2.09 | 0.32 | - | - | - | - |
| **muscle** | 1.48 | 0.18 | - | - | 0.53 | 0.12 | - | - | - | - |
| **pancreas** | 3.94 | 0.83 | 2.53 | 0.63 | 2.27 | 0.28 | - | - | - | - |
| **lungs** | 7.59 | 1.58 | 3.96 | 0.40 | 3.21 | 0.49 | - | - | - | - |
| **spleen** | 5.44 | 4.42 | 2.68 | 0.31 | 2.35 | 0.50 | - | - | - | - |
| **kidney** | 6.08 | 1.13 | 3.26 | 0.46 | 3.05 | 0.32 | - | - | 1.09 | 0.32 |
| **blood** | 6.67 | 1.00 | 4.22 | 0.29 | 3.71 | 0.62 | - | - | - | - |
| **testicles** | 4.11 | 0.01 | 2.43 | 0.39 | 2.01 | 0.24 | - | - | - | - |
| **thyroid** | 25.29 | | 59.09 | 34.36 | 7.92 | | 111.30 | | 178.80 | 86.38 |
| **uvea** | 16.66 | 4.94 | 12.69 | 2.16 | 11.50 | 1.21 | 6.07 | 1.70 | 2.54 | 0.32 |
| **GB** | 8.68 | | 6.78 | 2.13 | 4.90 | 1.24 | - | - | - | - |

Table 3 : Biodistribution compound **10** (dose=1.52 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **8.31** | 3.10 | **10.22** | 1.37 | **9.84** | 0.86 | **8.16** | 4.39 | **0.84** | 0.26 |
| **brain** | 4.84 | 0.69 | 1.73 | 0.29 | 0.74 | - | 0.00 | 0.00 | - | - |
| **Ic** | 76.74 | 137.12 | 112.56 | 80.06 | 79.85 | 62.59 | 3.31 | 2.34 | - | - |
| **Sc** | 35.41 | 21.04 | 18.52 | 16.68 | 21.84 | 16.62 | 2.10 | 1.10 | 0.04 | |
| **liver** | 11.86 | 1.23 | 5.21 | 0.59 | 3.63 | 0.41 | 1.61 | 0.38 | 0.61 | 0.10 |
| **muscle** | 1.50 | 0.47 | 0.49 | 0.13 | 0.31 | 0.18 | 0.19 | - | - | - |
| **pancreas** | 7.15 | 2.66 | 2.48 | 0.05 | 1.46 | - | 0.09 | 0.10 | - | - |
| **lungs** | 14.41 | 1.87 | 3.46 | 0.74 | 1.70 | 0.29 | 0.37 | 0.26 | - | - |
| **spleen** | 8.09 | 1.35 | 2.79 | 0.42 | 1.68 | 0.32 | 1.22 | 0.30 | - | - |
| **kidney** | 10.87 | 1.83 | 4.19 | 0.66 | 2.50 | 0.38 | 0.83 | 0.28 | 0.71 | - |
| **blood** | 1.60 | 0.27 | 0.79 | 0.24 | 0.67 | 0.18 | 0.10 | 0.11 | - | - |
| **uvea** | 11.70 | 1.29 | 12.78 | 3.31 | 13.20 | 2.81 | 8.81 | 2.48 | 5.86 | 0.63 |

Table 4 : Biodistribution compound **13** (dose= 0.71 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **12..47** | 4.78 | **9.98** | 4.29 | **8.72** | 4.08 | **7.28** | 2.29 | **1.73** | 0.47 |
| **brain** | 0.81 | 0.43 | - | - | - | - | - | - | - | - |
| **lc** | 71.13 | 64.79 | 73.02 | 61.60 | 37.70 | 35.50 | - | - | - | - |
| **Sc** | 20.00 | 12.68 | 5.93 | 3.24 | 18.77 | 8.42 | - | - | - | - |
| **liver** | 2.21 | 0.58 | 1.25 | 0.37 | - | - | - | - | - | - |
| **muscle** | 0.77 | 0.23 | - | - | - | - | - | - | - | - |
| **lungs** | 3.69 | 1.02 | - | - | - | - | - | - | - | - |
| **spleen** | 3.88 | 0.83 | - | - | - | - | - | - | - | - |
| **kidney** | 5.61 | 1.08 | - | - | - | - | - | - | - | - |
| **blood** | 1.37 | 0.30 | - | - | - | - | - | - | - | - |
| **thyroid** | 24.03 | - | 67.21 | 48.99 | 139.12 | 41.99 | 79.82 | 66.09 | - | - |
| **uvea** | 16.09 | 8.29 | 19.51 | 6.38 | 16.29 | 4.53 | 11.06 | 3.00 | 9.22 | 3.38 |
| **GB** | - | - | 65.56 | 47.19 | - | - | - | - | - | - |

Table 5 : Biodistribution compound **16** (dose=0.74 MBq)

| | **1H** | | **3H** | | **6H** | | **24 H** | | **72 H** | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **2.96** | 1.79 | **5.40** | 3.14 | **9.87** | 1.09 | **5.22** | 1.61 | - | - |
| **brain** | 0.33 | 0.07 | 0.36 | 0.29 | 0.65 | 0.41 | - | - | - | - |
| **lc** | 58.60 | 60.15 | 52.06 | 17.32 | 26.14 | 15.95 | - | - | - | - |
| **Sc** | 24.51 | 4.39 | 30.71 | 13.69 | 48.21 | 12.16 | - | - | 3.42 | |
| **liver** | 4.86 | 1.51 | 2.56 | 0.31 | - | - | - | - | - | - |
| **muscle** | 1.10 | 0.26 | 1.30 | 0.42 | - | - | - | - | - | - |
| **lungs** | 6.48 | - | 3.38 | 0.84 | 4.56 | 0.36 | - | - | - | - |
| **spleen** | 8.16 | 4.03 | 12.67 | 6.38 | | | - | - | - | - |
| **kidney** | 7.12 | 3.31 | 5.04 | 1.18 | 4.47 | 0.10 | - | - | - | - |
| **blood** | 2.20 | 2.20 | 3.93 | 1.01 | - | - | - | - | - | - |
| **uvea** | 28.30 | 11.11 | 16.60 | 3.65 | 22.16 | 11.57 | 11.17 | 1.79 | 2.41 | 0.05 |

Table 6 : Biodistribution compound **21** (dose=1.36 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **5.66** | 2.96 | **7.14** | 1.92 | **8.12** | 3.35 | **8.02** | 2.36 | **2.67** | 1.10 |
| **Ic** | 9.48 | 10.62 | 78.03 | 33.12 | 56.39 | 46.99 | 2.68 | 0.64 | - | - |
| **Sc** | 4.55 | 1.23 | 5.19 | 1.22 | 16.57 | 12.41 | - | - | - | - |
| **liver** | 3.97 | 0.69 | 0.99 | 0.16 | - | - | - | - | - | - |
| **muscle** | 0.51 | 0.17 | 0.09 | 0.02 | - | - | - | - | - | - |
| **pancreas** | 7.52 | - | - | - | - | - | - | - | - | - |
| **lungs** | 2.42 | 1.16 | - | - | - | - | - | - | - | - |
| **spleen** | 13.07 | 4.58 | - | - | - | - | - | - | - | - |
| **kidney** | 2.99 | 0.71 | - | - | - | - | - | - | - | - |
| **blood** | 0.68 | 0.28 | - | - | - | - | - | - | - | - |
| **uvea** | 17.43 | 4.63 | 19.18 | 5.76 | 26.81 | 10.20 | 11.54 | 4.32 | 9.55 | 2.12 |

Table 7 : Biodistribution compound **26** (dose=0.47 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **14.78** | 4.81 | **16.13** | 2.94 | **9.90** | 3.76 | **7.06** | 1.97 | **4.54** | 2.10 |
| **Ic** | 105.55 | 95.85 | 72.05 | 50.76 | 95.86 | 47.28 | 3.19 | 2.13 | - | - |
| **Sc** | 24.46 | 10.07 | 17.98 | 5.70 | 8.62 | 1.67 | 2.32 | 2.90 | - | - |
| **liver** | 13.08 | 1.74 | 7.15 | 1.67 | 3.38 | 0.77 | - | - | - | - |
| **muscle** | 0.55 | - | - | - | - | - | - | - | - | - |
| **pancreas** | 6.17 | - | - | - | - | - | - | - | - | - |
| **lungs** | 5.16 | 2.07 | 3.74 | 0.31 | - | - | - | - | - | - |
| **spleen** | 11.51 | 7.04 | - | - | 14.97 | 2.44 | - | - | - | - |
| **kidney** | 6.90 | 1.09 | 4.57 | 0.00 | - | - | - | - | - | - |
| **blood** | 3.31 | 1.68 | 1.33 | 0.26 | - | - | - | - | - | - |
| **thyroid** | | | 63.44 | 47.68 | 34.17 | 38.00 | 142.88 | 1.28 | 174.73 | 87.84 |
| **uvea** | 12.87 | 3.92 | 24.40 | 17.72 | 10.09 | 2.69 | 10.44 | | 7.26 | 1.59 |
| **GB** | - | - | 160.60 | 26.81 | 38.11 | - | - | - | - | - |

Table 8 : Biodistribution compound **31** (dose=1.47 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **19.63** | 8.99 | **25.35** | 9.58 | **17.21** | 4.64 | **12.13** | 5.18 | **5.93** | 1.68 |
| **brain** | 1.96 | 0.42 | 0.83 | 0.32 | 0.37 | 0.16 | - | - | - | - |
| **lc** | 15.78 | 17.68 | 20.65 | 11.13 | 23.36 | 10.68 | 1.45 | | - | - |
| **Sc** | 48.03 | 19.55 | 34.72 | 14.30 | 65.36 | 19.81 | 2.33 | 0.27 | - | - |
| **liver** | 10.92 | 1.52 | 8.89 | 1.74 | 7.11 | 1.48 | 2.25 | 0.77 | 1.23 | 0.45 |
| **muscle** | 1.79 | 0.22 | 1.14 | 0.37 | 0.95 | 0.21 | - | - | - | - |
| **pancreas** | 9.80 | 0.82 | 6.17 | 1.34 | - | - | - | - | - | - |
| **lungs** | 9.41 | 1.43 | 5.58 | 0.72 | 5.32 | 0.89 | 0.53 | - | - | - |
| **spleen** | 11.56 | 2.59 | 10.63 | 6.37 | 7.60 | 2.79 | 2.66 | 0.28 | 1.85 | 0.24 |
| **kidney** | 11.82 | 2.14 | 12.59 | 4.08 | 7.60 | 1.84 | 0.34 | - | - | - |
| **blood** | 7.01 | 0.82 | 5.57 | 0.98 | 5.91 | 0.78 | - | - | - | - |
| **uvea** | 32.71 | 14.88 | 33.10 | 8.22 | 36.62 | 10.60 | 22.27 | 4.72 | 18.30 | 6.97 |

Table 9 : Biodistribution compound **35** (dose=0,56 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **4.69** | 1.23 | **4.05** | 0.78 | **5.81** | 1.94 | **2.74** | 1.08 | **2.35** | 0.75 |
| **brain** | 0.07 | 0.08 | - | - | 0.08 | 0.14 | 0.04 | 0.07 | 0.10 | 0.12 |
| **lc** | 52.94 | 54.99 | 90.12 | 66.99 | 61.06 | 37.95 | 1.62 | 0.79 | 0.70 | 0.29 |
| **Sc** | 12.74 | 7.63 | 9.93 | 3.52 | 22.27 | 25.17 | 0.13 | 0.15 | - | - |
| **liver** | 5.16 | 0.72 | 1.84 | 0.78 | 0.32 | 0.29 | 0.07 | 0.08 | - | - |
| **muscle** | 0.92 | 0.32 | 0.13 | 0.00 | 0.01 | 0.02 | - | - | - | - |
| **pancreas** | 5.74 | 1.32 | 0.96 | 0.02 | 1.08 | 0.00 | - | - | - | - |
| **lungs** | 2.06 | 0.44 | 0.88 | 0.46 | 0.15 | 0.15 | 0.04 | 0.09 | - | - |
| **spleen** | 1.24 | 0.37 | 0.28 | 0.28 | 0.26 | 0.30 | 0.15 | 0.00 | - | - |
| **kidney** | 2.80 | 0.56 | 0.80 | 0.52 | 0.20 | 0.20 | 0.03 | 0.07 | - | - |
| **blood** | 0.42 | 0.14 | 0.23 | 0.22 | 0.17 | 0.22 | 0.22 | 0.36 | - | - |
| **thyroid** | | | 4.33 | 0.00 | 8.55 | 2.96 | 5.09 | 2.72 | 9.83 | 1.77 |
| **uvea** | 5.12 | 0.61 | 5.66 | 1.9 | 5.18 | 1.56 | 3.5 | 1.42 | 6.79 | 4.16 |

Table 10 : Biodistribution compound **39** (dose= 3.63 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | 7.27 | 2.43 | **7.12** | 5.45 | **7.92** | 2.17 | **7.05** | 2.42 | **3.13** | 1.92 |
| **brain** | 0.30 | 0.05 | 0.09 | - | - | - | - | - | - | - |
| **lc** | 42.97 | 59.32 | 68.64 | 52.65 | 29.16 | 11.21 | 1.70 | 0.46 | - | - |
| **Sc** | 23.01 | 17.75 | 19.05 | 7.60 | 28.44 | 20.08 | 0.75 | 0.29 | - | - |
| **liver** | 3.86 | 0.28 | 2.02 | 0.37 | 1.54 | 0.44 | 0.43 | - | - | - |
| **muscle** | 0.74 | 0.17 | 0.43 | 0.14 | - | - | - | - | - | - |
| **pancreas** | 2.39 | 0.31 | - | - | - | - | - | - | - | - |
| **lungs** | 3.68 | 0.28 | 2.76 | 0.41 | 2.05 | 0.55 | - | - | - | - |
| **spleen** | 2.83 | 0.25 | - | - | - | - | - | - | 1.62 | 1.43 |
| **kidney** | 4.62 | 0.47 | 2.66 | 0.35 | 2.11 | 0.47 | - | - | - | - |
| **blood** | 2.15 | 0.23 | 2.65 | 0.19 | 2.17 | 0.43 | - | - | - | - |
| **thyroid** | 16.88 | 2.66 | 35.71 | 6.74 | 48.93 | 38.15 | 88.65 | 66.65 | 132.79 | - |
| **uvea** | 18.08 | 6.80 | 13.87 | 4.59 | 18.02 | 4.89 | 14.55 | 5.32 | 9.73 | 1.39 |

Table 11: Biodistribution compound **46** (dose=1.17 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **17.00** | 11.06 | **27.68** | 7.01 | **40.70** | 7.76 | **21.67** | 10.77 | **12.45** | 1.61 |
| **brain** | 2.34 | 0.46 | 1.03 | 0.18 | - | - | - | - | - | - |
| **Ic** | 15.22 | 9.50 | 14.63 | 3.88 | 17.45 | 8.05 | - | - | - | - |
| **Sc** | 24.33 | 6.48 | 20.33 | 5.19 | 21.09 | 6.85 | - | - | 2.23 | 0.38 |
| **liver** | 13.18 | 1.32 | 8.87 | 1.11 | 7.53 | 1.40 | 1.49 | 0.18 | - | - |
| **BM** | 4.58 | 0.90 | - | - | - | - | - | - | - | - |
| **muscle** | 2.04 | 0.37 | 0.86 | 0.22 | 0.87 | 0.23 | - | - | - | - |
| **pancreas** | 7.60 | 0.76 | - | - | - | - | - | - | - | - |
| **lungs** | 7.83 | 0.59 | 5.68 | 1.05 | 4.46 | 0.60 | - | - | - | - |
| **spleen** | 14.21 | 14.32 | 4.69 | - | - | - | - | - | - | - |
| **kidney** | 16.46 | 2.98 | 9.30 | 3.31 | 7.69 | 0.80 | - | - | - | - |
| **blood** | 3.45 | 0.44 | 2.91 | 0.33 | 3.09 | 0.52 | - | - | - | - |
| **thyroid** | - | - | 39.76 | 23.47 | 83.77 | 26.14 | 94.22 | 27.48 | | |
| **uvea** | 33.82 | 3.47 | 24.79 | 6.78 | 32.30 | 6.52 | 32.44 | 5.98 | 25.26 | 8.38 |
| **GB** | - | - | - | - | 33.38 | 7.70 | - | - | - | - |

Table 12: Biodistribution compound **49** (dose=1.09 MBq)

| | **1H** | | **3H** | | **6H** | | **24 H** | | **72 H** | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **19.07** | 1.29 | **20.34** | 8.64 | **27.38** | 9.38 | **18.28** | 6.20 | **10.86** | 3.32 |
| **brain** | - | - | - | - | - | - | - | - | - | - |
| **Ic** | 24.45 | 11.24 | 23.54 | 8.25 | 26.72 | 9.87 | 1.21 | 0.22 | - | - |
| **Sc** | 25.53 | 12.50 | 24.89 | 9.33 | 22.21 | 9.54 | 2.16 | 0.44 | - | - |
| **liver** | 13.79 | 1.37 | 7.77 | 0.67 | 3.89 | 0.72 | - | - | - | - |
| **muscle** | 2.00 | 0.51 | 1.41 | 0.38 | 0.45 | 0.36 | - | - | - | - |
| **pancreas** | 21.47 | 4.78 | 9.71 | 0.94 | - | - | - | - | - | - |
| **lungs** | 8.33 | 1.07 | 6.88 | 1.23 | 3.34 | 0.56 | - | - | - | - |
| **spleen** | 18.88 | 7.30 | 16.71 | 15.26 | - | - | - | - | - | - |
| **kidney** | 18.29 | 3.49 | 13.10 | 2.90 | 7.98 | 1.65 | - | - | - | - |
| **blood** | 3.31 | 0.52 | 3.47 | 0.46 | 2.50 | | - | - | - | - |
| **thyroid** | 28.75 | | 125.51 | 113.55 | 16.38 | - | - | - | 53.26 | |
| **uvea** | 21.04 | 4.02 | 31.76 | 6.25 | 28.78 | 8.39 | 19.22 | 5.28 | 19.78 | 5.33 |
| **GB** | - | - | 50.82 | 14.16 | 55.80 | 3.92 | - | - | - | - |

Table 13: Biodistribution compound **84** (dose=1.60 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **12.64** | 2.85 | **21.05** | 8.81 | **26.27** | 8.64 | **27.45** | 8.26 | **17.49** | 2.67 |
| **brain** | 1.61 | 0.25 | 1.20 | 0.28 | 0.77 | 0.23 | 0.15 | 0.12 | - | - |
| **lc** | 22.68 | 19.59 | 27.80 | 10.94 | 32.99 | 8.82 | 5.16 | 2.77 | 0.31 | - |
| **Sc** | 23.12 | 5.13 | 13.44 | 3.07 | 24.42 | 4.89 | 3.13 | 1.34 | - | - |
| **liver** | 14.00 | 2.78 | 9.33 | 0.62 | 7.37 | 0.67 | 1.23 | 0.18 | 0.59 | 0.19 |
| **muscle** | 7.51 | 0.86 | 5.84 | 1.33 | 4.61 | 0.92 | 1.27 | 1.37 | - | - |
| **pancreas** | 18.67 | 3.31 | 23.73 | 7.93 | 23.46 | 4.68 | 19.96 | 6.32 | 16.83 | 8.20 |
| **lungs** | 1.87 | 0.37 | 1.39 | 0.19 | 0.92 | 0.16 | 0.22 | - | - | - |
| **spleen** | 8.69 | 0.64 | 6.66 | 1.05 | 5.14 | 0.70 | - | - | - | - |
| **kidney** | 23.94 | 2.76 | 13.99 | 2.72 | 9.99 | 1.47 | 0.68 | 0.09 | - | - |
| **blood** | 13.91 | 1.42 | 9.95 | 0.62 | 7.24 | 0.90 | 0.71 | 0.32 | - | - |
| **thyroid** | 17.60 | 2.28 | 12.97 | 1.99 | 8.95 | 1.38 | 0.65 | 0.38 | - | - |
| **uvea** | 12.88 | 5.29 | 16.32 | 3.71 | 36.25 | 8.52 | 76.78 | 44.05 | 14.93 | 2.68 |
| **GB** | 15.23 | 12.36 | 16.85 | 6.21 | 19.51 | 2.08 | 27.54 | 7.08 | 26.42 | 4.21 |

Table 14: Biodistribution compound **102** (dose=1.11 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **18.81** | 5.94 | **21.41** | 4.96 | **23.62** | 7.65 | **13.82** | 2.53 | **6.99** | 2.89 |
| **brain** | 1.05 | 0.44 | 0.50 | 0.22 | 0.38 | 0.13 | - | - | - | - |
| **lc** | 155.76 | 134.50 | 177.08 | 125.04 | 109.20 | 64.67 | 4.65 | 1.93 | - | - |
| **Sc** | 87.29 | 55.99 | 51.74 | 23.04 | 60.55 | 3.62 | 3.47 | 1.84 | - | - |
| **liver** | 17.89 | 1.82 | 11.30 | 1.38 | 7.64 | 1.19 | 1.07 | 0.19 | - | - |
| **muscles** | 2.34 | 0.49 | 1.34 | 0.53 | 1.36 | 0.60 | - | - | - | - |
| **pancreas** | 11.71 | 2.62 | 6.70 | 1.22 | 4.36 | 0.72 | - | - | - | - |
| **lungs** | 9.62 | 1.69 | 6.83 | 0.97 | 6.22 | 1.23 | - | - | - | - |
| **spleen** | 21.63 | 4.57 | 16.03 | 11.52 | 6.20 | 0.59 | - | - | - | - |
| **kidney** | 16.80 | 2.96 | 8.22 | 1.09 | 6.36 | 1.59 | - | - | - | - |
| **blood** | 4.07 | 0.86 | 5.37 | 0.72 | 6.46 | 1.90 | - | - | - | - |
| **thyroid** | 21.91 | 13.58 | 23.93 | - | 194.64 | - | 254.61 | - | 82.10 | 4.64 |
| **uvea** | 20.00 | 6.56 | 20.14 | 4.52 | 16.16 | 4.88 | 14.44 | 3.12 | 16.59 | 5.35 |
| **GB** | 281.34 | 63.99 | 150.10 | 55.85 | 281.20 | - | - | - | - | - |

Table 15 : Biodistribution compound **111** (dose = 1,54 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **4.78** | 0.64 | **3.98** | 1.16 | **4.57** | 0.70 | **2.77** | 1.16 | **1.67** | 1.06 |
| **Brain** | 0.28 | 0.15 | 0.27 | - | 0.11 | 0.08 | - | - | - | - |
| **Ic** | 23.92 | 19.42 | 30.20 | 9.86 | 28.54 | 11.25 | 1.83 | 0.80 | 0.75 | - |
| **Sc** | 10.16 | 4.07 | 9.56 | 2.96 | 6.06 | 3.06 | 0.73 | 0.27 | - | - |
| **Liver** | 5.38 | 1.12 | 3.62 | 0.80 | 2.16 | 0.19 | 0.48 | 0.21 | 0.42 | 0.10 |
| **muscles** | 0.30 | 0.07 | - | - | - | - | - | - | - | - |
| **pancreas** | 1.38 | 0.65 | 0.70 | 0.22 | - | | - | - | - | - |
| **lungs** | 1.70 | 0.48 | 0.88 | 0.17 | 0.59 | 0.02 | - | - | - | - |
| **spleen** | 3.11 | 0.97 | 1.29 | 0.39 | 0.87 | 0.27 | - | - | - | - |
| **kidney** | 3.07 | 0.64 | 1.27 | 0.19 | 0.77 | 0.15 | - | - | - | - |
| **blood** | 0.75 | 0.15 | 0.54 | 0.21 | 0.42 | 0.09 | - | - | - | - |
| **thyroid** | 21.00 | 15.49 | - | - | - | - | 40.73 | 45.83 | 38.07 | 76.44 |
| **uvea** | 3.83 | 0.62 | 4.69 | 3.01 | 3.51 | 0.60 | 3.45 | 1.87 | 1.96 | 1.14 |
| **GB** | 43.71 | - | - | - | 12.79 | - | - | - | - | - |

Table 16: Biodistribution compound **129** (dose=2.04 MBq)

| | 1H | | 3H | | 6H | | 24 H | | 72 H | |
|---|---|---|---|---|---|---|---|---|---|---|
| | Mean | Stanndard deviation | Mean | Standard deviation | Mean | Standard deviation | Mean | Standard deviation | Mean | Standard deviation |
| **B 16** | **0.95** | 0.43 | **1.55** | 0.11 | **1.25** | 0.29 | **1.19** | 0.25 | **1.05** | 0.39 |
| **brain** | 0.12 | 0.15 | 0.03 | - | - | - | - | - | - | - |
| **Ic** | 43.18 | 34.20 | 57.76 | 45.94 | 68.46 | 45.27 | 2.82 | 1.02 | - | - |
| **Sc** | 10.52 | 6.03 | 3.49 | 0.88 | 3.85 | 2.19 | 1.17 | 0.51 | 0.89 | - |
| **liver** | 8.88 | 1.04 | 4.96 | 0.50 | 4.95 | 0.35 | 1.53 | 0.21 | 1.11 | 0.08 |
| **muscles** | 0.84 | 0.24 | 0.48 | 0.12 | 0.44 | 0.14 | - | - | - | - |
| **pancreas** | 3.06 | 0.19 | 2.50 | - | 2.96 | 1.14 | - | - | - | - |
| **lungs** | 3.43 | 0.29 | 2.91 | 0.29 | 2.47 | 0.31 | 0.69 | 0.03 | - | - |
| **spleen** | 6.28 | 0.96 | 7.36 | 5.02 | 4.12 | 0.52 | 1.65 | 0.29 | 0.66 | - |
| **kidney** | 13.82 | 4.09 | 8.65 | 3.11 | 9.85 | 4.55 | 3.57 | 2.06 | 0.47 | 0.24 |
| **blood** | 0.53 | 0.12 | 0.24 | 0.05 | 0.24 | 0.05 | - | - | - | - |
| **thyroid** | - | - | - | - | 34.81 | 19.33 | 3.00 | - | 7.65 | - |
| **uvea** | 1.80 | 0.51 | 1.45 | - | 1.44 | 0.40 | 1.45 | 0.33 | 2.09 | 0.64 |
| **GB** | 20.19 | | 44.60 | 11.20 | 166.43 | 162.30 | - | - | - | - |

Table 17 : Biodistribution compound **142** (dose = 1.50 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **23.22** | 4.26 | **13.87** | 2.71 | **11.42** | 3.02 | **8.37** | 2.08 | **3.31** | 1.02 |
| **brain** | 0.35 | 0.05 | - | - | - | - | - | - | - | - |
| **lc** | 32.83 | 29.89 | 47.65 | 29.30 | 43.48 | 20.11 | 0.82 | - | - | - |
| **Sc** | 17.58 | 5.69 | 9.76 | 3.78 | 9.17 | 2.81 | 0.62 | - | 2.13 | - |
| **liver** | 3.92 | 0.83 | 2.27 | 0.25 | 1.49 | 0.31 | - | - | - | - |
| **muscle** | 0.86 | 0.15 | 0.43 | 0.20 | 0.24 | 0.07 | - | - | - | - |
| **pancreas** | 1.78 | 0.09 | - | - | - | - | - | - | - | - |
| **lungs** | 2.36 | 0.31 | 1.87 | 0.28 | 1.26 | 0.12 | - | - | - | - |
| **spleen** | 5.38 | 5.84 | 0.93 | - | - | - | - | - | - | - |
| **kidney** | 4.01 | 1.78 | 1.51 | 0.20 | 1.20 | 0.07 | - | - | - | - |
| **blood** | 2.21 | 0.31 | 1.88 | 0.21 | 1.36 | 0.06 | - | - | - | - |
| **thyroid** | 20.16 | 14.46 | 41.44 | 5.23 | 56.95 | 50.29 | 115.66 | 60.68 | - | - |
| **uvea** | 24.11 | 3.35 | 20.30 | 2.36 | 13.15 | 5.74 | 10.00 | 5.91 | 13.06 | 7.12 |
| **GB** | - | - | 41.51 | 20.52 | 28.67 | 10.68 | - | - | - | - |

Table 18 : Biodistribution compound **144** (dose = 1.09 MBq)

| | 1H | | 3H | | 24 H | | 72 H | |
|---|---|---|---|---|---|---|---|---|
| | Mean | Standard deviation | Mean | Standard deviation | Mean | Standard deviation | Mean | Standard deviation |
| **B 16** | **14.69** | 3.82 | **44.92** | 8.05 | **37.46** | 9.04 | **24.02** | 7.83 |
| **brain** | 0.75 | 0.12 | 0.46 | 0.10 | - | - | - | - |
| **lc** | 27.28 | 10.15 | 29.12 | 15.08 | - | - | - | - |
| **Sc** | 24.11 | 9.79 | 34.86 | 19.88 | - | - | - | - |
| **liver** | 12.84 | 0.70 | 7.84 | 0.76 | - | - | - | - |
| **muscle** | - | - | 0.79 | 0.10 | - | - | - | - |
| **pancreas** | 23.88 | 2.52 | 13.06 | 4.65 | - | - | - | - |
| **lungs** | 5.81 | 0.62 | 4.91 | 0.71 | - | - | - | - |
| **spleen** | 18.70 | 12.36 | 6.08 | 0.96 | - | - | - | - |
| **kidney** | 24.42 | 1.80 | 17.73 | 5.71 | - | - | - | - |
| **blood** | 1.85 | 0.18 | 1.61 | 0.30 | - | - | - | - |
| **thyroid** | - | - | 60.78 | - | 44.74 | - | - | - |
| **uvea** | 28.75 | 15.47 | 41.75 | 14.40 | 32.62 | 7.44 | 23.28 | 9.66 |
| **GB** | - | - | - | - | - | - | - | - |

Table 19: Biodistribution compound **151** (dose= 2.1 MBq)

| | 1H | | 3H | | 6H | | 24 H | | 72 H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **20.96** | 2.45 | **23.65** | 2.08 | **27.70** | 6.08 | **11.47** | 3.67 | **6.26** | 2.18 |
| **brain** | 1.79 | 0.07 | 0.44 | 0.11 | - | - | - | - | - | - |
| **Ic** | 56.33 | 51.00 | 52.72 | 24.17 | 52.58 | 28.94 | 2.30 | 1.57 | - | - |
| **Sc** | 11.25 | 3.96 | 14.52 | 6.14 | 16.09 | 7.94 | 2.95 | 1.28 | - | - |
| **liver** | 10.94 | 1.15 | 5.83 | 0.44 | 4.04 | 0.73 | 1.44 | 0.18 | - | - |
| **muscle** | 1.27 | 0.30 | 0.45 | 0.11 | 0.42 | - | - | - | - | - |
| **pancreas** | 6.63 | 1.40 | 1.53 | 0.05 | - | - | - | - | - | - |
| **lungs** | 8.77 | 2.62 | 2.84 | 0.46 | 1.40 | 0.17 | - | - | - | - |
| **spleen** | 14.02 | 7.40 | 13.84 | 8.93 | 18.88 | 3.12 | - | - | - | - |
| **kidney** | 9.45 | 1.53 | 3.42 | 0.52 | 1.55 | 0.22 | - | - | - | - |
| **blood** | 3.92 | 0.60 | 1.39 | 0.38 | - | - | - | - | - | - |
| **thyroid** | - | - | 8.63 | - | 54.71 | 21.06 | 214.42 | | 12.74 | 4.09 |
| **uvea** | 14.19 | 2.86 | 17.49 | 3.09 | 14.74 | 5.73 | 11.64 | 3.34 | 11.19 | 4.07 |
| **GB** | - | - | 107.29 | 41.74 | - | - | - | - | - | - |

Several molecules exhibit high tumour concentrations which are much higher than that of *N*-(2-diethylaminoethyl)-4-iodobenzamide (BZA), the reference compound already mentioned above. These values are particularly high for the compounds **26, 31, 46, 49, 84, 102, 144** and **151,** this being the case from the first hour with respective concentrations of 14.8, 19.6, 17, 19.1, 12.6, 18.8, 14.7 and 21% of the injected dose per gram of tumour (ID/g) whereas, for BZA, this value is 9.5% ID/g.

These tumour concentrations are much higher in comparison with the other organs and reflect a specific affinity for the melanoma, in particular with respect to the organs which are potentially the site of metastases. These data are illustrated in Figure 1 respectively at 3 hours (A) and at 72 hours (B) after the administration of 10 compounds **21, 26, 31, 46, 49, 84, 102, 142, 144** and **151,** in comparison with $^{125}$I-BZA.

For example, the compound **21** exhibits a novel behaviour as, while its tumour concentration is slightly less than that of BZA, the concentration is particularly specific **(Table 6** reported above, **Figure 1).** The product disappears very rapidly from the nontarget organs, which is in favour of use in imaging, this being the case even within a short period of time after the injection. The compound **142** is also noteworthy in this application, with a very high tumour concentration from one hour.

Furthermore, the compounds **26, 31, 46, 49, 84, 102, 144** and **151** are retained in the tumour with the greatest persistance with, at 72 hours, concentrations respectively 6, 8, 16, 14, 22, 9, 31 and 8 times higher than with BZA **(Figure 1B).**

**EXAMPLE 38: Elimination of the molecules**

For this study, the two mice monitored up to 72 hours are kept in metabolic cages in order to collect the urine and faeces for counting and determination of the cumulative urinary and faecal excretions **(Table 20).**

**Table 20:** Urinary and faecal elimination 0-72 hours

| | Urine | Faeces | Total |
|---|---|---|---|
| **BZA** | 83.1% | 4.8% | 87.9% |
| **3** | 43.7% | 26.7% | 70.6% |
| **7** | 67.3% | 5.3% | 72.6% |
| **10** | 21.5% | 61.7% | 83.2% |
| **13** | 52.6% | 46.7% | 99.3% |
| **16** | 52.6% | 24.0% | 76.6% |
| **21** | 36.8% | 43.5% | 80.3% |
| **26** | 17.1% | 40.7% | 57.8% |
| **31** | 80.7% | 11.4% | 91.2% |
| **35** | 23.8% | 69.2% | 93.0% |
| **39** | 49.9% | 21.2% | 71.1% |
| **46** | 70.2% | 18.0% | 88.2% |
| **49** | 67.2% | 19.9% | 87.1% |
| **84** | 10.4% | 38.4% | 48.8% |
| **102** | 52.8% | 46.1% | 98.9% |
| **111** | 8.5% | 23.2% | 31.7% |
| **129** | 4.20% | 33.2% | 37.4% |
| **142** | 68.9% | 29.4% | 98.3% |
| **144** | 45.7% | 34.8% | 80.5% |
| **151** | 5.8% | 61% | 66.8% |

**The** elimination from the body of the compounds studied is highly disparate first in terms of kinetics. It is found that, for some molecules, the elimination kinetics are complete at 72 hours (e.g.: compound **13** or compound **102).** If,

for the majority of the compounds, significant elimination is observed at 72 hours (>70%), a lower elimination was measured in the case of the molecules **26, 84, 111** and **129.** This fact, for the compound **84,** is in agreement with the strong tumour retention demonstrated.

Furthermore, in terms of elimination route, a great disparity also exists according to the compounds. Some exhibit, like BZA, elimination predominantly in the urine; this is the case more particularly of the compounds 7,**31** and **46.** The two routes are about equivalent for **13** and **102.** On the other hand, elimination by the faecal route predominates for the compounds **10, 35** or **151** and for the compound **84,** which is the slowest eliminated.

## EXAMPLE 39: Dosimetry

The dosimetry parameters with regard to the tumour were evaluated, from the experimental data of biodistribution of each molecule, using the MIRD programme and extrapolated to the case of use of the molecules labelled with $^{131}I$. The results given in **Table 21** below show for several novel compounds, due to their kinetics, an increased potentiality with respect to BZA in terms of dose delivered to the tumour and in particular with the compounds **26, 31, 46, 49, 84, 102** and **151** (x 3, 3.7, 4.9, 5.8, 8.4, 3.9 and 3.7 respectively).

**Table 21**

| | Biological period hours | Effective period hours | Dose absorbed cGy/μCi injected |
|---|---|---|---|
| **BZA** | 19.6 | 17.8 | 1.00 |
| 3 | 24.0 | 21.4 | 1.63 |
| 7 | 19.0 | 17.3 | 1.58 |
| 10 | 20.1 | 18.2 | 0.89 |
| 13 | 26.7 | 23.5 | 1.72 |
| 16 | 7.8 | 7.5 | 0.13 |
| 21 | 52.6 | 41.3 | 1.38 |
| **26** | **42.9** | **35.1** | **3.05** |
| **31** | **38.0** | **31.8** | **3.67** |
| 35 | 66.3 | 49.4 | 1.36 |
| 39 | 56.3 | 43.6 | 1.86 |
| **46** | **66.1** | **49.3** | **4.93** |
| **49** | **69.7** | **51.2** | **5.75** |
| **84** | **268.9** | **112.5** | **8.37** |
| **102** | **43.3** | **35.4** | **3.91** |
| **111** | nd | nd | nd |
| **129** | **162.3** | **88.3** | **0.49** |
| **142** | **30.2** | **26.1** | **3.56** |
| **144** | nd | nd | nd |
| **151** | **35.5** | **30.0** | **3.70** |
| nd = not determined | | | |

## EXAMPLE 40: Antitumour effectiveness

37.1. Antitumour effectiveness of the compound **31** by systemic administration after labelling with $^{131}I$:

The study relates to 20 C57B16 mice bearing a melanoma grafted subcutaneously by injection of 50 000 B16 F0 cells (0.1 ml). On the 17th day, the animals are weighed and the tumours are measured in two dimensions (L, 1), the tumour volume being expressed by $L \times 1^2 \times 1/2$. On the 18th day (D18), the mice are divided into two groups, the

labelled compound [$^{131}$I]-**31** with a high specific activity is administered by the i.v. route (37 MBq; 0.2 ml) to 10 mice and 10 constitute the control batch. The monitoring of the animals is carried out with all the necessary radioprotection measures and in particular the removal of the contaminated bedding, the animals are weighed and the tumours are measured every two days. Taking into account the volume of the tumours and ethical considerations, the surviving animals were euthenized on the 20th day after injection, i.e. 38 days after the tumour graft. Tumour growth is markedly slowed down in the treated animals and the difference from the controls, significant from the 6th day after the single administration, persists throughout the duration of the study. The evaluation of the effectiveness of an i.v. administration of [$^{131}$I]-**31** on tumour growth of C57BL6 mice which have received a subcutaneous graft of B 16 melanoma 18 days before treatment is represented in **Figure 2.** The times on the abscissa are given with respect to the day of inoculation of the tumours and with respect to the day of treatment. Under these conditions, a significant difference regarding the median of survival was not demonstrated.

37.2. Antitumour effectiveness of the compound **46** by systemic administration after labelling with $^{131}$I :

Administered with regard to the same model, in two doses of 18.5 MBq respectively 6 and 10 days after induction of the tumours, that is to say with regard to lesions which are less developed, growth is clearly slowed down. The evaluation of the effectiveness of an i.v. administration of [$^{131}$I]-**46** on tumour growth of C57BL6 mice which have received a subcutaneous graft of B16 melanoma is represented in **Figure 3.** The times on the abscissa are given with respect to the day of inoculation of the tumours and with respect to the first day of treatment. Under these conditions, the median of survival is extended and changes from 32 days for the control animals to 39 days for the treated animals. This experiment was repeated according to the same protocol and fully superimposable results were obtained. Unquestionably, effectiveness of the treatment is definitely demonstrated and should be made clear by other models and protocols.

**EXAMPLE 41 : Determination of the cytotoxicity of the compounds of the invention**

The compounds of the invention have formed the subject of a cytotoxicity study on murine melanoma (B16 $F_0$), human melanoma (M4Beu) and human fibroblast cell lines in comparison with DACA and with amsacrine, according to the Hoechst test.

*Experimental protocol :* the human fibroblasts were purchased from Biopredic International (Rennes, France). The M4Beu melanoma line originates from the laboratory of Dr. J. F. Doré (INSERM, Unit 218, Lyons, France). The B16 $F_0$, M4Beu and fibroblast lines are cultured in 75 cm$^2$ dishes comprising 12 ml of Eagle's essential medium with Earle's salts and Glutamax (Gibco-BRL, Paisley, Scotland) supplemented with 10% foetal calf serum, a solution of vitamins at 1X (Gibco), 1 mM sodium pyruvate (Gibco), a solution of nonessential amino acids at 1X (Gibco) and 4 μg/ml of gentamicin (antibiotic).

The cells are maintained at 37°C in an incubator under an atmosphere comprising 5%$CO_2$.

The cells ($5 \times 10^3$) in 150 μl of culture medium are inoculated in 96-well plates (Nunclon™, Nunc, Roskilde, Denmark). The plates are incubated for 16 hours (attachment of the cells) before their treatment. The stock solutions (200 X) are prepared with DMSO and are then stored at -20°C. 50 μl of a solution comprising stock solution and culture medium are subsequently added to the various wells (taking into account the different dilutions). Each test is carried out in triplicate.

After incubating for 48 hours, the plates are turned over on an absorbent paper and then frozen at -80 °C. The amount of cellular DNA is subsequently measured by the Hoechst test. The plates are then defrosted at ambient temperature for 10 minutes. 100 μl of a 0.01% (w/v) solution of SDS (sodium dodecyl sulphate) in sterile distilled water are subsequently added using a microvolume dispenser. The plates (96 well) are incubated with stirring at ambient temperature for 30 minutes and are then frozen at -80 °C for one hour. The plates are subsequently defrosted at ambient temperature for 20 minutes. 100 μl of a solution of Hoechst 33 342 (30 μg/ml) in TNE 2X (10 mM tris-HCl, 1 mM EDTA, 2 M NaCl, pH = 7.4) are subsequently added. The plates are then incubated for one hour at ambient temperature, with stirring and with the exclusion of light. The fluorescence obtained is measured using a Fluoroskan 96-well at the excitation wavelength at 360 nm and emission wavelength at 460 nm. Under these conditions, the fluorescence is proportional to the cell biomass in each well. The percentage of survival is defined by the fluorescence in each well (treated) with respect to the fluorescence of the control wells (drug-free), the blanks being subtracted (drug-free and cell-free wells).

The $IC_{50}$ values (μM) for the compounds of the invention are summarized in the following **Table 22,** in comparison with DACA and with amsacrine.

Table 22

| Compound | M4Beu | B 16 | Fibroblast |
|---|---|---|---|
| DACA | 1.8 | 0.19 | 7.1 |
| amsacrine | 0.40 | 0.035 | 2.0 |
| **55** | 4.3 | 2.0 | 2.7 |
| **60** | 3.7 | 1.5 | 1.9 |
| **65** | 30 | 13 | 56 |
| **69** | 4.1 | 3.0 | 3.7 |
| **78** | 3.6 | 1.7 | 2.8 |
| **84** | 4.1 | 3.6 | 3.2 |
| **87** | 3.5 | 5.1 | 9.5 |
| **91** | 2.4 | 1.1 | 2.2 |
| **95** | 2.3 | 0.28 | 1.8 |
| **98** | 44 | 23 | nd |
| **102** | 0.79 | 0.44 | 0.71 |
| **107** | 3.5 | 2.1 | 2.2 |
| **111** | 3.9 | 2.2 | 3.3 |
| **115** | 3.1 | 1.2 | 3.0 |
| **120** | 28 | 9.5 | nd |
| **122** | 5.6 | 21 | 3.9 |
| **125** | 2.0 | 1.0 | 2.0 |
| **127** | 2.3 | 0.45 | 0.78 |
| **129** | 0.68 | 1.0 | 0.91 |
| **137** | 2.4 | 1.0 | 1.4 |
| **151** | 3.6 | 3.6 | 2.7 |
| nd = not determined | | | |

Except for the compound **65,** the acridone derivatives **55** to **87** exhibit a mean cytotoxicity of the order of 3 μM without obvious specificity for the melanoma cells.

In the case of the iodinated analogues of DACA **91-122,** the compound **102,** with a greater activity than the parent compound with regard to the various cell lines studied, exhibits a noteworthy profile.

Among the structures of amsacrine type **125** to **137,** it is the compound **129** which exhibits the best effectiveness with regard to the M4Beu human cells, close to the values obtained for amscrine.

Finally, for the phenazine derivative **151,** activities similar to that of DACA were observed.

**EXAMPLE 42: *in vitro* study of the chemotherapeutic and radiotherapeutic effects of the compounds 84, 102 and 151:**

An *in vitro* study was carried out in order to evaluate the concept of radiotherapy by irradiation of the Auger electron ($^{125}I$) and to study the chemotherapeutic effects of the compounds **84, 102** and **151.** The colony forming technique is involved, that is to say the growth of colonies of clones starting from tumour cells (B16 $F_0$ murine melanoma cells) inoculated at a low concentration. This method makes it possible to visualize the cytotoxic and cytostatic properties of the compounds studied. The study was carried out in three parts :

1/ determination of the effects/doses range of the cold compound
2/ determination of the effects/doses range of the compound labelled with iodine-125 with a high specific activity

(HSA) in comparison with $Na^{125}I$ (without effect under these conditions)
3/ study of the overall activity of a moderately active dose of cold product in combination with a range of radiotoxic doses of the same product labelled with iodine-125.

The experiment is carried out on 6-well plates where 200 cells are inoculated in 2 ml of DMEM medium. After 20 hours, the medium is withdrawn and replaced with 2 ml of medium comprising the labelled compound at the desired concentration. This study is carried out in comparison with control wells comprising DMEM medium alone. After 48 hours of contact, the medium is withdrawn and 2 ml of DMEM medium are added. The plates are incubated for 8 days and then the medium is withdrawn. The wells are rinsed with PBS and methanol is added for 3 minutes. Once attached, the cells are coloured with crystal violet with a contact time of 3 minutes. Subsequently, the colonies comprising more than 50 cells are counted using a dedicated counter. The growth of the treated colonies of cells is quantified in comparison with the controls. The activity of the product is expressed by a percentage of inhibition :

$$100-[(\text{number of treated colonies})/(\text{number of control colonies})] \times 100$$

The results obtained are summarized in the following Tables 23 to 31 :

**Table 23 (Chemotherapeutic effects alone)**

| Concentration cold compound **84** | 47 nM | 187.5 nM | 750 nM | 3.0 μM |
|---|---|---|---|---|
| % of inhibition | 8 | 11 | 25 | 100 |

**Table 24 (Radiotherapeutic effects alone)**

| Activity compound [$^{125}$I]-**84** | 1.5 kBq | 6 kBq | 12 kBq | 24 kBq | 48 kBq |
|---|---|---|---|---|---|
| Concentration of the compound [$^{125}$I]-**84** | 9.31 pM | 37.23 pM | 74.5 pM | 148.9 pM | 297.8 pM |
| % of inhibition | 4.5 | 12.4 | 29 | 30 | 69 |

**Table 25 (Radiotherapeutic and chemotherapeutic effects)**

| Activity compound [$^{125}$I]-**84** | 0 | 1.5 kBq | 6 kBq | 12 kBq | 24 kBq | 48 kBq |
|---|---|---|---|---|---|---|
| Cumulative effect * (% inhib) | 17.3 | 18.3 | 22 | 40.3 | 54 | 88 |
| *Cumulative effect: Cold compound **84** at the dose of 0.75 μM with labelled [$^{125}$I]-**84** with a high specific activity. | | | | | | |

**Table 26 (Chemotherapeutic effects alone)**

| Concentration cold compound **102** | 3.125 nM | 6.25 nM | 12.5 nM | 25 nM | 50 nM |
|---|---|---|---|---|---|
| % of inhibition | 9.5 | 21 | 40.5 | 78.5 | 89.9 |

**Table 27 (Radiotherapeutic effects alone)**

| Activity compound [$^{125}$I]-**102** | 1.39 kBq | 2.78 kBq | 5.55 kBq | 11.1 kBq | 16.7 kBq | 22.2 kBq |
|---|---|---|---|---|---|---|
| Concentration of the compound [$^{125}$I]-**102** | 8.625 pM | 17.25 pM | 34.5 pM | 69 pM | 103.5 pM | 138 pM |
| % of inhibition | 11 | 14.3 | 40.3 | 65.5 | 79 | 96.1 |

**Table 28 (Radiotherapeutic and chemotherapeutic effects)**

| Activity compound [$^{125}$I]-**102** | 0 | 1.39 kBq | 2.78 kBq | 5.55 kBq | 11.1 kBq | 16.7 kBq |
|---|---|---|---|---|---|---|

(continued)

| | | | | | | |
|---|---|---|---|---|---|---|
| Cumulative effect * (% inhib) | 183 | 30 | 37.5 | 59.5 | 76.5 | 89 |
| *Cumulative effect: cold compound **102** at a dose of 6.25 nM with labelled [$^{125}$I]-**102** with a high specific activity. | | | | | | |

**Table 29 (Chemotherapeutic effects alone)**

| Concentration cold compound **151** | 2 μM | 4 μM | 6 μM | 8 μM | 10 μM |
|---|---|---|---|---|---|
| % of inhibition | 21 | 74 | 87 | 97 | 100 |

**Table 30 (Radiotherapeutic effects alone)**

| Activity compound [$^{125}$I]-**151** | 1.5 kBq | 3 kBq | 6 kBq | 12 kBq | 24 kBq | 48 kBq | 96 kBq |
|---|---|---|---|---|---|---|---|
| Concentration of the compound [$^{125}$I]-**151** | 9.31 pM | 18.6 pM | 37.2 pM | 74.5 pM | 148.9 pM | 297.8 pM | 595.6 pM |
| % of inhibition | 7 | 21 | 25 | 28 | 36 | 45 | 74 |

**Table 31 (Radiotherapeutic and chemotherapeutic effects)**

| Activity compound [$^{125}$I]-**151** | 0 | 1.5 kBq | 3 kBq | 6kBq | 12 kBq | 24kBq | 48 kBq | 96 kBq |
|---|---|---|---|---|---|---|---|---|
| Cumulative effect * (% inhib) | 19 | 28 | 29 | 32 | 39 | 40 | 49 | 82 |
| *Cumulative effect: cold compound **151** at the dose of 2 μM with labelled [$^{125}$I]-**151** with a high specific activity. | | | | | | | | |

A dose/effect study on the compounds **84, 102** and **151** was carried out, on the one hand with the cold products and, on the other hand, with the labelled products with a high specific activity **(Tables 23 and 24, 26 and 27, and 29 and 30).** It is apparent, with regard to the study model, which takes into account the inhibition of growth, that the activity of the cold compound **102** is much greater in comparison with the derivatives **84** and **151,** with a more marked difference than that observed during the acute cytotoxicity study (from 15 to 40×). For the group of the labelled compounds with an HSA, the activity observed is clearly related to a radiotoxicity mechanism as the ranges of concentrations differ by a factor of between 300 and $10^5$.

The third part of the *in vitro* study, in which a specific dose with an inhibiting effect of approximately 20% is combined with increasing concentrations of labelled compound with an HSA, clearly shows the effects to be additive **(Tables 25, 28 and 31).**

According to another of its aspects, the present invention relates to a radiopharmaceutical composition comprising, as active principle, a compound of formula (I), (I'), (I"), (Ia), (Ib), (Ic), (Id), (Ie), (If), (Ig), (Ih), (Ii), (Ij), (Ik), (Im), (In), (Io), (Ip), (Iq) or (Ir) or a compound of formula (II) comprising a radionuclide according to the invention or one of its pharmaceutically acceptable salts.

The said radiopharmaceutical composition advantageously comprises an effective amount of such a compound of formula (I), (I'), (I"), (Ia), (Ib), (Ic), (Id), (Ie), (If), (Ig), (Ih), (Ii), (Ij), (Ik), (Im), (In), (Io), (Ip), (Iq) or (Ir) or of a compound of formula (II) comprising a radionuclide, and also one or more excipients. Such excipients are chosen according to the type of formulation.

The present invention furthermore relates to the use of a compound of formula (Ip), (Ir), (Ii), (In), (Ih), (Im), (Ig), (Ik), (Io) or (Iq) or of a compound of formula (II) or one of its pharmaceutically acceptable salts in the preparation of a radiopharmaceutical composition intended for medical imaging, more particularly for the diagnosis of melanomas.

The present invention also relates to a product chosen from a labelled compound of formula (Ip), (Ir), (Ii), (In), (Ih), (Im), (Ig), (Ik), (Io) or (Iq) or a labelled compound of formula (II) or one of its pharmaceutically acceptable salts for the diagnosis of melanomas.

Likewise, the present invention relates to the use of a compound of formula (Ic), (If), (Ij), (Ib), (Ie), (Ia) or (Id) or of a compound of formula (II) or one of its pharmaceutically acceptable salts in the preparation of a radiopharmaceutical composition intended for the treatment of melanomas.

The present invention also relates to a product chosen from a labelled compound of formula (Ic), (If), (Ij), (Ib), (Ie), (Ia) or (Id), or a labelled compound of formula (II) or one of its pharmaceutically acceptable salts for the treatment of melanomas.

The present invention, according to another of its aspects, relates to a method for the treatment of melanomas which comprises the administration to a patient suffering from melanomas and more particularly from disseminated melanomas of an effective amount of a compound of formula (I), (I'), (I"), (Ia), (Ib), (Ic), (Id), (Ie), (If), (Ig), (Ih), (Ii), (Ij), (Ik), (Im), (In), (Io), (Ip), (Iq) or (Ir) or of a compound of formula (II) comprising a radionuclide or one of its pharmaceutically acceptable salts.

Lastly, a subject-matter of the present invention is a noninvasive method for the determination of the tissue distribution of tumour cells of melanomas on the human body, comprising the stages of at least one injection of a radiopharmaceutical composition comprising at least one compound of formula (I), (I'), (I"), (Ia), (Ib), (Ic), (Id), (Ie), (If), (Ig), (Ih), (Ii), (Ij), (Ik), (Im), (In), (Io), (Ip), (Iq) or (Ir) or one compound of formula (II) comprising a radionuclide or one of its pharmaceutically acceptable salts and of at least one determination of the concentration of the radioactivity.

## Claims

1. Use of a compound of formula (I):

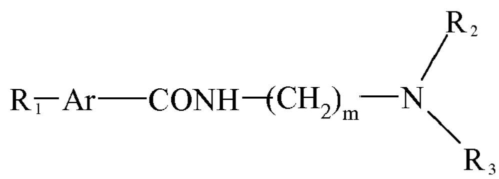

(I)

in which
$R_1$ represents a radionuclide, and in particular an iodine atom chosen from $^{123}I$, $^{125}I$ and $^{131}I$,
Ar represents an aromatic nucleus,
m is an integer varying from 2 to 4,
$R_2$ and $R_3$ represent, independently of one another, a hydrogen atom, a $(C_1-C_6)$alkyl group, a $(C_1-C_6)$alkenyl group or an aryl group chosen from a phenyl, benzyl, imidazolyl, pyridyl, pyrimidinyl, pyrazinyl, indolyl, indazolyl, furyl and thienyl group,
in which the aromatic nucleus denotes a heteroaryl group, it being possible for the said heteroaryl group to be

- mono- or disubstituted by:

  - an optionally labelled halogen atom,
  - a hydroxyl group,
  - a $(C_1-C_4)$alkyl group
  - a $(C_1-C_4)$alkoxy group,
  - an $-NO_2$ group,
  - an $-NR_5R_6$ group, where $R_5$ and $R_6$ can independently represent a hydrogen atom or a $(C_1-C_4)$alkyl group,
  - an $-NHCONH_2$ group,
  - an -SH group,
  - an $-NHCOOR_7$, $-NHCONHR_7$ or $-SR_7$ group, where $R_7$ represents a $(C_1-C_4)$alkyl group,
  - an oxo group, or

- monosubstituted by an anilino group which can itself be substituted by 1 to 3 groups which can be chosen from a $(C_1-C_4)$alkyl or $(C_1-C_4)$alkoxy group, a hydroxyl group, a halogen atom or an $NHR^e$ group where $R^e$ represents a hydrogen, or a $COR^a$ group, a $COOR^a$ group or an $SO_2R^a$ group, where $R^a$ represents an aryl group or a $(C_1-C_{10})$alkyl group optionally substituted by an oxo group,

and in which $R_1$ is bonded to the aromatic nucleus as such or, when the substituent of the aromatic nucleus is an anilino group, $R_1$ can be bonded to the phenyl group of the anilino group,
and their addition salts with pharmaceutically acceptable acids, in the preparation of a radiopharmaceutical composition intended for the diagnosis and/or treatment of melanoma.

**2.** Use according to Claim 1, **characterized in that** the heteroaryl group comprises from 1 to 4 nitrogen atoms, in particular 2 or 3 nitrogen atoms, and more particularly the heteroaryl comprises only nitrogen as heteroatom(s), preferably from 1 to 4 nitrogen atoms, in particular 2 or 3 nitrogen atoms.

**3.** Use according to Claim 1, **characterized in that** the heteroaryl is a 5- or 6-membered aromatic ring comprising 1 or 2 heteroatom(s) chosen from pyrrole, imidazole, pyrimidine, pyridine, pyrazine, pyridazine and thiazole or else a bi- or tricyclic aromatic nucleus, one of the rings of which is benzene, chosen from indole, isoindole, quinoline, isoquinoline, quinoxaline, benzimidazole, indazole, phthalazine, quinazoline, cinnoline, benzothiophene, carbazole, phenanthridine, acridine, phenothiazine, phenoxazine, phenazine, phenanthroline, carboline, perimidine and benzisoquinoline or else a bi- or tricyclic aromatic nucleus, each one of the rings of which, taken separately, is an aromatic nucleus comprising at least one heteroatom, chosen from naphthyridine, quinolizine, purine, imidazopyridine, indolizine, pteridine, imidazotriazine and pyrazinopyridazine.

**4.** Use according to any one of Claims 1 to 3, **characterized in that** the aromatic nucleus is bi- or tricyclic and the $R_1$ group is bonded to one of these rings and the group

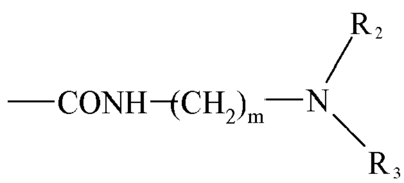

is bonded to the other ring or to one of the other rings constituting the bi- or tricyclic group.

**5.** Use of a compound of formula (I) as defined in any one of Claims 1 to 4, in which
$R_1$ represents a radionuclide,
Ar is a heteroaryl group,
m is an integer varying from 2 to 4,
$R_2$ and $R_3$ represent, independently of one another, a hydrogen atom, a $(C_1-C_6)$alkyl group or a $(C_1-C_6)$alkenyl group, and
the heteroaryl group being a 5- or 6-membered aromatic ring comprising 1 or 2 nitrogen atoms or a bi- or tricyclic aromatic nucleus comprising from 1 to 4 nitrogen atoms or comprising a sulphur atom, at least one of the rings of which has 6 ring members, the other fused ring or rings having 5 or 6 ring members, it being possible for the said heteroaryl group to be monosubstituted by:

- an optionally labelled halogen atom,
- a $(C_1-C_4)$alkoxy group,
- a $(C_1-C_4)$alkyl group,
- an oxo group or
- an anilino group which can itself be substituted by 1 to 3 groups which can be chosen from a $(C_1-C_4)$alkyl or $(C_1-C_4)$alkoxy group, a hydroxyl group, a halogen atom or an $NHR^e$ group where $R^e$ represents a hydrogen, or a $COR^a$ group, a $COOR^a$ group or an $SO_2R^a$ group, where $R^a$ represents an aryl group or a $(C_1-C_{10})$alkyl group optionally substituted by an oxo group;

and in which $R_1$ is bonded to the aromatic nucleus as such or, when the substituent of the aromatic nucleus is an anilino group, $R_1$ can be bonded to the phenyl group of the anilino group,
and its addition salts with pharmaceutically acceptable acids, in the preparation of a radiopharmaceutical composition intended for the diagnosis and/or treatment of melanoma.

**6.** Use according to Claim 5, **characterized in that** the heteroaryl group is chosen from an indolyl, isoindolyl, quinolyl, isoquinolyl, quinoxalinyl, benzimidazolyl, indazolyl, phthalazinyl, quinazolinyl, cinnolinyl, carbazolyl, phenanthridinyl, acridinyl, phenazinyl, phenanthrolinyl, carbolinyl, perimidinyl, benzisoquinolinyl, naphthyridinyl, quinolizinyl, purinyl, imidazopyridyl, indolizinyl, pteridinyl, imidazotriazinyl and pyrazinopyridazinyl group, it being possible for the said group to be substituted as defined according to Claim 6.

**7.** Use according to any one of Claims 5 or 6, **characterized in that** Ar is chosen from a pyridyl, phenazinyl, naphthyridinyl, indolyl, imidazopyridyl, benzimidazolyl, quinolyl, quinolonyl, isoquinolyl, quinoxalinyl, benzothienyl, acridinyl or acridonyl group, it being possible for the said group to be monosubstituted by a methyl group, a methoxy group or an optionally labelled halogen atom, and an acridinyl group substituted by an anilino group which can itself be substituted by 1 to 3 groups which can be chosen from a $(C_1-C_4)$alkyl or $(C_1-C_4)$alkoxy group, a hydroxyl group, a halogen atom or an $NHR^e$ group where $R^e$ represents a hydrogen, or a $COR^a$ group, a $COOR^a$ group or an $SO_2R^a$ group, where $R^a$ represents an aryl group or a $(C_1-C_{10})$alkyl group optionally substituted by an oxo group.

**8.** Use according to any one of Claims 5 to 7, **characterized in that** alternatively:

- the $R_1$ group is in the para position with respect to the group

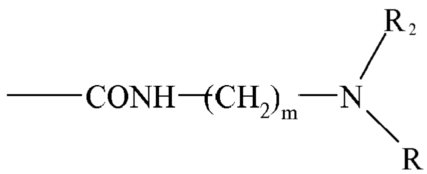

when Ar comprises only one ring and
**in that** the $R_1$ group is bonded to one of the rings and the group

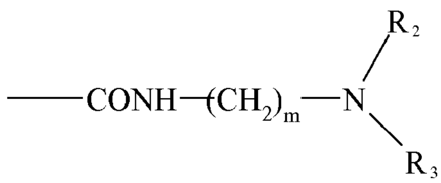

is bonded to the other ring or to one of the other rings when Ar is a bi- or tricycle, or
- Ar is a bi- or tricyclic heteroaryl and **in that** $R_1$ is bonded to the ring, taken in isolation, not comprising a heteroatom or comprising the least thereof and the group

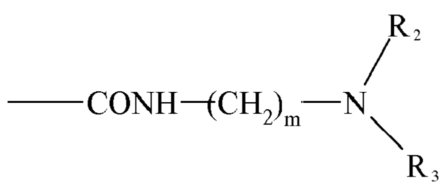

is bonded to another ring comprising the greater number of heteroatom(s).

**9.** Use according to any one of Claims 1 to 8, **characterized in that** the radionuclide is a radioisotope chosen from $^{123}I$, $^{124}I$, $^{125}I$, $^{131}I$, $^{75}Br$, $^{76}Br$ , $^{77}Br$, $^{18}F$, $^{210}At$ and $^{211}At$.

**10.** Compound of formula (II)

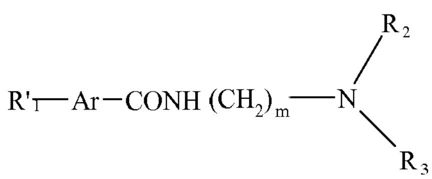
(II)

in which

R'$_1$ represents a labelled halogen atom,
$R_2$, $R_3$ and m have the same meaning as that defined in any one of Claims 1 to 7, and
Ar is chosen from the pyridyl, benzothienyl, indolyl, isoindolyl, quinolyl,
isoquinolyl, quinoxalinyl, benzimidazolyl, indazolyl, phthalazinyl, quinazolinyl, cinnolinyl, carbazolyl, phenanthrolinyl, carbolinyl, perimidinyl, benzisoquinolinyl, naphthyridinyl, quinolizinyl, purinyl, imidazopyridinyl, indolizinyl, pteridinyl, imidazotriazinyl and pyrazinopyridazinyl group, it being possible for the said group to be mono- or disubstituted by a $(C_1\text{-}C_4)$alkyl or $(C_1\text{-}C_4)$alkoxy group or an optionally labelled halogen atom and in particular Ar is chosen from a pyridyl, indolyl, imidazopyridinyl, benzimidazolyl, quinolyl, quinolonyl, isoquinolyl, quinoxalinyl, naphthyridinyl and benzothienyl group, and in that the said group is monosubstituted by a labelled halogen atom said compound comprising in particular a radionuclide as defined according to Claim 9.

**11.** Compound chosen from:

- *N*-(2-diethylaminoethyl)-6-iodoimidazo[1,2-*a*]pyridine-2-carboxamide;
- *N*-(2-diethylaminoethyl)-6-iodoquinoline-2-carboxamide;
- *N*-(4-dipropylaminobutyl)-6-iodoquinoline-2-carboxamide;
- *N*-(2-diethylaminoethyl)-6-iodoquinoxaline-2-carboxamide;
- *N*-(2-diethylaminoethyl)-5-iodobenzimidazole-2-carboxamide;
- *N*-(2-diethylaminoethyl)-9,10-dihydro-7-iodo-9-oxoacridine-4-carboxamide;
- *N*-(2-diethylaminoethyl)-5-iodoacridine-4-carboxamide;
- *N*-(2-diethylaminoethyl)-7-iodoacridine-4-carboxamide;
- *N*-(2-diethylaminoethyl)-8-iodonaphthyridine-2-carboxamide;
- *N*-(4-dipropylaminobutyl)-6-iodoquinoxaline-2-carboxamide;
- *N*-(2-diethylaminoethyl)-7-iodophenazine-1-carboxamide;
- and their pharmaceutically acceptable salts.

**12.** Radiopharmaceutical composition comprising, as active principle, a compound of formula (I) or (II) or one of its pharmaceutically acceptable salts as defined according to any one of Claims 1 to 10.

**13.** Use of a compound of formula (II) or of one of its pharmaceutically acceptable salts according to Claim 10, the said compound comprising a radionuclide, in the preparation of a radiopharmaceutical composition intended for medical imaging, and in particular for the diagnosis of melanomas.

**14.** Use of a compound of formula (II) or of one of its pharmaceutically acceptable salts according to Claim 10 in the preparation of a radiopharmaceutical composition intended for the treatment of melanomas.

**15.** Noninvasive method for the determination of the tissue distribution of tumour cells of melanomas of the human body, comprising the stages of at least one injection of a radiopharmaceutical composition comprising at least one compound of formula (I) or one compound of formula (II) comprising a radionuclide or one of its pharmaceutically acceptable salts as defined according to any one of Claims 1 to 10 and of at least one determination of the concentration of the radioactivity.

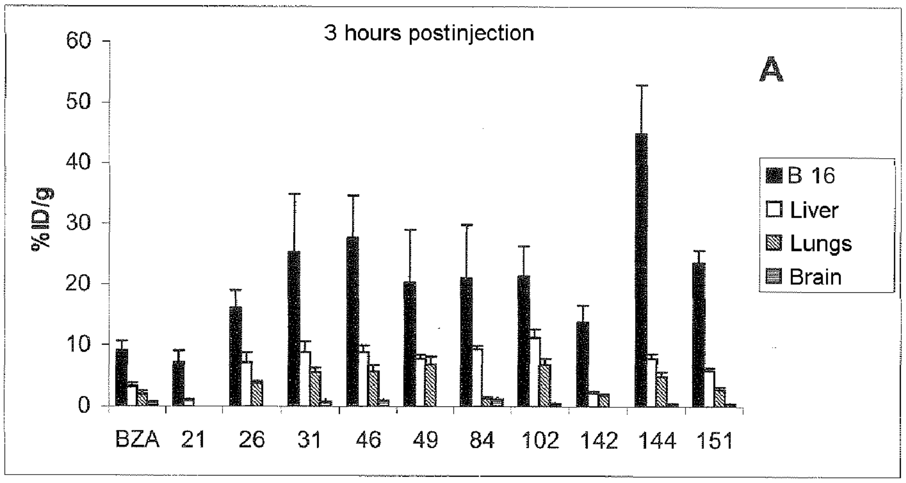


Figure 1A

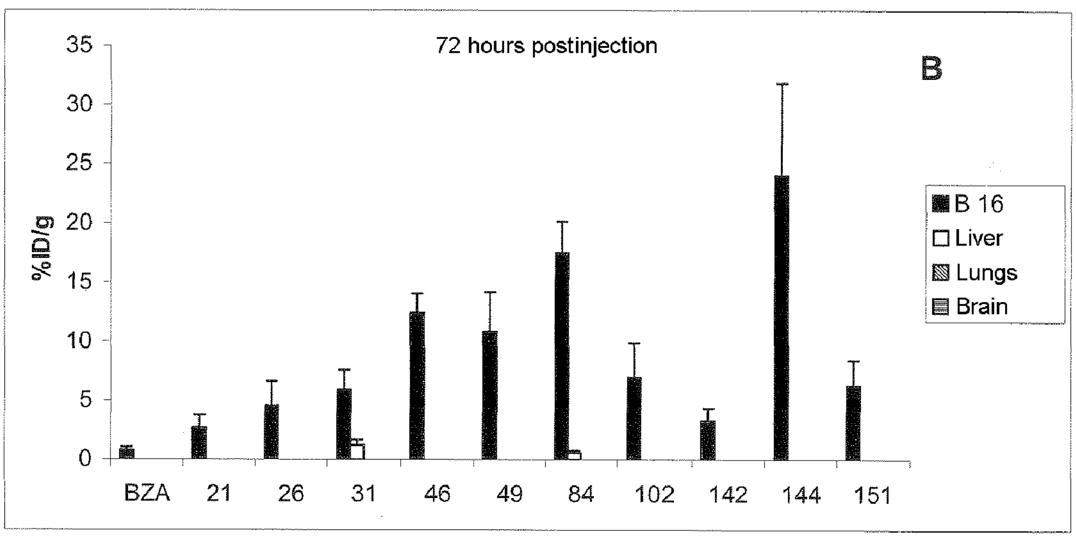


Figure 1B

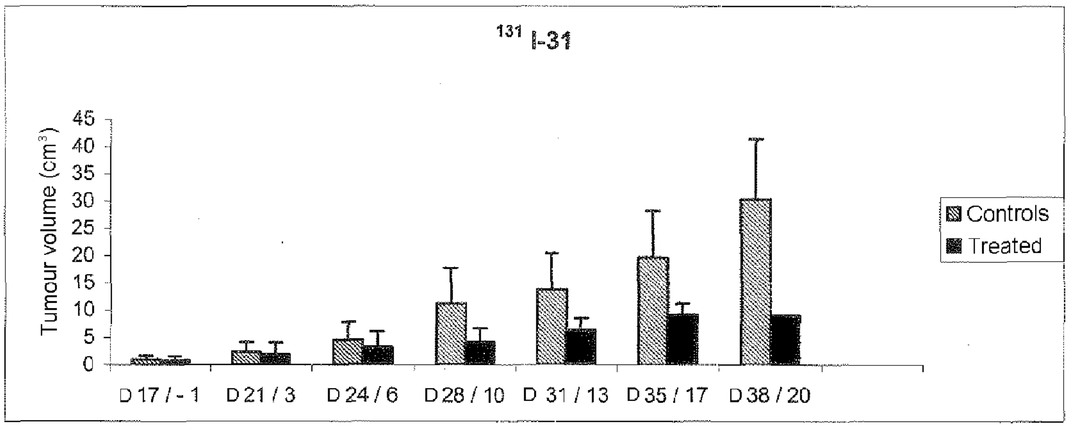


Figure 2

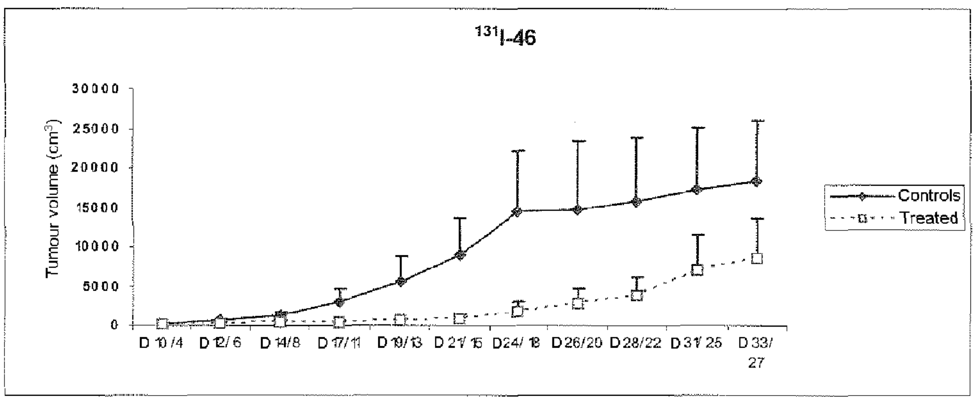


Figure 3

Europäisches Patentamt
European Patent Office
Office européen des brevets

# EUROPEAN SEARCH REPORT

Application Number
EP 11 16 7370

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 0 458 886 A1 (INST NAT SANTE RECH MED [FR]; CIS BIO INT [FR]) 4 December 1991 (1991-12-04) * page 2, line 3 * * page 21, lines 5-10; claim 1 * * page 21, lines 45-50; claim 3 * ----- | 1-15 | INV. C07D471/04 C07D219/06 A61K31/165 C07D209/42 C07D215/54 C07D217/26 C07D235/24 C07D237/26 C07D333/68 C07C233/62 |
| A | SHOWALTER H D ET AL: "Tyrosine kinase inhibitors. 6. Structure-activity relationships among N- and 3-substituted 2,2'-diselenobis(1H-indoles) for inhibition of protein tyrosine kinases and comparative in vitro and in vivo studies against selected sulfur congeners.", JOURNAL OF MEDICINAL CHEMISTRY, vol. 40, no. 4, 14 February 1997 (1997-02-14), pages 413-426, XP002469707, ISSN: 0022-2623 * page 415; compound 16 * ----- | 1-15 | |
| A | EP 0 082 665 A (ICI PLC [GB]) 29 June 1983 (1983-06-29) * page 12, line 30; example 1 * ----- | 1-15 | TECHNICAL FIELDS SEARCHED (IPC) C07D A61K C07C |
| A | OLSSON R ET AL: "Microwave-assisted solvent-free parallel synthesis of thioamides", TETRAHEDRON LETTERS, ELSEVIER, AMSTERDAM, NL, vol. 41, no. 41, 7 October 2000 (2000-10-07), pages 7947-7950, XP004235906, ISSN: 0040-4039 * page 7949; table 1; compounds A4B3, A4B4 * ----- -/-- | 1-15 | |

The present search report has been drawn up for all claims

| Place of search | Date of completion of the search | Examiner |
|---|---|---|
| Munich | 22 July 2011 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 11 16 7370

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 4 764 522 A (IMHOF RENE [CH] ET AL) 16 August 1988 (1988-08-16) * column 19, lines 40,41; example 18 * ----- | 1-15 | |
| A | WO 2005/042505 A (SCHERING AG [DE]; SCHULZE VOLKER [DE]; EIS KNUT [DE]; WORTMANN LARS [D) 12 May 2005 (2005-05-12) * page 71; compound INT108 * ----- | 1-15 | |
| | | | TECHNICAL FIELDS SEARCHED (IPC) |

The present search report has been drawn up for all claims

2

| Place of search | Date of completion of the search | Examiner |
|---|---|---|
| Munich | 22 July 2011 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 16 7370

This annex lists the patent family members relating to the patent documents cited in the above-mentioned European search report.
The members are as contained in the European Patent Office EDP file on
The European Patent Office is in no way liable for these particulars which are merely given for the purpose of information.

22-07-2011

| Patent document cited in search report | Publication date | Patent family member(s) | Publication date |
|---|---|---|---|
| EP 0458886 A1 | 04-12-1991 | AU 5191390 A | 05-09-1990 |
| | | DE 69003937 D1 | 18-11-1993 |
| | | DE 69003937 T2 | 05-05-1994 |
| | | FR 2642972 A1 | 17-08-1990 |
| | | WO 9009170 A2 | 23-08-1990 |
| | | JP 2846459 B2 | 13-01-1999 |
| | | JP 4506057 T | 22-10-1992 |
| | | US 5190741 A | 02-03-1993 |
| EP 0082665 A | 29-06-1983 | AU 553110 B2 | 03-07-1986 |
| | | AU 9183182 A | 30-06-1983 |
| | | CA 1189510 A1 | 25-06-1985 |
| | | DD 207712 A5 | 14-03-1984 |
| | | DE 3264796 D1 | 22-08-1985 |
| | | DK 542582 A | 24-06-1983 |
| | | ES 8402557 A1 | 01-05-1984 |
| | | ES 8502964 A1 | 01-05-1985 |
| | | ES 8502965 A1 | 01-05-1985 |
| | | FI 824336 A | 24-06-1983 |
| | | GR 77042 A1 | 04-09-1984 |
| | | HU 186650 B | 28-08-1985 |
| | | IE 54220 B1 | 19-07-1989 |
| | | JP 58110567 A | 01-07-1983 |
| | | NO 824335 A | 24-06-1983 |
| | | NZ 202890 A | 11-10-1985 |
| | | PT 76020 A | 01-01-1983 |
| | | US 4520027 A | 28-05-1985 |
| | | US 4568691 A | 04-02-1986 |
| | | ZA 8208850 A | 28-03-1984 |
| | | ZW 25182 A1 | 20-06-1984 |
| US 4764522 A | 16-08-1988 | AR 244209 A1 | 29-10-1993 |
| | | AT 391694 B | 12-11-1990 |
| | | AU 582381 B2 | 23-03-1989 |
| | | AU 4663685 A | 06-03-1986 |
| | | BE 903110 A1 | 24-02-1986 |
| | | BG 60401 B1 | 28-02-1995 |
| | | CA 1261335 A1 | 26-09-1989 |
| | | DE 3530046 A1 | 13-03-1986 |
| | | DK 391485 A | 01-03-1986 |
| | | ES 8705372 A1 | 16-07-1987 |
| | | ES 8800158 A1 | 01-01-1988 |
| | | FI 853140 A | 01-03-1986 |
| | | FR 2569694 A1 | 07-03-1986 |
| | | GB 2163746 A | 05-03-1986 |
| | | GR 852071 A1 | 24-12-1985 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 11 16 7370

This annex lists the patent family members relating to the patent documents cited in the above-mentioned European search report.
The members are as contained in the European Patent Office EDP file on
The European Patent Office is in no way liable for these particulars which are merely given for the purpose of information.

22-07-2011

| Patent document cited in search report | Publication date | Patent family member(s) | Publication date |
|---|---|---|---|
| US 4764522 A | | HU 38317 A2 | 28-05-1986 |
| | | IE 58788 B1 | 17-11-1993 |
| | | IL 76176 A | 28-02-1989 |
| | | IT 1190385 B | 16-02-1988 |
| | | JP 7300417 A | 14-11-1995 |
| | | JP 8025879 B | 13-03-1996 |
| | | JP 2030307 C | 19-03-1996 |
| | | JP 7064817 B | 12-07-1995 |
| | | JP 61060657 A | 28-03-1986 |
| | | LU 86049 A1 | 11-09-1986 |
| | | MC 1696 A | 18-07-1986 |
| | | NL 8502374 A | 17-03-1986 |
| | | NO 853390 A | 03-03-1986 |
| | | NZ 213193 A | 27-10-1989 |
| | | PH 22813 A | 27-12-1988 |
| | | PT 81041 A | 01-09-1985 |
| | | SE 464083 B | 04-03-1991 |
| | | SE 8504014 A | 01-03-1986 |
| WO 2005042505 A | 12-05-2005 | AR 046347 A1 | 07-12-2005 |
| | | AU 2004285682 A1 | 12-05-2005 |
| | | BR PI0416005 A | 02-01-2007 |
| | | CA 2544267 A1 | 12-05-2005 |
| | | CN 1902185 A | 24-01-2007 |
| | | DE 10351744 A1 | 16-06-2005 |
| | | EA 200600833 A1 | 27-02-2007 |
| | | EC SP066588 A | 17-10-2006 |
| | | EP 1678153 A1 | 12-07-2006 |
| | | JP 2007509892 A | 19-04-2007 |
| | | KR 20060098374 A | 18-09-2006 |
| | | PE 09242005 A1 | 25-11-2005 |
| | | RS P20060294 A | 07-08-2008 |
| | | US 2007037862 A1 | 15-02-2007 |
| | | ZA 200604432 A | 30-09-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 458886 A
- US 5911970 A
- WO 9324096 A
- WO 9817649 A
- US 6821983 B
- WO 2005118580 A
- US 656417 A, Lee, C. H., Bayburt, E. K., DiDomenico, S., Drizin, I., Gomtsyan, A. R., Koenig, J. R., Perner, R. J., Schmidt, R. G., Turner, S. C., White, T. K. and Zheng, G. Z.,
- US 2003 A
- EP 0587311 A, Higashida, S., Sakurai, M., Yabe, Y., Nishihgaki, T., Komai, T. and Handa, H.
- EP 1994 A
- US 20050038032 A, Allison, B. D., Hack, M. D., Phuong, V. K., Rabinowitz, M. H. and Rosen, M. D.


### Non-patent literature cited in the description


- **OSMAN, S. ; ROWLINSON-BUSZA, G. ; LUTHRA, K. S. ; ABOAGYE, E. O. ; BROWN, G. D. ; BRADY, F. ; MYERS, R. ; GAMAGE, S. A. ; DENNY, W. A. ; BAGULEY, B. C.** *Cancer Res.,* 2001, vol. 61, 2935-2944
- **SALEEM, A. ; HARTE, R. J. ; MATTHEWS, J. C. ; OSMAN, S. ; BRADY, F. ; LUTHRA, S. K. ; BROWN, G. D. ; BLEEHEN, N. ; CONNORS, T. ; JONES, T.** *J. Clin. Oncology,* 2001, vol. 19, 1421-1429
- **S. A. GAMAGE et al.** *Bioorganic & Medicinal Chemistry,* 2006, vol. 14, 1160-1168
- **ADCOCK, W. ; WELLS, P. R.** *Aust. J. Chem.,* 1965, vol. 18, 1351-1364
- **NEWKOME, G. R. ; MOORFIELD, C. N. ; SABBAGHIAN, B.** *J. Org. Chem.,* 1986, vol. 51, 953-954
- **BESHORE, D. C. ; DINSMORE, C. J.** *Synth. Commun.,* 2003, vol. 33, 2423-2427
- **BRIDGES, A. J. ; LEE, A. ; MADUAKOR, E. C. ; SCHWARTZ, C. E.** *Tetrahedron Lett.,* 1992, vol. 33, 7499-7502
- **ENGUEHARD, C. ; RENOU, J. L. ; COLLOT, V. ; HERVET, M. ; RAULT, S. ; GUEIFFIER, A.** *J. Org. Chem.,* 2000, vol. 65, 6572-6575
- **SUNBERG, R. J. ; BISWAS, S. ; MURTHI, K. K. ; ROWE, D. ; MCDALL, J. W. ; DZIMIANSKI, M. T.** *J. Med. Chem.,* 1998, vol. 41, 4317-4328
- **BUCHEL, K. H. Z.** *Naturforsch., B.,* 1970, vol. 25, 945-953
- **PETROW, V. ; STURGEON, B.** *J. Chem. Soc.,* 1954, 570-574
- **LIN, A. J. ; LOO, T. L.** *J. Med. Chem.,* 1978, vol. 21, 268-272
- **SEGUIN, H. ; GARDETTE, D. ; MOREAU, M. F. ; MADELMONT, J. C. ; GRAMAIN, J. C.** *Synth. Commun.,* 1998, vol. 28, 4257-4272
- **FIESER, L. F. ; HADDADIN, M. J.** *Org. Synth.,* vol. 46, 107-112
- **SY, W. W.** *Synth. Commun.,* 1992, vol. 22, 3215-3219
- **REWCASTTLE, G. W. ; DENNY, W. A.** *Synthesis,* 1985, 220-222
- **SELTZMAN, H. H. ; BERRANG, B. D.** *Tetrahedron Lett.,* 1993, vol. 34, 3083-3086
- **REWCASTLE, G. W. ; DENNY, W. A.** *Synthesis,* 1985, vol. 2, 217-220
- **REWCASTLE G. W. ; DENNY, W. A.** *Synthesis,* 1985, 217-220
- **ATWELL, G. J. ; REWCASTLE, G. W. ; BAGULEY, B. C. ; DENNY, W. A.** *J. Med. Chem.,* 1987, vol. 30, 664-669
- **RENOTTE, R. ; SARLET, G. ; THUNUS, L. ; LEJEUNE, R.** *Luminescence,* 2000, vol. 15, 311-320
- **FERLIN, M. G. ; MARZANO, C. ; CHIARELOTTO, G. ; BACCICHETTI, F. ; BORDIN, F.** *Eur. J. Med. Chem.,* 2000, vol. 35, 827-837
- **KAJIGAESHI, S. ; KAKINAMI, T. ; YAMASAKI, H. ; FUJISAKI, S. ; OKAMOTO, T.** *Bull. Chem. Soc. Jpn.,* 1988, vol. 61, 600-602
- **ATWELL, G. J. ; CAIN, B. F. ; BAGULEY, B. C. ; FINLAY, G. J. ; DENNY, W. A.** *J. Med. Chem.,* 1984, vol. 27, 1481-1485
- **CHAN, L. ; JIN, H. ; STEFANAC, T. ; LAVALLÉE, J.F. ; FALARDEAU, G. ; WANG, W. ; BÉDARD, J. ; MAY, S. ; YUEN, L.** *J. Med. Chem.,* 1999, vol. 42, 3023-3025

- **CULHANE, P.J.** Organic Syntheses. Wiley, 1944, vol. I, 125